(19) Europäisches Patentamt
European Patent Office
Office européen des brevets

(11) **EP 4 484 426 A1**

(12) **EUROPEAN PATENT APPLICATION**
published in accordance with Art. 153(4) EPC

(43) Date of publication:
**01.01.2025 Bulletin 2025/01**

(21) Application number: **23759152.4**

(22) Date of filing: **21.02.2023**

(51) International Patent Classification (IPC):
***C07D 471/04*** *(2006.01)* ***A61P 35/00*** *(2006.01)*
***A61P 35/02*** *(2006.01)* ***A61P 31/14*** *(2006.01)*
***A61P 31/20*** *(2006.01)* ***A61K 31/519*** *(2006.01)*

(52) Cooperative Patent Classification (CPC):
**A61K 31/517; A61K 31/519; A61K 31/5377; A61K 31/541; A61P 3/04; A61P 11/06; A61P 17/00; A61P 19/02; A61P 29/00; A61P 31/14; A61P 31/16; A61P 31/18; A61P 31/20; A61P 31/22; A61P 35/00;**
(Cont.)

(86) International application number:
**PCT/CN2023/077368**

(87) International publication number:
**WO 2023/160527 (31.08.2023 Gazette 2023/35)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **24.02.2022 CN 202210172494**

(71) Applicant: **Sunshine Lake Pharma Co., Ltd.**
**Dongguan, Guangdong 523000 (CN)**

(72) Inventors:
- **REN, Qingyun**
  **Dongguan, Guangdong 523871 (CN)**
- **LIU, Weishun**
  **Dongguan, Guangdong 523871 (CN)**
- **ZHANG, Yingjun**
  **Dongguan, Guangdong 523871 (CN)**
- **YAN, Guanghua**
  **Dongguan, Guangdong 523871 (CN)**

(74) Representative: **Hoffmann Eitle**
**Patent- und Rechtsanwälte PartmbB**
**Arabellastraße 30**
**81925 München (DE)**

# (54) PYRIMIDO AROMATIC RING COMPOUND AND USE THEREOF IN DRUG

(57) A pyrimido aromatic ring compound and a use thereof in a drug, especially a use thereof as a TLR8 agonist. Specifically, the present invention relates to a compound represented by general formula (I), or a stereoisomer, tautomer, nitrogen oxide, solvate, metabolite, pharmaceutically acceptable salt or prodrug thereof, and a use thereof as a drug, especially a use thereofas the TLR8 agonist.



Processed by Luminess, 75001 PARIS (FR)

**(Cont. next page)**

(52) Cooperative Patent Classification (CPC): (Cont.)
**A61P 35/02; A61P 37/00; A61P 37/08;
C07D 401/04; C07D 401/14; C07D 471/04**

## Description

### FIELD OF THE INVENTION

The present invention belongs to the field of medicine. Specifically, the present invention relates to a pyrimido aromatic ring compound and a use thereof in a drug, especially a use thereof as a TLR8 agonist. The present invention also relates to the composition of these pyrimido aromatic ring compounds with other therapeutic agents, and a use thereof in a drug, especially a use thereof as a TLR8 agonist.

### BACKGROUND ART

Toll-like receptors (TLRs) are important pattern recognition receptors for innate immune responses, which are widely distributed in mammalian myeloid dendritic cells, monocytes, and mononuclear macrophages. In one aspect, TLRs can recognize specific microbial PAMPs (e.g., lipopolysaccharides, flagellins, single/double-stranded RNA, etc.), thereby stimulating the body's innate immunity. In other aspect, different TLRs can induce gene expression in specific functional regions, thereby triggering antigen-specific acquired immune responses in the body.

In mammals, 13 members of TLRs have been found, of which TLR1-TLR9 and TLR11 are common to humans and mice, and TLR10, TLR12 and TLR13 are endemic to mice. TLR8 is a member of the subgroup of TLRs (TLRs 3, 7, 8, and 9) and is confined to an endosomal compartment that specifically recognizes cells that are not their own nucleic acids. TLR8 is predominantly expressed in humans through monocyte, NK cell, and myeloid dendritic cell (mDC). TLR8 agonists can lead to the release of a variety of different pro-inflammatory cytokines, such as IL-6, IL-12, TNF-$\alpha$ and IFN-$\gamma$.

After TLR8 activation, it mediates inflammatory immunity and promotes the clearance of virus-infected cells and tumor cells in vivo, and its agonist can be used as an independent immunotherapy drug or immune adjuvant, showing important clinical application prospects in immunotherapy. TLR8 activation is closely related to the innate immune response against infection, and can mediate the occurrence and development of viral infections such as HBV, HCV, HIV and herpesvirus, tumors, autoimmune diseases, and metabolic diseases.

At present, the dual agonists of TLR8 and TLR7 have been reported in many patents. Given the wide therapeutic potential of TLR8 agonists, there is still a need for new TLR8 agonists, especially those with high selectivity of TLR8 for the treatment and/or prevention of hepatitis B virus.

### SUMMARY

The present invention relates to novel pyrimido aromatic ring compounds and pharmaceutically acceptable compositions thereof, which have a good activating effect on TLR8 and a good selective activating effect on TLR8. At the same time, they also have the advantages of basically no induction effect on liver drug enzymes, basically no inhibitory effect on liver drug enzymes, and basically no toxicity to the heart. In addition, they have good solubility, good stability and very good pharmacokinetic properties. The compounds of the present invention can treat and/or prevent various diseases related to TLR8 activity, and especially have good application prospects in anti-hepatitis B virus (HBV).

In one aspect, the present invention relates to a compound having Formula (I) or a stereoisomer, a tautomer, an N-oxide, a solvate, a metabolite, a pharmaceutically acceptable salt or a prodrug thereof,

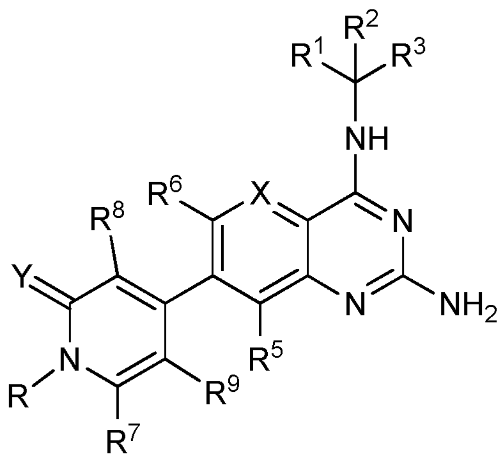

(I)

wherein, X is N or $CR^4$;

each $R^1$, $R^2$, $R^4$, $R^5$, $R^6$, $R^7$ and $R^8$ is independently H, D, F, Cl, Br, I, -OH, -CN, $-NH_2$, $C_{1-4}$ alkylamino, $C_{1-6}$ alkoxy or $C_{1-6}$ alkyl, wherein each $C_{1-4}$ alkylamino, $C_{1-6}$ alkoxy and $C_{1-6}$ alkyl is independently unsubstituted or substituted with 1, 2, 3 or 4 groups independently selected from F, Cl, Br, I, -OH, -CN, $-NH_2$ and $C_{1-4}$ alkyl;

Yis O or S;

$R^3$ is $-C_{1-6}$ alkylene-$R^{10}$, wherein the $-C_{1-6}$ alkylene of $-C_{1-6}$ alkylene-$R^{10}$ is unsubstituted or substituted with 1, 2, 3, 4 or 5 $R^{w1}$;

each $R^{10}$ is independently $C_{1-6}$ alkoxy, -OH, $-NH_2$, -O-C(=O)-$R^{10a}$ or -$NR^c$-C(=O)-$R^{10b}$, wherein the $C_{1-6}$ alkoxy is unsubstituted or substituted with 1, 2, 3 or 4 groups independently selected from F, Cl, Br, I, -OH, -CN, $-NH_2$ and $C_{1-4}$ alkyl;

each $R^c$ is independently H, D or $C_{1-4}$ alkyl, wherein the $C_{1-4}$ alkyl is unsubstituted or substituted with 1, 2, 3 or 4 groups independently selected from F, Cl, Br, I, -OH, -CN, $-NH_2$ and $C_{1-4}$ alkyl;

each $R^{10a}$ and $R^{10b}$ is independently $C_{1-6}$ alkyl, $C_{3-7}$ cycloalkyl, heteroaryl consisting of 5-12 ring atoms or heterocyclyl consisting of 3-12 ring atoms, wherein each $C_{1-6}$ alkyl, $C_{3-7}$ cycloalkyl, heteroaryl consisting of 5-12 ring atoms and heterocyclyl consisting of 3-12 ring atoms is independently unsubstituted or substituted with 1, 2, 3, 4 or 5 $R^{w2}$;

R is H, D, $C_{1-6}$ alkyl or heterocyclyl consisting of 3-6 ring atoms, wherein each $C_{1-6}$ alkyl and heterocyclyl consisting of 3-6 ring atoms is independently unsubstituted or substituted with 1, 2, 3, 4 or 5 $R^{w3}$.,

$R^9$ is H, D, -L-$R^{11}$, F, Cl, Br, I, -OH, -CN, $-NH_2$, $C_{1-4}$ alkylamino, $C_{1-6}$ alkoxy or $C_{1-6}$ alkyl, wherein each $C_{1-4}$ alkylamino, $C_{1-6}$ alkoxy and $C_{1-6}$ alkyl is independently unsubstituted or substituted with 1, 2, 3 or 4 groups selected from F, Cl, Br, I, -OH, -CN, $-NH_2$ and $C_{1-4}$ alkyl;

L is $-C_{1-6}$ alkylene or -C(=O)-, wherein the $-C_{1-6}$ alkylene is independently unsubstituted or substituted with 1, 2, 3 or 4 groups independently selected from F, Cl, Br, I, -OH, -CN, $-NH_2$, methyl, ethyl, n-propyl or isopropyl;

$R^{11}$ is $-NR^aR^b$, $C_{1-12}$ alkyl, -C(=O)$R^{9a}$, -$OR^{9b}$, $-S(=O)_tR^{9c}$, $C_{3-7}$ cycloalkyl, heteroaryl consisting of 5-12 ring atoms or heterocyclyl consisting of 3-12 ring atoms, wherein each $C_{1-12}$ alkyl, $C_{3-7}$ cycloalkyl, heteroaryl consisting of 5-12 ring atoms and heterocyclyl consisting of 3-12 ring atoms is independently unsubstituted or substituted with 1, 2, 3, 4 or 5 $R^{w4}$;

each $R^a$ and $R^b$ is independently H, D, -C(=O)$R^{9d}$, $C_{1-12}$ alkyl, $C_{2-12}$ alkenyl, $C_{2-12}$alkynyl or $-C_{1-6}$ alkylene-$R^{d1}$, wherein each $C_{1-12}$ alkyl, $C_{2-12}$ alkenyl, $C_{2-12}$ alkynyl and $-C_{1-6}$ alkylene of $-C_{1-6}$ alkylene-$R^{d1}$ is independently unsubstituted or substituted with 1, 2, 3, 4 or 5 $R^{w5}$;

or, $R^a$, $R^b$ and together with the N atom to which they are attached, form heterocyclyl consisting of 3-8 ring atoms, wherein the heterocyclyl consisting of 3-8 ring atoms is unsubstituted or substituted with 1, 2, 3 or 4 $R^{w4}$;

each $R^{9a}$, $R^{9b}$, $R^{9c}$ and $R^{9d}$ is independently $C_{1-12}$ alkyl, $C_{2-12}$ alkenyl, $C_{2-12}$ alkynyl or $-C_{1-6}$ alkylene-$R^{d2}$, wherein each $C_{1-12}$ alkyl, $C_{2-12}$ alkenyl, $C_{2-12}$ alkynyl and $-C_{1-6}$ alkylene of $-C_{1-6}$ alkylene-$R^{d2}$ is independently unsubstituted or substituted with 1, 2, 3, 4 or 5 $R^{w6}$;

each $R^{d1}$ and $R^{d2}$ is independently -OH, $C_{1-6}$ alkoxy, $C_{6-10}$ aryl, heteroaryl consisting of 5-12 ring atoms, $C_{3-6}$ cycloalkyl or $C_{1-6}$ alkylamino, wherein each $C_{1-6}$ alkoxy, $C_{6-10}$ aryl, heteroaryl consisting of 5-12 ring atoms, $C_{3-6}$ cycloalkyl and $C_{1-6}$ alkylamino is independently unsubstituted or substituted with 1, 2, 3, 4 or 5 $R^{w7}$;

each $R^{w1}$, $R^{w2}$, $R^{w3}$, $R^{w4}$ and $R^{w7}$ is independently D, F, Cl, Br, I, =O, -OH, -CN, $-NH_2$, $C_{1-6}$ alkylamino, $C_{1-6}$ alkoxy, $C_{1-6}$ alkyl, $C_{1-6}$ haloalkyl, -C(=O)-$C_{1-4}$ alkyl, $C_{3-6}$ cycloalkyl, heterocyclyl consisting of 3-6 ring atoms or $C_{6-10}$ aryl, wherein each $C_{1-6}$ alkylamino, $C_{1-6}$ alkoxy, $C_{1-6}$ alkyl, $C_{1-6}$ haloalkyl, -C(=O)-$C_{1-4}$ alkyl, $C_{3-6}$ cycloalkyl, heterocyclyl consisting of 3-6 ring atoms and $C_{6-10}$ aryl is independently unsubstituted or substituted with 1, 2, 3 or 4 groups independently selected from F, Cl, Br, I, -OH, -CN, $-NH_2$, $C_{1-4}$ alkyl and $C_{1-4}$ alkylamino;

each $R^{w5}$ and $R^{w6}$ is independently D, F, Cl, Br, I, =O, -OH, -CN, $-NH_2$, $C_{1-6}$ alkylamino, $C_{1-6}$ alkoxy, $C_{1-6}$ alkyl, $C_{1-6}$ haloalkyl, -C(=O)-$C_{1-4}$ alkyl, $C_{2-12}$ alkenyl, $C_{2-12}$ alkynyl, $C_{3-6}$ cycloalkyl, heterocyclyl consisting of 3-6 ring atoms, heteroaryl consisting of 5-6 ring atoms or $C_{6-10}$ aryl, wherein each $-NH_2$, $C_{1-6}$ alkylamino, $C_{1-6}$ alkoxy, $C_{1-6}$ alkyl, $C_{1-6}$ haloalkyl, -C(=O)-$C_{1-4}$ alkyl, $C_{2-12}$ alkenyl, $C_{2-12}$ alkynyl, $C_{3-6}$ cycloalkyl, heterocyclyl consisting of 3-6 ring atoms, heteroaryl consisting of 5-6 ring atoms and $C_{6-10}$ aryl is independently unsubstituted or substituted with 1, 2, 3 or 4 groups independently selected from F, Cl, Br, I, -OH, -CN, $-NH_2$, $C_{1-4}$ alkyl and $C_{1-4}$ alkylamino;

t is 0, 1 or 2.

In some embodiments, each $R^1$, $R^2$, $R^4$, $R^5$, $R^6$, $R^7$and $R^8$ is independently H, D, F, Cl, Br, I, -OH, -CN, $-NH_2$, N-methylamino, *N*-ethylamino, *N,N*-dimethylamino, *N,N*-diethylamino, methoxy, ethoxy, 1-propoxy, 2-propoxy, 1-butoxy, 2-methyl-1-propoxy, 2-butoxy, methyl, ethyl, *n*-propyl, isopropyl, *n*-butyl, *i*-butyl, *sec*-butyl, *tert*-butyl, *n*-pentyl or *n*-hexyl, wherein each *N*-methylamino, *N*-ethylamino, *N,N*-dimethylamino, *N,N*-diethylamino, methoxy, ethoxy, 1-propoxy, 2-propoxy, 1-butoxy, 2-methyl-1-propoxy, 2-butoxy, methyl, ethyl, n-propyl, isopropyl, *n*-butyl, *i*-butyl, *sec*-butyl, *tert*-butyl, *n*-pentyl and *n*-hexyl is independently unsubstituted or substituted with 1, 2, 3 or 4 groups independently selected from F, Cl, Br, I, -OH, -CN, $-NH_2$, methyl, ethyl, *n*-propyl, isopropyl, *n*-butyl, *i*-butyl and *sec*-butyl;

R is H, D, methyl, ethyl, *n*-propyl, isopropyl, *n*-butyl, *i*-butyl, *sec*-butyl, *tert*-butyl, *n*-pentyl, *n*-hexyl, aziridinyl, azetidinyl, oxetanyl, thietanyl, pyrrolidyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl or piperazinyl, wherein each methyl, ethyl, *n*-propyl, isopropyl, *n*-butyl, *i*-butyl, *sec*-butyl, *tert*-butyl, *n*-pentyl, *n*-hexyl, aziridinyl, azetidinyl, oxetanyl, thietanyl, pyrrolidyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl and piperazinyl is independently unsubstituted or substituted with 1, 2, 3, 4 or 5 $R^{w3}$;
wherein $R^{w3}$ is as defined herein.

In some embodiments, $R^3$ is $-CH_2-R^{10}$, $-(CH_2)_2-R^{10}$, $-(CH_2)_3-R^{10}$, $-CH(CH_3)CH_2-R^{10}$, $-CH_2CH(CH_3)-R^{10}$ or $-(CH_2)_4-R^{10}$, wherein each the $-CH_2-$ of $CH_2-R^{10}$, $-(CH_2)_2-$ of $-(CH_2)_2-R^{10}$, $-(CH_2)_3-$ of $-(CH_2)_3-R^{10}$, $-CH(CH_3)CH_2-$ of $-CH(CH_3)CH_2-R^{10}$, $-CH_2CH(CH_3)$ - of $-CH_2CH(CH_3)-R^{10}$ and $-(CH_2)_4-$ of $-(CH_2)_4-R^{10}$ is independently unsubstituted or substituted with 1, 2, 3, 4 or 5 $R^{w1}$;

each $R^{10}$ is independently methoxy, ethoxy, 1-propoxy, 2-propoxy, 1-butoxy, 2-methyl-1-propoxy, 2-butoxy, -OH, $-NH_2$, $-O-C(=O)-R^{10a}$ or $-NR^c-C(=O)-R^{10b}$, wherein each methoxy, ethoxy, 1-propoxy, 2-propoxy, 1-butoxy, 2-methyl-1-propoxy and 2-butoxy is independently unsubstituted or substituted with 1, 2, 3 or 4 groups independently selected from F, Cl, Br, I, -OH, -CN, $-NH_2$, methyl, ethyl, n-propyl, isopropyl, n-butyl, i-butyl and sec-butyl;
each $R^{10a}$ and $R^{10b}$ is independently $C_{1-4}$ alkyl, $C_{3-6}$ cycloalkyl, heteroaryl consisting of 5-6 ring atoms or heterocyclyl consisting of 3-6 ring atoms, wherein each $C_{1-4}$ alkyl, $C_{3-6}$ cycloalkyl, heteroaryl consisting of 5-6 ring atoms and heterocyclyl consisting of 3-6 ring atoms is independently unsubstituted or substituted with 1, 2, 3, 4 or 5 $R^{w2}$;
$R^c$ is H, D, methyl, ethyl, n-propyl or isopropyl, wherein each methyl, ethyl, n-propyl and isopropyl is independently unsubstituted or substituted with 1, 2, 3 or 4 groups independently selected from F, Cl, Br, I, -OH, -CN, $-NH_2$, methyl, ethyl, n-propyl and isopropyl;
wherein each $R^{w1}$ and $R^{w2}$ is as defined herein.

In some embodiments, each $R^{10a}$ and $R^{10b}$ is independently methyl, ethyl, n-propyl, isopropyl, n-butyl, i-butyl, sec-butyl, tert-butyl, cyclopropyl, cyclobutyl, cyclopentyl, cyclohexyl, furyl, pyrrolyl, pyridinyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidyl, aziridinyl, azetidinyl, oxetanyl, thietanyl, pyrrolidyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl or piperazinyl, wherein each methyl, ethyl, n-propyl, isopropyl, n-butyl, i-butyl, sec-butyl, tert-butyl, cyclopropyl, cyclobutyl, cyclopentyl, cyclohexyl, furyl, pyrrolyl, pyridinyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidyl, aziridinyl, azetidinyl, oxetanyl, thietanyl, pyrrolidyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl and piperazinyl is independently unsubstituted or substituted with 1, 2, 3, 4 or 5 $R^{w2}$;
wherein $R^{w2}$ is as defined herein.

In some embodiments, $R^9$ is H, D, $-L-R^{11}$, F, Cl, Br, I, -OH, -CN, $-NH_2$, $C_{1-4}$ alkylamino, $C_{1-4}$ alkoxy or $C_{1-4}$ alkyl, wherein each $C_{1-4}$ alkylamino, $C_{1-4}$ alkoxy and $C_{1-4}$ alkyl is independently unsubstituted or substituted with 1, 2, 3 or 4 groups independently selected from F, Cl, Br, I, -OH, -CN, $-NH_2$ and $C_{1-4}$ alkyl;

L is $-CH_2-$, $-(CH_2)_2-$, $-(CH_2)_3-$, $-CH_2CH(CH_3)-$, $-CH(CH_3)CH_2-$, $-(CH_2)_4-$ or $-C(=O)-$, wherein each $-CH_2-$, $-(CH_2)_2-$, $-(CH_2)_3-$, $-CH_2CH(CH_3)-$, $-CH(CH_3)CH_2-$ and $-(CH_2)_4-$is independently unsubstituted or substituted with 1, 2, 3 or 4 groups independently selected from F, Cl, Br, I, -OH, -CN, $-NH_2$, methyl, ethyl, n-propyl or isopropyl;
$R^{11}$ is $-NR^aR^b$, $C_{1-10}$ alkyl, $-C(=O)R^{9a}$, $-OR^{9b}$, $-S(=O)_tR^{9c}$, $C_{3-6}$ cycloalkyl, heteroaryl consisting of 5-6 ring atoms or heterocyclyl consisting of 3-6 ring atoms, wherein each $C_{1-10}$ alkyl, $C_{3-6}$ cycloalkyl, heteroaryl consisting of 5-6 ring atoms and heterocyclyl consisting of 3-6 ring atoms is independently unsubstituted or substituted with 1, 2, 3, 4 or 5 $R^{w4}$;
wherein, each $R^a$, $R^b$, $R^{9a}$, t, $R^{9c}$ and $R^{w4}$ is as defined herein.

In some embodiments, $R^9$ is independently H, D, $-L-R^{11}$, F, Cl, Br, I, -OH, -CN, $-NH_2$, N-methylamino, *N*-ethylamino, *N,N*-dimethylamino, *N,N*-diethylamino, methoxy, ethoxy, 1-propoxy, 2-propoxy, 1-butoxy, 2-methyl-1-propoxy, 2-butoxy, methyl, ethyl, n-propyl, isopropyl, n-butyl, i-butyl, sec-butyl, tert-butyl, n-pentyl or n-hexyl, wherein each N-methylamino, *N*-ethylamino, *N,N*-dimethylamino, *N,N*-diethylamino, methoxy, ethoxy, 1-propoxy, 2-propoxy, 1-butoxy, 2-methyl-l-propoxy, 2-butoxy, methyl, ethyl, *n*-propyl, isopropyl, *n*-butyl, *i*-butyl, *sec*-butyl, *tert*-butyl, *n*-pentyl and *n*-hexyl is independently unsubstituted or substituted with 1, 2, 3 or 4 groups independently selected from F, Cl, Br, I, -OH, -CN, $-NH_2$, methyl, ethyl, n-propyl, isopropyl, *n*-butyl, *i*-butyl and *sec*-butyl;

$R^{11}$ is $-NR^aR^b$, methyl, ethyl, *n*-propyl, isopropyl, *n*-butyl, *i*-butyl, *sec*-butyl, *tert*-butyl, n-pentyl, n-hexyl, $-C(=O)R^{9a}$, $-OR^{9b}$, $-S(=O)_tR^{9c}$, cyclopropyl, cyclobutyl, cyclopentyl, cyclohexyl, furyl, pyrrolyl, pyridinyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidyl, aziridinyl, azetidinyl, oxetanyl, thietanyl, pyrrolidyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl or piperazinyl, wherein each methyl, ethyl, *n*-propyl, isopropyl, *n*-butyl, *i*-butyl, *sec*-butyl, *tert*-butyl, *n*-pentyl, *n*-hexyl, cyclopropyl, cyclobutyl, cyclopentyl, cyclohexyl, furyl, pyrrolyl, pyridinyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidyl, aziridinyl, azetidinyl, oxetanyl, thietanyl, pyrrolidyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl and piperazinyl is independently unsubstituted or substituted with 1, 2, 3, 4 or 5 $R^{w4}$;

wherein, each $R^a$, $R^b$, $R^{9a}$, $R^{9b}$, L, t, $R^{9c}$ and $R^{w4}$ is as defined herein.

In some embodiments, each $R^a$ and $R^b$ is independently H, D, $-C(=O)R^{9d}$, $C_{1-6}$ alkyl, $C_{2-6}$ alkenyl, $C_{2-6}$ alkynyl or $-C_{1-4}$ alkylene-$R^{d1}$, wherein each $C_{1-6}$ alkyl, $C_{2-6}$ alkenyl, $C_{2-6}$alkynyl and $-C_{1-4}$ alkylene of $-C_{1-4}$ alkylene-$R^{d1}$ is independently unsubstituted or substituted with 1, 2, 3, 4 or 5 $R^{w5}$;

or, $R^a$, $R^b$ and together with the N atom to which they are attached, form heterocyclyl consisting of 3-6 ring atoms, wherein the heterocyclyl consisting of 3-6 ring atoms is unsubstituted or substituted with 1, 2, 3 or 4 $R^{w4}$;

wherein, each $R^{9d}$, $R^{d1}$, $R^{w5}$ and $R^{w4}$ is as defined herein.

In some embodiments, each $R^a$ and $R^b$ is independently H, D, $-C(=O)R^{9d}$, methyl, ethyl, n-propyl, isopropyl, n-butyl, i-butyl, sec-butyl, tert-butyl, n-pentyl, n-hexyl, $-CH=CH_2$, $-CH=CHCH_3$, $-CH_2CH=CH_2$, $-CH_2CH_2CH=CH_2$, $-C\equiv CH$, $-CH_2C\equiv CH$, $-C\equiv C-CH_3$, $-CH_2CH_2C\equiv CH$, $-CH_2\equiv CCH_3$, $-C\equiv CCH_2CH_3$, $-CH_2-R^{d1}$, $-(CH_2)_2-R^{d1}$, $-(CH_2)_3-R^{d1}$, $-CH_2CH(CH_3)-R^{d1}$, $-CH(CH_3)CH_2-R^{d1}$ or $-(CH_2)_4-R^{d1}$, wherein, each methyl, ethyl, n-propyl, isopropyl, n-butyl, i-butyl, sec-butyl, tert-butyl, n-pentyl, n-hexyl, $-CH=CH_2$, $-CH=CHCH_3$, $-CH_2CH=CH_2$, $-CH_2CH_2CH=CH_2$, $-C\equiv CH$, $-CH_2C\equiv CH$, $-C\equiv C-CH_3$, $-CH_2CH_2C\equiv CH$, $-CH_2\equiv CCH_3$, $-C\equiv CCH_2CH_3$, $-CH_2-$ of $-CH_2-R^{d1}$, $-(CH_2)_2-$ of $-(CH_2)_2-R^{d1}$, $-(CH_2)_3-$ of $-(CH_2)_3-R^{d1}$, $-CH_2CH(CH_3)-$ of $-CH_2CH(CH_3)-R^{d1}$, $-CH(CH_3)CH_2-$ of $-CH(CH_3)CH_2-R^{d1}$ and $-(CH_2)_4-$ of $-(CH_2)_4-R^{d1}$ is independently unsubstituted or substituted with 1, 2, 3, 4 or 5 $R^{w5}$;

or, $R^a$, $R^b$ and together with the N atom to which they are attached, form aziridinyl, azetidinyl, pyrrolidyl, pyrazolidinyl, imidazolidinyl, piperidinyl, morpholinyl, thiomorpholinyl or piperazinyl, wherein each aziridinyl, azetidinyl, pyrrolidyl, pyrazolidinyl, imidazolidinyl, piperidinyl, morpholinyl, thiomorpholinyl and piperazinyl is independently unsubstituted or substituted with 1, 2, 3 or 4 $R^{w4}$;

wherein, each $R^{9d}$, $R^{d1}$, $R^{w5}$ and $R^{w4}$ is as defined herein.

In some embodiments, each $R^{9a}$, $R^{9b}$, $R^{9c}$ and $R^{9d}$ is independently $C_{1-6}$ alkyl, $C_{2-6}$ alkenyl, $C_{2-6}$ alkynyl or $-C_{1-4}$ alkylene-$R^{d2}$, wherein each $C_{1-6}$ alkyl, $C_{2-6}$ alkenyl, $C_{2-6}$ alkynyl and $-C_{1-4}$ alkylene of $-C_{1-4}$ alkylene-$R^{d2}$ is independently unsubstituted or substituted with 1, 2, 3, 4 or 5 $R^{w6}$;

each $R^{d1}$ and $R^{d2}$ is independently -OH, $C_{1-4}$ alkoxy, phenyl, heteroaryl consisting of 5-6 ring atoms, cyclopropyl, cyclobutyl, cyclopentyl, cyclohexyl or $C_{1-4}$ alkylamino, wherein each $C_{1-4}$ alkoxy, phenyl, heteroaryl consisting of 5-6 ring atoms, cyclopropyl, cyclobutyl, cyclopentyl, cyclohexyl and $C_{1-4}$ alkylamino is independently unsubstituted or substituted with 1, 2, 3, 4 or 5 $R^{w7}$;

wherein $R^6$, $R^{w6}$ and $R^{w7}$ are as defined herein.

In some embodiments, each $R^{9a}$, $R^{9b}$, $R^{9c}$ and $R^{9d}$ is independently methyl, ethyl, n-propyl, isopropyl, n-butyl, i-butyl, sec-butyl, tert-butyl, n-pentyl, n-hexyl, $-CH=CH_2$, $-CH=CHCH_3$, $-CH_2CH=CH_2$, $-CH_2CH_2CH=CH_2$, ($-C\equiv CH$), $-CH_2C\equiv CH$, $-C\equiv C-CH_3$, $-CH_2CH_2C\equiv CH$, $-CH_2\equiv CCH_3$, $-C\equiv CCH_2CH_3$, $-CH_2-R^{d2}$, $-(CH_2)_2-R^{d2}$, $-(CH_2)_3-R^{d2}$, $-CH_2CH(CH_3)-R^{d2}$, $-CH(CH_3)CH_2-R^{d2}$ or $-(CH_2)_4-R^{d2}$, wherein each methyl, ethyl, n-propyl, isopropyl, *n*-butyl, *i*-butyl, *sec*-butyl, *tert*-butyl, *n*-pentyl, *n*-hexyl, $-CH=CH_2$, $-CH=CHCH_3$, $-CH_2CH=CH_2$, $-CH_2CH_2CH=CH_2$, $-C\equiv CH$, $-CH_2C\equiv CH$, $-C\equiv C-CH_3$, $-CH_2CH_2C\equiv CH$, $-CH_2\equiv CCH_3$, $-C\equiv CCH_2CH_3$, $-CH_2-$ of $-CH_2-R^{d2}$, $-(CH_2)_2-$ of $-(CH_2)_2-R^{d2}$, $-(CH_2)_3-$ of $-(CH_2)_3-R^{d2}$, $-CH_2CH(CH_3)-$ of $-CH_2CH(CH_3)-R^{d2}$, $-CH(CH_3)CH_2-$ of $-CH(CH_3)CH_2-R^{d2}$ and $-(CH_2)_4-$ of $-(CH_2)_4-R^{d2}$ is independently unsubstituted or substituted with 1, 2, 3, 4 or 5 $R^{w6}$;

each $R^{d1}$ and $R^{d2}$ is independently -OH, methoxy, ethoxy, 1-propoxy, 2-propoxy, 1-butoxy, 2-methyl-l-propoxy, 2-butoxy, phenyl, furyl, pyrrolyl, pyridinyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidyl, cyclopropyl, cyclobutyl, cyclopentyl, cyclohexyl, *N*-methylamino, *N*-ethylamino, *N,N*-dimethylamino or *N,N*-diethylamino, wherein each methoxy, ethoxy, 1-propoxy, 2-propoxy, 1-butoxy, 2-methyl-1-propoxy, 2-butoxy, phenyl, furyl, pyrrolyl, pyridinyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidyl, cyclopropyl, cyclobutyl, cyclopentyl, cyclohexyl, *N*-methylamino, *N*-ethylamino, *N,N*-dimethylamino and *N,N*-diethylamino is independently unsubstituted or substituted with 1, 2, 3, 4 or 5 $R^{w7}$;
wherein $R^6$, $R^{w6}$ and $R^{w7}$ are as defined herein.

In some embodiments, each $R^{w1}$, $R^{w2}$, $R^{w3}$, $R^{w4}$ and $R^{w7}$ is independently D, F, Cl, Br, I, =O, -OH, -CN, $-NH_2$, *N*-methylamino, *N*-ethylamino, *N,N*-dimethylamino, *N,N*-diethylamino, methoxy, ethoxy, 1-propoxy, 2-propoxy, 1-butoxy, 2-methyl-1-propoxy, 2-butoxy, methyl, ethyl, *n*-propyl, isopropyl, *n*-butyl, *i*-butyl, *sec*-butyl, *tert*-butyl, $-CH_2F$, $-CH_2Cl$, $-CF_3$, $-CHF_2$, $-CHCl_2$, $-CH_2CH_2F$, $-CH_2CH_2Cl$, $-CH_2CHF_2$, $-CH_2CHCl_2$, $-CHFCH_2F$, $-CHClCH_2Cl$, $-CH_2CF_3$, $-CH(CF_3)_2$, $-CF_2CH_2CH_3$, $-CH_2CH_2CH_2F$, $-CH_2CH_2CHF_2$, $-CH_2CH_2CF_3$, $-C(=O)-CH_3$, $-C(=O)-CH_2CH_3$, cyclopropyl, cyclobutyl, cyclopentyl, cyclohexyl, aziridinyl, azetidinyl, oxetanyl, thietanyl, pyrrolidyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl, piperazinyl or phenyl, wherein each N-methylamino, *N*-ethylamino, *N,N*-dimethylamino, *N,N*-diethylamino, methoxy, ethoxy, 1-propoxy, 2-propoxy, 1-butoxy, 2-methyl-1-propoxy, 2-butoxy, methyl, ethyl, n-propyl, isopropyl, n-butyl, i-butyl, sec-butyl, tert-butyl, $-CH_2F$, $-CH_2Cl$, $-CHF_2$, $-CHCl_2$, $-CH_2CH_2F$, $-CH_2CH_2Cl$, $-CH_2CHF_2$, $-CH_2CHCl_2$, $-CHFCH_2F$, $-CHClCH_2Cl$, $-CH_2CF_3$, $-CH(CF_3)_2$, $-CF_2CH_2CH_3$, $-CH_2CH_2CH_2F$, $-CH_2CH_2CHF_2$, $-CH_2CH_2CF_3$, $-C(=O)-CH_3$, $-C(=O)-CH_2CH_3$, cyclopropyl, cyclobutyl, cyclopentyl, cyclohexyl, aziridinyl, azetidinyl, oxetanyl, thietanyl, pyrrolidyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl, piperazinyl and phenyl is independently unsubstituted or substituted with 1, 2, 3 or 4 groups independently selected from F, Cl, Br, I, -OH, -CN, $-NH_2$, methyl, ethyl, n-propyl, isopropyl, n-butyl, i-butyl, $-N(CH_3)_2$, $-NHCH_3$, $-N(CH_2CH_3)_2$, $-N(CH_3)CH_2CH_3$ and $-NHCH_2CH_3$;
each $R^{w5}$ and $R^{w6}$ is independently D, F, Cl, Br, I, =O, -OH, -CN, $-NH_2$, $C_{1-4}$ alkylamino, $C_{1-4}$ alkoxy, $C_{1-4}$ alkyl, $C_{1-4}$ haloalkyl, $-C(=O)-C_{1-4}$ alkyl, $C_{2-6}$ alkenyl, $C_{2-6}$ alkynyl, cyclopropyl, cyclobutyl, cyclopentyl, cyclohexyl, heterocyclyl consisting of 3-6 ring atoms, heteroaryl consisting of 5-6 ring atoms or phenyl, wherein each $-NH_2$, $C_{1-4}$ alkylamino, $C_{1-4}$ alkoxy, $C_{1-4}$ alkyl, $C_{1-4}$ haloalkyl, $-C(=O)-C_{1-4}$ alkyl, $C_{2-6}$ alkenyl, $C_{2-6}$ alkynyl, cyclopropyl, cyclobutyl, cyclopentyl, cyclohexyl, heterocyclyl consisting of 3-6 ring atoms, heteroaryl consisting of 5-6 ring atoms and phenyl is independently unsubstituted or substituted with 1, 2, 3 or 4 groups independently selected from F, Cl, Br, I, -OH, -CN, $-NH_2$, $C_{1-4}$ alkyl and $C_{1-4}$ alkylamino.

In some embodiments, each $R^{w5}$ and $R^{w6}$ is independently D, F, Cl, Br, I, =O, -OH, -CN, $-NH_2$, *N*-methylamino, *N*-ethylamino, *N,N*-dimethylamino, *N,N*-diethylamino, methoxy, ethoxy, 1-propoxy, 2-propoxy, 1-butoxy, 2-methyl-l-propoxy, 2-butoxy, methyl, ethyl, *n*-propyl, isopropyl, *n*-butyl, *i*-butyl, *sec*-butyl, *tert*-butyl, $-CH_2F$, $-CH_2Cl$, $-CF_3$, $-CHF_2$, $-CHCl_2$, $-CH_2CH_2F$, $-CH_2CH_2Cl$, $-CH_2CHF_2$, $-CH_2CHCl_2$, $-CHFCH_2F$, $-CHClCH_2Cl$, $-CH_2CF_3$, $-CH(CF_3)_2$, $-CF_2CH_2CH_3$, $-CH_2CH_2CH_2F$, $-CH_2CH_2CHF_2$, $-CH_2CH_2CF_3$, $-C(=O)-CH_3$, $-C(=O)-CH_2CH_3$, $-C(=O)-CH_2CH_2CH_3$, $-CH=CH_2$, $-CH=CHCH_3$, $-CH_2CH=CH_2$, $-CH_2CH_2CH=CH_2$, $-C\equiv CH$, $-CH_2C\equiv CH$, $-C\equiv C-CH_3$, $-CH_2CH_2C\equiv CH$, $-CH_2\equiv CCH_3$, $-C\equiv CCH_2CH_3$, cyclopropyl, cyclobutyl, cyclopentyl, cyclohexyl, aziridinyl, azetidinyl, oxetanyl, thietanyl, pyrrolidyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl, piperazinyl, furyl, pyrrolyl, pyridinyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidyl or phenyl, wherein each $-NH_2$, *N*-methylamino, *N*-ethylamino, *N,N*-dimethylamino, *N,N*-diethylamino, methoxy, ethoxy, 1-propoxy, 2-propoxy, 1-butoxy, 2-methyl-1-propoxy, 2-butoxy, methyl, ethyl, n-propyl, isopropyl, n-butyl, i-butyl, sec-butyl, tert-butyl, $-CH_2F$, $-CH_2Cl$, $-CHF_2$, $-CHCl_2$, $-CH_2CH_2F$, $-CH_2CH_2Cl$, $-CH_2CHF_2$, $-CH_2CHCl_2$, $-CHFCH_2F$, $-CHClCH_2Cl$, $-CH_2CF_3$, $-CH(CF_3)_2$, $-CF_2CH_2CH_3$, $-CH_2CH_2CH_2F$, $-CH_2CH_2CHF_2$, $-CH_2CH_2CF_3$, $-C(=O)-CH_3$, $-C(=O)-CH_2CH_3$, $-C(=O)-CH_2CH_2CH_3$, $-CH=CH_2$, $-CH=CHCH_3$, $-CH_2CH=CH_2$, $-CH_2CH_2CH=CH_2$, $-C\equiv CH$, $-CH_2C\equiv CH$, $-C\equiv C-CH_3$, $-CH_2CH_2C\equiv CH$, $-CH_2\equiv CCH_3$, $-C\equiv CCH_2CH_3$, cyclopropyl, cyclobutyl, cyclopentyl, cyclohexyl, aziridinyl, azetidinyl, oxetanyl, thietanyl, pyrrolidyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl, piperazinyl, furyl, pyrrolyl, pyridinyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidyl and phenyl is independently unsubstituted or substituted with 1, 2, 3 or 4 groups independently selected from F, Cl, Br, I, -OH, -CN, $-NH_2$, methyl, ethyl, *n*-propyl, isopropyl, *n*-butyl, *i*-butyl, $-N(CH_3)_2$, $-NHCH_3$, $-N(CH_2CH_3)_2$, $-N(CH_3)CH_2CH_3$ and $-NHCH_2CH_3$.

In other aspect, provided herein is a pharmaceutical composition comprising the compound and the pharmaceutically acceptable excipient disclosed herein.

In some embodiments, the pharmaceutical composition of the present invention further comprises one or more other therapeutic agent, wherein the therapeutic agent is HBV DNA polymerase inhibitor, Toll Like Receptor 7 conditioning agent, Toll Like Receptor 8 conditioning agent, Toll Like Receptor 7 and 8 conditioning agent, Toll Like Receptor 3 conditioning agent, interferon $\alpha$ ligand, HBsAg inhibitor, compound of HbcAg-targeting, cyclophilin inhibitor, HBV therapeutic vaccine, HBV prophylactic vaccine, HBV virus entry inhibitor, NTCP inhibitor, antisense oligonucleotides

targeting viral mRNA, short interfering RNA (siRNA), hepatitis Be antigen inhibitor, HBx inhibitor, cccDNA inhibitor, HBV antibody, thymosin agonist, cytokine, nuclear protein inhibitor, stimulator of retinoic acid-inducible gene 1, NOD2 stimulator, recombinant thymosin α-1, hepatitis Be antigen replication inhibitor, hepatitis B surface antigen (HBsAg) secretion or assembly inhibitor, IDO inhibitor or combination thereof.

In some embodiments, the pharmaceutical composition of the present invention, wherein one or more other therapeutic agent is Lamivudine, Sebivo, Tenofovir, Entecavir, Adefovir Dipivoxil, Tenofovir Alafenamide, Tenofovir disoproxil, Tenofovir alafenamide fumarate, Tenofovir alafenamide hemifumarate, Alfaferone, Alloferon, Celmoleukin, Clevudine, Emtricitabine, Famciclovir, interferon, hepatect CP, Intefen, Interleukin-2, Mivotilate, Nitazoxanide, Ribavirin, Roferon-A, Schizophyllan, Euforavac, Ampligen, Phosphazid, Heplisav, Recombinant Human Interleukin-2 Injection, levamisole or Proxigermanium.

In some embodiments, the pharmaceutical composition of the present invention, wherein one or more other therapeutic agent is Lamivudine, Sebivo, Tenofovir, Entecavir, Adefovir Dipivoxil, Tenofovir Alafenamide, Tenofovir disoproxil, Tenofovir alafenamide fumarate, Tenofovir alafenamide hemifumarate, Alfaferone, Alloferon, Celmoleukin, Clevudine, Emtricitabine, Famciclovir, hepatect CP, Intefen, interferon α-1b, interferon α, interferon α-2a, interferon β-1a, interferon α-2, Interleukin-2, Mivotilate, Nitazoxanide, polyethylene glycol interferon α-2a, Ribavirin, Roferon-A, Schizophyllan, Euforavac, Ampligen, Phosphazid, Heplisav, interferon α-2b, Recombinant Human Interleukin-2 Injection, levamisole or Proxigermanium.

In other aspect, provided herein is the use of the compound or the pharmaceutical composition thereof in activating TLR8.

In other aspect, provided herein is the use of the compound or the pharmaceutical composition thereof in the manufacture a kit, wherein the kit is used for activating TLR8.

In other aspect, provided herein is the use of the compound or the pharmaceutical composition thereof in the manufacture a medicament for preventing, managing, treating or lessening diseases mediated by TLR8 in patients.

In some embodiments, the diseases mediated by TLR8 are hepatitis B virus infection, hepatitis C virus infection, influenza virus infection, herpes virus infection, HIV infection, allergic diseases, rheumatoid arthritis, allergic asthma, chronic fatigue, type II diabetes mellitus, hay fever, lupus erythrosus, multiple sclerosis, melanoma, lung cancer, liver cancer, basal cell carcinoma, kidney cancer, myeloma, biliary tract cancer, brain cancer, breast cancer, cervical cancer, chorionic cancer, colon cancer, rectal cancer, head and neck cancer, Peritoneal tumors, fallopian tube cancer, endometrial cancer, esophageal cancer, gastric cancer, leukemia, lymphoma, sarcoma, neuroblastoma, oral cancer, ovarian cancer, pancreatic cancer, prostate cancer, testicular cancer, skin cancer or thyroid cancer.

In other aspect, provided herein is the use of the compound or the pharmaceutical composition thereof in the manufacture a medicament for treating or preventing diseases of the immunomodulatory system.

In other aspect, provided herein is the use of the compound or the pharmaceutical composition thereof in the manufacture a medicament for treating or preventing viral infections or tumors.

In other aspect, provided herein is the use of the compound or the pharmaceutical composition thereof in the manufacture a medicament for treating or preventing hepatitis B virus infection, hepatitis C virus infection, influenza virus infection, herpes virus infection, HIV infection, allergic diseases, rheumatoid arthritis, allergic asthma, chronic fatigue, type II diabetes mellitus, hay fever, lupus erythrosus, multiple sclerosis, melanoma, lung cancer, liver cancer, basal cell carcinoma, kidney cancer, myeloma, biliary tract cancer, brain cancer, breast cancer, cervical cancer, chorionic cancer, colon cancer, rectal cancer, head and neck cancer, Peritoneal tumors, fallopian tube cancer, endometrial cancer, esophageal cancer, gastric cancer, leukemia, lymphoma, sarcoma, neuroblastoma, oral cancer, ovarian cancer, pancreatic cancer, prostate cancer, testicular cancer, skin cancer and thyroid cancer.

In another aspect, provided herein are methods for preparing, separating, and purifying the compounds represented by Formula (I).

The foregoing merely summarizes certain aspects disclosed herein and is not intended to be limiting in nature. These aspects and other aspects and embodiments are described more fully below.

## DETAILED DESCRIPTION OF THE INVENTION

## DEFINITIONS AND GENERAL TERMINOLOGY

The present invention will be listed in detail the literature corresponding to the determined concretized content, and the embodiments are accompanied by diagrams of structural and chemical formulas. The present invention is expected to cover all options, variants, and equivalents that may be included in the field of the prior invention as defined in the claims. A person skilled in the art will identify a number of similar or equivalent methods and substances described herein, which may be applied in practice to the present invention. The present invention is in no way limited to the description of methods and substances. There are many literature and similar substances that distinguish or contradict the application for the present invention, including, but not limited to, the definition of terms, the usage of terms, the techniques

described, or the extent controlled by the application for the present invention.

The following definitions shall apply to the present invention, unless otherwise indicated. For the purposes of the present invention, chemical elements are defined according to the periodic table, CAS version and Handbook of Chemical Agents, 75, thed, 1994. Additionally, general principles of organic chemistry are described in "Organic Chemistry", Thomas Sorrell, University Science Books, Sausalito: 1999, and "March's Advanced Organic Chemistry," by Michael B. Smith and Jerry March, John Wiley&Sons, New York: 2007, thus all contents are incorporated into the present invention by reference.

As described herein, compounds disclosed herein may optionally be substituted with one or more substituents, such as are illustrated generally below, or as exemplified by particular classes, subclasses, and species of the invention.

Furthermore, unless otherwise stated, the phrase "each...is independently" is used interchangeably with the phrase "each (of)...and...is independently". It should be understood broadly that the specific options expressed by the same symbol are independently of each other in different radicals; or the specific options expressed by the same symbol are independently of each other in same radicals.

At various places in the present specification, substituents of compounds disclosed herein are disclosed in groups or in ranges. It is specifically intended that the invention include each and every individual subcombination of the members of such groups and ranges. For example, the term "$C_1$-$C_6$ alkyl" is specifically intended to individually disclose methyl, ethyl, $C_3$ alkyl, $C_4$ alkyl, $C_5$ alkyl, and $C_6$ alkyl.

The term "alkyl" or "alkyl group" refers to a saturated linear or branched-chain monovalent hydrocarbon radical of 1 to 20 carbon atoms, wherein the alkyl may be optionally and independently substituted with one or more substituents described herein. In some embodiments, the alkyl group contains 1-12 carbon atoms; in other embodiments, the alkyl group contains 1-10 carbon atoms; in other embodiments, the alkyl group contains 1-8 carbon atoms; in still other embodiments, the alkyl group contains 1-6 carbon atoms; in yet other embodiments, the alkyl group contains 1-4 carbon atoms and in still yet other embodiments, the alkyl group contains 1-3 carbon atoms. Some non-limiting examples of the alkyl group include, methyl (Me, $-CH_3$), ethyl (Et, $-CH_2CH_3$), n-propyl (*n*-Pr, $-CH_2CH_2CH_3$), isopropyl (*i*-Pr, $-CH(CH_3)_2$), *n*-butyl (*n*-Bu, $-CH_2CH_2CH_2CH_3$), 2-methyl-propyl or isobutyl (*i*-Bu, $-CH_2CH(CH_3)_2$), 1-methyl-propyl or sec-butyl (*s*-Bu, $-CH(CH_3)CH_2CH_3$), tert-butyl (*t*-Bu, $-C(CH_3)_3$), *n*-pentyl ($-CH_2CH_2CH_2CH_2CH_3$), 2-pentyl ($-CH(CH_3)CH_2CH_2CH_3$), 3-pentyl ($-CH(CH_2CH_3)_2$), 2-methyl-2-butyl ($-C(CH_3)_2CH_2CH_3$), 3-methyl-2-butyl ($-CH(CH_3)CH(CH_3)_2$), 3-methyl-l-butyl ($-CH_2CH_2CH(CH_3)_2$), 2-methyl-l-butyl ($-CH_2CH(CH_3)CH_2CH_3$), *n*-hexyl ($-CH_2CH_2CH_2CH_2CH_2CH_3$), 2-hexyl ($-CH(CH_3)CH_2CH_2CH_2CH_3$), 3-hexyl ($-CH(CH_2CH_3)(CH_2CH_2CH_3)$), 2-methyl-2-pentyl ($-C(CH_3)_2CH_2CH_2CH_3$), 3-methyl-2-pentyl ($-CH(CH_3)CH(CH_3)CH_2CH_3$), 4-methyl-2-pentyl ($-CH(CH_3)CH_2CH(CH_3)_2$), 3-methyl-3-pentyl ($-C(CH_3)(CH_2CH_3)_2$), 2-methyl-3-pentyl ($-CH(CH_2CH_3)CH(CH_3)_2$), 2,3-dimethyl-2-butyl ($-C(CH_3)_2CH(CH_3)_2$), 3,3-dimethyl-2-butyl ($-CH(CH_3)C(CH_3)_3$, *n*-heptyl and *n*-octyl, etc.

The term "alkylene" refers to a saturated divalent or polyvalent hydrocarbon group derived from a straight or branched chain saturated hydrocarbon by the removal of two or more hydrogen atoms. Unless otherwise specified, the alkylene group contains 1-12 carbon atoms. In some embodiments, the alkylene group contains 1-6 carbon atoms; in other embodiments, the alkylene group contains 1-4 carbon atoms; in still other embodiments, the alkylene group contains 1-3 carbon atoms; in other embodiments, the alkylene group contains 1-2 carbon atoms. Some non-limiting examples of the alkylene include methylene ($-CH_2-$), ethylene ($-CH_2CH_2-$), *N*-propyl ($-CH_2CH_2CH_2-$), isopropylidene ($-CH(CH_3)CH_2-$), and the like.

The term "alkenyl" refers to linear or branched-chain monovalent hydrocarbon radical of 2 to 12 carbon atoms or 2 to 8 carbon atoms or 2 to 6 carbon atoms or 2 to 4 carbon atoms, wherein at least one position of C-C is $sp^2$ double bond, wherein the alkenyl radical may be optionally unsubstituted or substituted independently with one or more substituents described herein, and includes radicals having *"cis"* and *"trans"* orientations, or alternatively, "*E*" and "*Z*" orientations, some non-limiting examples of the alkenyl include vinyl ($-CH=CH_2$), propenyl ($-CH=CHCH_3$), allyl ($-CH_2CH=CH_2$), $-CH_2CH_2CH=CH_2$, and the like, wherein the alkenyl radical may be optionally unsubstituted or substituted independently with one or more substituents described herein

The term "alkynyl" refers to linear or branched-chain monovalent hydrocarbon radical of 2 to 12 carbon atoms or 2 to 8 carbon atoms or 2 to 6 carbon atoms or 2 to 4 carbon atoms, wherein at least one position of C-C is sp triple bond, wherein the alkynyl radical may be optionally unsubstituted or substituted independently with one or more substituents described herein, some non-limiting examples of the alkynyl include ethynyl ($-C\equiv CH$), propargyl ($-CH_2C\equiv CH$), propynyl ($-CH_2C\equiv CH$), 1-butynyl ($-CH_2CH_2C\equiv CH$), 2-butynyl ($-CH_2C\equiv CCH_3$), 3-butynyl ($-C\equiv CCH_2CH_3$), and the like, wherein the alkynyl radical may be optionally unsubstituted or substituted independently with one or more substituents described herein

The terms "haloalkyl", "haloalkenyl" or "haloalkoxy" refer to alkyl, alkenyl or alkoxy substituted with one or more halogen atoms. Wherein the alkyl, alkenyl and alkoxy are as defined herein. Some non-limiting examples include difluoroethyl ($-CH_2CHF_2$, $-CF_2CH_3$, $-CHFCH_2F$), trifluoroethyl ($-CH_2CF_3$, $-CF_2CH_2F$, $-CFHCHF_2$), trifluoromethyl ($-CF_3$), trifluoromethoxy ($-OCF_3$), fluorovinyl ($-CH=CHF$, $-CF=CH_2$), and the like.

The term "alkoxy" refers to an alkyl group, as previously defined, attached to the parent molecular moiety via an

oxygen atom. Unless otherwise specified, the alkoxy group contains 1-12 carbon atoms. In some embodiments, the alkoxy group contains 1-8 carbon atoms; in other embodiments, the alkoxy group contains 1-6 carbon atoms; in still other embodiments, the alkoxy group contains 1-4 carbon atoms; in yet other embodiments, the alkoxy group contains 1-3 carbon atoms. The alkoxy group may be optionally substituted with one or more substituents disclosed herein.

Some non-limiting examples of the alkoxy group include, but are not limited to, methoxy (MeO, $-OCH_3$), ethoxy (EtO, $-OCH_2CH_3$), 1-propoxy (*n*-PrO, *n*-propoxy, $-OCH_2CH_2CH_3$), 2-propoxy (*i*-PrO, *i*-propoxy, $-OCH(CH_3)_2$), 1-butoxy (*n*-BuO, *n*-butoxy, $-OCH_2CH_2CH_2CH_3$), 2-methyl-1-propoxy (*i*-BuO, *i*-butoxy, $-OCH_2CH(CH_3)_2$), 2-butoxy (*s*-BuO, *s*-butoxy, $-OCH(CH_3)CH_2CH_3$), 2-methyl-2-propoxy (*t*-BuO, t-butoxy, $-OC(CH_3)_3$), 1-pentoxy (*n*-pentoxy, $-OCH_2CH_2CH_2CH_2CH_3$), 2-pentoxy ($-OCH(CH_3)CH_2CH_2CH_3$), 3-pentoxy ($-OCH(CH_2CH_3)_2$), 2-methyl-2-butoxy ($-OC(CH_3)_2CH_2CH_3$), 3-methyl-2-butoxy ($-OCH(CH_3)CH(CH_3)_2$), 3-methyl-l-butoxy ($-OCH_2CH_2CH(CH_3)_2$), 2-methyl-l-butoxy ($-OCH_2CH(CH_3)CH_2CH_3$), and the like.

The term "aryl" used alone or as part of a larger moiety as in "arylalkyl", "arylalkoxy" or "aryloxyalkyl" refers to monocyclic, bicyclic and tricyclic carbocyclic ring systems having a total of 6 to 14 carbon atoms, or 6 to 12 carbon atoms, or 6 to 10 carbon atoms, wherein at least one ring in the system is aromatic, wherein each ring in the system contains 3 to 7 carbon atoms and that has a single point or multipoint of attachment to the rest of the molecule. The term "aryl" may be used interchangeably with the term "aryl ring" or "aromatic ring", such as the aryl group include phenyl, naphthyl and anthracene. The aryl group may be optionally substituted with one or more substituents disclosed herein.

The term "heteroaryl" refers to monocyclic, bicyclic or tricyclic ring containing 5-16 ring atoms, wherein at least one ring in the system is aromatic, and in which at least one ring member contains one or more heteroatom, and wherein each ring in the system contains 5 to 7 ring atoms and that has a single point or multipoint of attachment to the rest of the molecule. The term "heteroaryl" may be used interchangeably with the term "heteroaryl ring", "aromatic heterocyclic" or the term "heteroaromatic compound", such as the heteroaryl group include monocyclic heteroaryl, fused bicyclic heteroaryl, or polycyclic fused heteroaryl. In some embodiments, heteroaryl is a heteroaryl group consisting of 5-14 ring atoms comprising 1, 2, 3 or 4 heteroatoms independently selected from O, S and N. In other embodiments, heteroaryl is a heteroaryl group consisting of 5-12 ring atoms comprising 1, 2, 3 or 4 heteroatoms independently selected from O, S and N. In other embodiments, heteroaryl is a heteroaryl group consisting of 5-10 ring atoms comprising 1, 2, 3 or 4 heteroatoms independently selected from O, S and N. In other embodiments, heteroaryl is a fused bicyclic heteroaryl group consisting of 8-10 ring atoms comprising 1, 2, 3 or 4 heteroatoms independently selected from O, S and N. In other embodiments, heteroaryl is a heteroaryl group consisting of 5-8 ring atoms comprising 1, 2, 3 or 4 heteroatoms independently selected from O, S and N. In other embodiments, heteroaryl is a heteroaryl group consisting of 5-7 ring atoms comprising 1, 2, 3 or 4 heteroatoms independently selected from O, S and N. In other embodiments, heteroaryl is a monocyclic heteroaryl group consisting of 5-6 ring atoms comprising 1, 2, 3 or 4 heteroatoms independently selected from O, S and N. In other embodiments, heteroaryl is a heteroaryl group consisting of 5 ring atoms comprising 1, 2, 3 or 4 heteroatoms independently selected from O, S and N. In other embodiments, heteroaryl is a heteroaryl group consisting of 6 ring atoms comprising 1, 2, 3 or 4 heteroatoms independently selected from O, S and N.

Other embodiments are that heteroaryls include, but are not limited to, the following monocyclic groups: 2-furanyl, 3-furanyl, *N*-imidazolyl, 2-imidazolyl, 4-imidazolyl, 5-imidazolyl, 3-isoxazolyl, 4-isoxazolyl, 5-isoxazolyl, 2-oxazolyl, 4-oxazolyl, 5-oxazolyl, *N*-pyrrolyl, 2-pyrrolyl, 3-pyrrolyl, 2-pyridyl, 3-pyridyl, 4-pyridyl, 2-pyrimidinyl, 4-pyrimidinyl, 5-pyrimidinyl, pyridazinyl (e.g., 3-pyridazinyl), 2-thiazolyl, 4-thiazolyl, 5-thiazolyl, tetrazolyl, e.g., 5-tetrazolyl, 2-tetrazolyl), triazolyl (e.g., 2-triazolyl, 5-triazolyl, 4*H*-1,2,4-triazolyl, 1*H*-1,2,4-triazolyl, 1,2,3-triazolyl), 2-thienyl, 3-thienyl, pyrazolyl (e.g., 2-pyrazolyl and 3-pyrazolyl), isothiazolyl, 1,2,3-oxadiazolyl, 1,2,5-oxadiazolyl, 1,2,4-oxadiazolyl, 1,3,4-oxadiazolyl, 1,2,3-thiadiazolyl, 1,3,4-thiadiazolyl, 1,2,5-thiadiazolyl, pyrazinyl, 1,3,5-triazinyl; heteroaryls also include, but are not limited to, the following bicyclic or tricyclic groups: benzimidazolyl, benzofuryl, benzothiophenyl, indolyl (e.g., 2-indolyl), purinyl, quinolinyl (e.g., 2-quinolinyl, 3-quinolinyl, 4-quinolinyl), isoquinolinyl (e.g., 1-isoquinolinyl, 3-isoquinolinyl or 4-isoquinolinyl), phenoxothiyl, dibenzimidazolyl, dibenzofuryl, dibenzothiophenyl. The heteroaryl group is optionally substituted with one or more substituents disclosed herein.

The term "consisting of $M-M_1$ ring atoms" refers to that the ring group is composed of $M-M_1$ ring atoms, and the ring atoms include carbon atoms and/or heteroatoms such as O, N, S, P, etc. For example, "heteroaryl groups consisting of 5-10 ring atoms" means that they include heteroaryl groups consisting of 5, 6, 7, 8, 9 or 10 ring atoms.

The terms "heterocyclyl" and "heterocycle" are used interchangeably herein, refer to a saturated or partially unsaturated non-aromatic monocyclic, bicyclic or tricyclic ring system containing 3-12 ring atoms, in which at least one ring member is selected from nitrogen, sulfur and oxygen, and the ring system has one or more junctions that connect to the rest of the molecule. The terms "heterocyclyl" include monocyclic heterocyclyl, bicyclic fused heterocyclyl or polycyclic fused heterocyclyl, spirocyclic heterocyclyl or bridged heterocyclyl, it also includes a polycyclic ring system in which heterocyclyls may be fused with one or more non-aromatic carbon rings or heterocycles or one or more aromatic rings or a combination thereof, where connected atomic clusters or dots are on the heterocycles. Bicyclic heterocyclyl include bridged bicyclic heterocyclyl, fused bicyclic heterocyclyl, and spiral bicyclic heterocyclyl. Unless otherwise specified, $-CH_2$-group of the heterocyclyl group can be optionally replaced by a -C(=O)- group. In which, the sulfur atom of the ring

can be optionally oxygenized to S-oxide. and the nitrogen atom of the ring can be optionally oxygenized to N oxide. In some embodiments, heterocyclyl is ring system consisting of 3-12 ring atoms; in some embodiments, heterocyclyl is monocyclic heterocyclyl consisting of 4-7 ring atoms; in some embodiments, heterocyclyl is monocyclic heterocyclyl consisting of 3-7 ring atoms; in some embodiments, heterocyclyl is monocyclic heterocyclyl consisting of 4-6 ring atoms; in some embodiments, heterocyclyl is monocyclic heterocyclyl consisting of 3-6 ring atoms; in some embodiments, heterocyclyl is monocyclic heterocyclyl consisting of 5-6 ring atoms; in some embodiments, heterocyclyl is bicyclic heterocyclyl consisting of 7-12 ring atoms; in some embodiments, heterocyclyl is fused bicyclic heterocyclyl consisting of 7-12 ring atoms; in some embodiments, heterocyclyl is fused bicyclic heterocyclyl consisting of 7-10 ring atoms; in some embodiments, heterocyclyl is fused bicyclic heterocyclyl consisting of 8-10 ring atoms; in some embodiments, heterocyclyl is bridged bicyclic heterocyclyl consisting of 6-10 ring atoms; in other embodiments, heterocyclyl is ring system consisting of 3-8 ring atoms; in other embodiments, heterocyclyl is ring system consisting of 3-6 ring atoms; in other embodiments, heterocyclyl is ring system consisting of 5-7 ring atoms; in other embodiments, heterocyclyl is ring system consisting of 5-8 ring atoms; in other embodiments, heterocyclyl is ring system consisting of 6-8 ring atoms; heterocyclyl is ring system consisting of 3 ring atoms; in other embodiments, heterocyclyl is ring system consisting of 4 ring atoms; in other embodiments, heterocyclyl is ring system consisting of 5 ring atoms; in other embodiments, heterocyclyl is ring system consisting of 6 ring atoms; in other embodiments, heterocyclyl is ring system consisting of 7 ring atoms; in other embodiments, heterocyclyl is ring system consisting of 8 ring atoms.

Some non-limiting examples of the heterocyclyl group include pyrrolidinyl, tetrahydrofuranyl, dihydrofuranyl, tetrahydrothienyl, tetrahydropyranyl, dihydropyranyl, tetrahydrothiopyranyl, piperidyl, piperidinyl, morpholinyl, thiomorpholinyl, thioxanyl, piperazinyl, homopiperazinyl, azetidinyl, oxetanyl, thietanyl, homopiperidinyl, oxiranyl, azepanyl, oxepanyl, thiepanyl, oxazepinyl, diazepinyl, thiazepinyl, 2-pyrrolinyl, 3-pyrrolinyl, dihydroindolyl, 2*H*-pyranyl, 4*H*-pyranyl, dioxanyl, 1,3-dioxolanyl, pyrazolinyl, dithianyl, dithiolanyl, dihydrothienyl, pyrazolidinyl, imidazolinyl, imidazolidinyl, 1,2,3,4-tetrahydroisoquinolinyl, 3-azabicyclo[3.1.0]hexyl, 3-azabicyclo[3.1.0]heptyl, azabicyclo[3.1.0]hexyl, 3*H*-indolylquinazinyl and *N*-pyridyl urea. Examples of heterocyclyl group also include 1,1-dioxothiomorpholinyl; wherein, some non-limiting examples of substitution of carbon atoms on rings by oxotype (=O) include pyrimidinedionyl, 1,2,4-thiadiazol-5(4*H*)-keto, 1,2,4-oxadiazol-5(4*H*)-keto, 1*H*-1,2,4-triazol-5(4*H*)-keto, etc.; wherein, some non-limiting examples of substitution of carbon atoms on rings by =O include 1,2,4-oxadiazol-5(4*H*)-thioneyl, 1,3,4-oxadiazol-2(3*H*)-thioneyl, and the like. The heterocyclyl group may be optionally substituted with one or more substituents disclosed herein.

The term "fused bicycle" refers to a monovalent or polyvalent, saturated, partially unsaturated, or completely unsaturated system of non-aromatic or aromatic ring system in which two rings share two adjacent ring atoms.

The term "spirocyclyl", "spirocycle", "spiro bicyclyl" or "spiro bicyclic" as used interchangeably herein refers to a monovalent or multivalent saturated or partially unsaturated of non-aromatic ring system, wherein a ring originating from a particular annular carbon atom of another ring, and the two rings share only one atom.

For example, as depicted in Formula a below, ring B and B' are termed as "fused bicyclic", while ring A' and ring B share a carbon atom and are called "spirocycle" or "spiro bicyclic". Each cyclic ring in the fused bicyclyl or spiro bicyclyl can be either a carbocyclic or a heteroalicyclic, and optionally substituted with one or more substituents disclosed herein.

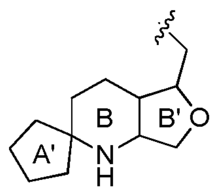

Formula a

The term "fused bicyclic heterocyclyl" and "bicyclic fused heterocyclyl" as used interchangeably refers to a monovalent saturated or partially unsaturated of non-aromatic parallel ring system. Such system can comprise independent or conjugated unsaturated units, but the core structure does not comprise aromatic ring or heteroaromatic ring (but aromatic group can be used as substituents of the above group). Each ring in the ring system comprises 3-7 atoms, and at least one ring contains one or more heteroatoms, i.e., comprises 1-6 carbon atoms and 1-3 heteroatoms selected from N, O, P, S, wherein S or P is optionally substituted with one or more oxygen atoms to obtain groups like SO, $SO_2$, PO, $PO_2$, in some embodiments, fused bicyclic heterocyclyl is fused bicyclic heterocyclyl consisting of 7-10 ring atoms; in some embodiments, fused bicyclic heterocyclyl is fused bicyclic heterocyclyl consisting of 8-10 ring atoms; examples include, but are not limited to, 3-aza-dicyclo[3.3.0]hexane, 3-azabicyclo[3.3.0]octane, hexahydro-furan[3,4-c]pyrrolyl, hexahydrothienyl[3,4-c]pyrrolyl, 3,4,5,6-tetrahydro-cyclopentane[c]thienyl, etc. The fused heterobicyclo group is optionally substituted with one or more substituents disclosed herein.

The term "bridged bicyclyl" or "bridged bicyclic" refers to a saturated or partially unsaturated non-aromatic

bridged ring system, as shown in formula b, that is, ring A1 and ring A2 share an alkane chain, a heteroatom or a heteroalkane chain, wherein j is 1, 2, 3 or 4, and $X^3$ is an alkane chain, a heteroatom or a heteroalkane chain. Such system can comprise independent or conjugated unsaturated units, but the core structure does not comprise aromatic ring or heteroaromatic ring (but aromatic group can be used as substituents of the above group). In which each ring, such as A1 or A2, contains 3 to 7 ring members, some non-limiting examples of the bridged bicyclyl group include bicyclo[2.2.1]heptyl, 2-methyl-diazabicyclo[2.2.1]heptyl, and the like. The bridged bicyclyl is optionally substituted with one or more substituents described herein.

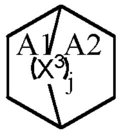

式 b

The term "bridged bicyclic heterocyclyl" refers to saturated or partially unsaturated non-aromatic bridged bicyclic system, wherein each ring comprises 3-7 atoms, and at least one ring contains one or more heteroatoms, i.e., comprises 1-6 carbon atoms and 1-3 heteroatoms selected from N, O, P, S, wherein S or P is optionally substituted with one or more oxygen atoms to obtain groups like SO, $SO_2$, PO, $PO_2$, in some embodiments, bridged bicyclic heterocyclyl is bridged bicyclic heterocyclyl consisting of 6-10 ring atoms, some non-limiting examples include 2-oxa-5-azabicyclo[2.2.1]heptyl, 2-thio-5-azabicyclo[2.2.1]heptyl, 2-oxo-5-azabicyclo[2.2.1]heptyl, 2,5-diazabicyclo[2.2.1]heptyl, 2-methyl-2,5-diazabicyclo[2.2.1]heptyl, and the like. Wherein the bridged bicyclic heterocyclyl is optionally substituted with one or more substitutents described herein.

The term "cycloalkyl" refers to a saturated, monocyclic, bicyclic or tricyclic ring system containing 3 to 12 ring carbon atoms having one or more points of attachment to the rest of the molecule, including monocyclic, bicyclic or polycyclic fused, spiro or bridged ring systems. In some embodiments, the cycloalkyl is a spiro bicycloalkyl consisting of 6-10 atoms; in other embodiments, the cycloalkyl is a fused bicycloalkyl group consisting of 6-10 atoms; in other embodiments, the cycloalkyl is a ring system consisting 3-10 ring carbon atoms; in other embodiments, the cycloalkyl is a ring system consisting 3-8 ring carbon atoms; in other embodiments, the cycloalkyl is a ring system consisting 3-7 ring carbon atoms; in other embodiments, the cycloalkyl is a ring system consisting 5-8 ring carbon atoms; in other embodiments, the cycloalkyl is a ring system consisting 3-6 ring carbon atoms; in other embodiments, the cycloalkyl is a ring system consisting 5-6 ring carbon atoms; some non-limiting examples of cycloalkyl include cyclopropyl, cyclobutyl, cyclopentyl, cyclohexyl, etc., and the cycloalkyl can be independently unsubstituted or substituted with one or more substituents described herein.

The term "alkylamino" refers to "*N*-alkylamino" and "*N*,*N*-dialkylamino" wherein amino group is independently substituted with one alkyl or two alkyl radicals, respectively. In some embodiments, the alkylamino is lower alkylamino group formed by one or two $C_{1-12}$ alkyl groups attached to nitrogen atom. In some embodiments, the alkylamino is lower alkylamino group formed by one or two $C_{1-6}$ alkyl groups attached to nitrogen atom. In some embodiments, the alkylamino is lower alkylamino group formed by one or two $C_{1-4}$ alkyl groups attached to nitrogen atom. In some embodiments, the alkylamino is lower alkylamino group formed by one or two $C_{1-3}$ alkyl groups attached to nitrogen atom. Suitable alkylamino groups may be monoalkylamino or dialkylamino, some non-limiting examples of the alkylamino group include *N*-methylamino ($-NHCH_3$), *N*-ethylamino ($-NHCH_2CH_3$), *N,N*-dimethylamino ($-N(CH_3)_2$), *N,N*-diethylamino ($-N(CH_2CH_3)_2$), and the like.

Unless otherwise stated, structures depicted herein are also meant to include all isomeric (e.g., enantiomers, diastereomers, and geometric isomers (or conformational isomers)) forms of the structure; for example, the *R* and *S* configurations for each asymmetric center, (*Z*) and (*E*) double bond isomers, and (*Z*) and (*E*) conformational isomers. Therefore, single stereochemical isomers as well as enantiomeric, diastereomeric, orgeometric isomer (or conformational isomer) mixtures of the present compounds are within the scope disclosed herein.

The term "prodrug" refers to a compound that is transformed *in vivo* into a compound of Formula (I). Such a transformation can be affected, for example, by hydrolysis of the prodrug form in blood or enzymatic transformation to the parent form in blood or tissue. Prodrugs of the compounds disclosed herein may be, for example, esters. Some common esters which have been utilized as prodrugs are phenyl esters, aliphatic ($C_{1-24}$) esters, acyloxymethyl esters, carbonates, carbamates and amino acid esters. For example, a compound disclosed herein that contains a hydroxy group may be acylated at this position in its prodrug form. Other prodrug forms include phosphates, such as, those phosphate compounds derived from the phosphonation of a hydroxy group on the parent compound. A thorough discussion of prodrugs is provided in T. Higuchi and V. Stella, Pro-drugs as Novel Delivery Systems, Vol. 14 of the A.C.S. Symposium Series, Edward B. Roche, ed., Bioreversible Carriers in Drug Design, American Pharmaceutical Association and Pergamon Press, 1987, J. Rautio et al., Prodrugs: Design and Clinical Applications, Nature Review Drug Discovery,

2008, 7, 255-270, and S. J. Hecker et al., Prodrugs of Phosphates and Phosphonates, Journal of Medicinal Chemistry, 2008, 51, 2328-2345, all of which are incorporated herein by reference in their entireties.

Unless otherwise stated, all tautomeric forms of the compounds disclosed herein are within the scope of the invention. Additionally, unless otherwise stated, structures depicted herein are also meant to include compounds that differ only in the presence of one or more isotopically enriched atoms.

A "metabolite" is a product produced through metabolism in the body of a specified compound or salt thereof. The metabolites of a compound may be identified using routine techniques known in the art and their activities determined using tests such as those described herein. Such products may result for example from oxidation, reduction, hydrolysis, amidation, deamidation, esterification, deesterification, enzyme cleavage, and the like, of the administered compound. Accordingly, the invention includes metabolites of compounds disclosed herein, including metabolites produced by contacting a compound disclosed herein with a mammal for a sufficient time period.

Stereochemical definitions and conventions used herein generally follow S. P. Parker Ed., McGraw-Hill Dictionary of Chemical Terms (1984) McGraw-Hill Book Company, New York and Eliel et al., "Stereochemistry of Organic Compounds", John Wiley & Sons, Inc., New York, 1994. The compounds disclosed herein may contain asymmetric or chiral centers, and therefore exist in different stereoisomeric forms. It is intended that all stereoisomeric forms of the compounds disclosed herein, including, but not limited to, diastereomers, enantiomers and atropisomers, as well as mixtures thereof such as racemic mixtures, form part of the present invention. Many organic compounds exist in optically active forms, i.e., they have the ability to rotate the plane of plane-polarized light. In describing an optically active compound, the prefixes *D, L* or *R,* S, are used to denote the absolute configuration of the molecule about its chiral center(s). The prefixes d and 1 or (+) and (-) are employed to designate the sign of rotation of plane-polarized light by the compound, with (-) or *l* meaning that the compound is levorotatory. A compound prefixed with (+) or *d* is dextrorotatory. For a given chemical structure, these stereoisomers are identical except that they are mirror images of one another. A specific stereoisomer may also be referred to as an enantiomer, and a mixture of such isomers is often called an enantiomeric mixture. A 50:50 mixture of enantiomers is referred to as a racemic mixture or a racemate, which may occur where there has been no stereoselection or stereospecificity in a chemical reaction or process. The term "racemic mixture" or "racemate" refers to an equimolar mixture of two enantiomeric species, devoid of optical activity.

The term "tautomer" or "tautomeric form" refers to structural isomers of different energies which are interconvertible via a low energy barrier. Some non-limiting examples of proton tautomers (also known as prototropic tautomers) include interconversions via migration of a proton, such as keto-enol and imine-enamine isomerizations. Valence tautomers include interconversions by reorganization of some of the bonding electrons. Unless otherwise stated, all tautomeric forms of the compounds disclosed herein are within the scope of the invention.

A "pharmaceutically acceptable salts" refers to organic or inorganic salts of a compound disclosed herein. Pharmaceutically acceptable salts are well known in the art. For example, Berge et al., describe pharmaceutically acceptable salts in detail in J. Pharmacol Sci, 1977, 66:1-19, which is incorporated herein by reference. Some non-limiting examples of pharmaceutically acceptable and nontoxic salts include salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid and malonic acid or by using other methods used in the art such as ion exchange. Other pharmaceutically acceptable salts include adipate, 2-hydroxy propionate, alginate, ascorbate, aspartate, benzenesulfonate, benzoate, bisulfate, borate, butyrate, camphorate, camphorsulfonate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, laurylsulfate, malate, malonate, methanesulfonate, 2-naphthalenesulfonate, nicotinate, nitrate, oleate, palmitate, pamoate, pectinate, persulfate, 3-phenylpropionate, picrate, pivalate, propionate, stearate, thiocyanate, p-toluenesulfonate, undecanoate, valerate salts, and the like. Salts derived from appropriate bases include alkali metal, alkaline earth metal, ammonium and $N^+(C_{1-4}\ alkyl)_4$ salts. This invention also envisions the quaternization of any basic nitrogen-containing groups of the compounds disclosed herein. Water or oil soluble or dispersable products may be obtained by such quaternization. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, $C_{1-8}$ sulfonate or aryl sulfonate.

The term "solvate" refers to an association or complex of one or more solvent molecules and a compound disclosed herein. Some non-limiting examples of the solvent that form solvates include water, isopropanol, ethanol, methanol, dimethylsulfoxide (DMSO), ethyl acetate, acetic acid and ethanolamine. The term "hydrate" refers to the complex where the solvent molecule is water.

The term "protecting group" or "Pg" refers to a substituent that is commonly employed to block or protect a particular functionality while reacting with other functional groups on the compound. For example, an "amino-protecting group" is a substituent attached to an amino group that blocks or protects the amino functionality in the compound. Suitable amino-protecting groups include acetyl, trifluoroacetyl, t-butoxy-carbonyl (BOC), benzyloxycarbonyl (CBZ) and 9-fluorenylmethylenoxy-carbonyl (Fmoc). Similarly, a "hydroxy-protecting group" refers to a substituent of a hydroxy group that blocks or protects the hydroxy functionality. Suitable protecting groups include acetyl and silyl. A "carboxy-protecting group" refers to a substituent of the carboxy group that blocks or protects the carboxy functionality. Common carboxy-protecting groups include $-CH_2CH_2SO_2Ph$, cyanoethyl, 2-(trimethylsilyl)-ethyl, 2-(trimethylsilyl) ethoxy-methyl, 2-(p-toluenesulfonyl) ethyl, 2-(p-nitrophenylsulfonyl)-ethyl, 2-(diphenylphosphino)-ethyl, nitroethyl and the like. For a general description of protecting groups and their use, see T. W. Greene, Protective Groups in Organic Synthesis, John Wiley & Sons, New York, 1991; and P. J. Kocienski, Protecting Groups, Thieme, Stuttgart, 2005.

**DESCRIPTION OF COMPOUNDS OF THE INVENTION**

The compound and pharmaceutically acceptable composition thereof involved in the present invention can effectively activate TLR8 and inhibit HBV infection.

In one aspect, the present invention relates to a compound having Formula (I) or a stereoisomer, a tautomer, an N-oxide, a solvate, a metabolite, a pharmaceutically acceptable salt or a prodrug thereof,

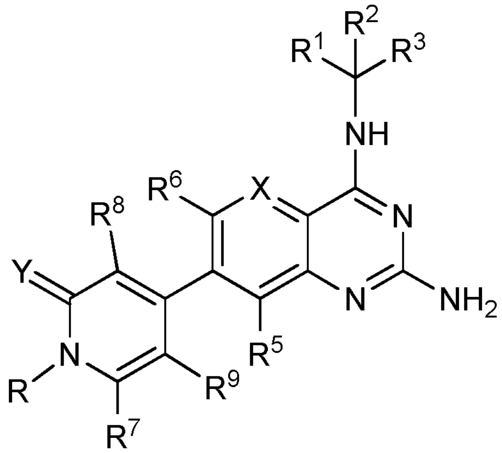

(I)

wherein, X is N or $CR^4$;

each $R^1$, $R^2$, $R^4$, $R^5$, $R^6$, $R^7$ and $R^8$ is independently H, D, F, Cl, Br, I, -OH, -CN, $-NH_2$, $C_{1-4}$ alkylamino, $C_{1-6}$ alkoxy or $C_{1-6}$ alkyl, wherein each $C_{1-4}$ alkylamino, $C_{1-6}$ alkoxy and $C_{1-6}$ alkyl is independently unsubstituted or substituted with 1, 2, 3 or 4 groups independently selected from F, Cl, Br, I, -OH, -CN, $-NH_2$ and $C_{1-4}$ alkyl;

Yis O or S;

$R^3$ is $-C_{1-6}$ alkylene-$R^{10}$, wherein the $-C_{1-6}$ alkylene of $-C_{1-6}$ alkylene-$R^{10}$ is unsubstituted or substituted with 1, 2, 3, 4 or 5 $R^{w1}$;

each $R^{10}$ is independently $C_{1-6}$ alkoxy, -OH, $-NH_2$, -O-C(=O)-$R^{10a}$ or -$NR^c$-C(=O)-$R^{10b}$, wherein the $C_{1-6}$ alkoxy is unsubstituted or substituted with 1, 2, 3 or 4 groups independently selected from F, Cl, Br, I, -OH, -CN, $-NH_2$ and $C_{1-4}$ alkyl;

each $R^c$ is independently H, D or $C_{1-4}$ alkyl, wherein the $C_{1-4}$ alkyl is unsubstituted or substituted with 1, 2, 3 or 4 groups independently selected from F, Cl, Br, I, -OH, -CN, $-NH_2$ and $C_{1-4}$ alkyl;

each $R^{10a}$ and $R^{10b}$ is independently $C_{1-6}$ alkyl, $C_{3-7}$ cycloalkyl, heteroaryl consisting of 5-12 ring atoms or heterocyclyl consisting of 3-12 ring atoms, wherein each $C_{1-6}$ alkyl, $C_{3-7}$ cycloalkyl, heteroaryl consisting of 5-12 ring atoms and heterocyclyl consisting of 3-12 ring atoms is independently unsubstituted or substituted with 1, 2, 3, 4 or 5 $R^{w2}$;

R is H, D, $C_{1-6}$ alkyl or heterocyclyl consisting of 3-6 ring atoms, wherein each $C_{1-6}$ alkyl and heterocyclyl consisting of 3-6 ring atoms is independently unsubstituted or substituted with 1, 2, 3, 4 or 5 $R^{w3}$.,

$R^9$ is H, D, -L-$R^{11}$, F, Cl, Br, I, -OH, -CN, $-NH_2$, $C_{1-4}$ alkylamino, $C_{1-6}$ alkoxy or $C_{1-6}$ alkyl, wherein each $C_{1-4}$ alkylamino, $C_{1-6}$ alkoxy and $C_{1-6}$ alkyl is independently unsubstituted or substituted with 1, 2, 3 or 4 groups independently selected from F, Cl, Br, I, -OH, -CN, $-NH_2$ and $C_{1-4}$ alkyl;

L is $-C_{1-6}$ alkylene or -C(=O)-, wherein the $-C_{1-6}$ alkylene is independently unsubstituted or substituted with 1, 2, 3 or 4 groups independently selected from F, Cl, Br, I, -OH, -CN, $-NH_2$, methyl, ethyl, n-propyl and isopropyl;

$R^{11}$ is $-NR^aR^b$, $C_{1-12}$ alkyl, -C(=O)$R^{9a}$, -$OR^{9b}$, $-S(=O)_tR^{9c}$, $C_{3-7}$ cycloalkyl, heteroaryl consisting of 5-12 ring atoms or heterocyclyl consisting of 3-12 ring atoms, wherein each $C_{1-12}$ alkyl, $C_{3-7}$ cycloalkyl, heteroaryl consisting of 5-12 ring atoms and heterocyclyl consisting of 3-12 ring atoms is independently unsubstituted or substituted with 1, 2, 3, 4 or 5 $R^{w4}$;

each $R^a$ and $R^b$ is independently H, D, -C(=O)$R^{9d}$, $C_{1-12}$ alkyl, $C_{2-12}$ alkenyl, $C_{2-12}$ alkynyl or -$C_{1-6}$ alkylene-$R^{d1}$, wherein each $C_{1-12}$ alkyl, $C_{2-12}$ alkenyl, $C_{2-12}$ alkynyl and -$C_{1-6}$ alkylene of -$C_{1-6}$ alkylene-$R^{d1}$ is independently unsubstituted or substituted with 1, 2, 3, 4 or 5 $R^{w5}$;

or, $R^a$, $R^b$ and together with the N atom to which they are attached, form heterocyclyl consisting of 3-8 ring atoms, wherein the heterocyclyl consisting of 3-8 ring atoms is unsubstituted or substituted with 1, 2, 3 or 4 $R^{w4}$;

each $R^{9a}$, $R^{9b}$, $R^{9c}$ and $R^{9d}$ is independently $C_{1-12}$ alkyl, $C_{2-12}$ alkenyl, $C_{2-12}$ alkynyl or -$C_{1-6}$ alkylene-$R^{d2}$, wherein each $C_{1-12}$ alkyl, $C_{2-12}$ alkenyl, $C_{2-12}$ alkynyl and -$C_{1-6}$ alkylene of -$C_{1-6}$ alkylene-$R^{d2}$ is independently unsubstituted or substituted with 1, 2, 3, 4 or 5 $R^{w6}$;

each $R^{d1}$ and $R^{d2}$ is independently -OH, $C_{1-6}$ alkoxy, $C_{6-10}$ aryl, heteroaryl consisting of 5-12 ring atoms, $C_{3-6}$ cycloalkyl or $C_{1-6}$ alkylamino, wherein each $C_{1-6}$ alkoxy, $C_{6-10}$ aryl, heteroaryl consisting of 5-12 ring atoms, $C_{3-6}$ cycloalkyl and $C_{1-6}$ alkylamino is independently unsubstituted or substituted with 1, 2, 3, 4 or 5 $R^{w7}$;

each $R^{w1}$, $R^{w2}$, $R^{w3}$, $R^{w4}$ and $R^{w7}$ is independently D, F, Cl, Br, I, =O, -OH, -CN, -$NH_2$, $C_{1-6}$ alkylamino, $C_{1-6}$ alkoxy, $C_{1-6}$ alkyl, $C_{1-6}$ haloalkyl, -C(=O)-$C_{1-4}$ alkyl, $C_{3-6}$ cycloalkyl, heterocyclyl consisting of 3-6 ring atoms or $C_{6-10}$ aryl, wherein each $C_{1-6}$ alkylamino, $C_{1-6}$ alkoxy, $C_{1-6}$ alkyl, $C_{1-6}$ haloalkyl, -C(=O)-$C_{1-4}$ alkyl, $C_{3-6}$ cycloalkyl, heterocyclyl consisting of 3-6 ring atoms and $C_{6-10}$ aryl is independently unsubstituted or substituted with 1, 2, 3 or 4 groups independently selected from F, Cl, Br, I, -OH, -CN, -$NH_2$, $C_{1-4}$ alkyl and $C_{1-4}$ alkylamino;

each $R^{w5}$ and $R^{w6}$ is independently D, F, Cl, Br, I, =O, -OH, -CN, -$NH_2$, $C_{1-6}$ alkylamino, $C_{1-6}$ alkoxy, $C_{1-6}$ alkyl, $C_{1-6}$ haloalkyl, -C(=O)-$C_{1-4}$ alkyl, $C_{2-12}$ alkenyl, $C_{2-12}$ alkynyl, $C_{3-6}$ cycloalkyl, heterocyclyl consisting of 3-6 ring atoms, heteroaryl consisting of 5-6 ring atoms or $C_{6-10}$ aryl, wherein each -$NH_2$, $C_{1-6}$ alkylamino, $C_{1-6}$ alkoxy, $C_{1-6}$ alkyl, $C_{1-6}$ haloalkyl, -C(=O)-$C_{1-4}$ alkyl, $C_{2-12}$ alkenyl, $C_{2-12}$ alkynyl, $C_{3-6}$ cycloalkyl, heterocyclyl consisting of 3-6 ring atoms, heteroaryl consisting of 5-6 ring atoms and $C_{6-10}$ aryl is independently unsubstituted or substituted with 1, 2, 3 or 4 groups independently selected from F, Cl, Br, I, -OH, -CN, -$NH_2$, $C_{1-4}$ alkyl and $C_{1-4}$ alkylamino;

t is 0, 1 or 2.

In some embodiments, each $R^1$, $R^2$, $R^4$, $R^5$, $R^6$, $R^7$ and $R^8$ is independently H, D, F, Cl, Br, I, -OH, -CN, -$NH_2$, N-methylamino, *N*-ethylamino, *N,N*-dimethylamino, *N,N*-diethylamino, methoxy, ethoxy, 1-propoxy, 2-propoxy, 1-butoxy, 2-methyl-1-propoxy, 2-butoxy, methyl, ethyl, *n*-propyl, isopropyl, *n*-butyl, *i*-butyl, *sec*-butyl, *tert*-butyl, *n*-pentyl or *n*-hexyl, wherein each *N*-methylamino, *N*-ethylamino, *N,N*-dimethylamino, *N,N*-diethylamino, methoxy, ethoxy, 1-propoxy, 2-propoxy, 1-butoxy, 2-methyl-1-propoxy, 2-butoxy, methyl, ethyl, n-propyl, isopropyl, *n*-butyl, *i*-butyl, *sec*-butyl, *tert*-butyl, *n*-pentyl and *n*-hexyl is independently unsubstituted or substituted with 1, 2, 3 or 4 groups independently selected from F, Cl, Br, I, -OH, -CN, -$NH_2$, methyl, ethyl, *n*-propyl, isopropyl, *n*-butyl, *i*-butyl and *sec*-butyl.

In some embodiments, R is H, D, methyl, ethyl, *n*-propyl, isopropyl, *n*-butyl, *i*-butyl, *sec*-butyl, *tert*-butyl, *n*-pentyl, *n*-hexyl, aziridinyl, azetidinyl, oxetanyl, thietanyl, pyrrolidyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl or piperazinyl, wherein each methyl, ethyl, *n*-propyl, isopropyl, *n*-butyl, *i*-butyl, *sec*-butyl, *tert*-butyl, *n*-pentyl, *n*-hexyl, aziridinyl, azetidinyl, oxetanyl, thietanyl, pyrrolidyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl and piperazinyl is independently unsubstituted or substituted with 1, 2, 3, 4 or 5 $R^{w3}$;

wherein $R^{w3}$ is as defined herein.

In some embodiments, $R^3$ is -$CH_2$-$R^{10}$, -$(CH_2)_2$-$R^{10}$, -$(CH_2)_3$-$R^{10}$, -$CH(CH_3)CH_2$-$R^{10}$, -$CH_2CH(CH_3)$-$R^{10}$ or -$(CH_2)_4$-$R^{10}$, wherein each the -$CH_2$- of $CH_2$-$R^{10}$, -$(CH_2)_2$- of -$(CH_2)_2$-$R^{10}$, -$(CH_2)_3$- of -$(CH_2)_3$-$R^{10}$, -$CH(CH_3)CH_2$- of -$CH(CH_3)CH_2$-$R^{10}$, -$CH_2CH(CH_3)$- of -$CH_2CH(CH_3)$-$R^{10}$ and -$(CH_2)_4$- of -$(CH_2)_4$-$R^{10}$ is independently unsubstituted or substituted with 1, 2, 3, 4 or 5 $R^{w1}$;

wherein each $R^{10}$ and $R^{w1}$ is as defined herein.

In some embodiments, each $R^{10}$ is independently methoxy, ethoxy, 1-propoxy, 2-propoxy, 1-butoxy, 2-methyl-1-propoxy, 2-butoxy, -OH, -$NH_2$, -O-C(=O)-$R^{10a}$ or -$NR^c$-C(=O)-$R^{10b}$, wherein each methoxy, ethoxy, 1-propoxy, 2-propoxy, 1-butoxy, 2-methyl-1-propoxy and 2-butoxy is independently unsubstituted or substituted with 1, 2, 3 or 4 groups independently selected from F, Cl, Br, I, -OH, -CN, -$NH_2$, methyl, ethyl, *n*-propyl, isopropyl, *n*-butyl, *i*-butyl and *sec*-butyl; wherein each $R^c$, $R^{10a}$ and $R^{10b}$ is as defined herein.

In some embodiments, each $R^{10a}$ and $R^{10b}$ is independently $C_{1-4}$ alkyl, $C_{3-6}$ cycloalkyl, heteroaryl consisting of 5-6 ring atoms or heterocyclyl consisting of 3-6 ring atoms, wherein each $C_{1-4}$ alkyl, $C_{3-6}$ cycloalkyl, heteroaryl consisting of 5-6 ring atoms and heterocyclyl consisting of 3-6 ring atoms is independently unsubstituted or substituted with 1, 2, 3, 4 or 5 $R^{w2}$;

wherein $R^{w2}$ is as defined herein.

In some embodiments, $R^c$ is H, D, methyl, ethyl, *n*-propyl or isopropyl, wherein each methyl, ethyl, n-propyl and isopropyl is independently unsubstituted or substituted with 1, 2, 3 or 4 groups independently selected from F, Cl, Br, I, -OH, -CN, -$NH_2$, methyl, ethyl, n-propyl and isopropyl.

In some embodiments, each $R^{10a}$ and $R^{10b}$ is independently methyl, ethyl, n-propyl, isopropyl, n-butyl, i-butyl,

sec-butyl, tert-butyl, cyclopropyl, cyclobutyl, cyclopentyl, cyclohexyl, furyl, pyrrolyl, pyridinyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidyl, aziridinyl, azetidinyl, oxetanyl, thietanyl, pyrrolidyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl or piperazinyl, wherein each methyl, ethyl, n-propyl, isopropyl, n-butyl, i-butyl, *sec*-butyl, *tert*-butyl, cyclopropyl, cyclobutyl, cyclopentyl, cyclohexyl, furyl, pyrrolyl, pyridinyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidyl, aziridinyl, azetidinyl, oxetanyl, thietanyl, pyrrolidyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl and piperazinyl is independently unsubstituted or substituted with 1, 2, 3, 4 or 5 $R^{w2}$;
wherein $R^{w2}$ is as defined herein.

In some embodiments, $R^9$ is H, D, -L-$R^{11}$, F, Cl, Br, I, -OH, -CN, -$NH_2$, $C_{1-4}$ alkylamino, $C_{1-4}$ alkoxy or $C_{1-4}$ alkyl, wherein each $C_{1-4}$ alkylamino, $C_{1-4}$ alkoxy and $C_{1-4}$ alkyl is independently unsubstituted or substituted with 1, 2, 3 or 4 groups independently selected from F, Cl, Br, I, -OH, -CN, -$NH_2$ and $C_{1-4}$ alkyl;
Wherein each L and $R^{11}$ is as defined herein.

In some embodiments, L is -$CH_2$-, -$(CH_2)_2$-, -$(CH_2)_3$-, -$CH_2CH(CH_3)$-, -$CH(CH_3)CH_2$-, -$(CH_2)_4$- or -C(=O)-, wherein -$CH_2$-, -$(CH_2)_2$-, -$(CH_2)_3$-, -$CH_2CH(CH_3)$-, -$CH(CH_3)CH_2$- and -$(CH_2)_4$- is independently unsubstituted or substituted with 1, 2, 3 or 4 groups independently selected from F, Cl, Br, I, -OH, -CN, -$NH_2$, methyl, ethyl, n-propyl or isopropyl.

In some embodiments, $R^{11}$ is -$NR^aR^b$, $C_{1-10}$ alkyl, -C(=O)$R^{9a}$, -O$R^{9b}$, -S(=O)$_t$$R^{9c}$, $C_{3-6}$ cycloalkyl, heteroaryl consisting of 5-6 ring atoms or heterocyclyl consisting of 3-6 ring atoms, wherein each $C_{1-10}$ alkyl, $C_{3-6}$ cycloalkyl, heteroaryl consisting of 5-6 ring atoms and heterocyclyl consisting of 3-6 ring atoms is independently unsubstituted or substituted with 1, 2, 3, 4 or 5 $R^{w4}$;
wherein, each $R^a$, $R^b$, $R^{9a}$, t, $R^{9c}$ and $R^{w4}$ is as defined herein.

In some embodiments, $R^9$ is independently H, D, -L-$R^{11}$, F, Cl, Br, I, -OH, -CN, -$NH_2$, *N*-methylamino, *N*-ethylamino, *N,N*-dimethylamino, *N,N*-diethylamino, methoxy, ethoxy, 1-propoxy, 2-propoxy, 1-butoxy, 2-methyl-1-propoxy, 2-butoxy, methyl, ethyl, *n*-propyl, isopropyl, *n*-butyl, *i*-butyl, *sec*-butyl, *tert*-butyl, *n*-pentyl or *n*-hexyl, wherein each *N*-methylamino, *N*-ethylamino, *N,N*-dimethylamino, *N,N*-diethylamino, methoxy, ethoxy, 1-propoxy, 2-propoxy, 1-butoxy, 2-methyl-1-propoxy, 2-butoxy, methyl, ethyl, *n*-propyl, isopropyl, *n*-butyl, *i*-butyl, *sec*-butyl, *tert*-butyl, *n*-pentyl and *n*-hexyl is independently unsubstituted or substituted with 1, 2, 3 or 4 groups independently selected from F, Cl, Br, I, -OH, -CN, -$NH_2$, methyl, ethyl, *n*-propyl, isopropyl, *n*-butyl, *i*-butyl and *sec*-butyl;
Wherein each L and $R^{11}$ is as defined herein.

In some embodiments, $R^{11}$ is -$NR^aR^b$, methyl, ethyl, *n*-propyl, isopropyl, *n*-butyl, *i*-butyl, *sec*-butyl, *tert*-butyl, *n*-pentyl, *n*-hexyl, -C(=O)$R^{9a}$, -O$R^{9b}$, -S(=O)$_t$$R^{9c}$, cyclopropyl, cyclobutyl, cyclopentyl, cyclohexyl, furyl, pyrrolyl, pyridinyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidyl, aziridinyl, azetidinyl, oxetanyl, thietanyl, pyrrolidyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl or piperazinyl, wherein each methyl, ethyl, n-propyl, isopropyl, *n*-butyl, *i*-butyl, *sec*-butyl, *tert*-butyl, *n*-pentyl, *n*-hexyl, cyclopropyl, cyclobutyl, cyclopentyl, cyclohexyl, furyl, pyrrolyl, pyridinyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidyl, aziridinyl, azetidinyl, oxetanyl, thietanyl, pyrrolidyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl and piperazinyl is independently unsubstituted or substituted with 1, 2, 3, 4 or 5 $R^{w4}$;
wherein, each $R^a$, $R^b$, $R^{9a}$, $R^b$, t, $R^{9c}$ and $R^{w4}$ is as defined herein.

In some embodiments, each $R^a$ and $R^b$ is independently H, D, -C(=O)$R^{9d}$, $C_{1-6}$ alkyl, $C_{2-6}$ alkenyl, $C_{2-6}$ alkynyl or -$C_{1-4}$ alkylene-$R^{d1}$, wherein each $C_{1-6}$ alkyl, $C_{2-6}$ alkenyl, $C_{2-6}$ alkynyl and -$C_{1-4}$ alkylene of -$C_{1-4}$ alkylene-$R^{d1}$ is independently unsubstituted or substituted with 1, 2, 3, 4 or 5 $R^{w5}$;

or, $R^a$, $R^b$ and together with the N atom to which they are attached, form heterocyclyl consisting of 3-6 ring atoms, wherein the heterocyclyl consisting of 3-6 ring atoms is unsubstituted or substituted with 1, 2, 3 or 4 $R^{w4}$;
wherein, each $R^{9d}$, $R^{d1}$, $R^{w5}$ and $R^{w4}$ is as defined herein.

In some embodiments, each $R^a$ and $R^b$ is independently H, D, -C(=O)$R^{9d}$, methyl, ethyl, *n*-propyl, isopropyl, *n*-butyl, *i*-butyl, *sec*-butyl, *tert*-butyl, *n*-pentyl, *n*-hexyl, -CH=$CH_2$, -CH=$CHCH_3$, -$CH_2CH$=$CH_2$, -$CH_2CH_2CH$=$CH_2$, -C≡CH, -$CH_2$C≡CH, -C≡C-$CH_3$, -$CH_2CH_2$C≡CH, -$CH_2$C≡$CCH_3$, -C≡$CCH_2CH_3$, -$CH_2$-$R^{d1}$, -$(CH_2)_2$-$R^{d1}$, -$(CH_2)_3$-$R^{d1}$, -$CH_2CH(CH_3)$-$R^{d1}$, -$CH(CH_3)CH_2$-$R^{d1}$ or -$(CH_2)_4$-$R^{d1}$, wherein, each methyl, ethyl, *n*-propyl, isopropyl, *n*-butyl, *i*-butyl, *sec*-butyl, *tert*-butyl, *n*-pentyl, *n*-hexyl, -CH=$CH_2$, -CH=$CHCH_3$, -$CH_2CH$=$CH_2$, -$CH_2CH_2CH$=$CH_2$, -C≡CH, -$CH_2$C≡CH, -C≡C-$CH_3$, -$CH_2CH_2$C≡CH, -$CH_2$C≡$CCH_3$, -C≡$CCH_2CH_3$, -$CH_2$- of -$CH_2$-$R^{d1}$, -$(CH_2)_2$- of -$(CH_2)_2$-$R^{d1}$, -$(CH_2)_3$- of -$(CH_2)_3$-$R^{d1}$, -$CH_2CH(CH_3)$- of -$CH_2CH(CH_3)$-$R^{d1}$, -$CH(CH_3)CH_2$- of -$CH(CH_3)CH_2$-$R^{d1}$ and -$(CH_2)_4$- of -$(CH_2)_4$-$R^{d1}$

is independently unsubstituted or substituted with 1, 2, 3, 4 or 5 $R^{w5}$;

or, $R^a$, $R^b$ and together with the N atom to which they are attached, form aziridinyl, azetidinyl, pyrrolidyl, pyrazolidinyl, imidazolidinyl, piperidinyl, morpholinyl, thiomorpholinyl or piperazinyl, wherein each aziridinyl, azetidinyl, pyrrolidyl, pyrazolidinyl, imidazolidinyl, piperidinyl, morpholinyl, thiomorpholinyl and piperazinyl is independently unsubstituted or substituted with 1, 2, 3 or 4 $R^{w4}$;
wherein, each $R^{9d}$, $R^{d1}$, $R^{w5}$ and $R^{w4}$ is as defined herein.

In some embodiments, each $R^{9a}$, $R^{9b}$, $R^{9c}$ and $R^{9d}$ is independently $C_{1-6}$ alkyl, $C_{2-6}$ alkenyl, $C_{2-6}$ alkynyl or -$C_{1-4}$ alkylene-$R^{d2}$, wherein each $C_{1-6}$ alkyl, $C_{2-6}$ alkenyl, $C_{2-6}$ alkynyl and -$C_{1-4}$ alkylene of -$C_{1-4}$ alkylene-$R^{d2}$ is independently unsubstituted or substituted with 1, 2, 3, 4 or 5 $R^{w6}$;
wherein each $R^{d2}$ and $R^{w6}$ is as defined herein.

In some embodiments, each $R^{d1}$ and $R^{d2}$ is independently -OH, $C_{1-4}$ alkoxy, phenyl, heteroaryl consisting of 5-6 ring atoms, cyclopropyl, cyclobutyl, cyclopentyl, cyclohexyl or $C_{1-4}$ alkylamino, wherein each $C_{1-4}$ alkoxy, phenyl, heteroaryl consisting of 5-6 ring atoms, cyclopropyl, cyclobutyl, cyclopentyl, cyclohexyl and $C_{1-4}$ alkylamino is independently unsubstituted or substituted with 1, 2, 3, 4 or 5 $R^{w7}$;
wherein $R^{w7}$ is as defined herein.

In some embodiments, each $R^{9a}$, $R^{9b}$, $R^{9c}$ and $R^{9d}$ is independently methyl, ethyl, *n*-propyl, isopropyl, *n*-butyl, *i*-butyl, *sec*-butyl, *tert*-butyl, *n*-pentyl, *n*-hexyl, $-CH=CH_2$, $-CH=CHCH_3$, $-CH_2CH=CH_2$, $-CH_2CH_2CH=CH_2$, $-C\equiv CH$, $-CH_2C\equiv CH$, $-C\equiv C-CH_3$, $-CH_2CH_2C\equiv CH$, $-CH_2C\equiv CCH_3$, $-C\equiv CCH_2CH_3$, $-CH_2-R^{d2}$, $-(CH_2)_2-R^{d2}$, $-(CH_2)_3-R^{d2}$, $-CH_2CH(CH_3)-R^{d2}$, $-CH(CH_3)CH_2-R^{d2}$ or $-(CH_2)_4-R^{d2}$, wherein, each methyl, ethyl, *n*-propyl, isopropyl, *n*-butyl, *i*-butyl, *sec*-butyl, *tert*-butyl, *n*-pentyl, *n*-hexyl, $-CH=CH_2$, $-CH=CHCH_3$, $-CH_2CH=CH_2$, $-CH_2CH_2CH=CH_2$, $-C\equiv CH$, $-CH_2C\equiv CH$, $-C\equiv C-CH_3$, $-CH_2CH_2C\equiv CH$, $-CH_2C\equiv CCH_3$, $-C\equiv CCH_2CH_3$, $-CH_2-$ of $-CH_2-R^{d2}$, $-(CH_2)_2-$ of $-(CH_2)_2-R^{d2}$, $-(CH_2)_3-$ of $-(CH_2)_3-R^{d2}$, $-CH_2CH(CH_3)-$ of $-CH_2CH(CH_3)-R^{d2}$, $-CH(CH_3)CH_2-$ of $-CH(CH_3)CH_2-R^{d2}$ and $-(CH_2)_4-$ of $-(CH_2)_4-R^{d2}$ is independently unsubstituted or substituted with 1, 2, 3, 4 or 5 $R^{w6}$;
wherein each $R^{d2}$ and $R^{w6}$ is as defined herein.

In some embodiments, each $R^{d1}$ and $R^{d2}$ is independently -OH, methoxy, ethoxy, 1-propoxy, 2-propoxy, 1-butoxy, 2-methyl-l-propoxy, 2-butoxy, phenyl, furyl, pyrrolyl, pyridinyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidyl, cyclopropyl, cyclobutyl, cyclopentyl, cyclohexyl, *N*-methylamino, *N*-ethylamino, *N*,*N*-dimethylamino or *N*,*N*-diethylamino, wherein each methoxy, ethoxy, 1-propoxy, 2-propoxy, 1-butoxy, 2-methyl-l-propoxy, 2-butoxy, phenyl, furyl, pyrrolyl, pyridinyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidyl, cyclopropyl, cyclobutyl, cyclopentyl, cyclohexyl, *N*-methylamino, *N*-ethylamino, *N,N*-dimethylamino and *N*,*N*-diethylamino is independently unsubstituted or substituted with 1, 2, 3, 4 or 5 $R^{w7}$;
wherein $R^{w7}$ is as defined herein.

In some embodiments, each $R^{w1}$, $R^{w2}$, $R^{w3}$, $R^{w4}$ and $R^{w7}$ is independently D, F, Cl, Br, I, =O, -OH, -CN, $-NH_2$, *N*-methylamino, *N*-ethylamino, *N,N*-dimethylamino, *N,N*-diethylamino, methoxy, ethoxy, 1-propoxy, 2-propoxy, 1-butoxy, 2-methyl-l-propoxy, 2-butoxy, methyl, ethyl, *n*-propyl, isopropyl, *n*-butyl, *i*-butyl, *sec*-butyl, *tert*-butyl, $-CH_2F$, $-CH_2Cl$, $-CF_3$, $-CHF_2$, $-CHCl_2$, $-CH_2CH_2F$, $-CH_2CH_2Cl$, $-CH_2CHF_2$, $-CH_2CHCl_2$, $-CHFCH_2F$, $-CHClCH_2Cl$, $-CH_2CF_3$, $-CH(CF_3)_2$, $-CF_2CH_2CH_3$, $-CH_2CH_2CH_2F$, $-CH_2CH_2CHF_2$, $-CH_2CH_2CF_3$, $-C(=O)-CH_3$, $-C(=O)-CH_2CH_3$, cyclopropyl, cyclobutyl, cyclopentyl, cyclohexyl, aziridinyl, azetidinyl, oxetanyl, thietanyl, pyrrolidyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl, piperazinyl or phenyl, wherein each *N*-methylamino, *N*-ethylamino, *N*,*N*-dimethylamino, *N*,*N*-diethylamino, methoxy, ethoxy, 1-propoxy, 2-propoxy, 1-butoxy, 2-methyl-l-propoxy, 2-butoxy, methyl, ethyl, *n*-propyl, isopropyl, *n*-butyl, *i*-butyl, *sec*-butyl, *tert*-butyl, $-CH_2F$, $-CH_2Cl$, $-CHF_2$, $-CHCl_2$, $-CH_2CH_2F$, $-CH_2CH_2Cl$, $-CH_2CHF_2$, $-CH_2CHCl_2$, $-CHFCH_2F$, $-CHClCH_2Cl$, $-CH_2CF_3$, $-CH(CF_3)_2$, $-CF_2CH_2CH_3$, $-CH_2CH_2CH_2F$, $-CH_2CH_2CHF_2$, $-CH_2CH_2CF_3$, $-C(=O)-CH_3$, $-C(=O)-CH_2CH_3$, cyclopropyl, cyclobutyl, cyclopentyl, cyclohexyl, aziridinyl, azetidinyl, oxetanyl, thietanyl, pyrrolidyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl, piperazinyl and phenyl is independently unsubstituted or substituted with 1, 2, 3 or 4 groups independently selected from F, Cl, Br, I, -OH, -CN, $-NH_2$, methyl, ethyl, *n*-propyl, isopropyl, n-butyl, i-butyl, $-N(CH_3)_2$, $-NHCH_3$, $-N(CH_2CH_3)_2$, $-N(CH_3)CH_2CH_3$ and $-NHCH_2CH_3$.

In some embodiments, each $R^{w5}$ and $R^{w6}$ is independently D, F, Cl, Br, I, =O, -OH, -CN, $-NH_2$, $C_{1-4}$ alkylamino, $C_{1-4}$ alkoxy, $C_{1-4}$ alkyl, $C_{1-4}$ haloalkyl, -C(=O)-$C_{1-4}$ alkyl, $C_{2-6}$ alkenyl, $C_{2-6}$ alkynyl, cyclopropyl, cyclobutyl, cyclopentyl, cyclohexyl, heterocyclyl consisting of 3-6 ring atoms, heteroaryl consisting of 5-6 ring atoms or phenyl, wherein each $-NH_2$, $C_{1-4}$ alkylamino, $C_{1-4}$ alkoxy, $C_{1-4}$ alkyl, $C_{1-4}$ haloalkyl, -C(=O)-$C_{1-4}$ alkyl, $C_{2-6}$ alkenyl, $C_{2-6}$ alkynyl, cyclopropyl, cyclobutyl, cyclopentyl, cyclohexyl, heterocyclyl consisting of 3-6 ring atoms, heteroaryl consisting of 5-6 ring atoms and phenyl is independently unsubstituted or substituted with 1, 2, 3 or 4 groups selected from F, Cl, Br, I, -OH, -CN, $-NH_2$, $C_{1-4}$ alkyl and $C_{1-4}$ alkylamino.

In some embodiments, each $R^{w5}$ and $R^{w6}$ is independently D, F, Cl, Br, I, =O, -OH, -CN, $-NH_2$, *N*-methylamino, *N*-ethylamino, *N,N*-dimethylamino, *N,N*-diethylamino, methoxy, ethoxy, 1-propoxy, 2-propoxy, 1-butoxy, 2-methyl-l-propoxy, 2-butoxy, methyl, ethyl, *n*-propyl, isopropyl, *n*-butyl, *i*-butyl, *sec*-butyl, *tert*-butyl, $-CH_2F$, $-CH_2Cl$, $-CF_3$, $-CHF_2$, $-CHCl_2$, $-CH_2CH_2F$, $-CH_2CH_2Cl$, $-CH_2CHF_2$, $-CH_2CHCl_2$, $-CHFCH_2F$, $-CHClCH_2Cl$, $-CH_2CF_3$, $-CH(CF_3)_2$, $-CF_2CH_2CH_3$, $-CH_2CH_2CH_2F$, $-CH_2CH_2CHF_2$, $-CH_2CH_2CF_3$, $-C(=O)-CH_3$, $-C(=O)-CH_2CH_3$, $-C(=O)-$ $CH_2CH_2CH_3$, $-CH=CH_2$, $-CH=CHCH_3$, $-CH_2CH=CH_2$, $-CH_2CH_2CH=CH_2$, $-C\equiv CH$, $-CH_2C\equiv CH$, $-C\equiv C-CH_3$, $-CH_2CH_2C\equiv CH$, $-CH_2C\equiv CCH_3$, $-C\equiv CCH_2CH_3$, cyclopropyl, cyclobutyl, cyclopentyl, cyclohexyl, aziridinyl, azetidinyl, oxetanyl, thietanyl, pyrrolidyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl, piperazinyl, furyl, pyrrolyl, pyridinyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidyl or phenyl, wherein each $-NH_2$, *N*-methylamino, *N*-ethylamino, *N,N*-dimethylamino, *N,N*-diethylamino, methoxy, ethoxy, 1-propoxy, 2-propoxy, 1-butoxy, 2-methyl-l-propoxy, 2-butoxy, methyl, ethyl, *n*-propyl, isopropyl, *n*-butyl, *i*-butyl, *sec*-butyl, *tert*-butyl, $-CH_2F$, $-CH_2Cl$, $-CHF_2$, $-CHCl_2$, $-CH_2CH_2F$, $-CH_2CH_2Cl$, $-CH_2CHF_2$, $-CH_2CHCl_2$, $-CHFCH_2F$, $-CHClCH_2Cl$, $-CH_2CF_3$, $-CH(CF_3)_2$, $-CF_2CH_2CH_3$, $-CH_2CH_2CH_2F$, $-CH_2CH_2CHF_2$, $-CH_2CH_2CF_3$, $-C(=O)-CH_3$, $-C(=O)-CH_2CH_3$, $-C(=O)-$ $CH_2CH_2CH_3$, $-CH=CH_2$, $-CH=CHCH_3$, $-CH_2CH=CH_2$, $-CH_2CH_2CH=CH_2$, $-C\equiv CH$, $-CH_2C\equiv CH$, $-C\equiv C-CH_3$, $-CH_2CH_2C\equiv CH$, $-CH_2C\equiv CCH_3$, $-C\equiv CCH_2CH_3$, cyclopropyl, cyclobutyl, cyclopentyl, cyclohexyl, aziridinyl, azetidinyl, oxetanyl, thietanyl, pyrrolidyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl, piperazinyl, furyl, pyrrolyl, pyridinyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidyl and phenyl is independently unsubstituted or substituted with 1, 2, 3 or 4 groups independently selected from F, Cl, Br, I, -OH, -CN, $-NH_2$, methyl, ethyl, *n*-propyl, isopropyl, *n*-butyl, *i*-butyl, $-N(CH_3)_2$, $-NHCH_3$, $-N(CH_2CH_3)_2$, $-N(CH_3)CH_2CH_3$ and $-NHCH_2CH_3$.

In some embodiments, the present invention comprises a compound having one of the following structures or a stereoisomer, a tautomer, an *N*-oxide, a solvate, a metabolite, a pharmaceutically acceptable salt or a prodrug thereof:

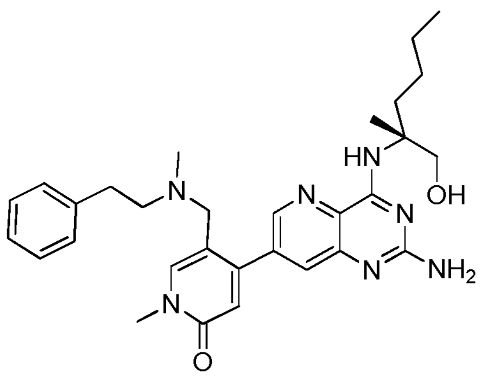

(1),

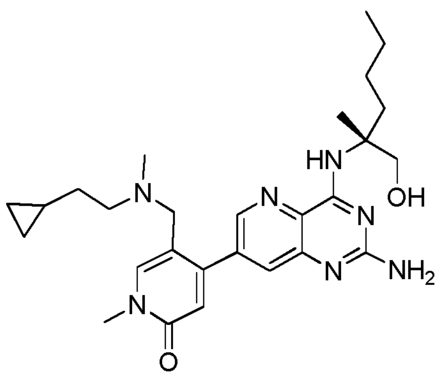

(2),

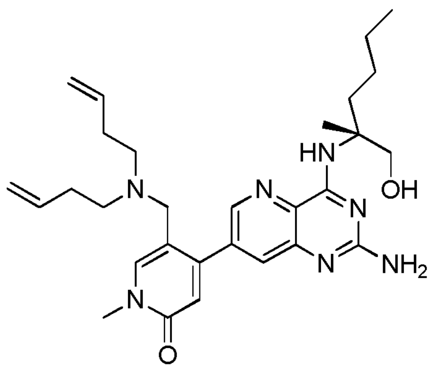

(3),

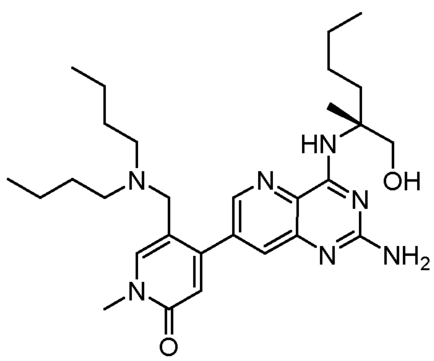

(4),

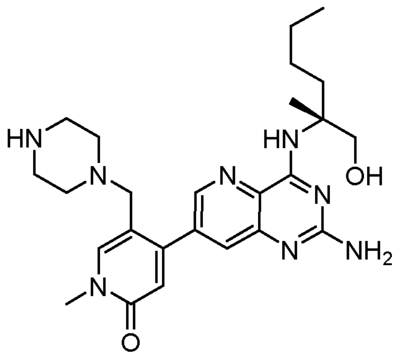

(5),

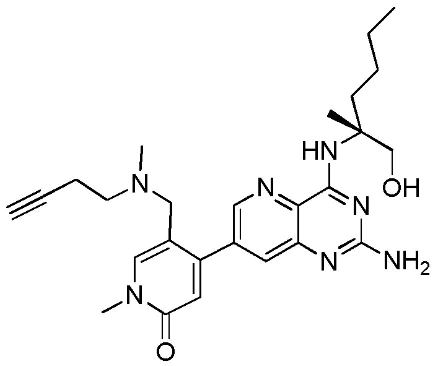

(6),

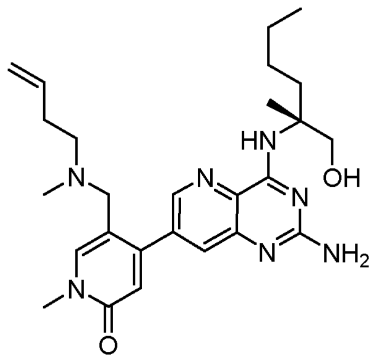

(7),

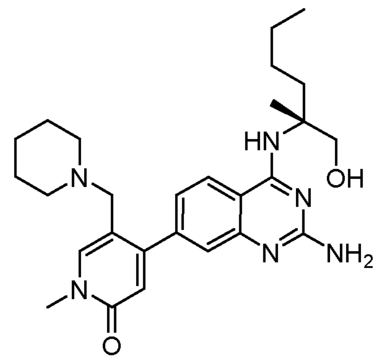

(8),

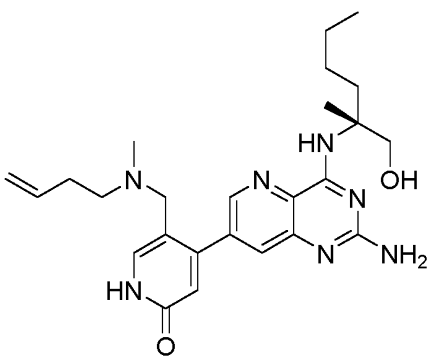

(9),

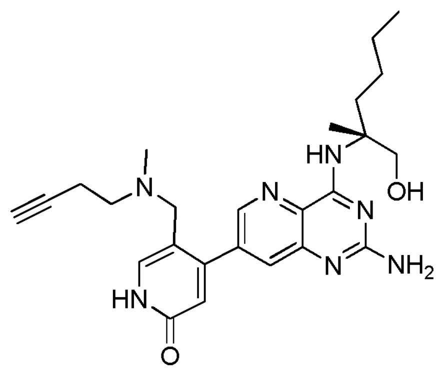

(10),

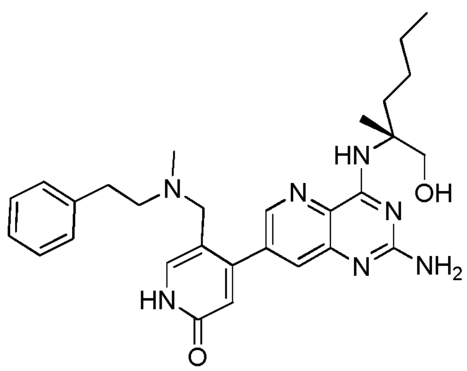

(11),

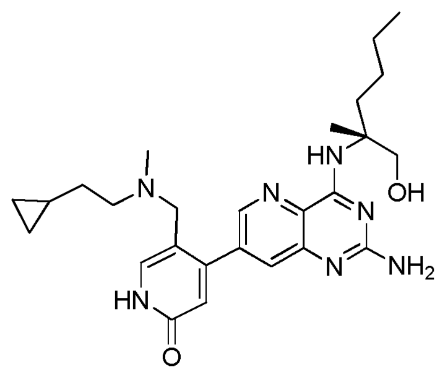

(12),

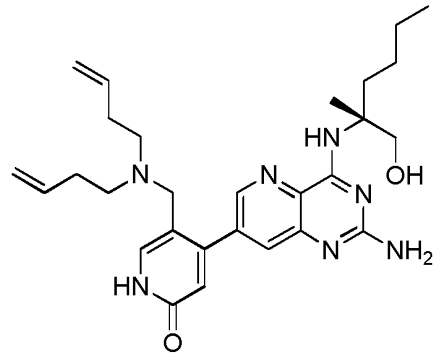
(13),

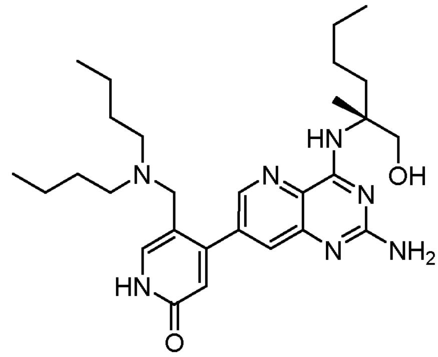
(14),

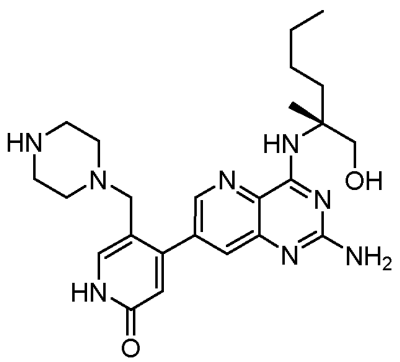
(15),

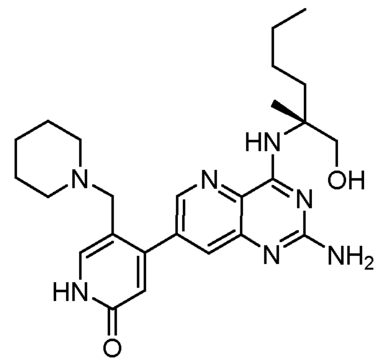
(16),

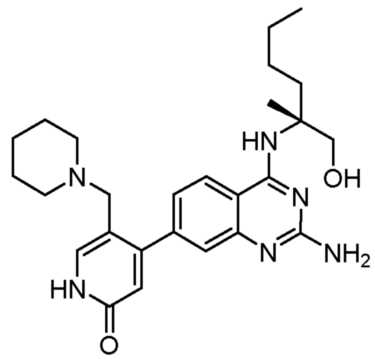
(17),

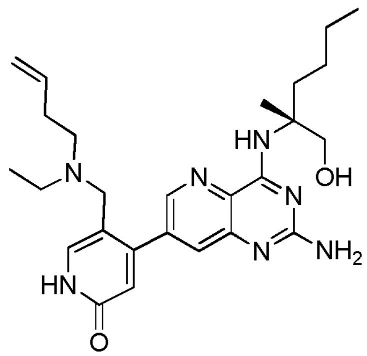
(18),

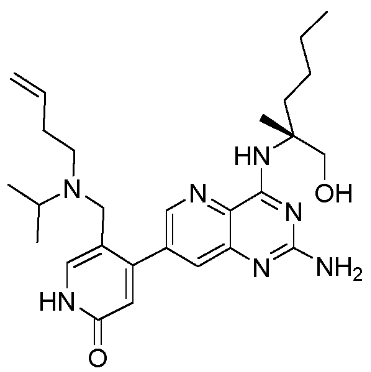
(19),

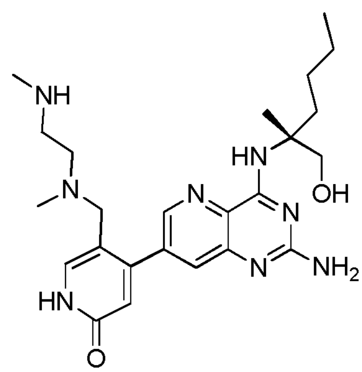
(20),

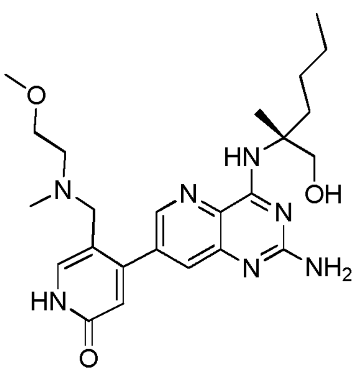

(21),

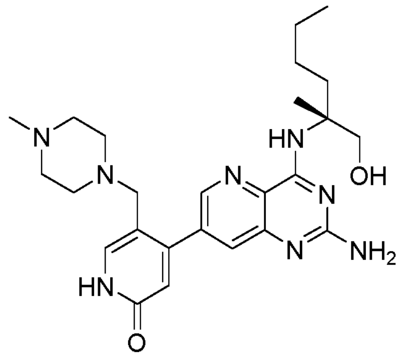

(22),

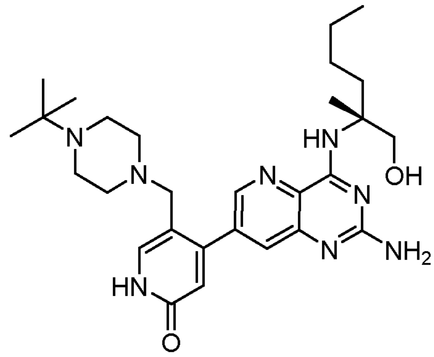

(23),

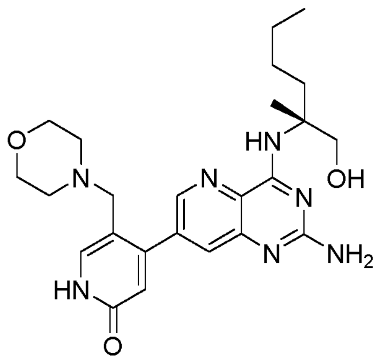

(24),

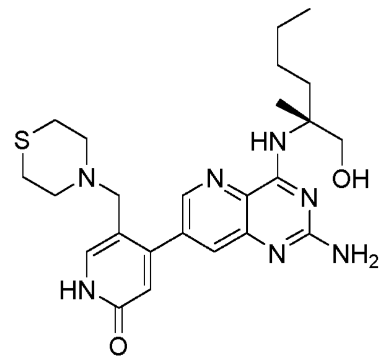

(25),

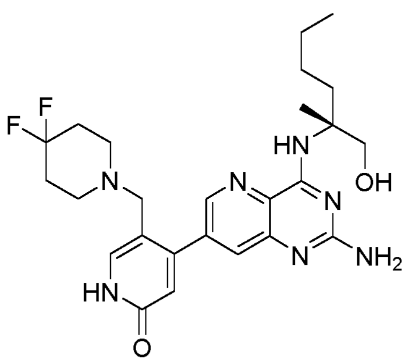

(26),

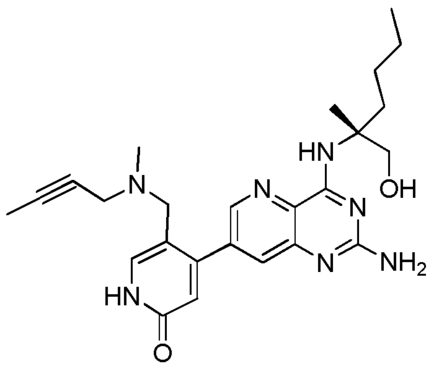

(27),

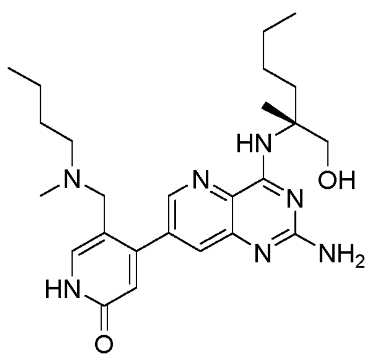

(28),

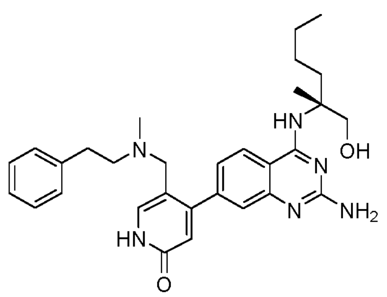

(29),

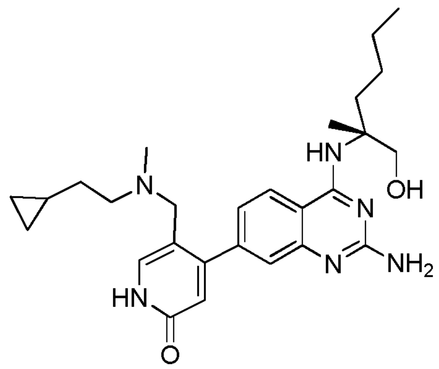

(30),

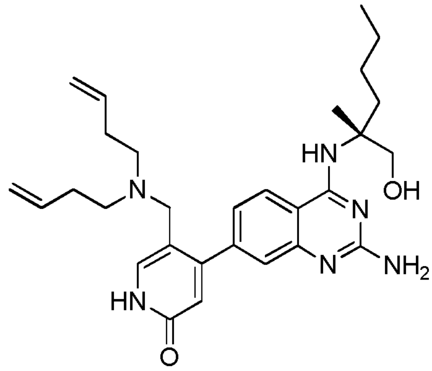

(31),

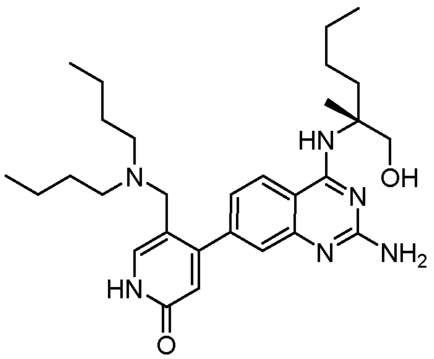

(32),

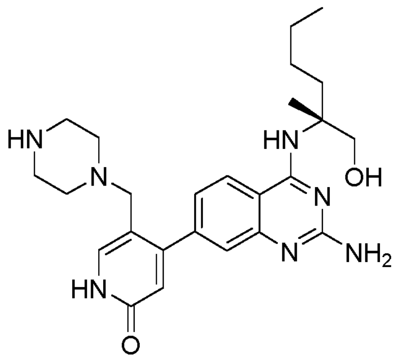

(33),

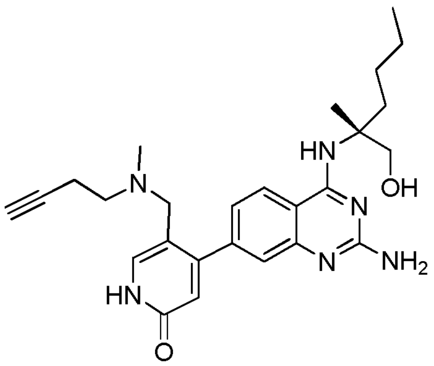

(34),

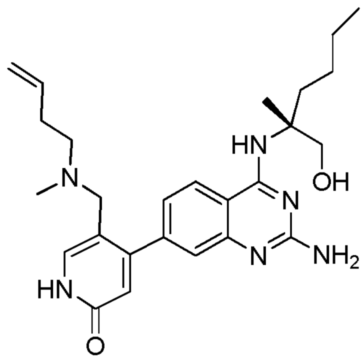

(35),

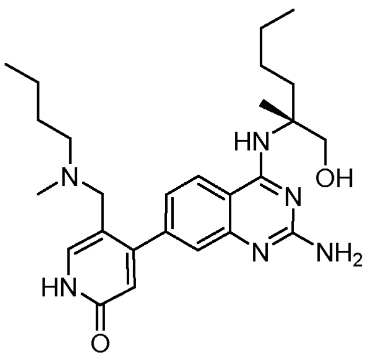

(36),

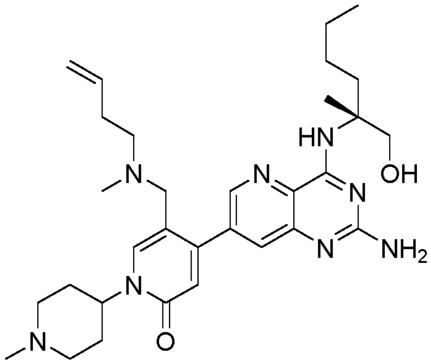

(37),

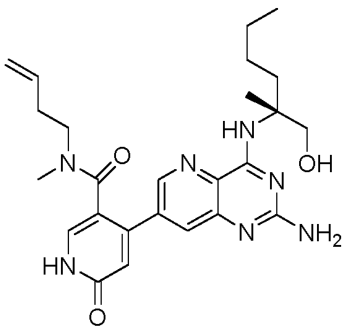

(38),

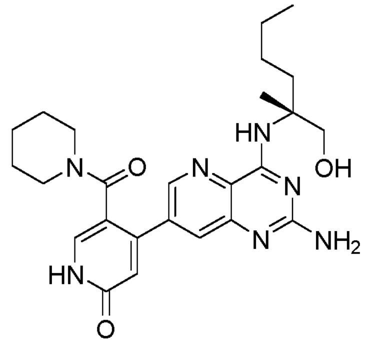

(39),

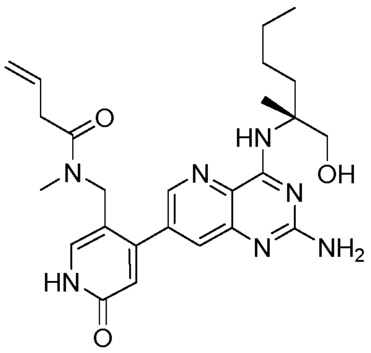

(40),

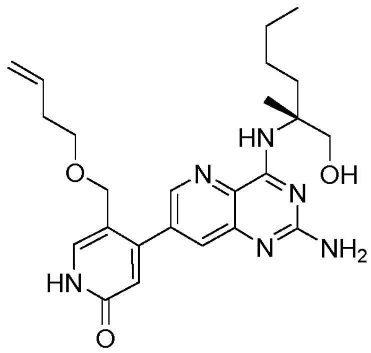

(41),

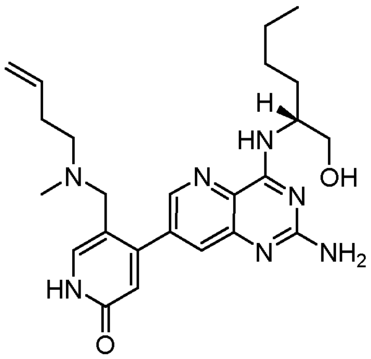

(42),

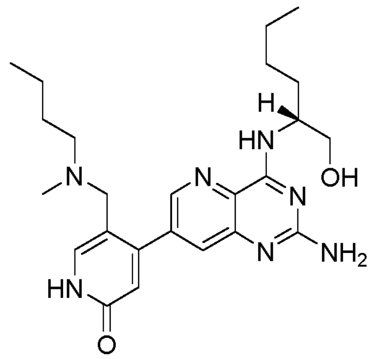

(43),

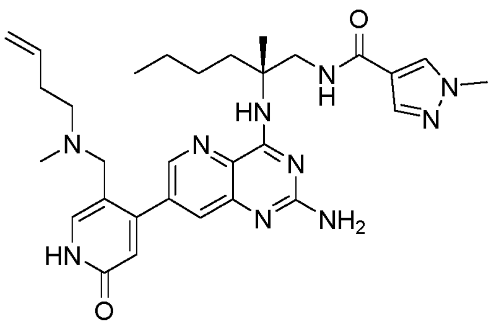

(44),

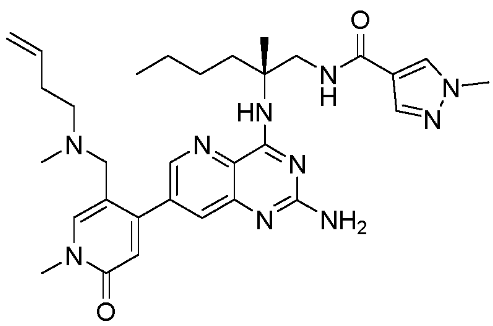

(45),

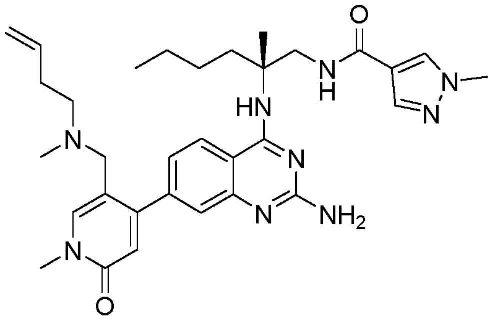

(46),

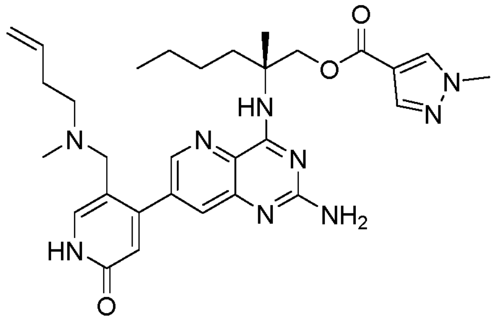

(47),

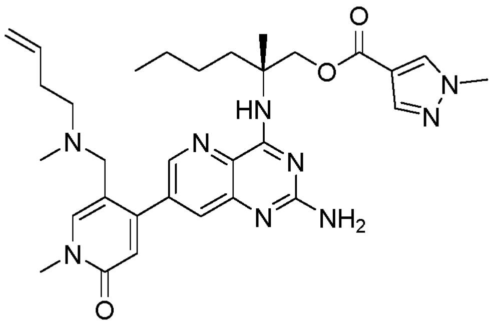

(48),

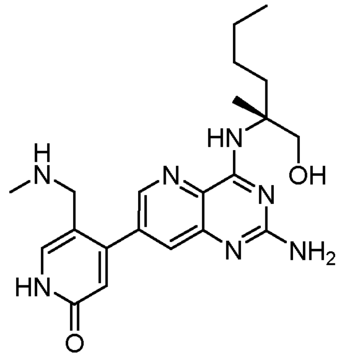

(49),

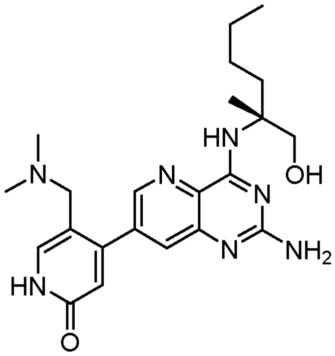

(50),

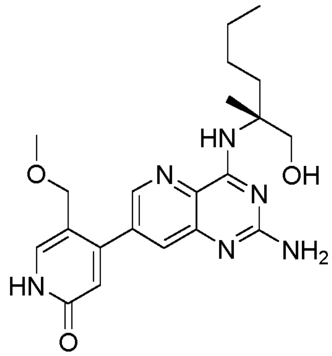

(51),

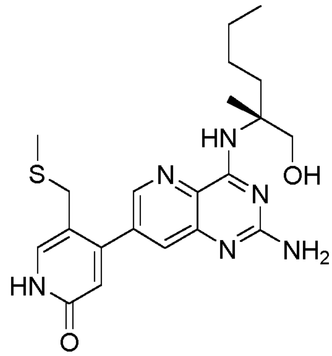

(52),

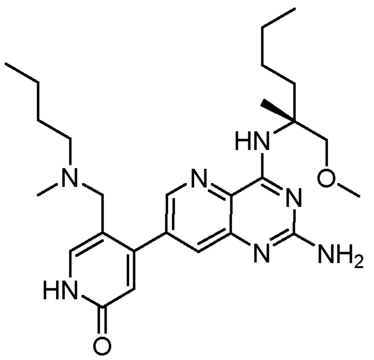

(53),

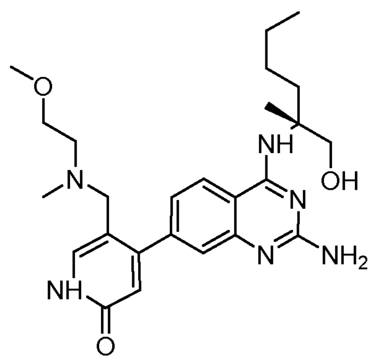

(54),

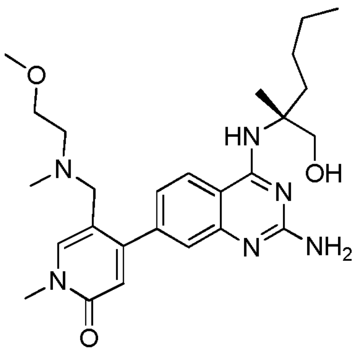
(55),

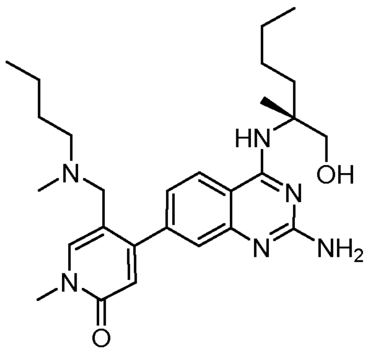
(56),

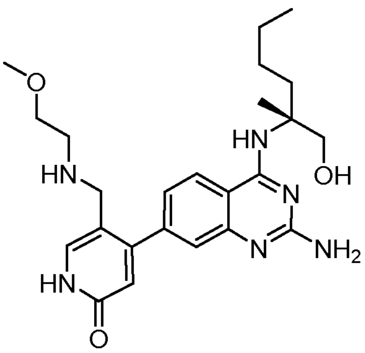
(57),

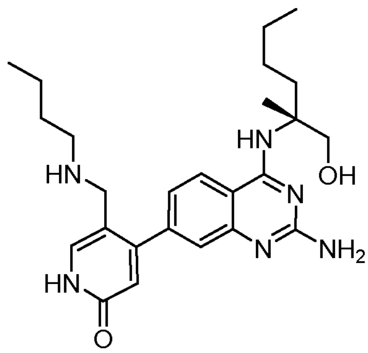
(58),

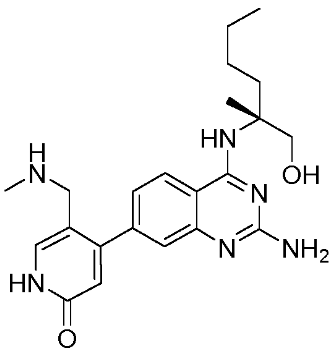
(59),

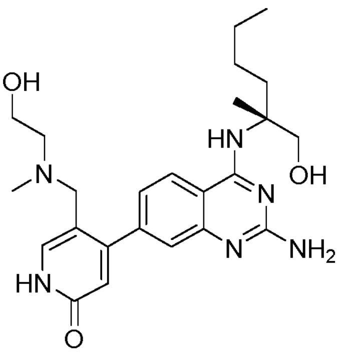
(60),

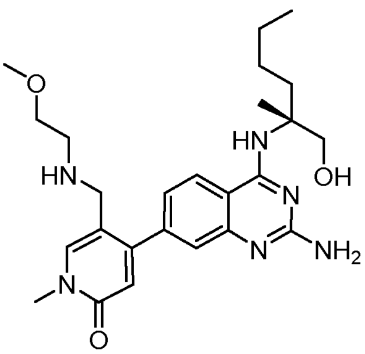
(61),

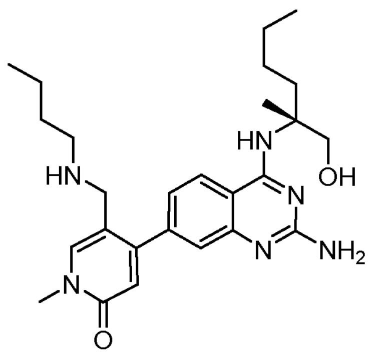
(62),

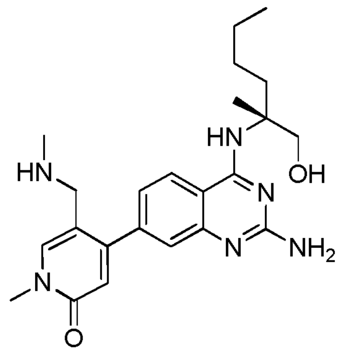

(63),

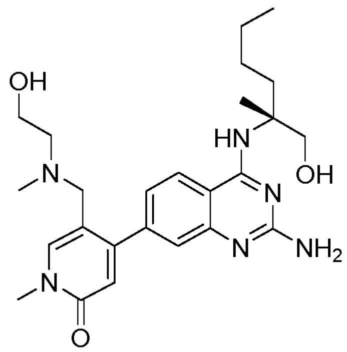

(64),

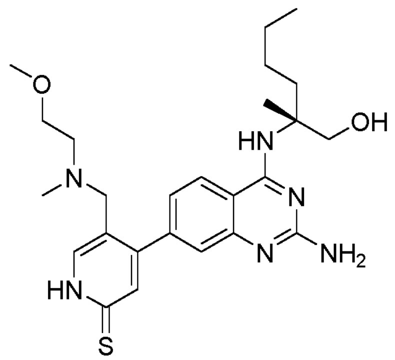

(65),

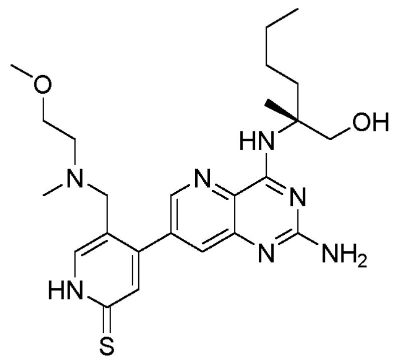

(66),

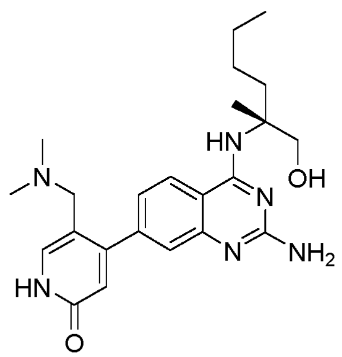

(67),

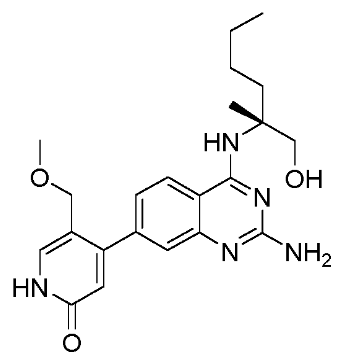

(68),

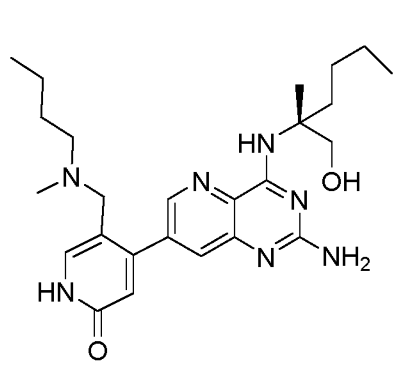

(69),

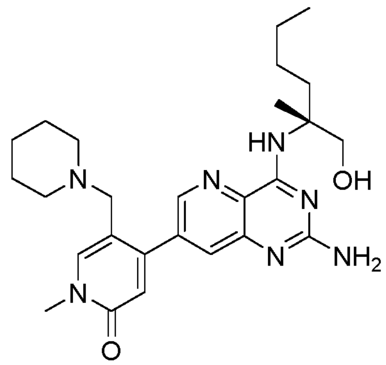

(70)

and

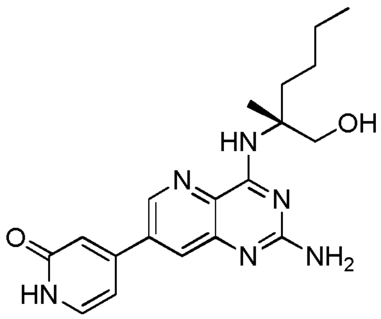

(71).

In other aspect, provided herein is a pharmaceutical composition comprising the compound and the pharmaceutically acceptable excipient disclosed herein.

In some embodiments, the pharmaceutical composition of the present invention further comprises one or more other therapeutic agent, wherein the therapeutic agent is HBV DNA polymerase inhibitor, Toll Like Receptor 7 conditioning agent, Toll Like Receptor 8 conditioning agent, Toll Like Receptor 7 and 8 conditioning agent, Toll Like Receptor 3 conditioning agent, interferon α ligand, HBsAg inhibitor, HbcAg-targeting compound, cyclophilin inhibitor, HBV therapeutic vaccine, HBV prophylactic vaccine, HBV virus entry inhibitor, NTCP inhibitor, antisense oligonucleotides targeting viral mRNA, short interfering RNA (siRNA), hepatitis Be antigen inhibitor, HBx inhibitor, cccDNA inhibitor, HBV antibody, thymosin agonist, cytokine, nuclear protein inhibitor, stimulator of retinoic acid-inducible gene 1, NOD2 stimulator, recombinant thymosin α-1, hepatitis Be antigen replication inhibitor, hepatitis B surface antigen (HBsAg) secretion or assembly inhibitor, IDO inhibitor or combination thereof.

In some embodiments, the pharmaceutical composition of the present invention, wherein one or more other therapeutic agent is Lamivudine, Sebivo, Tenofovir, Entecavir, Adefovir Dipivoxil, Tenofovir Alafenamide, Tenofovir disoproxil, Tenofovir alafenamide fumarate, Tenofovir alafenamide hemifumarate, Alfaferone, Alloferon, Celmoleukin, Clevudine, Emtricitabine, Famciclovir, interferon, hepatect CP, Intefen, Interleukin-2, Mivotilate, Nitazoxanide, Ribavirin, Roferon-A, Schizophyllan, Euforavac, Ampligen, Phosphazid, Heplisav, Recombinant Human Interleukin-2 Injection, levamisole or Proxigermanium.

In some embodiments, the interferon of the present invention is interferon α-1b, interferon α, interferon α-2a, interferon β-1a, interferon α-2, pegylated interferon α-2a or interferon α-2b.

In other aspect, provided herein is the use of the compound or the pharmaceutical composition thereof in activating TLR8.

In other aspect, provided herein is the use of the compound or the pharmaceutical composition thereof in the manufacture a kit, wherein the kit is used for activating TLR8.

In other aspect, provided herein is the use of the compound or the pharmaceutical composition thereof in the manufacture a medicament for preventing, managing, treating or lessening diseases mediated by TLR8 in patients.

In some embodiments, the diseases mediated by TLR8 are hepatitis B virus infection, hepatitis C virus infection, influenza virus infection, herpes virus infection, HIV infection, allergic diseases, rheumatoid arthritis, allergic asthma, chronic fatigue, type II diabetes mellitus, hay fever, lupus erythrosus, multiple sclerosis, melanoma, lung cancer, liver cancer, basal cell carcinoma, kidney cancer, myeloma, biliary tract cancer, brain cancer, breast cancer, cervical cancer, chorionic cancer, colon cancer, rectal cancer, head and neck cancer, Peritoneal tumors, fallopian tube cancer, endometrial cancer, esophageal cancer, gastric cancer, leukemia, lymphoma, sarcoma, neuroblastoma, oral cancer, ovarian cancer, pancreatic cancer, prostate cancer, testicular cancer, skin cancer or thyroid cancer.

In other aspect, provided herein is the use of the compound or the pharmaceutical composition thereof in the manufacture a medicament for treating or preventing diseases of the immunomodulatory system.

In other aspect, provided herein is the use of the compound or the pharmaceutical composition thereof in the manufacture a medicament for treating or preventing viral infections or tumors.

In other aspect, provided herein is the use of the compound or the pharmaceutical composition thereof in the manufacture a medicament for treating or preventing hepatitis B virus infection, hepatitis C virus infection, influenza virus infection, herpes virus infection, HIV infection, allergic diseases, rheumatoid arthritis, allergic asthma, chronic fatigue, type II diabetes mellitus, hay fever, lupus erythrosus, multiple sclerosis, melanoma, lung cancer, liver cancer, basal cell carcinoma, kidney cancer, myeloma, biliary tract cancer, brain cancer, breast cancer, cervical cancer, chorionic cancer, colon cancer, rectal cancer, head and neck cancer, Peritoneal tumors, fallopian tube cancer, endometrial cancer, esophageal cancer, gastric cancer, leukemia, lymphoma, sarcoma, neuroblastoma, oral cancer, ovarian cancer, pancreatic cancer, prostate cancer, testicular cancer, skin cancer and thyroid cancer.

In other aspect, provided herein is the use of the compound or the pharmaceutical composition thereof in the manufacture a medicament for preventing, treating or lessening diseases of the immunomodulatory system, comprising

administering an effective therapeutic dose of the compound or pharmaceutical composition of the present invention to a patient.

In other aspect, provided herein is the use of the compound or the pharmaceutical composition thereof in the manufacture a medicament for preventing, treating or lessening diseases mediated by TLR8 in patients, the method comprising administering a pharmaceutically acceptable effective dose of the compound of the present invention to the patient.

In other aspect, provided herein is the use of the compound or the pharmaceutical composition thereof in the manufacture a medicament for treating or preventing viral infections or tumors, which comprises administering a pharmaceutical composition containing the compound of the present invention in a pharmaceutically acceptable effective dose to a patient.

In other aspect, provided herein is a method of the compound or the pharmaceutical composition thereof in the manufacture a medicament for treating or preventing hepatitis B virus infection, hepatitis C virus infection, influenza virus infection, herpes virus infection, HIV infection, allergic diseases, rheumatoid arthritis, allergic asthma, chronic fatigue, type II diabetes mellitus, hay fever, lupus erythrosus, multiple sclerosis, melanoma, lung cancer, liver cancer, basal cell carcinoma, kidney cancer, myeloma, biliary tract cancer, brain cancer, breast cancer, cervical cancer, chorionic cancer, colon cancer, rectal cancer, head and neck cancer, Peritoneal tumors, fallopian tube cancer, endometrial cancer, esophageal cancer, gastric cancer, leukemia, lymphoma, sarcoma, neuroblastoma, oral cancer, ovarian cancer, pancreatic cancer, prostate cancer, testicular cancer, skin cancer and thyroid cancer, which comprises administering a pharmaceutical composition containing the compound of the present invention in a pharmaceutically acceptable effective dose to a patient.

In other aspect, provided herein is a method of the compound or the pharmaceutical composition thereof in the manufacture a medicament for preventing, treating or lessening diseases of the immunomodulatory system, comprising administering an effective therapeutic dose of the compound or pharmaceutical composition of the present invention to a patient.

In other aspect, provided herein is a use of the compound or the pharmaceutical composition thereof in the manufacture a medicament for preventing, treating or lessening diseases mediated by TLR8 in patients.

In other aspect, provided herein is a use of the compound or the pharmaceutical composition thereof in the manufacture a medicament for preventing, treating or lessening diseases of the immunomodulatory system.

In other aspect, provided herein is a use of the compound or the pharmaceutical composition thereof in the manufacture a medicament for treating or preventing viral infections or tumors.

In other aspect, provided herein is the use of the compound or the pharmaceutical composition thereof in the manufacture a medicament for treating or preventing hepatitis B virus infection, hepatitis C virus infection, influenza virus infection, herpes virus infection, HIV infection, allergic diseases, rheumatoid arthritis, allergic asthma, chronic fatigue, type II diabetes mellitus, hay fever, lupus erythrosus, multiple sclerosis, melanoma, lung cancer, liver cancer, basal cell carcinoma, kidney cancer, myeloma, biliary tract cancer, brain cancer, breast cancer, cervical cancer, chorionic cancer, colon cancer, rectal cancer, head and neck cancer, Peritoneal tumors, fallopian tube cancer, endometrial cancer, esophageal cancer, gastric cancer, leukemia, lymphoma, sarcoma, neuroblastoma, oral cancer, ovarian cancer, pancreatic cancer, prostate cancer, testicular cancer, skin cancer and thyroid cancer.

In some embodiments, the patient is a mammal, and in other embodiments, the patient is a human. In some other embodiments, the use further comprises contacting the cell with a therapeutic agent.

In another aspect, provided herein are methods for preparing, separating, and purifying the compounds represented by Formula (I).

The present invention also relates to the use of the compound and pharmaceutically acceptable salts thereof for producing pharmaceutical products for effectively treating the diseases mentioned in the present invention. The compounds of the present invention are also useful in the manufacture of a medicament for reducing, preventing, controlling or treating symptoms of a disease described herein in a patient.

Unless otherwise stated, all stereoisomers, tautomers, N-oxides, solvates, metabolites and pharmaceutically acceptable salts and prodrugs of the compounds disclosed herein are within the scope of the invention.

The phrase "pharmaceutically acceptable" refers to that the substance or composition must be compatible chemically and/or toxicologically, with the other ingredients comprising a formulation, and/or the mammal being treated therewith.

The compounds disclosed herein also include salts of the compounds which are not necessarily pharmaceutically acceptable salts, and which may be useful as intermediates for preparing and/or purifying compounds of Formula (I), and/or for separating enantiomers of compounds of Formula (I).

If the compound disclosed herein is a base, the desired salt may be prepared by any suitable method available in the art, for example, treatment of the free base with an inorganic acid, such as hydrochloric acid, hydrobromic acid, sulfuric acid, nitric acid, phosphoric acid, and the like. or with an organic acid, such as acetic acid, maleic acid, succinic acid, mandelic acid, fumaric acid, malonic acid, pyruvic acid, malic acid, 2-hydroxypropionic acid, citric acid, oxalic acid, glycolic

acid and salicylic acid; a pyranosidyl acid, such as glucuronic acid and galacturonic acid; an $\alpha$-hydroxy acid, such as citric acid and tartaric acid; an amino acid, such as aspartic acid and glutamic acid; an aromatic acid, such as benzoic acid and cinnamic acid; a sulfonic acid, such as p-toluenesulfonic acid, benzenesulfonic acid, methanesulfonic acid, ethanesulfonic acid,trifluoromethanesulfonic acid, and the like; or the combination thereof.

If the compound disclosed herein is an acid, the desired salt may be prepared by any suitable method, for example, treatment of the free acid with an inorganic or organic base, such as an amine (primary, secondary or tertiary), an alkali metal hydroxide, ammonium, $N^+(R^{14})_4$ salt or alkaline earth metal hydroxide, and the like. Some non-limiting examples of suitable salts include organic salts derived from amino acids, such as glycine and arginine; ammonia, such as primary, secondary and tertiary amine, $N^+(R^{14})_4$ salt, wherein $R^{14}$ is H, $C_{1-4}$ alkyl, $C_{6-10}$ aryl, $C_{6-10}$ aryl-$C_{1-4}$-alkyl, and the like; and cyclic amines, such as piperidine, morpholine and piperazine, and inorganic salts derived from sodium, calcium, potassium, magnesium, manganese, iron, copper, zinc, aluminum, lithium, and the like, and further include, when appropriate, nontoxic ammonium, quaternary ammonium and amine cations formed using counterions such as halide, hydroxide, carboxylate, sulfate, phosphate, nitrate, $C_{1-8}$ sulfonate or aryl sulfonate.

**The pharmaceutical compositions of compounds, formulations, administration and use of the compounds and pharmaceutical compositions of the present invention**

According to a further aspect, the pharmaceutical composition of the invention comprises the compound of formula (I), the compounds listed herein, or the compounds of Example, and a pharmaceutically acceptable adjuvant.

The compounds in the pharmaceutical composition of the present invention can be effectively used for the treatment of TLR8-mediated diseases. Areas of disease treatment that may be mentioned for the compounds or pharmaceutical compositions of the present invention are, for example, immune diseases, diseases caused by viral infections and tumors, e.g. hepatitis B virus infection, hepatitis C virus infection, influenza virus infection, herpes virus infection, HIV infection, allergic diseases, rheumatoid arthritis, allergic asthma, chronic fatigue, type II diabetes mellitus, hay fever, lupus erythrosus, multiple sclerosis, melanoma, lung cancer, liver cancer, basal cell carcinoma, kidney cancer, myeloma, biliary tract cancer, brain cancer, breast cancer, cervical cancer, chorionic cancer, colon cancer, rectal cancer, head and neck cancer, Peritoneal tumors, fallopian tube cancer, endometrial cancer, esophageal cancer, gastric cancer, leukemia, lymphoma, sarcoma, neuroblastoma, oral cancer, ovarian cancer, pancreatic cancer, prostate cancer, testicular cancer, skin cancer and thyroid cancer.

The present invention includes pharmaceutical formulations which contain, in addition to non-toxic, inert pharmaceutically suitable excipients, one or more compounds of formula (I) of the present invention or pharmaceutical compositions thereof.

The above-mentioned pharmaceutical formulations may also comprise other active pharmaceutical ingredients besides the compound represented by formula (I).

It will also be appreciated that certain of the compounds disclosed herein can exist in free form for treatment, or where appropriate, as a pharmaceutically acceptable derivative thereof. Some non-limiting examples of the pharmaceutically acceptable derivative include pharmaceutically acceptable prodrugs, salts, esters, salts of such esters, or any other adducts or derivatives which upon administration to a patient in need is capable of providing, directly or indirectly, a compound as otherwise described herein, or a metabolite or residue thereof.

As described above, the pharmaceutical compositions disclosed herein comprises any one of the compounds represented by formula (I) of the present invention, and further comprise a pharmaceutically acceptable an adjuvant, such as those used in the present invention, including any solvent, solid excipient, diluent, binder, disintegrant, or other liquid excipient, dispersant, flavoring agent or suspending agent, surfactant, isotonic agent, thickener, emulsifier, preservative, solid binder or lubricant, etc., suitable for a specific target dosage form. As described in the following: In Remington: Troy et al., Remington: The Science and Practice of Pharmacy, 21st ed., 2005, Lippincott Williams & Wilkins, Philadelphia, and Swarbrick et al., Encyclopedia of Pharmaceutical Technology, eds. 1988-1999, Marcel Dekker, New York, both of which are herein incorporated by reference in their entireties, discloses various adjuvants used in formulating pharmaceutically acceptable compositions and known techniques for the preparation thereof. Except insofar as any conventional adjuvant incompatible with the compounds disclosed herein, such as by producing any undesirable biological effect or otherwise interacting in a deleterious manner with any other components of the pharmaceutically acceptable composition, its use is contemplated to be within the scope of this invention.

Some non-limiting examples of materials which can serve as pharmaceutically acceptable adjuvants include ion exchangers; aluminium; aluminum stearate; lecithin; serum proteins such as human serum albumin; buffer substances such as phosphates; glycine; sorbic acid; potassium sorbate; partial glyceride mixtures of saturated vegetable fatty acids; water; salts or electrolytes such as protamine sulfate, disodium hydrogen phosphate, potassium hydrogen phosphate, sodium chloride and zinc salts; colloidal silica; magnesium trisilicate; polyvinyl pyrrolidone; polyacrylates; waxes; polyethylene-polyoxypropylene-block polymers; wool fat; sugars such as lactose, glucose and sucrose; starches such as corn starch and potato starch; cellulose and its derivatives such as sodium carboxymethyl cellulose, ethyl cellulose and

cellulose acetate; powdered tragacanth; malt; gelatin; talc; excipients such as cocoa butter and suppository waxes; oils such as peanut oil, cottonseed oil, safflower oil, sesame oil, olive oil, corn oil and soybean oil; glycols such as propylene glycol and polyethylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol; and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants.

The pharmaceutical compositions of the compounds of the present invention can be administered in any of the following ways: orally, by inhalation spray, topically, rectally, nasally, topically, vaginally, parenterally such as subcutaneously, intravenously, intramuscularly, intraperitoneal, intrathecal, intraventricular, intrasternal, or intracranial injection or infusion, or administration via an external implanted reservoir. The preferred methods are administered orally, intramuscularly, intraperitoneally or intravenously.

The compounds or pharmaceutical compositions thereof of the present invention may be administered in the form of a unit dose. The dosage form for administration may be a liquid dosage form or other dosage forms. Liquid dosage forms can be true solutions, colloids, microparticles, and suspensions. Other dosage forms include tablets, capsules, pellets, aerosols, pills, powders, solutions, suspensions, emulsions, granules, suppositories, lyophilized powder injections, inclusion compounds, implants, patches, ointments, etc.

Oral tablets and capsules may contain excipients such as binders, for example, syrup, acacia, sorbitol, tragacanth, or polyvinyl pyrrolidone; fillers, for example, lactose, sucrose, corn starch, calcium phosphate, sorbitol, glycine; lubricants, for example, magnesium stearate, talc, polyethylene glycol, silica; disintegrants, for example, potato starch; or acceptable wetting agents, for example, sodium lauryl sulfate. Tablets may also be coated by methods well known in the pharmaceutical industry.

Oral liquids can be prepared into aqueous oil suspensions, solutions, emulsions, syrups or elixirs, or can be prepared into dry products and supplemented with water or other suitable media before use. Such liquid preparations may contain conventional additives, such as suspending agents, sorbitol, methyl cellulose, glucose syrup, gelatin, hydroxyethylcellulose, carboxymethyl cellulose, aluminum tristearate gel, hydrogenated edible oils and fats, emulsifiers, such as lecithin, sorbitan monooleate, gum arabic; or non-aqueous excipients (which may contain edible oils), such as almond oil, oils such as glycerol, ethylene glycol, or ethanol; preservatives, such as methyl 4-hydroxybenzoate or propyl 4-hydroxybenzoate, sorbic acid. Flavoring or coloring agents can be added if desired.

Suppositories may contain conventional suppository bases such as cocoa butter or other glycerides.

For parenteral administration, liquid dosage form is usually prepared by combining the compound with a sterile adjuvant. The preferred adjuvant is water. Depending on the selected adjuvants and drug concentrations, the compound can be dissolved in the adjuvants or made into a suspension solution. When making a solution for injection, the compound is first dissolved in water, filtered and sterilized, and then filled into a sealed bottle or ampoule.

When applied topically to the skin, the compounds of the present invention can be made into the form of suitable ointments, lotions, or creams, in which the active ingredient is suspended or dissolved in one or more adjuvants, wherein the adjuvants that can be used for ointment preparations include but are not limited to: mineral oil, liquid petrolatum, white petrolatum, propylene glycol, polyoxyethylene, polyoxypropylene, emulsifying wax and water; the adjuvants that can be used for lotions and creams include but are not limited to: mineral oil, sorbitan monostearate, Tween 60, cetyl ester wax, hexadecylene aromatic alcohol, 2-octyl dodecanol, benzyl alcohol and water.

The amount of active ingredient that may be combined with the inactive ingredients to produce a dosage form may vary depending upon the intended subject to be treated and the particular mode of administration. For example, in some embodiments, dosage forms for oral administration to humans may contain about 1 to 1000 mg of active material formulated with an appropriate and convenient amount of a pharmaceutically acceptable excipient. In certain embodiments, the pharmaceutically acceptable excipient varies from about 5% to about 95% (weight: weight) of the total composition.

The compounds disclosed herein, such as compounds represented by formula (I), can be administered to an individual for a desired period of time or duration according to an effective dosing regimen, such as at least about one month, at least about 2 months, at least about 3 months, at least about 6 months, or at least about 12 months or longer. In one variation, the compound is administered on a daily or intermittent schedule during the life of the individual.

The dosage or frequency of administration of the compounds disclosed herein may be adjusted during the course of treatment based on the judgment of the administering physician.

The compound can be administered to an individual (e.g., a human) in an effective amount. In certain embodiment, a dose is administered once per day.

In certain embodiments, methods are provided for treating or preventing a disease or condition in a human, comprising administering to a human a therapeutically effective amount of a compound disclosed herein, or a pharmaceutically acceptable salt thereof, in combination with a therapeutically effective amount of one or more (e.g., one, two, three, four, one or two, one to three, or one to four) additional therapeutic agents. Because agonists of TLR-8 are useful in

treating a variety of diseases or conditions, the specific identity of the additional therapeutic agent will depend on the particular disease or condition being treated.

The compound represented by formula (I) can be administered by any useful route and mode, for example, orally or parenterally (eg, intravenously). The therapeutically effective amount of the compound represented by formula (I) is about 0.00001 mg/kg body weight/day to about 10 mg/kg body weight/day, for example, about 0.0001 mg/kg body weight/day to about 10 mg/kg/day or about 0.001 mg/kg body weight/day to about 1 mg/kg body weight/day, or for example, about 0.01 mg/kg body weight/day to about 1 mg/g body weight/day or for example, about 0.05 mg/kg body weight/day to about 0.5 mg/kg body weight/day.

The therapeutically effective amount of the compound represented by formula (I) is about 0.01 mg/kg body weight/day to about 1000 mg/kg body weight/day, for example, about 0.01 mg/kg body weight/day to about 100 mg/kg/day or about 0.1 mg/kg body weight/day to about 100 mg/kg body weight/day, or for example, about 1 mg/kg body weight/day to about 100 mg/g body weight/day or for example, about 1 mg/kg body weight/day to about 10 mg/kg body weight/day. Other therapeutically effective amount of the compound represented by formula (I) is about 1 mg/dose, or about 2, 3, 4, 5, 6, 7, 8, 9, 10, 15, 20, 25, 30, 35, 40, 45, 50, 55, 60, 65, 70, 75, 80, 85, 90, 95 or 100 mg/dose. Other therapeutically effective amount of the compound represented by formula (I) is about 100 mg/dose, or about 125, 150, 175, 200, 225, 250, 275, 300, 350, 400, 450 or 500 mg/dose. Single doses may be administered hourly, daily, or weekly. For example, a single dose can be administered once every 1 hour, once every 2, 3, 4, 6, 8, 12, 16 hours, or once every 24 hours. A single dose can also be administered once every 1 day, once every 2, 3, 4, 5, 6 days, or once every 7 days. A single dose can also be administered once every 1 week, once every 2, 3, or 4 weeks. In certain embodiments, a single dose may be administered once per week. A single dose may also be administered monthly.

The frequency of administration of the compound represented by formula (I) will be determined by the needs of the individual patient, for example, it may be once a day or twice a day or more. For example, the compound can be administered to a human infected with HBV or HCV for a period of 20 to 180 days, or for example, 20 to 90 days, or for example, 30 to 60 days, depending on the length of time required to continue treating the HBV or HCV infection.

Administration can be intermittent, wherein the patient receives a daily dose of a compound of formula (I) over a period of several or more days, followed by a period of several or more days in which the patient does not receive a daily dose of the compound. For example, a patient may receive doses of the compound every other day or three times per week. Again by way of example, a patient may receive daily doses of the compound for a period of 1 to 14 days, followed by a period of 7 to 21 days during which the patient does not receive doses of the compound, followed by a subsequent period (e.g., from 1 to 14 days) during which the patient again receives daily doses of the compound. Administration of the compound, followed by periods of time without administration of the compound, can be repeated as clinically required to treat the patient.

In one embodiment, a pharmaceutical composition is provided, comprising a compound or a pharmaceutically acceptable salt thereof and a pharmaceutically acceptable excipient disclosed herein in combination with one or more (e.g., one, two, three, four, one or two, one to three, or one to four) additional therapeutic agents.

In one embodiment, a kit is provided, comprising a compound or a pharmaceutically acceptable salt thereof disclosed herein in combination with one or more (e.g., one, two, three, four, one or two, one to three, or one to four) additional therapeutic agents.

In certain embodiments, a compound or a pharmaceutically acceptable salt thereof disclosed herein, is combined with one, two, three, four or more additional therapeutic agents. In certain embodiments, a compound or a pharmaceutically acceptable salt thereof disclosed herein, is combined with two additional therapeutic agents. In other embodiments, a compound or a pharmaceutically acceptable salt thereof disclosed herein, is combined with three additional therapeutic agents. In additional embodiments, a compound or a pharmaceutically acceptable salt thereof disclosed herein, is combined with four additional therapeutic agents. The one, two, three, four or more additional therapeutic agents can be different therapeutic agents selected from the same class of therapeutic agents, and/or they can be selected from different classes of therapeutic agents.

In certain embodiments, when the compounds disclosed herein are combined with one or more additional therapeutic agents described herein, the components of the composition are administered in a simultaneous or sequential regimen. When administered sequentially, the combination may be administered in two or more administrations.

In certain embodiments, the compounds disclosed herein are combined with one or more additional therapeutic agents in a single dosage form for simultaneous administration to a patient, for example, as a solid dosage form for oral administration.

In certain embodiments, the compounds disclosed herein are administered with one or more additional therapeutic agents. Co-administering a compound disclosed herein with one or more additional therapeutic agents generally refers to administering a compound disclosed herein and one or more additional therapeutic agents simultaneously or sequentially such that therapeutically effective amounts of both the compound disclosed herein and the one or more additional therapeutic agents are present in the patient’s body.

Co-administration includes administering a unit dose of a compound disclosed herein before or after administration of a unit dose of one or more additional therapeutic agents, for example, administering a compound disclosed herein within seconds, minutes or hours of administration of one or more additional therapeutic agents. For example, in some embodiments, a unit dose of a compound disclosed herein is administered first, followed within seconds or minutes by a unit dose of one or more additional therapeutic agents. Or, in other embodiments, a unit dose of one or more additional therapeutic agents is administered first, followed by administration of a unit dose of a compound disclosed herein within seconds or minutes. In some embodiments, a unit dose of a compound disclosed herein is administered first, followed by administration of a unit dose of one or more additional therapeutic agents after a period of several hours (e.g., 1-12 hours). In other embodiments, a unit dose of one or more additional therapeutic agents is administered first, followed by administration of a unit dose of a compound disclosed herein after a period of several hours (e.g., 1-12 hours).

The pharmaceutical composition provided by the present invention comprises a compound shown in formula (I) of the present invention or a pharmaceutically acceptable salt thereof and a pharmaceutically acceptable excipient, and further comprises one or more other therapeutic agents. Wherein the therapeutic agent is HBV DNA polymerase inhibitor, Toll Like Receptor 7 conditioning agent, Toll Like Receptor 8 conditioning agent, Toll Like Receptor 7 and 8 conditioning agent, Toll Like Receptor 3 conditioning agent, interferon $\alpha$ ligand, HBsAg inhibitor, HbcAg-targeting compound, cyclophilin inhibitor, HBV therapeutic vaccine, HBV prophylactic vaccine, HBV virus entry inhibitor, NTCP inhibitor, antisense oligonucleotides targeting viral mRNA, short interfering RNA (siRNA), hepatitis Be antigen inhibitor, HBx inhibitor, cccDNA inhibitor, HBV antibody, thymosin agonist, cytokine, nuclear protein inhibitor, stimulator of retinoic acid-inducible gene 1, NOD2 stimulator, recombinant thymosin $\alpha$-1 and hepatitis Be antigen replication inhibitor, hepatitis B surface antigen (HBsAg) secretion or assembly inhibitor, IDO inhibitor or combination thereof.

Wherein one or more other therapeutic agent is Lamivudine, Sebivo, Tenofovir, Entecavir, Adefovir Dipivoxil, Tenofovir Alafenamide, Tenofovir disoproxil, Tenofovir alafenamide fumarate, Tenofovir alafenamide hemifumarate, Alfaferone, Alloferon, Celmoleukin, Clevudine, Emtricitabine, Famciclovir, interferon, hepatect CP, Intefen, interferon $\alpha$-1b, interferon $\alpha$, interferon $\alpha$-2a, interferon $\beta$-1a, interferon $\alpha$-2, Interleukin-2, Mivotilate, Nitazoxanide, polyethylene glycol interferon $\alpha$-2a, Ribavirin, Roferon-A, Schizophyllan, Euforavac, Ampligen, Phosphazid, Heplisav, interferon $\alpha$-2b, Recombinant Human Interleukin-2 Injection, levamisole or Proxigermanium.

In other aspect, provided herein relates to the use of a compound or pharmaceutical composition of the present invention to prepare medicines for immune diseases, diseases caused by viral infections and tumors, especially for preventing, treating or alleviating hepatitis B in patients, comprising administering a pharmaceutically acceptable effective dose to the patient. Hepatitis B disease refers to liver disease caused by hepatitis B virus infection or hepatitis B infection, including acute hepatitis, chronic hepatitis, cirrhosis and hepatocellular carcinoma. Acute hepatitis B virus infection may be asymptomatic or present with symptoms of acute hepatitis. Patients with chronic viral infections have active disease that can progress to cirrhosis and liver cancer.

## GENERAL SYNTHETIC PROCEDURES

Generally, the compounds disclosed herein may be prepared by methods described herein, wherein the substituents are as defined for Formula (I) above, except where further noted. The following non-limiting schemes and examples are presented to further exemplify the invention.

Persons skilled in the art will recognize that the chemical reactions described may be readily adapted to prepare a number of other compounds disclosed herein, and alternative methods for preparing the compounds disclosed herein are deemed to be within the scope disclosed herein. For example, the synthesis of non-exemplified compounds according to the invention may be successfully performed by modifications apparent to those skilled in the art, e.g., by appropriately protecting interfering groups, by utilizing other suitable reagents known in the art other than those described, and/or by making routine modifications of reaction conditions. Alternatively, other reactions disclosed herein or known in the art will be recognized as having applicability for preparing other compounds disclosed herein.

In the examples described below, unless otherwise indicated all temperatures are set forth in degrees Celsius (°C). Reagents were purchased from commercial suppliers such as Aldrich Chemical Company, Arco Chemical Company and Alfa Chemical Company, and were used without further purification unless otherwise indicated. Common solvents were purchased from commercial suppliers such as Shantou XiLong Chemical Factory, Guangdong Guanghua Reagent Chemical Factory Co. Ltd., Guangzhou Reagent Chemical Factory, Tianjin YuYu Fine Chemical Ltd., Qingdao Tenglong Reagent Chemical Ltd., and Qingdao Ocean Chemical Factory.

Column chromatography was conducted using a silica gel column. Silica gel (200 - 300 mesh) was purchased from Qingdao Ocean Chemical Factory. 1H NMR spectra were obtained by using $CDCl_3$, DMSO-$d_6$, $CD_3OD$ or $d_6$-acetone solutions (reported in ppm), with TMS (0 ppm) or chloroform (7.25 ppm) as the reference standard. When peak multiplicities were reported, the following abbreviations were used: s (singlet), q (quartet), d (doublet), t (triplet), m (multiplet), br (broadened), dd (doublet of doublets,), dt (doublet of triplets), ddd (doublet of doublet of doublets), tt (triplet of triplets), br.s (broadened singlet). Coupling constants *J*, when given, were reported in Hertz (Hz).

Low-resolution mass spectral (MS) data were also determined on an Agilent 6320 series LC-MS spectrometer

equipped with G1312A binary pumps, a G1316A TCC (Temperature Control of Column, maintained at 30 °C), a G1329A autosampler and a G1315B DAD detector were used in the analysis. An ESI source was used on the LC-MS spectrometer.

Low-resolution mass spectral (MS) data were also determined on an Agilent 6120 series LC-MS spectrometer equipped with G1311A binary pumps, a G1316A TCC (Temperature Control of Column, maintained at 30 °C), a G1329A autosampler and a G1315D DAD detector were used in the analysis. An ESI source was used on the LC-MS spectrometer.

Both LC-MS spectrometers were equipped with an Agilent Zorbax SB-C18, 2.1 * 30 mm, 5 μm column. Injection volume was decided by the sample concentration. The flow rate was 0.6 mL/min. The HPLC peaks were recorded by UV-Vis wavelength at 210 nm and 254 nm. The mobile phase was 0.1% formic acid in acetonitrile (phase A) and 0.1% formic acid in ultrapure water (phase B). The gradient elution conditions were showed in Table 1: The gradient elution conditions were showed in Table a:

Table a: the gradient elution conditions

| Time (min) | A ($CH_3CN$, 0.1% HCOOH) | B ($H_2O$, 0.1% HCOOH) |
|---|---|---|
| 0 - 3 | 5 - 100 | 95 - 0 |
| 3 - 6 | 100 | 0 |
| 6 - 6.1 | 100 - 5 | 0 - 95 |
| 6.1 - 8 | 5 | 95 |

Purities of compounds were assessed by Agilent 1100 Series high performance liquid chromatography (HPLC) with UV detection at 210 nm and 254 nm (Zorbax SB-C18, 2.1 * 30 mm, 4 micron). The run time was 10 min, and the flow rate was 0.6 mL/min. The elution was performed with a gradient of 5 to 95% phase A (0.1% formic acid in $CH_3CN$) in phase B (0.1% formic acid in $H_2O$). Column was operated at 40 °C.

The following abbreviations are used throughout the specification:

| | | | |
|---|---|---|---|
| MeOH | methanol | *t*-BuOH | *t*-butanol |
| MeOH-$d_4$ | methanol -$d_4$ | $DMBNH_2$ | 2,4-Dimethoxybenzylamine |
| MsCl | methylsufonyl chloride | HCl/EA, | HCl/EtOAc a solution of hydrogen chloride in ethyl acetate |
| MeI | iodomethane | | |
| DIAD | diisopropyl azodicarboxylate | | |
| DCM, $CH_2Cl_2$ | dichloromethane | HAUT | 2-(7-aza-1H-benzotriazole-1-yl)-1,1,3,3-tetramethylu ronium hexafluorophosphate |
| DCE | dichloroethane | | |
| $PdCl_2$(dppf) | [1,1'-bis(diphenylphosphino)ferro-cene]dichloropal -ladium(II) | NBS | *N*-bromosuccinimide |
| | | ACN | acetonitrile |
| $CDC1_3$ | chloroform-d | DIPEA | *N,N*- diisopropylethylamine |
| Boc | *tert*-butoxycarbonyl | DMF | *N*,*N*-dimethylformamide |
| $(Boc)_2O$ | Di-tert-butyl dicarbonate | EDCI | 1-(3-dimethylaminopropyl)-3-ethylcarbodiimide hydrochloride |
| PE | petroleum ether | | |
| EtOAc, EA | ethyl acetate | | |
| 1atm | 101.325 kPa | DMF | *N,N*- dimethylformamide |
| h | hour, hours | AIBN | 2,2'-Azobis(2-methylpropionitrile) |
| RT, rt | room temperature | EtOH | ethyl alcohol |
| DME | glycol dimethyl ether | $Et_3N$, TEA | triethylamine |
| $B_2Pin_2$ | bis(pinacolato)diboron | mL, ml | milliliter |
| TFA | trifluoroacetic acid | THF | tetrahydrofuran |
| KOAc | potassium acetate | DMSO | dimethylsulfoxide |
| $MeNH_2$ | Aminomethane | $Ac_2O$ | Acetic anhydride |
| t-BuOK | potassium tert-butoxide | $t_{1/2}$ | half-life |
| | | Rt | retention time |

**Synthesis method**

The following synthetic schemes list the experimental procedures for preparing the compounds disclosed in this invention. wherein, each $R^1$, $R^2$, $R^3$, $R^5$, $R^6$, X, $R^7$, $R^8$, $R^9$, R and Y is as defined herein, each $X_1$ and $X_2$ is independently Cl, Br or I.

## Scheme 1

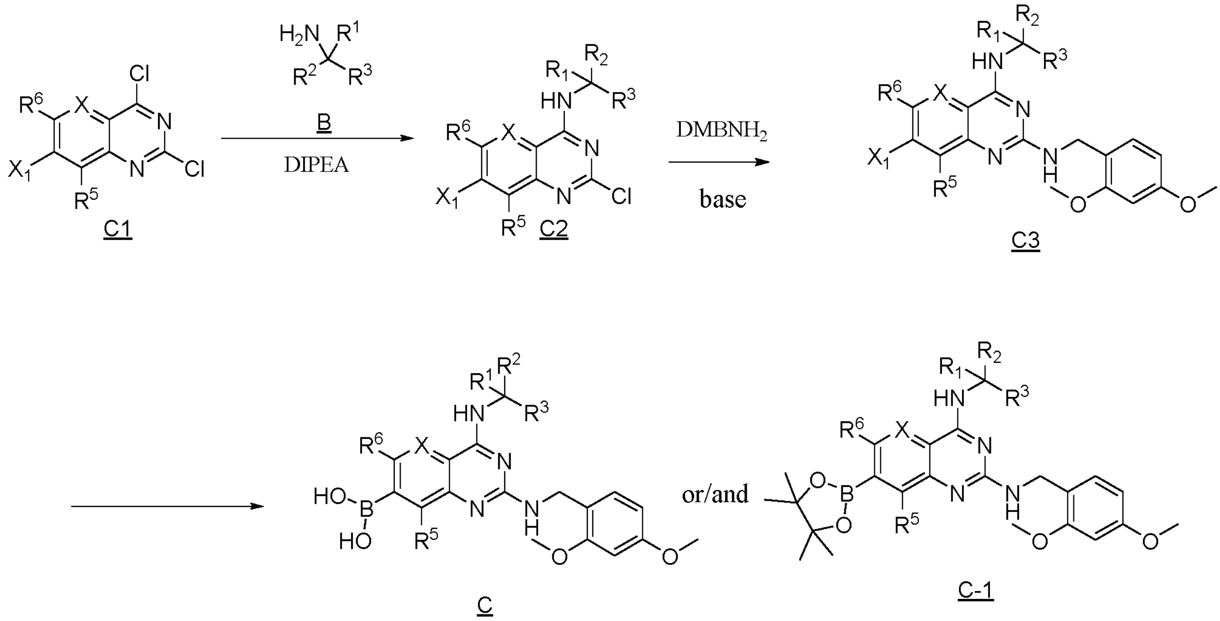


The intermediate of compound C and/or compound C-1 can be prepared by the process illustrated in scheme 1. Under basic conditions (such as N,N-diisopropylethylamine, etc.), compound C1 reacts with compound B or a salt thereof to obtain compound C2. Then, under the action of a base (such as potassium carbonate, sodium carbonate, etc.), compound C2 reacts with 2,4-dimethoxybenzylamine to obtain compound C3. Finally, under basic conditions (such as potassium acetate) and a catalyst (such as [1,1'-bis(diphenylphosphino)ferrocene] dichloropalladium dichloromethane complex, etc.), bis(pinacolato)diboron reacts with compound C3 to obtain compound C and/or compound C-1.

## Scheme 2

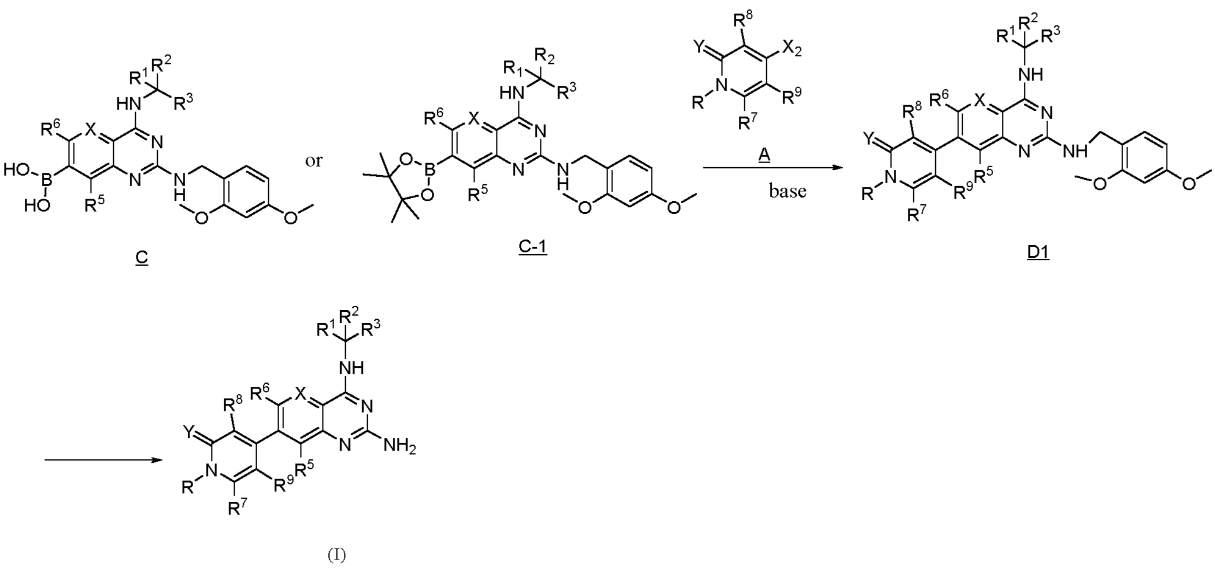


Compound (I) can be prepared by the process illustrated in scheme 2, First, compound C or compound C-1 reacts

with compound A in the presence of a base (such as potassium carbonate) and a catalyst (such as [1,1'-bis(diphenylphosphino)ferrocene] dichloropalladium dichloromethane complex, etc.) to obtain compound D1. Then, under acidic conditions (such as trifluoroacetic acid, etc.), compound D1 is removed the 2,4-dimethoxybenzyl protecting group on the amino group to obtain compound (I).

## EXAMPLES

The following examples are used to illustrate the present invention but are not intended to limit the scope of the present invention.

### Preparation Example

In the following preparation examples, the inventors describe in detail the preparation process of the compounds of the present invention by taking some of the compounds of the present invention as examples. In addition, in the following preparation examples, when there is a discrepancy between the compound name and the compound structure, the compound structure shall prevail.

**Example 1: (*R*)-4-(2-Amino-4-((1-hydroxy-2-m ethylhexan-2-yl)amino)pyrido[3,2-*d*]pyrimidin-7-yl)-1-met hyl-5-((methyl(phenethyl)amino)methyl)pyridin-2(1*H*)-one**

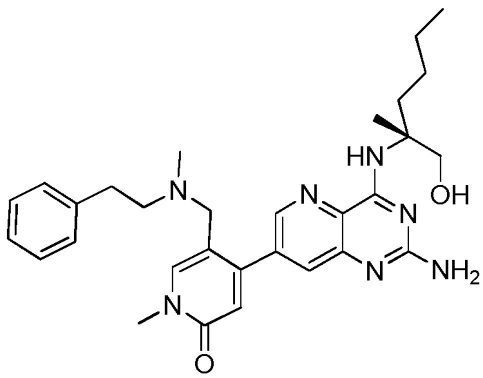

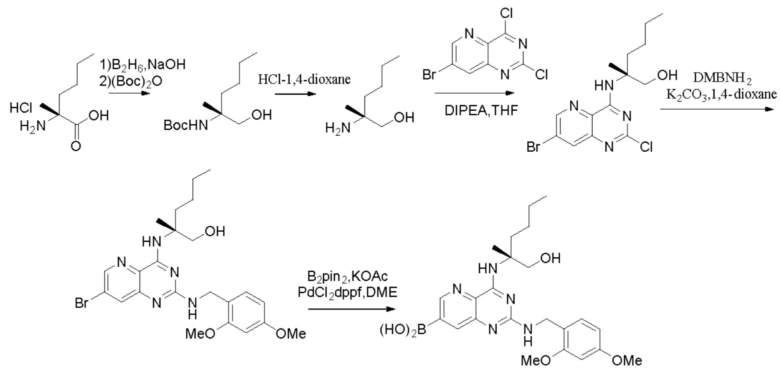


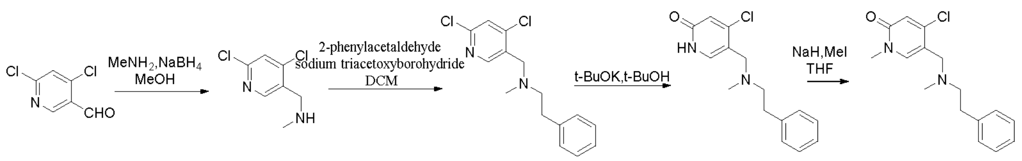

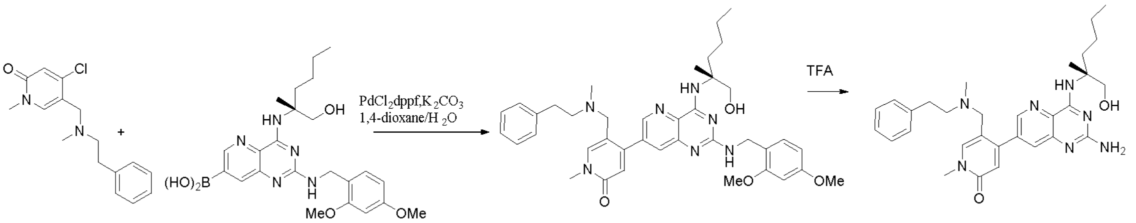


Step 1: Tert-butyl (*R*)-(1-hydroxy-2-methylhexan-2-yl)carbamate

(2R)-2-Amino-2-methylhexanoic acid hydrochloride (11.86 g, 65.29 mmol) was weighed and dissolved in THF (100 mL). Under $N_2$, 0 °C and stirring conditions, the Borane-tetrahydrofuran complex (196.00 mL, 195.87 mmol, 1 M) was slowly added dropwise. After the addition was completed, the resulted mixture was transferred to room temperature and stirred for about 3 hours, then transferred to 60 °C and stirred for about 2 hours. Then, the sodium hydroxide (13.06 g, 326.45 mmol) was slowly added dropwise under 0 °C and stirring. After the addition was completed, the resulted mixture was transferred to a 60 °C oil bath andstirred for about 3 hours. Then, $(Boc)_2O$ (15.68 g, 71.82 mmol) was added dropwise and the reaction was continued with stirring for about 3 hours. The reaction was stopped, water (200 mL) was added to dilute the mixture, and then the resulted mixture was extracted with ethyl acetate (120 mL × 3). The organic phases were combined, washed with saturated salt water (150 mL), dried over anhydrous sodium sulfate and concentrated *in vacuo.* The residue was purified by silica gel column chromatography (PE/EA (V/V) = 10/1) to obtain the title compound as a colorless oily liquid (15.10 g, yield 99.98%).
MS (ESI, pos.ion) *m/z:* 254.2 $[M+Na]^+$.

Step 2: (*R*)-2-Amino-2-methylhexan-1-ol

Tert-butyl (*R*)-(1-hydroxy-2-methylhexan-2-yl)carbamate (1.44 g, 6.22 mmol) was added to a solution of hydrochloric acid in 1,4-dioxane (20 mL, 4 M).The resulted mixture was stirred at room temperature for about 1 hour, and then concentrated under reduced pressure to obtain the title compound as an oily liquid (0.82 g, yield 100.47%), which was directly used in the next step.

Step 3: (*R*)-2-((7-Bromo-2-chloropyrido[3,2-*d*]pyrimidin-4-yl)amino)-2-methylhexan-1-ol

7-Bromo-2,4-dichloropyrido[3,2-*d*]pyrimidine (1.73 g, 6.20 mmol) and (R)-2-amino-2-methylhexan-1-ol (0.81 g, 6.2 mmol) were mixed in THF (30 mL), and DIPEA (4.01 g, 31.00 mmol) was added. The reaction mixture was heated in an oil bath at 60 °C overnight. The mixture was cooled to room temperature and concentrated under reduced pressure to remove the solvent. The residue was purified by silica gel column chromatography (PE/EA (V/V) = 5/1) to give the title compound as a yellow solid (1.63 g, yield 70.36%).
MS (ESI, pos.ion) *m/z:* 373.0, 375.0 $[M+H]^+$.

Step 4: (*R*)-2-((7-Bromo-2-((2,4-dimethoxybenzyl)amino)pyrido[3,2-*d*]pyrimidin-4-yl)amino)-2-methylhex an-1-ol

(*R*)-2-((7-Bromo-2-chloropyrido[3,2-*d*]pyrimidin-4-yl)amino)-2-methylhexan-1-ol (1.53 g, 4.09 mmol), $DMBNH_2$ (0.79 g, 4.70 mmol) and potassium carbonate (1.13 g, 8.18 mmol) were mixed in 1,4-dioxane (50 mL). The reaction mixture was heated in an oil bath at 100 °C for about 25 h. The mixture was cooled to room temperature and concentrated under reduced pressure. The residue was purified by silica gel column chromatography (PE/EA (V/V) = 3/1) to give the title compound as a light yellow solid (2.02 g, yield 97.91%).

Step 5: (*R*)-(2-((2,4-Dimethoxybenzyl)amino)-4-((1-hydroxy-2-methylhexan-2-yl)amino)pyrido[3,2-*d*]pyri midin-7-yl) boronic acid

(*R*)-2-((7-Bromo-2-((2,4-dimethoxybenzyl)amino)pyrido[3,2-*d*]pyrimidin-4-yl)am ino)-2-methylhexan-1-ol (1.15 g, 2.28 mmol), $B_2Pin_2$ (0.75 g, 2.96 mmol), potassium acetate (0.67 g, 6.84 mmol) and $PdCl_2(dppf)$ (0.17 g, 0.23 mmol) were weighed separately and mixed in DME (30 mL). Under $N_2$, the reaction mixture was heated and stirred in an oil bath at 80 °C for about 4 hours, then filtered through celite, and the filter cake was washed with dichloromethane (20 mL). The filtrate was then concentrated under reduced pressure, and the residue was dissolved with dichloromethane (30 mL). The resulting solution was washed with saturated brine (30 mL) and dried over anhydrous sodium sulfate, and concentrated under reduced pressure to obtain the title compound as a brown foamy solid (2.09 g, yield 97.65%), which was directly

used in the next step. MS (ESI, pos.ion) *m/z:* 470.4 [M+H]+.

Step 6: 1-(4,6-Dichloropyiidin-3-yl)-*N*-methylmethanamine

4,6-Dichloronicotinaldehyde (5.77 g, 32.78 mmol) was weighed and dissolved in anhydrous methanol (80 mL), under $N_2$ and stirring at 0 °C, 30% a solution of methylamine in methanol (33.94 g, 327.80 mmol) was slowly added dropwise, and the reaction was continued to stir; then sodium borohydride (1.86 g, 49.17 mmol) was added in batches, and after the addition was completed, the resulted mixture was transferred to room temperature and stirred for about 20 hours; the reaction was stopped, water (100 mL) was added to the reaction solution to dilute it, and then extracted with ethyl acetate (40 mL × 3), the organic phases were combined, washed with saturated brine (50 mL), dried over anhydrous sodium sulfate, and concentrated under reduced pressure. The residue was purified by silica gel column chromatography (PE/EA (V/V) = 5/1) to obtain the title compound as a light yellow oily liquid (4.0 g, yield 63.87%).
MS (ESI, pos.ion) *m/z:* 191.0 [M+H]+.

Step 7: *N*-((4,6-Dichloropyridin-3-yl)methyl)-*N*-methyl-2-phenylethan-1-amine

1-(4,6-Dichloropyridin-3-yl)-N-methylmethanamine (0.67 g, 3.51 mmol) and 2-phenylacetaldehyde (0.51 g, 4.21 mmol) were weighed separately and added to DCM (20 mL), then sodium triacetoxyborohydride (2.98 g, 14.08 mmol) was added. The mixture was stirred at room temperature for about 20 hours, then saturated sodium bicarbonate solution (30 mL) was added to the reaction solution to dilute it, and then extracted with DCM (30 mL × 3), the organic phases were combined, washed with saturated brine (30 mL), dried over anhydrous sodium sulfate, and concentrated under reduced pressure. The residue was purified by silica gel column chromatography (PE/EA (V/V) = 10/1) to obtain the title compound as a colorless oily liquid (0.35 g, yield 33.78%).
MS (ESI, pos.ion) *m/z:* 295.0 [M+H]+.

Step 8: 4-Chloro-5-((methyl(phenethyl)amino)methyl)pyridin-2(1*H*)-one

*N*-((4,6-Dichloropyridin-3-yl)methyl)-*N*-methyl-2-phenylethan-1-amine (0.35 g, 1.19 mmol), potassium tert-butyrate (0.54 g, 4.78 mmol) and tert-butanol (30 mL) were added into a single-necked bottle respectively. Under $N_2$, the mixture was placed in an oil bath at 100 °C and heated to reflux for about 48 hours. The stirring was stopped. The reaction mixture was adjusted the pH to 5-6 with 4 M hydrochloric acid in 1,4-dioxane, then concentrated under reduced pressure. The residue was purified by silica gel column chromatography (DCM/MeOH (V/V) = 15/1) to give the title compound as a white solid (0.17 g, yield 51.62%).
MS (ESI, pos.ion) *m/z:* 277.1 [M+H]+.

Step 9: 4-Chloro-1-methyl-5-((methyl(phenethyl)amino)methyl)pyridin-2(1*H*)-one

4-Chloro-5-((methyl(phenethyl)amino)methyl)pyridin-2(1*H*)-one (1.00 g, 2.72 mmol) was weighed and dissolved in THF (50 mL), the mixture was stirred at 0 °C, then NaH (0.22 g, 5.44 mmol) was slowly added and stirred for about 1 hour, then iodomethane (0.77 g, 5.42 mmol) was added , the resulted mixture was transferred to room temperature and stirred for about 4 hours. The reaction was stopped, water (30 mL) was added to the reaction solution to dilute it, and then extracted with ethyl acetate (30 mL × 3), the organic phases were combined, washed with saturated brine (40 mL), dried over anhydrous sodium sulfate, and concentrated under reduced pressure. The residue was purified by silica gel column chromatography (DCM/MeOH (V/V) = 15/1) to obtain the title compound as a brown-yellow oily liquid (0.51 g, yield 49.05%).
MS (ESI, pos.ion) *m/z*: 291.0 [M+H]+.

Step 10: (*R*)-4-(2-((2,4-Dimethoxybenzyl)amino)-4-((1-hydroxy-2-methylhexan-2-yl)amino)pyrido[3,2-*d*]py rimidin-7-yl)-1-methyl-5-((methyl(phenethyl)amino)methyl)pyridin-2(1*H*)-one

4-Chloro-1-methyl-5-((methyl(phenethyl)amino)methyl)pyridin-2(1*H*)-one (0.35 g, 0.92 mmol), (*R*)-(2-((2,4-Dimethoxybenzyl)amino)-4-((1-hydroxy-2-methylhexan-2-yl)amino)pyrido[3,2-*d*]pyri midin-7-yl)boronic acid (0.47 g, 1.01 mmol), $PdCl_2$(dppf) (0.067 g, 0.092 mmol) and potassium carbonate (0.38 g, 2.75 mmol) were mixed in 1,4-dioxane/$H_2O$ (V/V=10/1, 30 mL), under $N_2$, the reaction mixture was heated in an oil bath at 65 °C for about 18 h. The mixture was cooled to room temperature and concentrated under reduced pressure. The residue was purified by silica gel column chromatography (DCM/MeOH (V/V) = 15/1) to give the title compound as a light yellow solid (0.29 g, yield 46.37%).
MS (ESI, pos.ion) *m/z*: 680.4 [M+H]+.

Step 11: (*R*)-4-(2-Amino-4-((1-hydroxy-2-methylhexan-2-yl)amino)pyrido[3,2-*d*]pyrimidin-7-yl)-1-methyl-5-((methyl(phenethyl)amino)methyl)pyridin-2(1*H*)-one

(*R*)-4-(2-((2,4-Dimethoxybenzyl)amino)-4-((1-hydroxy-2-methylhexan-2-yl)amino)pyrido[3,2-*d*]pyrimidin-7-yl)-1-methyl-5-((methyl(phenethyl)amino)methyl)pyridin-2(1*H*)-one (0.29 g, 0.43 mmol) was weighed and added to TFA (5 mL), and the mixture was stirred at room temperature for about 2 hours. The solvent was blown dry with $N_2$, and saturated sodium bicarbonate solution (10 mL) was added to the reaction system. The resulted mixture was extracted with dichloromethane (10 mL × 3). The organic phases were combined, washed with saturated brine (20 mL), dried over anhydrous sodium sulfate, and concentrated under reduced pressure. The residue was purified by silica gel column chromatography (DCM/MeOH (V/V) = 15/1) to obtain the title compound as a light yellow solid (0.11 g, yield 48.30%).

MS (ESI, pos.ion) *m/z:* 530.5 $[M+H]^+$;
$^1H$ NMR (400 MHz, DMSO-$d_6$): δ (ppm) 13.38 (s, 1H), 9.84 (s, 1H), 8.60 (s, 3H), 8.32 (s, 1H), 8.20 (s, 1H), 7.77 (s, 1H), 7.23 (dd, *J* = 17.3, 7.0 Hz, 3H), 7.09 (d, *J* = 6.9 Hz, 2H), 6.47 (s, 1H), 3.74 (d, *J* = 10.6 Hz, 2H), 3.51 (s, 4H), 3.06 (s, 2H), 2.79 (s, 2H), 2.53 (s, 3H), 2.08 - 1.86 (m, 2H), 1.47 (s, 3H), 1.25 (s, 4H), 0.85 (s, 3H);
HR-MS (ESI): 530.3245 $[M+H]^+$.

**Examples 2-8 can be obtained by referring to the synthesis method of Example 1. The structures and characterization data of Examples 2-8 are shown in Table 1 below.**

Table 1: Structure and characterization data of Examples 2-8

| Example No. | Structure of Example | Status, colors and characterization data |
|---|---|---|
| 2 | 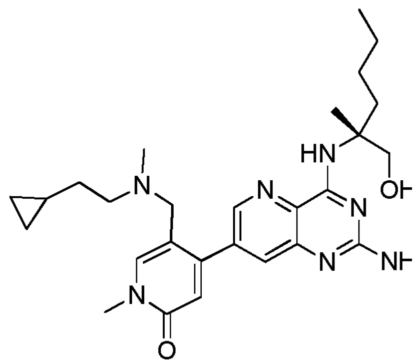 | White solid (yield 27.62%); MS (ESI, *pos.ion*) *m*/*z*: 494.5 $[M+H]^+$;<br><br>$^1H$ NMR (400 MHz, $CDCl_3$): δ (ppm) 8.28 (s, 1H), 7.64 (s, 1H), 7.49 (s, 1H), 7.37 (s, 1H), 6.49 (s, 1H), 5.51 (s, 2H), 3.84 - 3.73 (m, 3H), 3.64 (s, 2H), 3.61 (s, 3H), 2.35 - 2.27 (m, 2H), 2.07 (s, 3H), 1.99 - 1.89 (m, 1H), 1.79 (dd, *J* = 16.7, 8.7 Hz, 1H), 1.47 - 1.39 (m, 4H), 1.38 - 1.31 (m, 3H), 1.18 (dd, *J* = 14.6, 7.1 Hz, 2H), 0.91 (t, *J* = 6.9 Hz, 3H), 0.55 - 0.42 (m, 1H), 0.32 (q, *J* = 4.9 Hz, 2H), 0.09 (q, *J* = 4.7 Hz, 2H). |
| 3 | 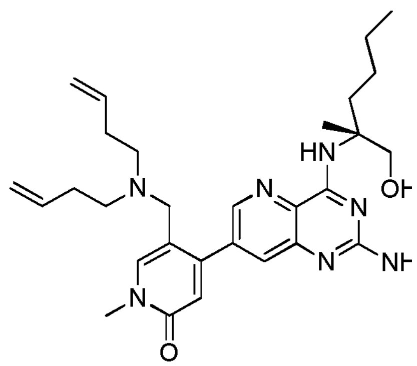 | White solid (yield 20.41%); MS (ESI, *pos.ion) m*/*z*: 520.6 $[M+H]^+$;<br><br>$^1H$ NMR (400 MHz, DMSO-$d_6$): δ (ppm) 8.62 (d, *J* = 1.4 Hz, 2H), 8.34 (s, 1H), 7.84 (s, 1H), 6.48 (s, 1H), 5.60 (d, *J* = 7.0 Hz, 2H), 5.00 (d, *J* = 14.9 Hz, 4H), 4.09 (s, 2H), 3.52 (s, 7H), 2.86 (s, 4H), 2.16 (s, 3H), 2.05 - 1.87 (m, 3H), 1.48 (s, 3H), 1.26 (s, 4H), 0.86 (t, *J* = 6.8 Hz, 3H). |

(continued)

| Example No. | Structure of Example | Status, colors and characterization data |
|---|---|---|
| 4 | 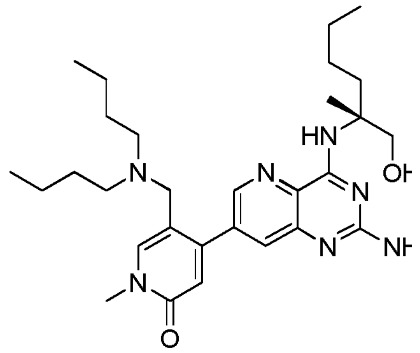 | Light yellow solid (yield 43.64%); MS (ESI, *pos.ion) m/z:* 524.3 $[M+H]^+$; $^1H$ NMR (400 MHz, DMSO-$d_6$): δ (ppm) 13.63 (s, 1H), 9.45 (s, 1H), 8.65 (d, *J* = 1.3 Hz, 3H), 8.37 (s, 1H), 8.23 (s, 1H), 7.85 (d, *J* = 1.3 Hz, 1H), 6.50 (s, 1H), 3.77 (d, *J* = 10.9 Hz, 1H), 3.54 - 3.44 (m, 4H), 2.90 - 2.69 (m, 4H), 2.09 - 1.88 (m, 2H), 1.49 (s, 3H), 1.36 - 1.06 (m, 12H), 0.86 (t, *J* = 6.8 Hz, 3H), 0.74 (t, *J* = 6.9 Hz, 6H). |
| 5 | 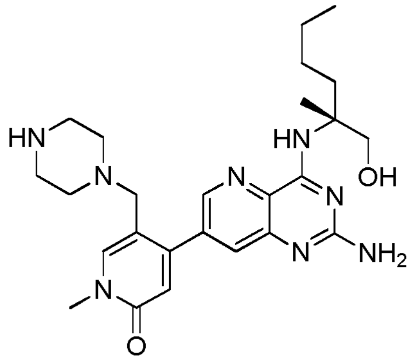 | Light yellow solid (yield 60.02%); MS (ESI, *pos.ion) m/z:* 481.2 $[M+H]^+$; $^1H$ NMR (400 MHz, $CD_3OD$): δ (ppm) 8.68 (d, *J* = 1.5 Hz, 1H), 8.01 (d, *J* = 1.5 Hz, 1H), 7.81 (s, 1H), 6.57 (s, 1H), 3.88 (dd, *J* = 100.9, 11.2 Hz, 2H), 3.66 (s, 3H), 3.37 (s, 2H), 3.15 - 3.14 (m, 4H), 2.63 (s, 4H), 2.21 - 1.86 (m, 2H), 1.58 (s, 3H), 1.39 - 1.35 (m, 4H), 0.95 (t, *J* = 6.7 Hz, 3H). |
| 6 | 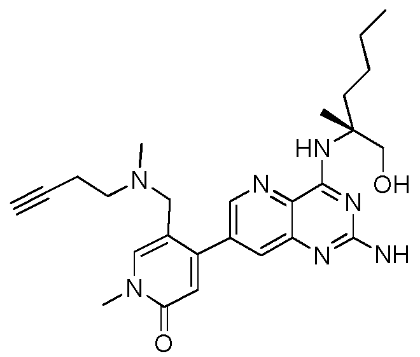 | Light yellow solid (yield 62.37%); MS (ESI, *pos.ion) m/z:* 478.5 $[M+H]^+$; $^1H$ NMR (400 MHz, $CD_3OD$): δ (ppm) 8.34 (s, 1H), 8.00 (s, 1H), 7.63 (s, 1H), 7.05 (s, 1H), 6.14 (s, 1H), 4.42 (s, 2H), 3.96 (d, *J* = 11.3 Hz, 1H), 3.83 - 3.46 (m, 6H), 3.11 - 2.85 (m, 5H), 2.14 - 1.85 (m, 2H), 1.52 (s, 3H), 1.46 - 1.31 (m, 4H), 0.92 (t, *J* = 6.9 Hz, 3H). |
| 7 | 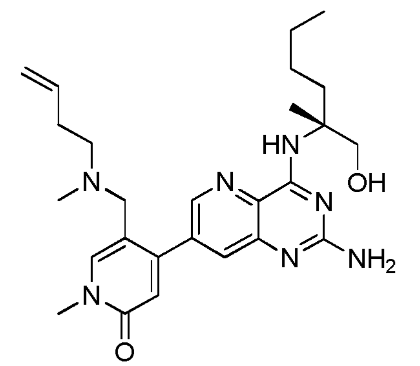 | Yellow solid (yield 47.39%); MS (ESI, *pos.ion) m/z*: 480.4 $[M+H]^+$; $^1H$ NMR (400 MHz, $CDCl_3$): δ (ppm) 8.26 (s, 1H), 7.60 (s, 1H), 7.39 (s, 1H), 7.31 (s, 1H), 6.50 (s, 1H), 5.67 - 5.57 (m, 1H), 5.20 (s, 2H), 4.92 (t, *J* = 13.0 Hz, 2H), 3.82 - 3.63 (m, 4H), 3.60 (s, 3H), 2.27 (t, *J* = 7.2 Hz, 2H), 2.09 - 2.01 (m, 5H), 1.99 - 1.89 (m, 1H), 1.81 - 1.74 (m, 1H), 1.50 - 1.29 (m, 8H), 0.91 (t, *J* = 6.9 Hz, 3H). |

(continued)

| Example No. | Structure of Example | Status, colors and characterization data |
|---|---|---|
| 8 | 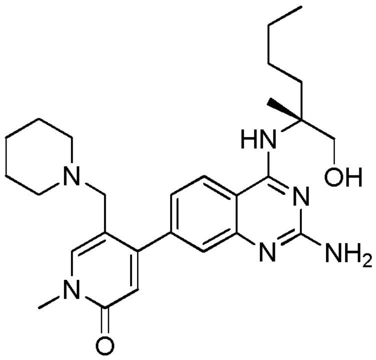 | Yellow solid (yield 31.34%); MS (ESI, *pos.ion) m/z*: 479.5 $[M+H]^+$;<br><br>$^1H$ NMR (400 MHz, DMSO-$d_6$): δ (ppm) 12.88 (s, 1H), 8.54 (d, *J* = 8.5 Hz, 1H), 8.13 (s, 1H), 7.97 (s, 1H), 7.39 (d, *J* = 8.7 Hz, 1H), 7.31 (s, 1H), 6.37 (s, 1H), 4.02 (s, 2H), 3.94 (d, *J* = 10.9 Hz, 1H), 3.58 (d, *J* = 10.9 Hz, 1H), 3.51 (s, 3H), 3.13 (d, *J* = 10.4 Hz, 2H), 2.52 (s, 1H), 2.17 (d, *J* = 8.9 Hz, 1H), 1.77 - 1.51 (m, 6H), 1.45 (s, 3H), 1.26 (s, 6H), 0.86 (t, *J* = 6.8 Hz, 3H). |

**Example** 9: **(*R*)-4-(2-Amino-4-((1-hydroxy-2-methylhexan-2-yl)amino)pyrido[3,2-*d*]pyrimidin-7-yl)-5-((but -3-en-1-yl(methyl)amino)methyl)pyridin-2(1*H*)-one**

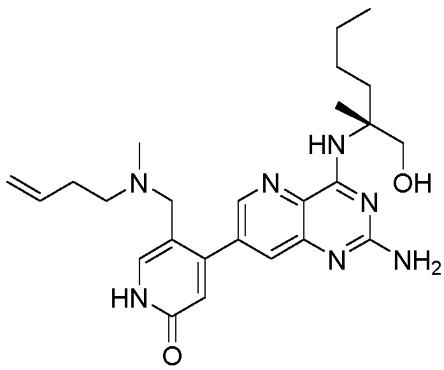

Step 1: 5-(Bromomethyl)-2-fluoro-4-iodopyridine

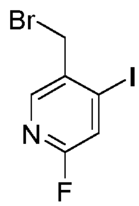

2-Fluoro-4-iodo-5-methylpyridine (20.00 g, 84.38 mmol), N-bromosuccinimide (16.52 g, 92.82 mmol) and 2,2'-azobis(2-methylpropionitrile) (1.39 g, 8.44 mmol) were weighed and mixed in carbon tetrachloride (200 mL). Under $N_2$, The resulted mixture was refluxed in an oil bath at 80 °C for about 14 hours. The stirring was stopped and saturated sodium thiosulfate solution (30 mL) was added to the reaction system, the mixture was extracted with dichloromethane (100 mL × 3). The organic phases were combined, washed with saturated brine (50 mL), dried over anhydrous sodium sulfate, and concentrated under reduced pressure. The residue was purified by silica gel column chromatography (PE/EA (V/V) = 20/1) to obtain the title compound as a white solid (19.56 g, yield 73.38%).
$^1H$NMR (400MHz, $CDCl_3$): δ (ppm) 8.25(s, 1H), 7.51 (d, *J* = 3.1Hz, 1H), 4.58 (s, 2H).

Step 2: 1-(6-Fluoro-4-iodopyridin-3-yl)-N-methylmethanamine

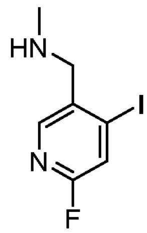

5-(Bromomethyl)-2-fluoro-4-iodopyridine (6.01 g, 19.02 mmol), aminomethane (5.97 g, 192.3 mmol) and methanol (120 mL) were added to the flask, followed by stirring for about 1.5 hours at room temperature. After the reaction was completed, the solvent was evaporated under reduced pressure, saturated aqueous sodium bicarbonate solution (200 mL) was added to the residue, and then the mixture was extracted with dichloromethane (200 mL × 3), the organic phase was dried over anhydrous sodium sulfate, and evaporated under reduced pressure to give the title compound as a yellow oil (5.04 g, yield 99.60%).
MS (ESI, *pos. ion*) *m*/*z*: 266.9 $[M+H]^+$.

Step 3: *N*-((6-Fluoro-4-iodopyridin-3-yl)methyl)-*N*-methylbut-3-en-1-amine

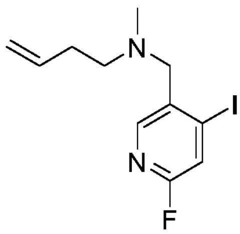

1-(6-Fluoro-4-iodopyridin-3-yl)-*N*-methylmethanamine (11.28 g, 42.40 mmol), 4-bromo-1-butene (22.90 g, 169.6 mmol), sodium iodide (0.64 g, 4.27 mmol) and potassium carbonate (29.30 g, 212.04 mmol) were weighed and mixed in acetonitrile (30 mL), and heated and stirred in an oil bath at 70°C for about 17 hours under $N_2$. The stirring was stopped and the mixture was concentrated under reduced pressure to obtain the title compound as a colorless oily liquid (11.00 g, yield 81.04%).
MS (ESI, *pos.ion*) *m*/*z:* 321.0 $[M+H]^+$.

Step 4: 5-((But-3-en-1-yl(methyl)amino)methyl)-4-iodopyridin-2(1H)-one

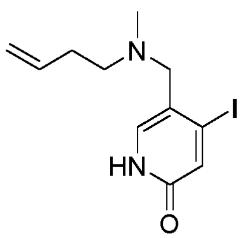

*N*-((6-Fluoro-4-iodopyridin-3-yl)methyl)-*N*-methylbut-3-en-1-amine (10.46 g, 32.68 mmol), potassium tert-butyrate (11.00 g, 98.04 mmol) and tert-butanol (30 mL) were added into a single-necked bottle respectively. Under $N_2$, the resulted mixture was placed in an oil bath at 100 °C and heated to reflux for about 27 hours, then adjusted the pH to 5-6 with 4 M solution of hydrochloric acid in 1,4-dioxane, and the mixture wasconcentrated under reduced pressure. The resulted residue was purified by silica gel column chromatography (DCM/MeOH (V/V) = 15/1) to give the title compound as a white

solid (6.20 g, yield 59.63%).
MS (ESI, *pos.ion*) *m*/*z:* 319.1 [M+H]$^+$.

Step 5: (*R*)-4-(2-Amino-4-((1-hydroxy-2-methylhexan-2-yl)amino)pyrido[3,2-*d*]pyrimidin-7-yl)-5-((but-3-e n-1-yl(methyl) amino)methyl)pyridin-2(1*H*)-one

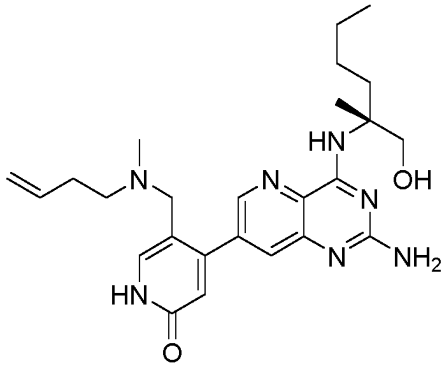

5-((But-3-en-1-yl(methyl)amino)methyl)-4-iodopyridin-2(1H)-one and (*R*)-(2-((2,4-dimethoxybenzyl)amino)-4-((1-hydroxy-2-methylhexan-2-yl)amino)pyrido[3,2-*d*]pyri midin-7-yl)boronic acid were used as starting materials and the title compound was obtained as a white solid by referring to steps 10 and 11 of Example 1 in a yield of 51%.

MS (ESI, *pos.ion*) *m*/*z*: 466.1 [M+H]$^+$;
$^1$H NMR(400 MHz, $CDCl_3$): δ (ppm) 8.35 (s, 1H), 7.70 (s, 1H), 7.49 (s, 1H), 7.37 (s, 1H), 6.53 (s, 1H), 5.73 - 5.63 (m, 1H), 5.42 (s, 2H), 4.92 (t, *J* = 14.1 Hz, 2H), 3.88 - 3.79 (m, 2H), 3.16 (s, 2H), 2.29 (t, *J* = 7.3 Hz, 2H), 2.10 - 1.96 (m, 6H), 1.81 (t, *J* = 10.6 Hz, 1H), 1.48 - 1.31 (m, 8H), 0.93 (d, *J* = 7.0 Hz, 3H).

**Examples 10-27 can be obtained by referring to the synthesis method of Example 9. The structures and characterization data of Examples 10-27 are shown in Table 2 below.**

| Example No. | Structure of Example | Status colors and characterization data |
|---|---|---|
| **10** | 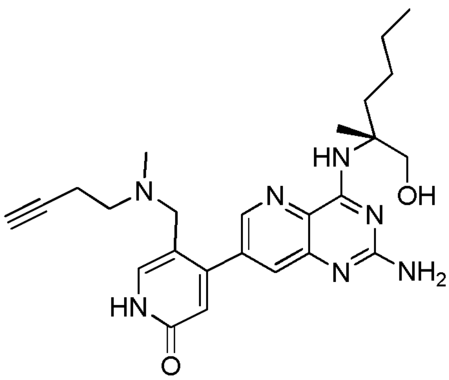 | Yellow solid (yield 31.56%); MS (ESI, *pos.ion*) *m*/*z*: 464.3 [M+H]$^+$;<br>Note: The compound was prepared by referring to the synthetic method of Example 9, wherein the reaction temperature of step 4 was changed to room temperature reaction. |

(continued)

| Example No. | Structure of Example | Status colors and characterization data |
|---|---|---|
| **11** | 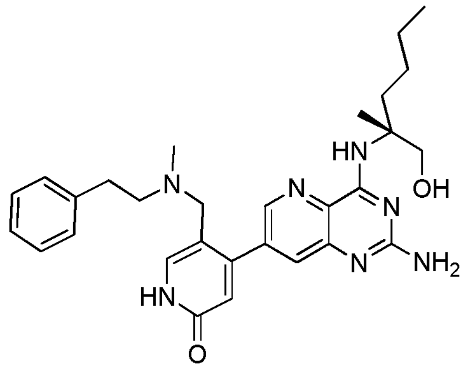 | Yellow solid (yield 67.88%); MS (ESI, *pos.ion*) *m*/*z*: 516.4 $[M+H]^+$; $^1H$ NMR (400 MHz, $CDCl_3$): δ (ppm) 8.37 (s, 1H), 7.88 (s, 1H), 7.71 (s, 1H), 7.43 (s, 1H), 7.21 (t, *J* = 7.3 Hz, 2H), 7.13 (t, *J* = 7.3 Hz, 1H), 7.05 (d, *J* = 7.3 Hz, 2H), 6.39 (s, 1H), 3.90 (d, *J* = 11.7 Hz, 1H), 3.84 - 3.75 (m, 2H), 3.22 |
| | | (s, 2H), 2.59 (d, *J* = 7.4 Hz, 2H), 2.52 (d, *J* = 7.5 Hz, 2H), 2.17 (s, 3H), 2.01 - 1.93 (m, 1H), 1.88 - 1.77 (m, 1H), 1.47 (s, 3H), 1.37 - 1.30 (m, 4H), 1.25 (s, 3H), 0.91 (t, *J* = 6.9 Hz, 3H). |
| **12** | 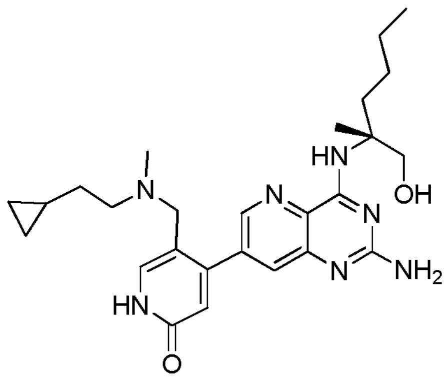 | Yellow solid (yield 45.87%); MS (ESI, *pos.ion*) *m*/*z*: 480.5 $[M+H]^+$; $^1H$ NMR (400 MHz, $CDCl_3$): δ (ppm) 8.36 (s, 1H), 7.73 (s, 1H), 7.53 (s, 1H), 7.42 (s, 1H), 6.50 (s, 1H), 5.84 (s, 2H), 3.81 (q, *J* = 11.9 Hz, 2H), 3.75 - 3.58 (m, 3H), 2.37 - 2.25 (m, 2H), 2.06 (s, 3H), 1.99 - 1.92 (m, 1H), 1.79 (t, *J* = 10.7 Hz, 1H), 1.43 (s, 3H), 1.40 - 1.31 (m, 4H), 1.18 - 112 (m, 2H), 0.92 (t, *J* = 6.9 Hz, 3H), 0.47 - 0.46 (m, 1H), 0.31 (d, *J* = 7.5 Hz, 2H), 0.11 (d, *J* = 4.4 Hz, 2H). |
| **13** | 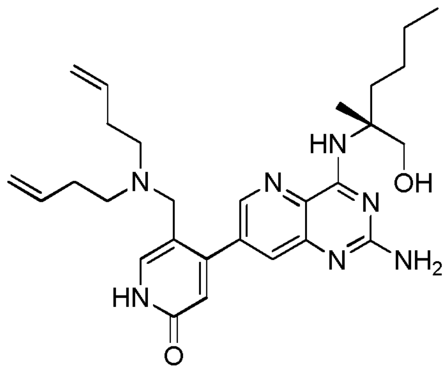 | Yellow solid (yield 29.66%); MS (ESI, *pos.ion*) *m*/*z*: 506.5 $[M+H]^+$; $^1H$ NMR (400 MHz, DMSO-$d_6$): δ (ppm) 8.63 (s, 2H), 8.34 (s, 2H), 7.83 (s, 2H), 6.41 (s, 1H), 5.60 (s, 2H), 5.03 (s, 4H), 4.15 (s, 2H), 3.40 - 3.25 (m, 3H), 2.89 (s, 3H), 2.18 (s, 3H), 2.05 - 1.88 (m, 2H), 1.49 (s, 3H), 1.26 (s, 4H), 0.86 (t, *J* = 6.8 Hz, 3H). |
| **14** | 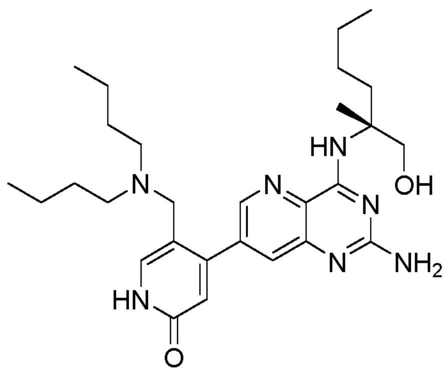 | Yellow solid (yield 71.78%); MS (ESI, *pos.ion*) *m*/*z*: 510.2 $[M+H]^+$; $^1H$ NMR (400 MHz, $CDCl_3$): δ (ppm) 8.31 (s, 1H), 7.62 (s, 1H), 7.56 (s, 1H), 7.37 (s, 1H), 6.51 (s, 1H), 5.36 (s, 2H), 3.81 (d, *J* = 9.4 Hz, 2H), 3.24 (s, 2H), 2.24 (s, 4H), 1.98 (t, *J* = 11.8 Hz, 1H), 1.82 (t, *J* = 12.5 Hz, 1H), 1.44 (s, 3H), 1.39 (d, *J* = 6.7 Hz, 2H), 1.27 (s, 3H), 1.14 (s, 8H), 0.94 (t, *J* = 6.6 Hz, 4H), 0.79 (d, *J* = 6.6 Hz, 6H). |

(continued)

| Example No. | Structure of Example | Status colors and characterization data |
|---|---|---|
| **15** | 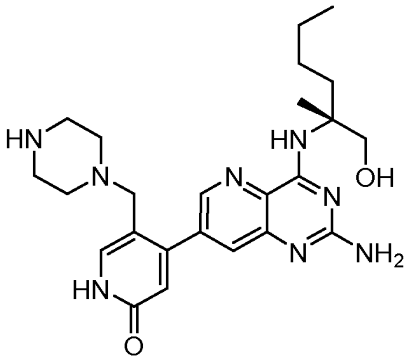 | Yellow solid (yield 99.37%); MS (ESI, *pos.ion*) *m*/*z*: 467.5 $[M+H]^+$; $^1H$ NMR (400 MHz, $CD_3OD$): δ (ppm) 8.68 (s, 1H), 8.02 (s, 1H), 7.61 (s, 1H), 6.57 (s, 1H), 4.01 (d, *J* = 11.2, 1H), 3.76 (d, *J* = 11.2, 1H), 3.41 (s, 2H), 3.16 (s, 4H), 2.68 - 2.65 (m, 4H), 2.25 - 1.84 (m, 2H), 1.58 (s, 3H), 1.49 - 1.33 (m, 4H), 0.95 (t, *J* = 6.6 Hz, 3H). |
| **16** | 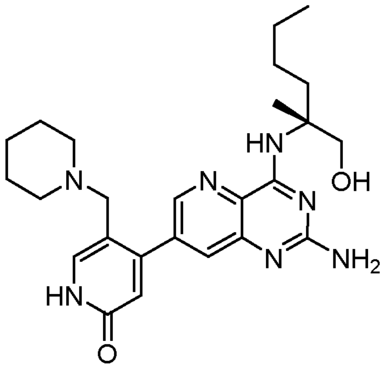 | Yellow solid (yield 16.52%); MS (ESI, *pos.ion*) *m*/*z*: 466.2 $[M+H]^+$; $^1H$ NMR (400 MHz, $CDCl_3$): δ (ppm) 8.32 (s, 1H), 7.70 (s, 1H), 7.62 (s, 1H), 7.26 (s, 1H), 6.43 (s, 1H), 3.81 (d, *J* = 11.8 Hz, 1H), 3.77 - 3.71 (m, 1H), 3.09 (dd, *J* = 14.6, 7.3 Hz, 2H), 2.33 (s, 4H), 1.51 (s, 4H), 1.42 (s, 3H), 1.33 (t, *J* = 7.3 Hz, 8H), 0.88 (t, *J* = 6.8 Hz, 3H). |
| **17** | 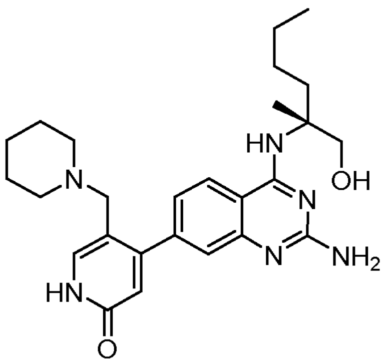 | White solid (yield 55.08%); MS (ESI, *pos.ion) m*/*z*: 465.4 $[M+H]^+$; $^1H$ NMR (400 MHz, DMSO-$d_6$): δ (ppm) 8.52 (d, *J* = 8.3 Hz, 1H), 7.97 (s, 1H), 7.85 (s, 1H), 7.45 - 7.24 (m, 2H), 6.29 (s, 1H), 4.03 (s, 3H), 3.94 (d, *J* = 10.8 Hz, 2H), 3.57 (d, *J* = 10.9 Hz, 3H), 3.13 (d, *J* = 9.0 Hz, 2H), 2.14 (s, 1H), 1.71 (d, *J* = 10.6 Hz, 1H), 1.67 - 1.47 (m, 5H), 1.44 (s, 3H), 1.25 (s, 5H), 0.84 (t, *J* = 6.2 Hz, 3H). |
| **18** | 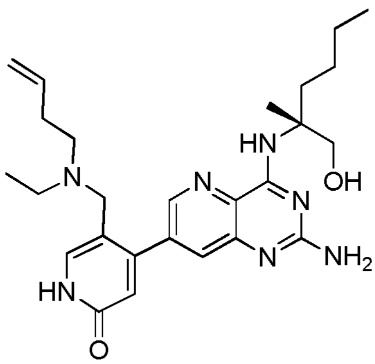 | Yellow solid (yield 31.27%); MS (ESI, *pos.ion*) *m*/*z*: 480.3 $[M+H]^+$; $^1H$ NMR (400 MHz, DMSO-$d_6$): δ (ppm) 8.62 (s, 2H), 8.33 (s, 1H), 8.02 (s, 1H), 7.83 (s, 1H), 6.38 (s, 1H), 5.68 - 5.58 (m, 1H), 5.10 - 4.95 (m, 2H), 4.06 (s, 2H), 3.48 (s, 2H), 2.85 (s, 4H), 2.21 (s, 2H), 2.08 - 1.86 (m, 2H), 1.47 (s, 3H), 1.26 (s, 4H), 0.98 (t, *J* = 6.9 Hz, 3H), 0.85 (t, *J* = 6.7 Hz, 3H). |

(continued)

| Example No. | Structure of Example | Status colors and characterization data |
|---|---|---|
| **19** | 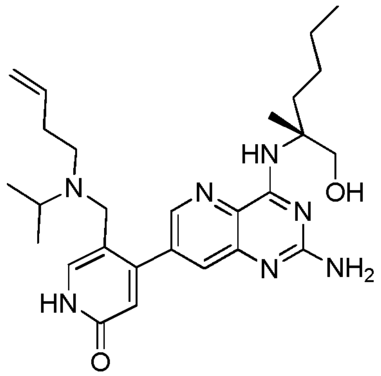 | Yellow solid (yield 17.87%); MS (ESI, *pos.ion*) *m/z*: 494.6 [M+H]$^+$; $^1$H NMR (400 MHz, DMSO-$d_6$): δ (ppm) 9.35 (s, 1H), 8.64 (s, 2H), 8.33 (s, 1H), 7.99 (s, 1H), 7.87 (s, 1H), 6.41 (s, 1H), 5.66 (d, *J* = 6.9 Hz, 1H), 5.05 (d, *J* = 11.2 Hz, 2H), 4.08 (s, 2H), 3.77 (d, *J* = 10.6 Hz, 2H), 2.85 (d, *J* = 59.4 Hz, 2H), 2.29 (s, 2H), 1.96 (d, *J* = 7.8 Hz, 2H), 1.49 (s, 3H), 1.27 (s, 4H), 1.14 (s, 3H), 0.87 - 0.81 (m, 6H). |
| **20** | 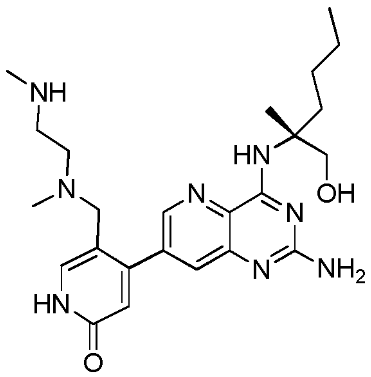 | Yellow solid (yield 66.23%); MS (ESI, *pos.ion*) *m/z*: 469.3 [M+H]$^+$; $^1$H NMR (400 MHz, DMSO-$d_6$): δ (ppm) 13.46 (s, 1H), 8.79 (s, 1H), 8.62 (d, *J* = 1.3 Hz, 3H), 8.32 (s, 1H), 7.87 (s, 1H), 7.78 (s, 1H), 6.38 (s, 1H), 3.78 (s, 1H), 3.51 (d, *J* = 10.9 Hz, 1H), 3.10 (s, 2H), 2.89 (s, 1H), 2.52 (d, *J* = 7.4 Hz, 4H), 2.32 (s, 3H), 2.07 - 1.87 (m, 2H), 1.49 (s, 3H), 1.30 - 1.25 (m, 4H), 0.87 (t, *J* = 6.7 Hz, 3H). |
| **21** | 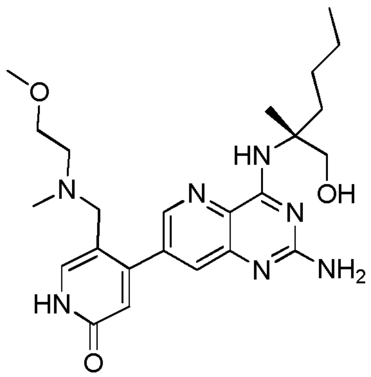 | White solid (yield 85.18%); MS (ESI, *pos.ion*) *m/z*: 470.3 [M+H]$^+$; $^1$H NMR (400 MHz, DMSO-$d_6$): δ (ppm) 13.51 (s, 1H), 12.31 (s, 1H), 9.39 (s, 1H), 8.62 (d, *J* = 1.6 Hz, 3H), 8.36 (s, 1H), 7.88 (s, 1H), 7.79 (d, *J* = 1.5 Hz, 1H), 6.40 (s, 1H), 4.20 (s, 1H), 4.03 (s, 2H), 3.77 (d, *J* = 10.9 Hz, 2H), 3.52 - 3.47 (m, 3H), 3.13 (s, 4H), 2.99 (d, *J* = 6.1 Hz, 1H), 1.97 (td, *J* = 15.8, 7.5 Hz, 2H), 1.49 (s, 3H), 1.28 (s, 4H), 0.87 (t, *J* = 6.8 Hz, 3H). |
| **22** | 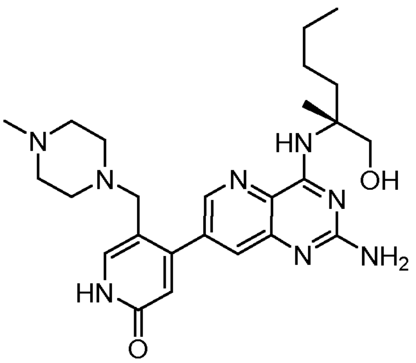 | Yellow solid (yield 98.25%); MS (ESI, *pos.ion) m/z*: 481.3 [M+H]$^+$; $^1$H NMR (400 MHz, DMSO-$d_6$): δ (ppm) 9.96 (s, 1H), 8.81 - 8.44 (m, 3H), 8.30 (s, 1H), 8.00 (d, *J* = 1.3 Hz, 1H), 7.52 (s, 1H), 6.36 (s, 1H), 3.77 (d, *J* = 10.9 Hz, 1H), 3.52 (d, *J* = 10.9 Hz, 1H), 3.36 (s, 2H), 3.19 (s, 2H), 3.07 - 2.79 (m, 4H), 2.75 (s, 3H), 2.26 (s, 2H), 1.97 (s, 2H), 1.49 (s, 3H), 1.28 - 1.24 (m, 4H), 0.88 (t, *J* = 6.7 Hz, 3H). |

(continued)

| Example No. | Structure of Example | Status colors and characterization data |
|---|---|---|
| **23** | 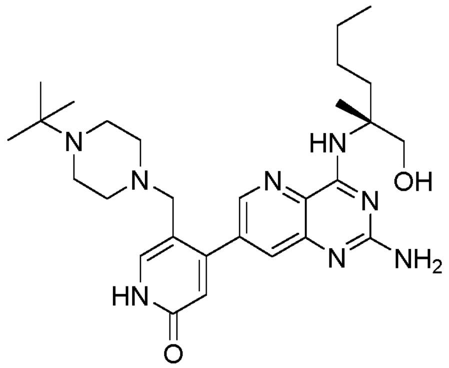 | Yellow solid (yield 85.23%); MS (ESI, *pos.ion) m/z*: 523.3 [M+H]+; $^1$H NMR (400 MHz, $CD_3OD$): δ (ppm) 8.69 (d, *J* = 1.4 Hz, 1H), 7.97 (d, *J* = 1.3 Hz, 1H), 7.58 (s, 1H), 6.57 (s, 1H), 4.00 (d, *J* = 11.2 Hz, 1H), 3.75 (d, *J* = 11.2 Hz, 1H), 3.48 (d, *J* = 12.0 Hz, 2H), 3.37 (s, 2H), 2.98 - 2.81 (m, 4H), 2.38 (t, *J* = 11.5 Hz, 2H), 2.19 - 2.09 (m, 1H), 2.03 - 1.93 (m, 1H), 1.59 (s, 3H), 1.39 (s, 13H), 0.96 (t, *J* = 6.7 Hz, 3H). |
| **24** | 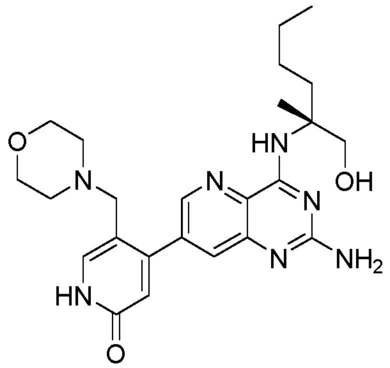 | White solid (yield 69.81%); MS (ESI, *pos.ion*) *m/z*: 468.1 [M+H]+; $^1$H NMR (400 MHz, DMSO-$d_6$): δ(ppm) 13.44 (s, 1H), 12.29 (s, 1H), 10.06 (s, 1H), 8.61 (d, *J* = 1.6 Hz, 3H), 8.35 (s, 1H), 7.81 (s, 2H), 6.38 (s, 1H), 4.01 (s, 2H), 3.77 (d, *J* = 10.9 Hz, 3H), 3.63 (s, 1H), 3.52 (d, *J* = 10.9 Hz, 2H), 3.11 (s, 1H), 2.76 (s, 2H), 2.12 - 1.87 (m, 2H), 1.49 (s, 3H), 1.28 (d, *J* = 3.7 Hz, 4H), 0.88 (t, *J* = 6.8 Hz, 3H). |
| **25** | 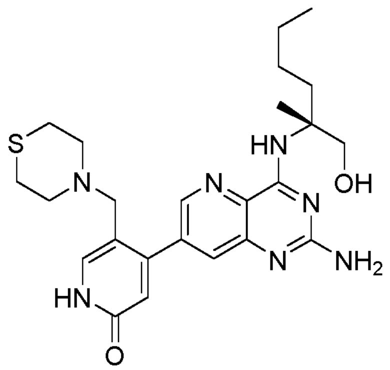 | White solid (yield 83.95%); MS (ESI, *pos.ion*) *m/z*: 484.1 [M+H]+; $^1$H NMR (400 MHz, DMSO-$d_6$): δ (ppm) 13.40 (s, 1H), 12.30 (s, 1H), 9.82 (s, 1H), 8.61 - 8.53 (m, 3H), 8.36 (s, 1H), 7.84 (s, 1H), 7.79 (s, 1H), 6.38 (s, 1H), 3.77 (d, *J* = 10.9 Hz, 1H), 3.52 (d, *J* = 10.9 Hz, 1H), 3.41 (s, 1H), 2.76 (s, 6H), 2.04 - 1.92 (m, 2H), 1.50 (s, 3H), 1.28 (d, *J* = 3.7 Hz, 4H), 0.88 (t, *J* = 6.8 Hz, 3H). |
| **26** | 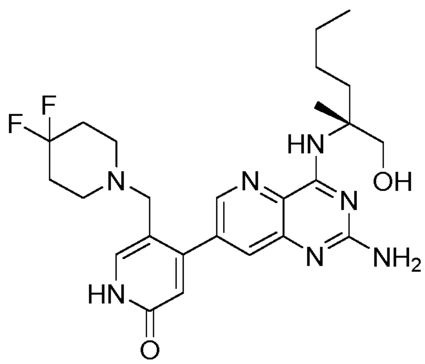 | White solid (yield 74.21%); MS (ESI, *pos.ion*) *m/z*: 502.3 [M+H]+; $^1$H NMR (400 MHz, DMSO-$d_6$): δ (ppm) 13.35 (s, 1H), 12.44 (s, 1H), 9.82 (s, 1H), 8.61 (s, 2H), 8.52 (s, 1H), 8.35 (s, 1H), 7.85 (s, 1H), 7.78 (s, 1H), 6.38 (s, 1H), 4.15 - 3.93 (m, 4H), 3.77 (d, *J* = 10.8 Hz, 1H), 3.52 (d, *J* = 10.9 Hz, 1H), 3.06 - 2.67 (m, 3H), 2.10 (s, 3H), 2.04 - 1.91 (m, 2H), 1.49 (s, 3H), 1.36 - 1.18 (m, 4H), 0.87 (t, *J* = 6.6 Hz, 3H). |

(continued)

| Example No. | Structure of Example | Status colors and characterization data |
|---|---|---|
| **27** | 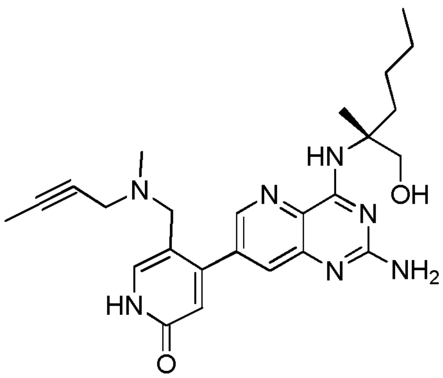 | Yellow solid (yield 26.40%); MS (ESI, *pos.ion*) *m/z*: 464.1 $[M+H]^+$; $^1H$ NMR (400 MHz, $CD_3OD$): δ (ppm) 8.25 (s, 1H), 7.70 (s, 1H), 7.38 (s, 1H), 5.73 (s, 1H), 3.96 (d, *J* = 11.3 Hz, 1H), 3.86 - 3.68 (m, 3H), 3.62 (s, 2H), 2.63 (s, 3H), 2.26 (s, 3H), 2.16 - 1.85 (m, 2H), 1.53 (s, 3H), 1.37 - 1.31 (m, 4H), 0.94 (t, *J* = 6.4 Hz, 3H). |

**Example 28: (*R*)-4-(2-Amino-4-((1-hydroxy-2-methylhexan-2-yl)amino)pyrido[3,2-*d*]pyrimidin-7-yl)-5-((but yl(methyl)amino)methyl)pyridin-2(1*H*)-one**

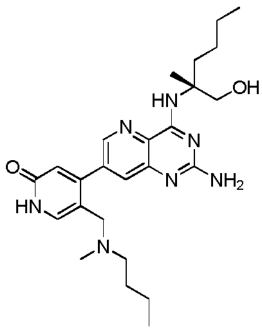

Step 1: (*R*)-5-((But-3-en-1-yl(methyl)amino)methyl)-4-(2-((2,4-dimethoxybenzyl)amino)-4-((1-hydroxy-2-methylhexan-2-yl)amino)pyrido[3,2-*d*]pyrimidin-7-yl)pyridin-2(1*H*)-one

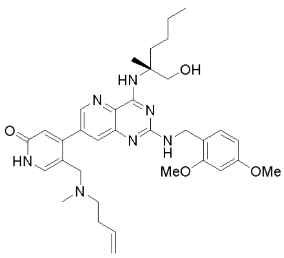

The compound was prepared using 5-((but-3-en-1-yl(methyl)amino)methyl)-4-iodopyridin-2(1H)-one and (*R*)-(2-((2,4-dimethoxybenzyl)amino)-4-((1-hydroxy-2-methylhexan-2-yl)amino)pyrido[3,2-*d*]pyri midin-7-yl)boronic acid as raw material, according to the synthetic method of step 10 of example 1.

Step 2: (*R*)-5-(Butyl(methyl)amino)methyl)-4-(2-((2,4-dimethoxybenzyl)amino)-4-((1-hydroxy-2-methylh exan-2-yl)amino)pyrido[3,2-*d*]pyrimidin-7-yl)pyridin-2(1*H*)-one

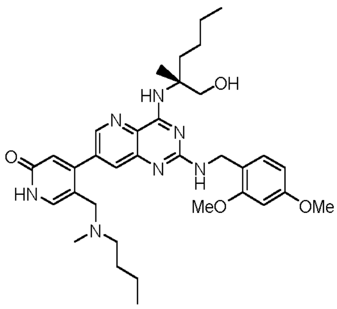

(*R*)-5-((But-3-en-1-yl(methyl)amino)methyl)-4-(2-((2,4-dimethoxybenzyl)amino)-4-((1-hydroxy-2-methylhexan-2-yl)amino)pyrido[3,2-*d*]pyrimidin-7-yl)pyridin-2(1*H*)-one (0.10 g, 0.16 mmol) and 10% palladium/carbon (20 mg, 0.16 mmol) were weighed and dissolved in THF/MeOH (V/V = 1/1) (20 mL), and the mixture was placed in a hydrogen balloon atmosphere and reacted for about 20 hours, then filtered to obtain the title compound as a light yellow solid (99 mg, yield 100%).
MS (ESI, *pos.ion*) *m*/*z*: 618.2 $[M+H]^+$.

Step 3: (*R*)-4-(2-Amino-4-((1-hydroxy-2-methylhexan-2-yl)amino)pyrido[3,2-*d*]pyrimidin-7-yl)-5-((butyl( methyl)amino) methyl)pyridin-2(1*H*)-one

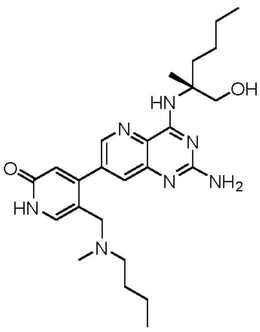

(*R*)-5-((Butyl(methyl)amino)methyl)-4-(2-((2,4-dimethoxybenzyl)amino)-4-((1-hy droxy-2-methylhexan-2-yl) amino)pyrido[3,2-*d*]pyrimidin-7-yl)pyridin-2(1*H*)-one (99 mg, 0.16 mmol) was weighed and added to TFA (15 mL), and the mixture was stirred at room temperature for about 2 hours. The stirring was stopped and the solvent was blown dry with $N_2$, and saturated sodium bicarbonate solution (10 mL) was added to the reaction system. The mixture was extracted with dichloromethane (10 mL × 3). The organic phases were combined, washed with saturated brine (10 mL), dried over anhydrous sodium sulfate, and concentrated under reduced pressure. The residue was purified by silica gel column chromatography (DCM/MeOH (V/V) = 15/1) to obtain the title compound as a light yellow solid (0.060 g, yield 80.20%).

MS (ESI, *pos.ion*) *m*/*z*: 468.2 $[M+H]^+$;
$^1H$ NMR (400 MHz, DMSO-$d_6$): δ (ppm) 13.31 (s, 1H), 12.31 (s, 1H), 9.23 (s, 1H), 8.63 (s, 2H), 8.47 (s, 1H), 8.37 (s, 1H), 7.88 (s, 1H), 7.82 (s, 1H), 6.42 (s, 1H), 4.19 (d, *J* = 13.4 Hz, 1H), 3.95 (dd, *J* = 13.7, 6.0 Hz, 1H), 3.76 (d, *J* = 10.6 Hz, 1H), 3.50 (d, *J* = 10.8 Hz, 1H), 2.86 (s, 1H), 2.72 (s, 1H), 2.47 (d, *J* = 3.8 Hz, 2H), 2.08 - 1.88 (m, 2H), 1.49 (s, 3H), 1.28 (s, 6H), 1.18 - 1.08 (m, 2H), 0.87 (t, *J* = 6.8 Hz, 3H), 0.75 (t, *J* = 7.2 Hz, 3H).

**Example 29: (*R*)-4-(2-Amino-4-((1-hydroxy-2-methylhexan-2-yl)amino)quinazolin-7-yl)-5-((methyl(pheneth yl) amino)methyl)pyridin-2(1H)-one**

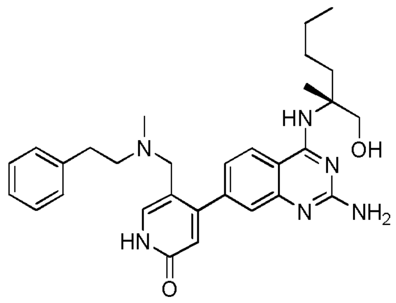

Step 1: (*R*)-(2-((2,4-Dimethoxybenzyl)amino)-4-((1-hydroxy-2-methylhexan-2-yl)amino)quinazolin-7-yl)b oronic acid

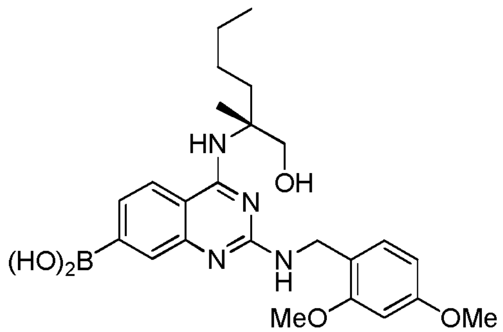

(*R*)-2-Amino-2-methylhexan-1-ol and 7-bromo-2,4-dichloroquinazoline were used as raw materials, according to the synthesis method of step 3 to step 5 of example 1, and the title compound can be obtained as a brown-black solid with a total yield of 42%.
MS (ESI, *pos.ion*) *m*/*z*: 469.1 $[M+H]^+$.

Step 2: (*R*)-4-(2-Amino-4-((1-hydroxy-2-methylhexan-2-yl)amino)quinazolin-7-yl)-5-((methyl(phenethyl)a mino)methyl) pyridin-2(1*H*)-one

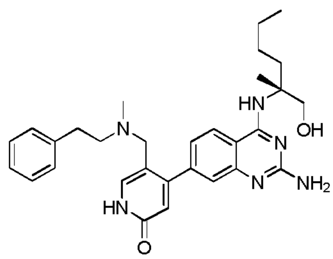

(*R*)-(2-((2,4-Dimethoxybenzyl)amino)-4-((1-hydroxy-2-methylhexan-2-yl)amino) quinazolin-7-yl)boronic acid and 4-chloro-5-((methyl(phenethyl)amino)methyl)pyridin-2(1*H*)-one were used as raw materials, according to the synthesis method of step 10 to step 11 of example 1, and the title compound can be obtained as a white solid with a total yield of 25%.

MS (ESI, *pos.ion*) *m*/*z*: 515.1 $[M+H]^+$;
$^1$H NMR(400 MHz, DMSO-$d_6$) δ (ppm): 12.82 (s, 1H), 12.28 (s, 1H), 9.45 (s, 1H), 8.53 (d, *J* = 8.4 Hz, 1H), 8.26 (s, 2H), 7.96 (s, 1H), 7.88 (s, 1H), 7.41(d, *J* = 8.4 Hz, 1H), 7.35 - 7.17 (m, 4H), 7.06 (d, *J* = 7.1 Hz, 2H), 6.33 (s, 1H), 4.89 (s, 1H), 4.29 (s, 1H), 4.05 (s, 1H), 3.95 (d, *J* = 10.9 Hz, 1H), 3.58 (d, *J* = 10.8 Hz, 1H), 2.99 (s, 2H), 2.73 (s, 2H), 2.16 (s, 1H), 1.81 - 1.68 (m, 1H), 1.45 (s, 3H), 1.27 (d, *J* = 7.1 Hz, 4H), 0.85 (t, *J* = 6.8 Hz, 3H).

**Examples 30-35 can be obtained by referring to the synthesis method of Example 29. The characterization data of Examples 30-35 are shown in Table 3 below.**

| Example No. | Structure of Example | Status, colors and characterization data |
|---|---|---|
| 30 | 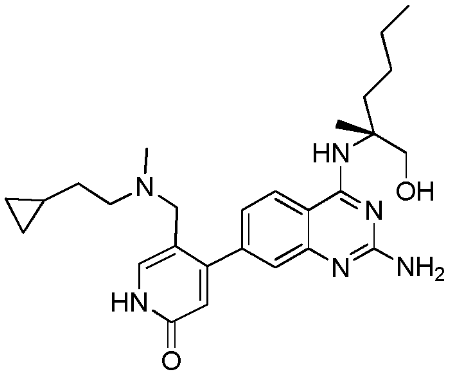 | Yellow solid (yield 10.20%); MS (ESI, *pos.ion) m/z:479.3* [M+H]+. |
| 31 | 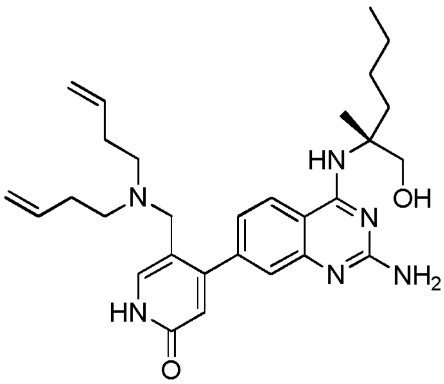 | White solid (yield 27.97%); MS (ESI, *pos.ion*) *m/z*: 505.6 [M+H]+; $^1$H NMR (400 MHz, DMSO-$d_6$): δ (ppm) 8.59 (s, 1H), 8.02 (s, 2H), 7.45 - 7.30 (m, 2H), 6.31 (s, 1H), 5.65 - 5.46 (m, 2H), 4.96 (d, *J* = 16.1 Hz, 4H), 4.14 (s, 2H), 3.54 (d, *J* = 11.0 Hz, 2H), 2.84 (s, 4H), 2.14 (s, 4H), 1.80 - 1.68 (m, 1H), 1.44 (s, 3H), 1.32 - 1.13 (m, 5H), 0.84 (t, *J* = 6.4 Hz, 3H). |
| 32 | 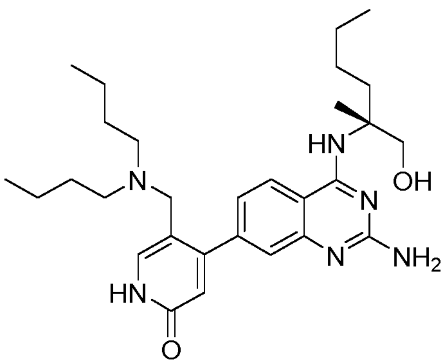 | White solid (yield 32.76%); MS (ESI, *pos.ion*) *m/z*:509.2 [M+H]+; $^1$H NMR (400 MHz, DMSO-$d_6$): δ (ppm) 13.26 (s, 1H), 9.19 (s, 1H), 8.60 (d, *J* = 8.4 Hz, 1H), 8.37 (s, 2H), 8.03 (s, 1H), 7.89 (s, 1H), 7.43 (d, *J* = 8.3 Hz, 1H), 7.37 (s, 1H), 6.35 (s, 1H), 4.11 (s, 2H), 3.97 (d, *J* = 10.7 Hz, 1H), 3.59 - 3.56 (m, 2H),2.81 - 2.70 (m, 4H), 2.24 - 2.11 (m, 1H), 1.73 (t, *J* = 10.5 Hz, 1H), 1.45 (s, 3H), 1.31 - 1.17 (m, 6H), 1.16 - 1.01 (m, 6H), 0.84 (t, *J* = 6.6 Hz, 3H), 0.71 (d, *J* = 3.3 Hz, 6H). |
| 33 | 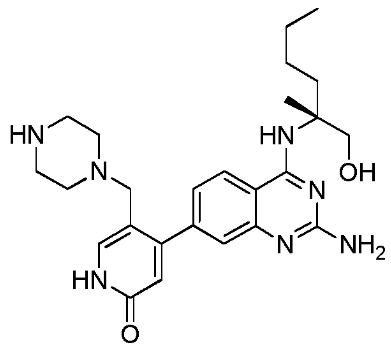 | White solid (yield 63.17%); MS (ESI, *pos.ion*) *m/z:466.3* [M+H]+; $^1$H NMR(400 MHz, $CD_3OD$): δ (ppm) 8.32 (d, *J* = 8.1 Hz, 1H), 7.61 (s, 1H), 7.49 - 7.47 (m, 2H), 6.51 (s, 1H), 4.18 (d, *J* = 11.3 Hz, 1H), 3.73 (d, *J* = 11.2 Hz, 1H), 3.47 (s, 2H), 3.14 (s, 4H), 2.66 (s, 4H), 2.27 (t, *J* = 11.1 Hz, 1H), 1.84 (t, *J* = 11.1 Hz, 1H), 1.55 (s, 3H), 1.43 - 1.27 (m, 4H), 0.92 (s, 3H). |

(continued)

| Example No. | Structure of Example | Status, colors and characterization data |
|---|---|---|
| 34 | 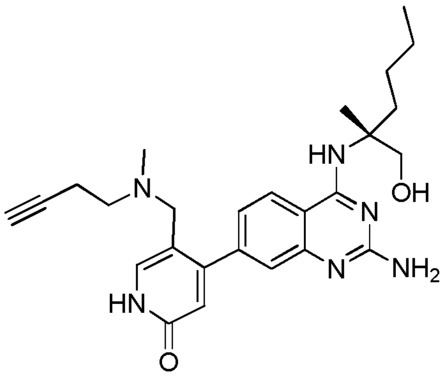 | Yellow solid (yield 42.13%); MS (ESI, *pos.ion*) *m*/*z*: 463.3 $[M+H]^+$;<br>Note: The compound was prepared by referring to the synthetic method of Example 9, wherein the reaction temperature in step 4 was replaced with room temperature. |
| 35 | 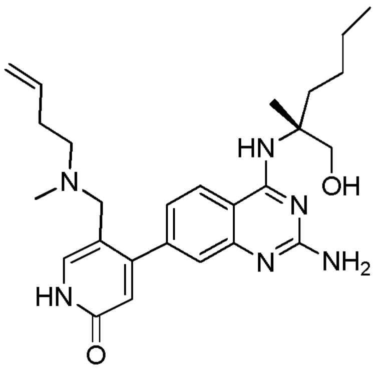 | Yellow solid (yield 29.89%); MS (ESI, *pos.ion) m*/*z*: 465.4 $[M+H]^+$.<br>$^1$H NMR (400 MHz, DMSO-$d_6$): δ (ppm) 8.56 (d, *J* = 8.5 Hz, 1H), 7.98 (s, 1H), 7.86 (s, 1H), 7.40 (d, *J* = 8.7 Hz, 1H), 7.31 (s, 1H), 6.32 (s, 1H), 5.65 - 5.53 (m, 1H), 5.04 - 4.95 (m, 2H), 4.18 (s, 1H), 4.05 - 3.91 (m, 2H), 3.57 (d, *J* = 10.8 Hz, 2H), 2.99 - 2.86 (m, 1H), 2.85 - 2.71 (m, 1H), 2.44 (s, 3H), 2.22 - 2.10 (m, 3H), 1.80 - 1.67 (m, 1H), 1.45 (s, 3H), 1.32 - 1.17 (m, 4H), 0.85 (t, *J* = 6.8 Hz, 3H). |

**Example 36: (*R*)-4-(2-Amino-4-((1-hydroxy-2-methylhexan-2-yl)amino)quinazolin-7-yl)-5-((butyl(methyl)a mino) methyl)pyridin-2(1*H*)-one**

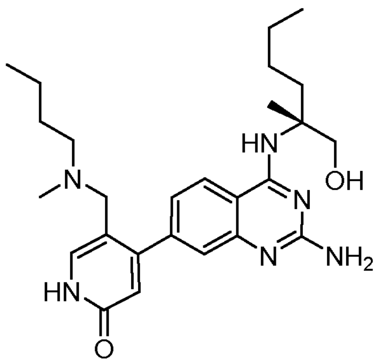

5-((Butyl(methyl)amino)methyl)-4-iodopyridin-2(1*H*)-one (prepared by the synthetic method of steps 1 to 3 of Example 56) and (*R*)-(2-((2,4-dimethoxybenzyl)amino)-4-((1-hydroxy-2-methylhexan-2-yl)amino)quinazolin-7-yl)bo ronic acid were used as raw materials, according to the synthesis method of step 5 of example 56, and the title compound can be obtained as a yellow solid with a total yield of 54%.

MS (ESI, *pos.ion*) *m*/*z:* 467.3 $[M+H]^+$;
$^1$H NMR (400 MHz, $CD_3OD$): δ (ppm) 8.42 (d, *J* = 8.4 Hz, 1H), 7.94 (s, 1H), 7.51 (s, 1H), 7.45 (d, *J* = 8.6 Hz, 1H), 6.57 (s, 1H), 4.36 (s, 1H), 4.20 (d, *J* = 11.2 Hz, 2H), 3.72 (d, *J* = 11.2 Hz, 1H), 2.99 - 2.79 (m, 2H), 2.62 (s, 3H), 2.35 - 2.19 (m, 1H), 1.29 - 1.77 (m, 1H), 1.57 (s, 3H), 1.47 - 1.27 (m, 8H), 0.94 (t, *J* = 6.7 Hz, 3H), 0.84 (t, *J* = 7.2 Hz, 3H).

**Example 37: (*R*)-4-(2-Amino-4-((1-hydroxy-2-methylhexan-2-yl)amino)pyrido[3,2-*d*]pyrimidin-7-yl)-5-((but -3-en-1-yl(methyl)amino)methyl)-1-(1-methylpiperidin-4-yl)pyridin-2(1*H*)-one**

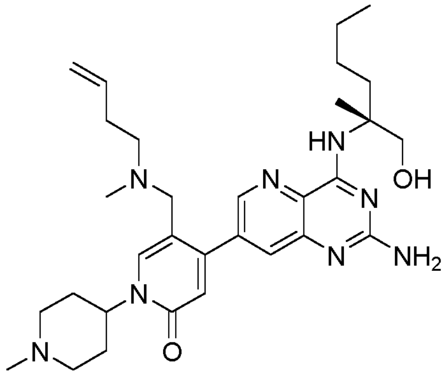

Step 1: 5-((But-3-en-1-yl(methyl)amino)methyl)-4-iodo-1-(1-methylpiperidin-4-yl)pyridin-2(1*H*)-one

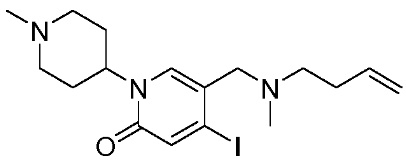

5-((But-3-en-1-yl(methyl)amino)methyl)-4-iodopyridin-2(1*H*)-one (0.55 g, 1.74 mmol, prepared by the synthetic method of steps 1-4 of Example 9), 1-methylpiperidin-4-ol (0.20 g, 1.74 mmol) and triphenylphosphine (0.68 g, 2.61 mmol) were dissolved in anhydrous tetrahydrofuran (10 mL). The mixture was degassed and refilled with $N_2$three times, then the mixture was cooled to 0 °C, and DIAD (0.53 g, 2.61 mmol)was added to the mixture. After the addition was completed, the reaction was moved to room temperature and stirred for about 13 hours. Post-treatment: n-hexane (5 mL) was added, solid was precipitated, then the mixture was filtered, and the filtrate was concentrated under reduced pressure. The residue was purified by silica gel column chromatography (DCM/MeOH (V/V) = 10/1) to obtain a yellow butter compound (0.52 g, yield 71.27%).
MS (ESI, *pos.ion*) *m*/*z*:208.7 [(M+H)/2]$^+$.

Step 2: (*R*)-4-(2-Amino-4-((1-hydroxy-2-methylhexan-2-yl)amino)pyrido[3,2-*d*]pyrimidin-7-yl)-5-((but-3-e n-1-yl(methyl) amino)methyl)-1-(1-methylpiperidin-4-yl)pyridin-2(1*H*)-one

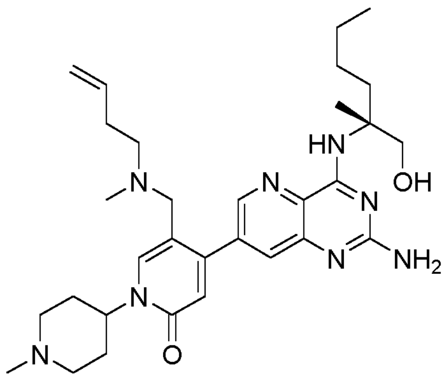

5-((But-3-en-1-yl(methyl)amino)methyl)-4-iodo-1-(1-methylpiperidin-4-yl)pyridi n-2(1*H*)-one and (*R*)-(2-((2,4-dimethoxybenzyl)amino)-4-((1-hydroxy-2-methylhexan-2-yl)amino)pyrido[3,2-*d*]pyri midin-7-yl)boronic acid were used as raw materials, according to the synthesis method of steps 10-11 of example 1, and the title compound can be obtained as a white solid with a total yield of 31%.

MS (ESI, *pos.ion*) *m*/*z*: 563.6 [M+H]$^+$;
$^1$H NMR (400 MHz, DMSO-$d_6$): δ (ppm) 8.63 (s, 2H), 8.36 (s, 1H), 7.83 (s, 1H), 6.94 (d, *J* = 3.9 Hz, 1H), 5.68 - 5.56 (m, 1H), 5.10 - 4.97 (m, 2H), 4.42 (s, 1H), 4.19 (s, 1H), 3.77 (d, *J* = 10.9 Hz, 1H), 3.39 (d, *J* = 11.7 Hz, 2H), 3.19 (s, 2H), 3.11 - 2.87 (m, 2H), 2.81 (s, 3H), 2.47 (s, 2H), 2.29 (s, 3H), 2.19 - 2.06 (m, 2H), 2.04 - 1.83 (m, 3H), 1.49 (s, 3H), 1.28 (s, 4H), 1.20 (d, *J* = 6.3 Hz, 1H), 0.87 (t, *J* = 6.7 Hz, 3H).

**Example** 38: **(*R*)-4-(2-Amino-4-((1-hydroxy-2-methylhexan-2-yl)amino)pyrido[3,2-*d*]pyrimidin-7-yl)-*N*-(but -3-en-1-yl)-*N*-methyl-6-oxo-1,6-dihydropyridine-3-carboxamide**

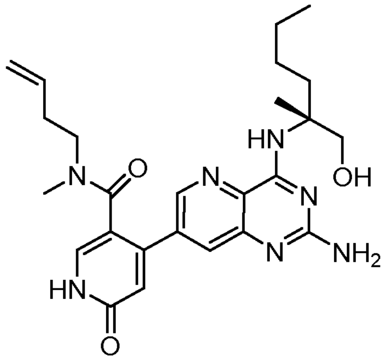

Step 1: *N*-(But-3-en-1-yl)-4,6-dichloronicotinamide

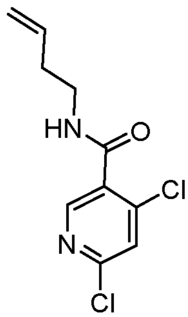

4,6-Dichloronicotinic acid (1.00 g, 5.21 mmol), but-3-en-1-amine hydrochloride (0.84 g, 7.81 mmol), HATU (3.96 g, 10.42 mmol) and DIPEA (4.04 g, 31.26 mmol) were weighed separately and mixed in dichloromethane (40 mL). The mixture was stirred at room temperature for about 12 hours. The stirring was stopped and the mixture was concentrated under reduced pressure. The residue was purified by silica gel column chromatography (DCM/MeOH (V/V) = 15/1) to obtain the title compound as a brown-black solid (1.28 g, yield 23%).
MS (ESI, *pos.ion*) *m*/*z*:245.0 [M+H]$^+$.

Step 2: *N*-(But-3-en-1-yl)-4,6-dichloro-*N*-methylnicotinamide

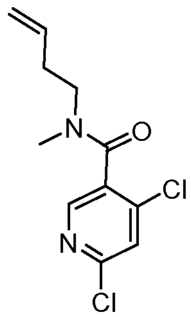

*N*-(but-3-en-1-yl)-4,6-dichloronicotinamide (1.28 g, 5.22 mmol) was weighed and dissolved in THF (40 mL), then 60% sodium hydride (0.42 g, 10.5 mmol) was added under stirring and 0 °C. The resulted mixture was stirred for about 15 minutes, then iodomethane (3.70 g, 26.07 mmol) was added, and after the addition, the reaction was transferred to room temperature and reacted for about 15 hours; the reaction was stopped, water (30 mL) was added to the reaction solution to dilute it, and then extracted with ethyl acetate (20 mL × 3), the organic phases were combined, washed with saturated brine (30 mL), dried over anhydrous sodium sulfate, and concentrated under reduced pressure. The residue was purified by silica gel column chromatography (PE/EA(V/V) = 10/1) to obtain the title compound as a light yellow oily liquid (0.90 g, yield 66.54%).
MS (ESI, *pos.ion*) *m*/*z*:259.1 $[M+H]^+$.

Step 3: *N*-(But-3-en-1-yl)-4-chloro-*N*-methyl-6-oxo-1,6-dihydropyridine-3-carboxamide

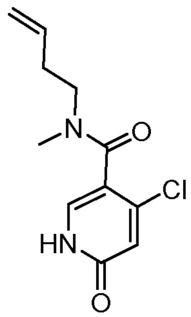

*N*-(But-3-en-1-yl)-4,6-dichloro-*N*-methylnicotinamide (0.40 g, 1.54 mmol) was weighed and added to tert-butyl alcohol (30 mL), and then potassium tert-butylate (0.69 g, 6.16 mmol) was added and stirred at room temperature for about 15 hours. The raw material was not completely reacted, potassium tert-butylate (0.23 g, 2.03 mmol) was added and the reaction was continued at the same temperature for about 12 hours. The stirring was stopped and the pH of the reaction mixture was adjusted to 2-3 with 4 M hydrochloric acid in 1,4-dioxane solution and stirred for about 30 minutes. Then, saturated sodium bicarbonate solution was added to adjust the pH to 8-9, and then extracted with EA (20 mL × 3). The organic phases were combined, washed with saturated brine (30 mL), dried over anhydrous sodium sulfate, and concentrated under reduced pressure. The residue was purified by silica gel column chromatography (DCM/MeOH (V/V) = 15/1) to obtain the title compound as a light yellow solid (0.17 g, yield 45.86%).
MS (ESI, *pos.ion*) *m*/*z*:241.1 $[M+H]^+$.

Step 4: (*R*)-4-(2-Amino-4-((1-hydroxy-2-methylhexan-2-yl)amino)pyrido[3,2-*d*]pyrimidin-7-yl)-*N*-(but-3-e n-1-yl)-*N*-methyl-6-oxo-1,6-dihydropyridine-3-carboxamide

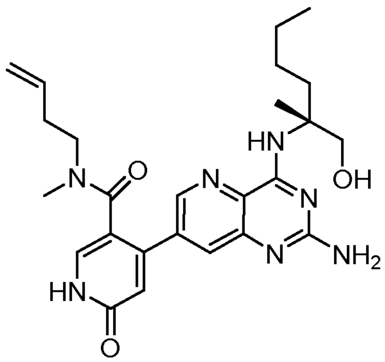

*N*-(But-3-en-1-yl)-4-chloro-*N*-methyl-6-oxo-1,6-dihydropyridine-3-carboxamide and (*R*)-(2-((2,4-dimethoxybenzyl)amino)-4-((1-hydroxy-2-methylhexan-2-yl)amino)pyrido[3,2-*d*]pyri midin-7-yl)boronic acid were used as raw materials, according to the synthesis method of steps 10-11 of example 1, and the title compound can be obtained as a white solid with a total yield of 30%.

MS (ESI, *pos.ion*) *m*/*z*:480.3 $[M+H]^+$;
$^1$H NMR (400 MHz, DMSO-$d_6$): δ (ppm) 13.08 (s, 1H), 9.12 (s, 1H), 8.57 (s, 1H), 8.35 (s, 2H), 8.30 (s, 1H), 7.50 (s, 1H), 5.87 (d, *J* = 7.0 Hz, 1H), 5.63 (d, *J* = 7.7 Hz, 1H), 5.16 (d, *J* = 17.0 Hz, 1H), 5.11 - 4.97 (m, 2H), 3.79 (d, *J* = 10.9 Hz, 1H), 3.61 - 3.47 (m, 3H), 3.25 (s, 1H), 2.99 (s, 1H), 2.88 (s, 1H), 2.36 (d, *J* = 7.4 Hz, 1H), 2.27 (d, *J* = 7.2 Hz, 1H), 1.97 (s, 2H), 1.50 (s, 3H), 1.28 (s, 4H), 0.88 (t, *J* = 6.7 Hz, 3H).

**Example 39: (*R*)-4-(2-Amino-4-((1-hydroxy-2-methylhexan-2-yl)amino)pyrido[3,2-*d*]pyrimidin-7-yl)-5-(pip eridine-1-carbonyl)pyridin-2(1*H*)-one**

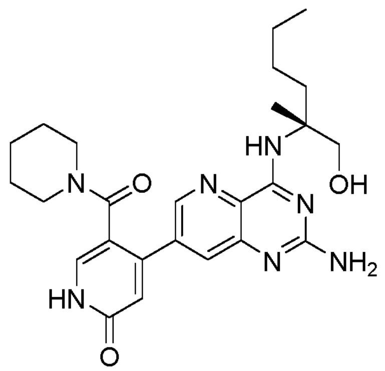

Step 1: (6-Bromo-4-chloropyridin-3-yl)(piperidin-1-yl)methanone

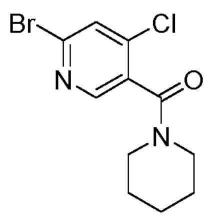

6-Bromo-4-chloropyridine-3-carboxylic acid (2.46 g, 10.42 mmol), piperidine (1.77 g, 20.84 mmol), HATU (7.92 g, 20.84 mmol) and DIPEA (8.08 g, 62.52 mmol) were weighed separately and mixed in DMF (30 mL). The mixture was stirred at room temperature for about 24 hours. Water (30 mL) was added for dilution, and then the mixture was extracted with ethyl acetate (30 mL × 3). The organic phases were combined, washed with saturated brine (25 mL × 3), dried over anhydrous sodium sulfate, and concentrated under reduced pressure. The residue was purified by silica gel column

chromatography (PE/EA(V/V) = 10/1) to obtain the title compound as a light yellow oily liquid (2.70 g, 85.35%).
MS (ESI, *pos.ion*) *m*/*z*:303.0 $[M+H]^+$.

Step 2: 4-Chloro-5-(piperidine-1-carbonyl)pyridin-2(1*H*)-one

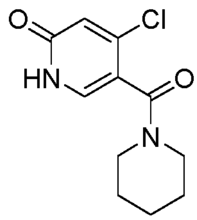

(6-Bromo-4-chloropyridin-3-yl)(piperidin-1-yl)methanone (1.50 g, 4.94 mmol) and potassium tert-butoxide (2.22 g, 19.76 mmol) were weighed and added to tert-butanol (50 mL), and the mixture was heated and stirred in an oil bath at 90°C for overnight. The stirring was stopped, and the pH of the reaction mixture was adjusted to 1-3 with 2 M a solution of hydrochloric acid in 1,4-dioxane. Then, saturated sodium bicarbonate solution (50 mL) was added to the system, and the resulted mixture was extracted with ethyl acetate (20 mL × 3). The combined organic phases were washed with saturated brine (30 mL)., dried over anhydrous sodium sulfate, concentrated under reduced pressure. The residue was purified by silica gel column chromatography (DCM/MeOH (V/V) = 15/1) to obtain the title compound as a brown-black oily liquid (0.31 g, yield 26.07%).
MS (ESI, *pos.ion*) *m*/*z*:241.0 $[M+H]^+$.

Step 3: (*R*)-4-(2-Amino-4-((1-hydroxy-2-methylhexan-2-yl)amino)pyrido[3,2-*d*]pyrimidin-7-yl)-5-(piperidi ne-1-carbonyl) pyridin-2(1*H*)-one

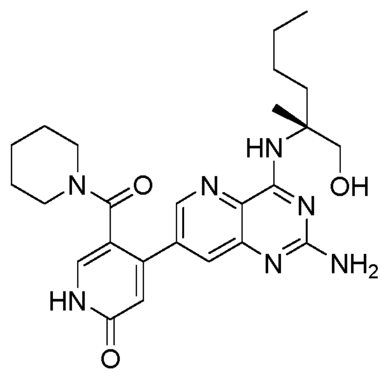

4-Chloro-5-(piperidine-1-carbonyl)pyridin-2(1*H*)-one and (*R*)-(2-((2,4-dimethoxybenzyl)amino)-4-((1-hydroxy-2-methylhexan-2-yl)amino)pyrido[3,2-*d*]pyri midin-7-yl)boronic acid were used as raw materials, according to the synthesis method of step 4 of example 38, and the title compound can be obtained as a white solid with a total yield of 44%.

MS (ESI, *pos.ion*) *m*/*z:* 480.3 $[M+H]^+$;
$^1$H NMR (400 MHz, DMSO-$d_6$): δ (ppm) 13.16 (s, 1H), 8.59 (d, *J* = 1.6 Hz, 1H), 8.27 (s, 1H), 7.71 (d, *J* = 1.6 Hz, 1H), 7.63 (s, 1H), 6.58 (s, 1H), 3.79 (d, *J* = 11.0 Hz, 2H), 3.51 (d, *J* = 11.0 Hz, 2H), 3.40 - 3.19 (m, 4H), 1.96 (d, *J* = 9.3 Hz, 2H), 1.48 (s, 5H), 1.29 (d, *J* = 15.4 Hz, 8H), 0.86 (t, *J* = 6.7 Hz, 3H).

**Example 40: (*R*)-*N*-((4-(2-Amino-4-((1-hydroxy-2-methylhexan-2-yl)amino)pyrido[3,2-*d*]pyrimidin-7-yl)-6-oxo-1,6-dihydropyridin-3-yl)methyl)-*N*-methylbut-3-enamide**

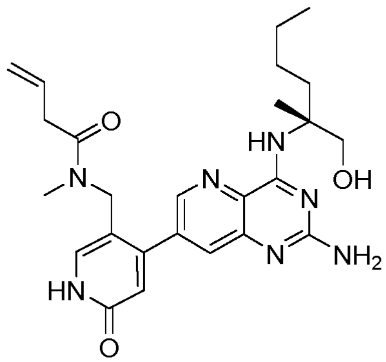

Step 1: (6-Fluoro-4-iodopyridin-3-yl)methanamine

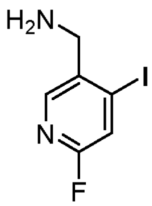

5-(Bromomethyl)-2-fluoro-4-iodopyridine (1.01 g, 3.19 mmol) was weighed and added to a solution of ammonia in methanol (1.08 g, 63.77 mmol, 7 M), and the mixture was stirred at room temperature for about 16 hours; then concentrated under reduced pressure, and the residue was purified by silica gel column chromatography (DCM/MeOH (V/V) = 15/1) to give the title compound as a colorless oily liquid (0.71 g, yield 88.31%).
MS (ESI, *pos.ion*) *m*/*z*:253.1 $[M+H]^+$.

Step 2: *N*-((6-Fluoro-4-iodopyridin-3-yl)methyl)but-3-enamide

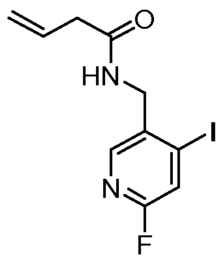

(6-Fluoro-4-iodopyridin-3-yl)methanamine (0.70 g, 2.78 mmol), vinylacetic acid (0.29 g, 3.36 mmol) and HATU (1.59 g, 4.17 mmol) were weighed and mixed in dichloromethane (50 mL), and then DIPEA (1.08 g, 8.34 mmol) was added and the resulted mixture was stirred at room temperature for about 42 hours. Stirring was stopped and the mixture was concentrated under reduced pressure. The residue was purified by silica gel column chromatography (PE/EA (V/V) = 5/1) to obtain the title compound as a colorless oily liquid (0.81 g, yield 91.02%).
MS (ESI, *pos.ion*) *m*/*z*:321.2 $[M+H]^+$.

Step 3: *N*-((6-fluoro-4-iodopyridin-3-yl)methyl)-*N*-methylbut-3-enamide

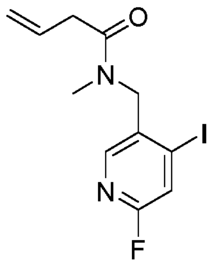

N ((6-fluoro-4-iodopyridin-3-yl)methyl)but-3-enamide (0.70 g, 7.06 mmol) was weighed and added to tetrahydrofuran (50 mL), under stirring and 0 °C; the sodium hydride (0.20 g, 5.06 mmol, purity: 60%) was added and stirred for about 15 minutes; then iodomethane (1.80 g, 12.68 mmol) was added, and after the addition was completed, the mixture was transferred to room temperature and stirred for about 28 hours, then concentrated under reduced pressure, and the residue was purified by silica gel column chromatography (PE/EA(V/V) = 5/1) to obtain the title compound as a light yellow solid (0.55 g, yield 65.06%).
MS (ESI, *pos.ion*) *m*/*z*: 334.9 $[M+H]^+$.

Step 4: (*R*)-*N*-((4-(2-Amino-4-((1-hydroxy-2-methylhexan-2-yl)amino)pyrido[3,2-*d*]pyrimidin-7-yl)-6-oxo-1,6-dihydropyridin-3-yl)methyl)-*N*-methylbut-3-enamide

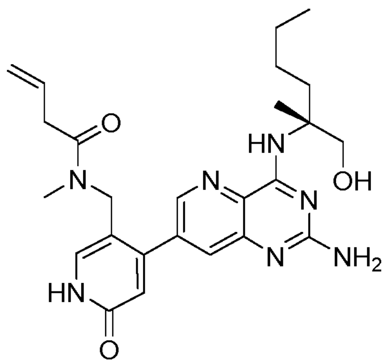

*N*-((6-Fluoro-4-iodopyridin-3-yl)methyl)-A-methylbut-3-enamide was used as the starting material, and the synthesis method of step 3 in example 38 was referred to prepare *N*-((4-iodo-6-oxo-1,6-dihydropyridin-3-yl)methyl)-N-methylbut-3-enamide. Then, *N*-((4-iodo-6-oxo-1,6-dihydropyridin-3-yl)methyl)-N-methylbut-3-enamide and (R)-(2-((2,4-dimethoxybenzyl)amino)-4-((1-hydroxy-2-methylhexan-2-yl)amino)pyrido[3,2-*d*]pyri midin-7-yl)boronic acid were used as raw materials, according to the synthesis method of steps 4 of example 38, and the title compound can be obtained as a white solid with a total yield of 28%.

MS (ESI, *pos.ion*) *m*/*z*: 480.2 $[M+H]^+$;
$^1$H NMR (400 MHz, DMSO-$d_6$): δ (ppm) 13.00 (s, 1H), 8.52 (d, *J* = 17.9 Hz, 2H), 8.29 (s, 2H), 7.78 - 7.66 (m, 1H), 7.51 - 7.36 (m, 1H), 6.32 (s, 1H), 6.30 - 5.94 (m, 2H), 4.32 (d, *J* = 13.8 Hz, 3H), 3.76 (d, *J* = 10.9 Hz, 1H), 3.50 (d, *J* = 10.9 Hz, 1H), 2.70 (s, 2H), 2.61 (s, 1H), 2.08 - 1.88 (m, 2H), 1.70 (d, *J* = 6.1 Hz, 2H), 1.60 (d, *J* = 5.0 Hz, 1H), 1.49 (s, 3H), 1.35 - 1.18 (m, 4H), 0.87 (t, *J* = 6.8 Hz, 3H).

**Example 41: (*R*)-4-(2-Amino-4-((1-hydroxy-2-methylhexan-2-yl)amino)pyrido[3,2-*d*]pyrimidin-7-yl)-5-((but -3-en-1-yloxy)methyl)pyridin-2(1*H*)-one**

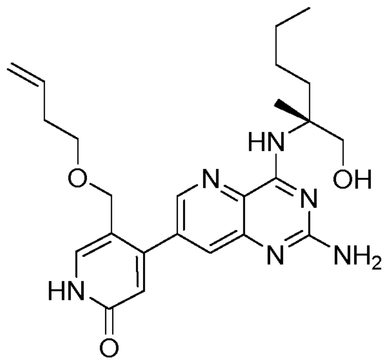

Step 1: 4-Iodo-2-methoxy-5-methylpyridine

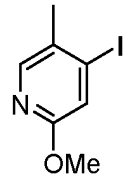

2-Fluoro-4-iodo-5-methylpyridine (2.00 g, 8.44 mmol) was weighed and dissolved in methanol (20 mL), and then 7 M sodium methoxide methanol solution (1.82 g, 33.76 mmol) was added, and the resulted mixture was heated in a 60 °C oil bath for about 12 hours; the reaction solution was diluted with water (30 mL), and then extracted with ethyl acetate (20 mL × 3), the organic phases were combined, washed with saturated brine (30 mL), dried over anhydrous sodium sulfate, and concentrated under reduced pressure to obtain the title compound as a light yellow oily liquid (2.10 g, yield 99.91%). MS (ESI, *pos.ion*) *m*/*z*:250.1 $[M+H]^+$.

Step 2: 5-(Bromomethyl)-4-iodo-2-methoxypyridine

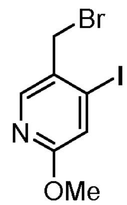

4-Iodo-2-methoxy-5-methylpyridine (2.10 g, 8.43 mmol), azobisisobutyronitrile (1.38 g, 8.43 mmol) and NBS (1.50 g, 8.43 mmol) were weighed and added to carbon tetrachloride (40 mL). Under $N_2$, the mixture was heated in an oil bath at 78 °C for about 2 hours, then filtered. Saturated sodium thiosulfate solution (20 mL) was added to the filtrate, and the resulted mixture was extracted with dichloromethane (30 mL × 3). The organic phases were combined, washed with saturated brine (30 mL), dried over anhydrous sodium sulfate, and concentrated under reduced pressure. The residue was purified by silica gel column chromatography (PE/EA (V/V) = 20/1) to obtain the title compound as an off-white solid (2.20 g, yield 79.58%).
MS (ESI, *pos.ion*) *m*/*z*: 327.8 $[M+H]^+$.

Step 3: 5-((But-3-en-1-yloxy)methyl)-4-iodo-2-methoxypyridine

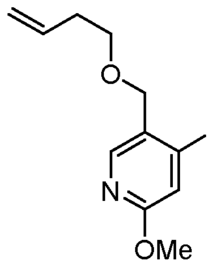

Potassium hydroxide (1.88 g, 33.55 mmol) was added to but-3-en-1-ol (9.68 g, 134.20 mmol), and the mixture was heated and stirred in an oil bath at 90 °C for about 1 hour. The temperature of the mixture was lowered to room temperature, and then a solution of 5-(bromomethyl)-4-iodo-2-methoxypyridine (2.20 g, 6.71 mmol) in tetrahydrofuran (20 mL) was added, and the reaction mixture was continued to stir at room temperature for about 17 hours. Water (50 mL) was added to dilute the mixture, and then the mixture was extracted with ethyl acetate (40 mL × 3). The organic phases were combined, washed with saturated brine (50 mL × 3), dried over anhydrous sodium sulfate, and concentrated under reduced pressure. The residue was purified by silica gel column chromatography (PE/EA (V/V) = 20/1) to obtain the title compound as a light yellow oily liquid (1.80 g, yield 84.06%).
MS (ESI, *pos.ion*) *m*/*z*:319.9 $[M+H]^+$.

Step 4: 5-((But-3-en-1-yloxy)methyl)-4-iodopyridin-2(1*H*)-one

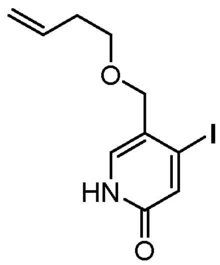

5-((But-3-en-1-yloxy)methyl)-4-iodo-2-methoxypyridine (1.80 g, 5.64 mmol) and pyridine hydrobromide (18.05 g, 112.8 mmol) were weighed and mixed in DMF (30 mL). The mixture was heated and stirred in an 80 °C oil bath for about 18 hours, then saturated sodium bicarbonate solution (30 mL) was added to the reaction system, and then the resulted mixture was extracted with ethyl acetate (20 mL × 3). The organic phases were combined, washed with saturated brine (30 mL × 3), dried over anhydrous sodium sulfate, and concentrated under reduced pressure. The residue was purified by silica gel column chromatography (DCM/MeOH (V/V) = 15/1) to obtain the title compound as a light yellow solid (0.40 g, yield 23.24%).
MS (ESI, *pos.ion*) *m*/*z*:306.0 $[M+H]^+$.

Step 5: (*R*)-4-(2-Amino-4-((1-hydroxy-2-methylhexan-2-yl)amino)pyrido[3,2-*d*]pyrimidin-7-yl)-5-((but-3-e n-1-yloxy) methyl)pyridin-2(1*H*)-one

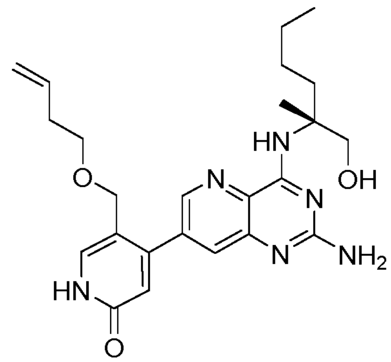

5-((But-3-en-1-yloxy)methyl)-4-iodopyridin-2(1*H*)-one and (*R*)-(2-((2,4-dimethoxybenzyl)amino)-4-((1-hydroxy-2-methylhexan-2-yl)amino)pyrido[3,2-*d*]pyri midin-7-yl)boronic acid were used as starting materials and the title compound was obtained as a white solid by referring to step 4 of Example 38 in a yield of 46%.

MS (ESI, *pos.ion*) *m*/*z*:453.2 $[M+H]^+$;
$^1H$ NMR (400 MHz, DMSO-$d_6$): δ (ppm) 13.34 (s, 1H), 11.93 (s, 1H), 8.62 (s, 1H), 8.27 (s, 1H), 7.87 (s, 1H), 7.58 (s, 1H), 6.38 (s, 1H), 5.64 - 5.50 (m, 1H), 5.04 - 4.84 (m, 2H), 4.10 (s, 2H), 3.78 (d, *J* = 10.8 Hz, 1H), 3.51 (d, *J* = 10.8 Hz, 1H), 3.26 (t, *J* = 6.6 Hz, 2H), 2.51 (s, 2H), 2.13 (q, *J* = 6.6 Hz, 2H), 1.97 (d, *J* = 11.9 Hz, 2H), 1.48 (s, 3H), 1.27 (d, *J* = 3.5 Hz, 4H), 0.86 (t, *J* = 6.7 Hz, 3H).

**Example 42: (*R*)-4-(2-Amino-4-((1-hydroxyhexan-2-yl)amino)pyrido[3,2-*d*]pyrimidin-7-yl)-5-((but-3-en-1-yl (methyl)amino)methyl)pyridin-2(1*H*)-one**

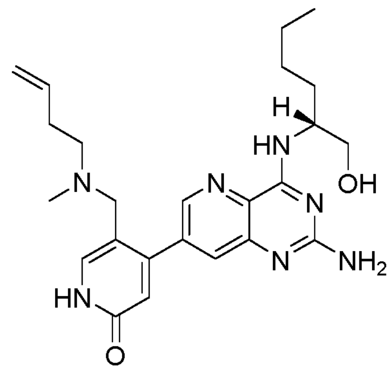

Step 1: (*R*)-(2-((2,4-Dimethoxybenzyl)amino)-4-((1-hydroxyhexan-2-yl)amino)pyrido[3,2-*d*]pyrimidin-7-yl )boronic acid

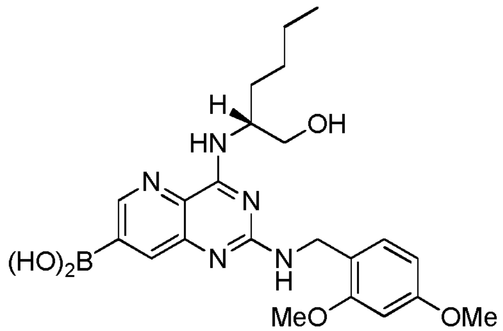

7-Bromo-2,4-dichloropyrido[3,2-*d*]pyrimidine and (R)-2-aminohexanoic acid were used as raw materials, according to the synthesis method of step 1 to step 5 of example 1, and the title compound can be obtained as a brown-black

solid with a total yield of 99%.
MS (ESI, *pos.ion) m/z:456.5* [M+H]$^+$.

Step 2: (*R*)-4-(2-Amino-4-((1-hydroxyhexan-2-yl)amino)pyrido[3,2-*d*]pyrimidin-7-yl)-5-((but-3-en-1-yl(m ethyl)amino) methyl)pyridin-2(1*H*)-one

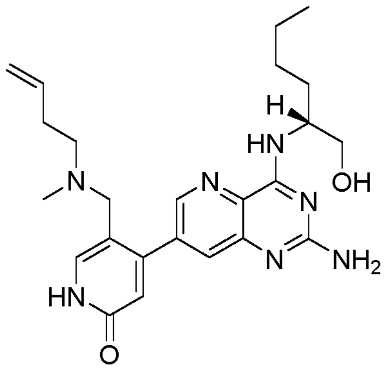

5-((But-3-en-1-yl(methyl)amino)methyl)-4-iodopyridin-2(1*H*)-one and (*R*)-(2-((2,4-dimethoxybenzyl)amino)-4-((1-hydroxyhexan-2-yl)amino)pyrido[3,2-*d*]pyrimidin-7-yl )boronic acid were used as raw materials and the title compound was obtained as a white solid by referring to step 4 of Example 38 in a yield of 77%.

MS (ESI, *pos.ion) m/z:452.2* [M+H]$^+$;
$^1$H NMR (400 MHz, $CD_3OD$): δ (ppm) 8.67 (d, *J* = 1.2 Hz, 1H), 7.97 (s, 2H), 6.62 (s, 1H), 5.75 - 5.62 (m, 1H), 5.18 - 5.08 (m, 2H), 4.64 - 4.55 (m, 1H), 4.27 (s, 1H), 3.77 (d, *J* = 5.3 Hz, 2H), 3.33 (s, 2H), 3.03 (s, 2H), 2.63 (s, 3H), 2.35 (q, *J* = 7.4 Hz, 2H), 1.85 - 1.67 (m, 2H), 1.42 (s, 4H), 0.93 (t, *J* = 6.8 Hz, 3H).

**Example 43: (*R*)-4-(2-Amino-4-((1-hydroxyhexan-2-yl)amino)pyrido[3,2-*d*]pyrimidin-7-yl)-5-((butyl(methyl )amino)methyl)pyridin-2(1*H*)-one**

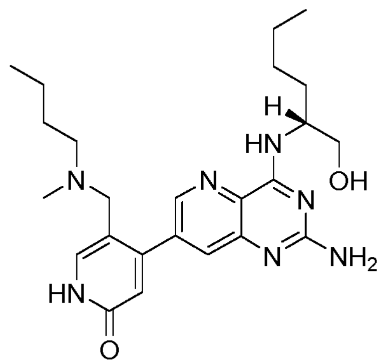

Step 1: (*R*)-5-((But-3-en-1-yl(methyl)amino)methyl)-4-(2-((2,4-dimethoxybenzyl)amino)-4-((1-hydroxyhex an-2-yl)amino)pyrido[3,2-*d*]pyrimidin-7-yl)pyridin-2(1*H*)-one

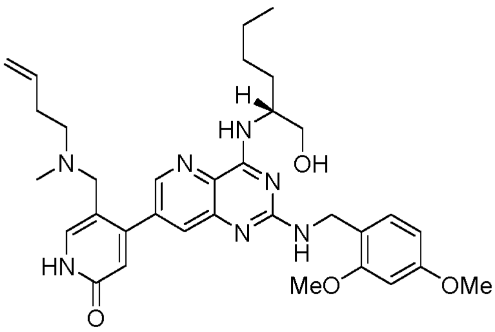

5-((But-3-en-1-yl(methyl)amino)methyl)-4-iodopyridin-2(1*H*)-one and (*R*)-(2-((2,4-dimethoxybenzyl)amino)-4-((1-hydroxyhexan-2-yl)amino)pyrido[3,2-*d*]pyrimidin-7-yl )boronic acid were used as raw materials and the title compound was obtained as a brown-black solid by referring to step 10 of Example 1 in a yield of 11%.
MS (ESI, *pos.ion) m/z:602.3* [M+H]+.

Step 2: (*R*)-4-(2-Amino-4-((1-hydroxyhexan-2-yl)amino)pyrido[3,2-d]pyrimidin-7-yl)-5-((butyl(methyl)am ino)methyl) pyridin-2(1*H*)-one

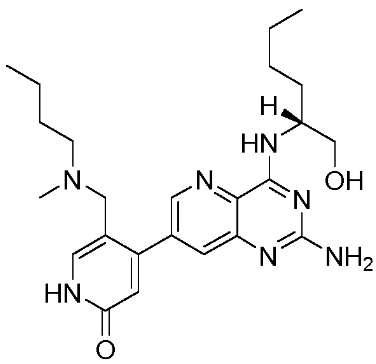

(*R*)-5-((But-3-en-1-yl(methyl)amino)methyl)-4-(2-((2,4-dimethoxybenzyl)amino)-4-((1-hydroxyhexan-2-yl)amino)pyrido[3,2-*d*]pyrimidin-7-yl)pyridin-2(1*H*)-one was used as raw materials and the title compound was obtained as a white solid by referring to steps 2-3 of Example 28 in a yield of 26%.

MS (ESI, *pos.ion)* m/z:454.3 [M+H]+;
$^1$H NMR (400 MHz, $CD_3OD$): δ (ppm) 8.68 (s, 1H), 7.99 (d, *J* = 2.5 Hz, 2H), 6.62 (s, 1H), 4.71 - 4.53 (m, 1H), 4.36 (s, 1H), 4.16 (s, 1H), 3.77 (d, *J* = 5.3 Hz, 2H), 3.33 (s, 3H), 2.93 (s, 2H), 2.63 (s, 3H), 1.88 - 1.69 (m, 2H), 1.43 (s, 6H), 1.29 - 1.20 (m, 2H), 0.94 (t, *J* = 6.4 Hz, 3H), 0.87 (t, *J* = 7.3 Hz, 3H).

**Example 44: (R)-N (2-((2-Amino-7-(5-((but-3-en-1-yl(methyl)amino)methyl)-2-oxo-1,2-dihydropyridin-4-yl) pyrido[3,2-*d*]pyrimidin-4-yl)amino)-2-methylhexyl)-1-methyl-1*H*-pyrazole-4-carboxamide**

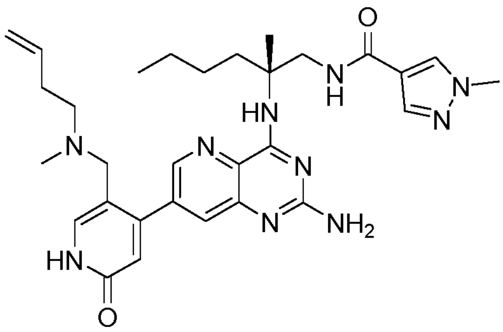

Step 1: (*R*)-2-((Tert-butoxycarbonyl)amino)-2-methylhexanoic acid

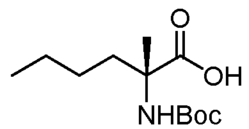

(2R)-2-Amino-2-methylhexanoic acid hydrochloride (20.00 g, 110.10 mmol) was weighed and added to water (400 mL), and then sodium hydroxide (14.53 g, 363.33 mmol) was added and stirred at room temperature until the mixture became clear. Then, a solution of $(Boc)_2O$ (28.83 g, 132.11 mmol) in tetrahydrofuran (100 mL) was slowly added dropwise. The mixture was stirred at room temperature overnight. Stirring was stopped and the pH of the reaction solution was adjusted to 4-5 with saturated citric acid solution. The mixture was extracted with ethyl acetate (100 mL × 3). The organic phases were combined, washed with saturated brine (80 mL), dried over anhydrous sodium sulfate, and concentrated under reduced pressure to obtain the title compound as a light yellow paste solid (22.50 g, yield 83.30%).
MS (ESI, *pos.ion) m*/*z*:146.4 $[M+H-Boc]^+$.

Step 2: Tert-butyl (*R*)-(1-amino-2-methyl-1-oxohexan-2-yl)carbamate

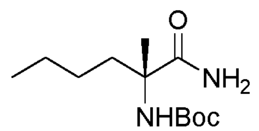

(*R*)-2-((*Tert*-butoxycarbonyl)amino)-2-methylhexanoic acid (19.40 g, 79.08 mmol) and triethylamine (10.40 g, 102.80 mmol) in tetrahydrofuran (130 mL) was added to a two-necked flask, and ethyl chloroformate (10.30 g, 94.90 mmol) was slowly added dropwise under stirring at -10°C. After the addition was completed, the reaction mixture was stirred at the same temperature for about 2 hours. The temperature of reaction mixture was lowered to -20°C, and the reaction mixture was stirred under an ammonia atmosphere for about 2 hours. The reaction mixture was transferred to room temperature and stirred for about 5 hours. The stirring was stopped, and then the mixture was extracted with ethyl acetate (20 mL × 3). The organic phases were combined, washed with saturated brine (30 mL), dried over anhydrous sodium sulfate, and concentrated under reduced pressure to obtain a colorless oily liquid as the title compound (19.32 g, yield 99.99%).
MS (ESI, *pos.ion*) *m*/*z*:145.1 $[M+H-Boc]^+$.

Step 3: Tert-butyl (*R*)-(1-amino-2-methylhexan-2-yl)carbamate

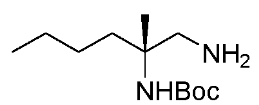

To a solution of lithium aluminum hydride in tetrahydrofuran (9.00 g, 237.21 mmol, 1 M) was slowly added a

solution of tert-butyl (R)-(1-amino-2-methyl-1-oxohexan-2-yl)carbamate (19.32 g, 79.07 mmol) in tetrahydrofuran (100 mL) at 0 °C; the addition was completed after about 1 hour, and the mixture was stirred at room temperature for about 3 hours; the mixture was transferred to an oil bath at 60 °C and heated with stirring for about 5 hours; stirring was stopped, 15% sodium hydroxide solution (20 mL) was added to the reaction system, and then extracted with ethyl acetate (10 mL × 3), the organic phases were combined, washed with saturated brine (20 mL), dried over anhydrous sodium sulfate, and concentrated under reduced pressure to obtain the title product as a colorless oily liquid (12.65 g, yield 69.45%).
MS (ESI, *pos.ion) m/z:231.2* [M+H]+.

Step 4: Tert-butyl (*R*)-(2-methyl-1-(1-methyl-1*H*-pyrazole-4-carboxamido)hexan-2-yl)carbamate

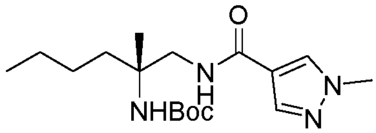

Tert-butyl (*R*)-(1-amino-2-methylhexan-2-yl)carbamate (11.00 g, 47.75 mmol), 1-methyl-1H-pyrazole-4-carboxylic acid (6.02 g, 47.75 mmol), EDCI (18.31 g, 95.50 mmol) and DIPEA (18.51 g, 143.25 mmol) were weighed and mixed in dichloromethane (150 mL), and the resulted mixiture was stirred at 39 °C. Stirring was stopped, water (100 mL) was added, and the liquids were separated. The aqueous phase was extracted with DCM (80 mL × 3). The organic phases were combined, washed with saturated brine (100 mL), dried over anhydrous sodium sulfate, and concentrated under reduced pressure. The residue was purified by silica gel column chromatography (DCM/MeOH (V/V) = 15/1) to obtain the title compound as a colorless oily liquid (12.12 g, yield 75.00%).
MS (ESI, *pos.ion) m/z:239.2* [M-Boc +H]+.

Step 5: (*R*)-*N*-(2-Amino-2-methylhexyl)-1-methyl-1*H*-pyrazole-4-carboxamide

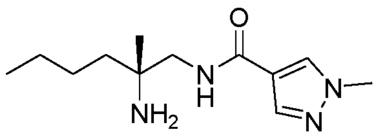

Tert-butyl (*R*)-(2-methyl-1-(1-methyl-1*H*-pyrazole-4-carboxamido)hexan-2-yl)carbamate (3.17 g, 9.37 mmol) was added to a solution of hydrochloric acid in 1,4-dioxane (4 M, 30 mL) and stirred at room temperature for about 1 hour; the mixture was concentrated under reduced pressure to remove the solvent, and the resulted residue was dissolved in dichloromethane (30 mL) and concentrated under reduced pressure. This process was repeated twice to give the title compound as a white solid (2.23 g, yield 99.86%).

Step 6: (*R*)-(2-((2,4-Dimethoxybenzyl)amino)-4-((2-methyl-l-(1-methyl-1*H*-pyrazole-4-carboxamido)hexa n-2-yl)arnino) pyrido[3,2-*d*]pyrimidin-7-yl)boronic acid

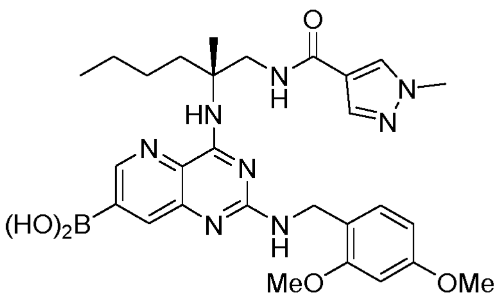

(*R*)-*N*-(2-Amino-2-methylhexyl)-1-methyl-1*H*-pyrazole-4-carboxamide and 7-bromo-2,4-dichloropyrido[3,2-d]pyrimidine were used as raw materials, according to the synthesis method of step 3 to step 5 of example 5, and the title compound can be obtained as a brown-black solid with a total yield of 27%.
MS (ESI, *pos.ion) m/z:577.5* [M+H]$^+$.

Step 7: LR)-N-(2-((2-Amino-7-(5-((but-3-en-1-yl(methyl)amino)methyl)-2-oxo-1,2-dihydropyridin-4-yl)pyr ido[3,2-*6*]pyrimidin-4-yl)amino)-2-methylhexyl)-1-methyl-1*H*-pyrazole-4-carboxamide

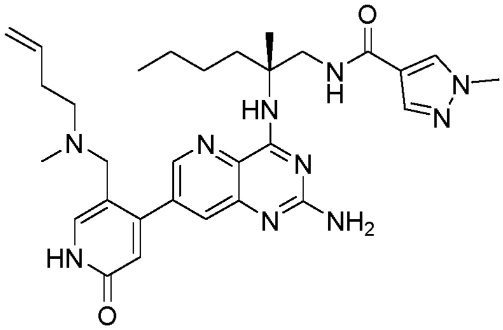

(*R*)-(2-((2,4-Dimethoxybenzyl)amino)-4-((2-methyl-1-(1-methyl-1*H*-pyrazole-4-c arboxamido)hexan-2-yl)amino)pyrido[3,2-d]pyrimidin-7-yl)boronic acid and 5-((but-3-en-1-yl(methyl)amino)methyl)-4-iodopyridin-2(1H)-one (prepared by the synthetic method of steps 1-4 of Example 9) were used as raw materials, according to the synthesis method of step 10 to step 11 of example 1, and the title compound can be obtained as a white solid with a total yield of 46%.

MS (ESI, *pos.ion*) m/z:573.3 [M+H]$^+$;
$^1$H NMR (400 MHz, DMSO-*d$_6$*): δ (ppm) 13.19 (s, 1H), 12.31 (s, 1H), 9.32(s, 1H), 8.94 (s, 1H), 8.60 (s, 1H), 8.36 (s, 1H), 8.15 (s, 1H), 7.87 (s, 1H), 7.84 (s, 1H), 7.79 (s, 1H), 6.40 (s, 1H), 5.71 - 5.52 (m, 1H), 5.08 - 4.97 (m, 2H), 4.20 (s, 1H), 4.03 - 3.88 (m, 2H), 3.84 (s, 3H), 2.99 (s, 1H), 2.84 (s, 1H), 2.47 (s, 3H), 2.22(s, 2H), 2.08 (s, 2H), 1.54 (s, 3H), 1.39 - 1.21 (m, 4H), 0.87 (t, *J*= 6.3 Hz, 3H).

**Examples 45-46 can be obtained by referring to the synthesis method of Example 44. The structures and characterization data of Examples 45-46 are shown in Table 4 below.**

| Example No. | Structure of Example | Status, colors and characterization data |
|---|---|---|
| 45 | 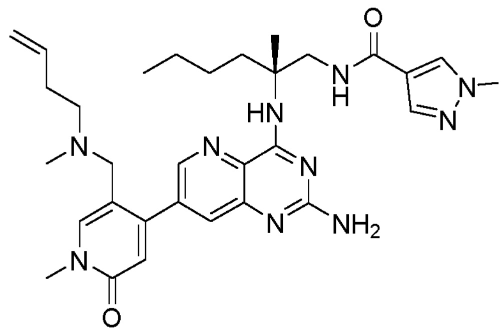 | Light yellow solid (yield 41.92%); MS (ESI, *pos.ion) m/z:*587.3 [M+H]$^+$; $^1$H NMR(400 MHz, DMSO-*d$_6$*): δ (ppm) 13.42 (s, 1H), 9.63 (s, 1H), 8.92 (s, 1H), 8.59 (s, 2H), 8.53 (s, 1H), 8.37 (d, *J* = 5.7 Hz, 1H), 8.17 (s, 1H), 8.15 (s, 1H), 7.84 (s, 1H), 7.78 (s, 1H), 6.47 (s, 1H), 5.71 - 5.55 (m, 1H), 5.10 - 4.95 (m, 2H), 4.19 (s, 1H), 3.83 (s, 6H), 3.52 (s, 3H), 3.44 (d, *J* = 8.6 Hz, 1H), 2.93 (d, *J* = 51.3 Hz, 2H), 2.23 (d, *J* = 7.0 Hz, 2H), 2.07 (s, 2H), 1.53 (s, 3H), 1.29 (d, *J* = 5.9 Hz, 4H), 0.86 (t, *J*= 6.3 Hz, 3H). |

(continued)

| Example No. | Structure of Example | Status, colors and characterization data |
|---|---|---|
| 46 | 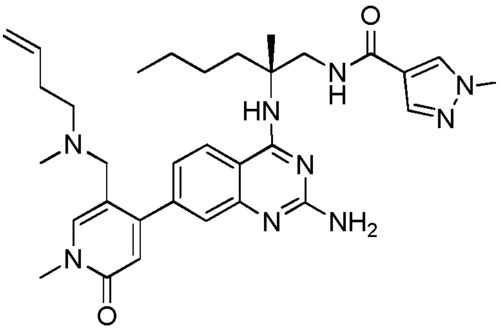 | Light yellow solid (yield 54.42%); MS (ESI, *pos.ion) m/z:586.4* [M+H]+; $^1$H NMR (400 MHz, DMSO-$d_6$): δ (ppm) 13.04 (s, 1H), 9.43 (s, 1H), 9.19 (s, 1H), 8.70 (s, 1H), 8.29 (d, *J* = 8.5 Hz, 1H), 8.21 (s, 1H), 8.14 (s, 1H), 7.91 (s, 1H), 7.43 (d, *J* = 8.3 Hz, 1H), 7.33 (s, 1H), 6.39 (s, 1H), 5.66 - 5.50 (m, 1H), 5.03 - 4.93 (m, 2H), 4.18 (s, 1H), 4.00 (s, 1H), 3.90 (d, *J* = 5.8 Hz, 1H), 3.85 (s, 3H), 3.52 (s, 3H), 3.34 (d, *J* = 4.7 Hz, 2H), 2.91 (s, 1H), 2.80 (s, 1H), 2.48 (s, 2H), 2.15 (d, *J* = 6.5 Hz, 3H), 2.05 (s, 1H), 1.52 (s, 3H), 1.38 - 1.16 (m, 4H), 0.86 (t, *J* = 6.8 Hz, 3H). |

**Example** 47: **(*R*)-2-((2-Amino-7-(5-((but-3-en-1-yl(methyl)amino)methyl)-2-oxo-1,2-dihydropyridin-4-yl)py rido [3,2-*d*]pyrimidin-4-yl)amino)-2-methylhexyl 1-methyl-1*H*-pyrazole-4-carboxylate**

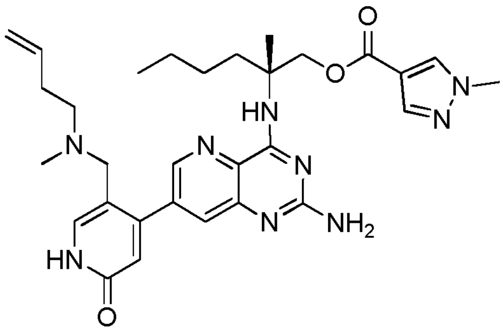

Step 1: (*R*)-2-((Tert-butoxycarbonyl)amino)-2-methylhexyl 1-methyl-1*H*-pyrazole-4-carboxylate

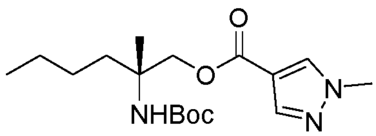

Tert-butyl (R)-(1-hydroxy-2-methylhexan-2-yl)carbamate (1.00 g, 4.32 mmol) and 1-methyl-1H-pyrazole-4-carboxylic acid (0.82 g, 6.48 mmol) were weighed and dissolved in 1,2-dichloroethane (10 mL), and then EDCI (1.24 g, 6.48 mmol) and DMAP (0.053 g, 0.43 mmol) were added in sequence. The resulted mixture washeated to 60°C and stirred for about 18 hours. The reaction was stopped, the temperature was lowered to room temperature, water (100 mL) was added, and then extracted with ethyl acetate (100 mL×3). The organic phase was collected, washed with saturated brine (100 mL), dried over anhydrous sodium sulfate, and the solvent was removed by spin drying. The residue was purified by silica gel column chromatography (PE/EA (V/V) = 1/1) to obtain the title compound as a colorless oil product (0.64 g, yield 43.65%).
MS (ESI, *pos. ion) m*/z: 240.4 [M+H-Boc]+.

Step 2: (*R*)-2-((2-Amino-7-(5-((but-3-en-1-yl(methyl)amino)methyl)-2-oxo-1,2-dihydropyridin-4-yl)pyrido [3,2-*d*]pyrimidin-4-yl)amino)-2-methylhexyl 1-methyl-1*H*-pyrazole-4-carboxylate

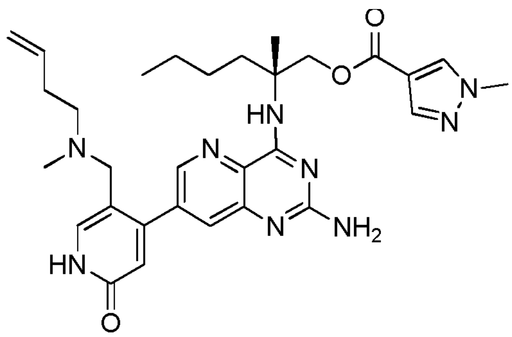

(R)-2-((Tert-butoxycarbonyl)amino)-2-methylhexyl 1-methyl-1*H*-pyrazole-4-carboxylate was used as a raw material to synthesize (R)-2-amino-2-methylhexyl 1-methyl-1*H*-pyrazole-4-carboxylate, which was prepared by referring to the synthesis method of step 5 of example 44, and then (R)-2-amino-2-methylhexyl 1-methyl-1*H*-pyrazole-4-carboxylate and 7-bromo-2,4-dichloropyrido[3,2-d]pyrimidine were used as raw materials, according to the synthesis method of step 6 to step 7 of example 44, and the title compound can be obtained as a white solid with a yield of 8.7%.

MS (ESI, *pos.ion*) *m*/*z*: 574.5 [M+H]$^+$,
$^1$H NMR (400 MHz, $CD_3OD$): δ (ppm) 8.66 (s, 1H), 8.17 (s, 1H), 7.97 (s, 2H), 7.85 (s, 1H), 6.61 (s, 1H), 5.75 - 5.59 (m, 1H), 5.17 - 5.06 (m, 2H), 4.61 (d, *J* = 11.3 Hz, 1H), 4.26 (s, 2H), 3.92 (s, 3H), 3.03 (s, 2H), 2.62 (s, 3H), 2.34 (d, *J* = 7.5 Hz, 3H), 1.99 (t, *J* = 10.3 Hz, 1H), 1.70 (s, 3H), 1.53 - 1.36 (m, 5H), 0.96 (t, *J* = 6.7 Hz, 3H).

**Example 48: Examples 48 can be obtained by referring to the synthesis method of Example 47. The structures and characterization data of Examples 48 are shown in Table 5 below.**

| Example No. | Structure of Example | Status, color and characterization data |
|---|---|---|
| 48 | 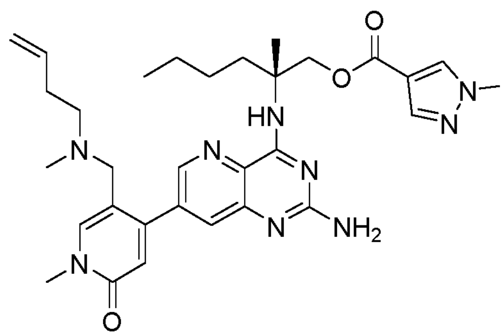 | White solid (yield 90.39%); MS (ESI, *pos.ion) m/z:* 588.5 [M+H]$^+$, $^1$H-NMR (400 MHz, $CD_3OD$): δ (ppm) 8.66 (s, 1H), 8.17 (d, *J* = 7.0 Hz, 2H), 7.97 (s, 1H), 7.84 (s, 1H), 6.62 (s, 1H), 5.72 - 5.61 (m, 1H), 5.13 - 5.08 (m, 2H), 4.61 (d, *J* = 11.3 Hz, 1H), 4.25 (s, 2H), 3.92 (s, 3H), 3.69 |
| | | (s, 3H), 3.03 (s, 2H), 2.63 (s, 3H), 2.38 - 2.34 (m, 3H), 2.08 - 1.91 (m, 1H), 1.70 (s, 3H), 1.56 - 1.37 (m, 4H), 0.96 (t, *J* = 6.7 Hz, 3H). |

**Example** 49: **(*R*)-4-(2-Amino-4-((1-hydroxy-2-methylhexan-2-yl)amino)pyrido[3,2-*d*]pyrimidin-7-yl)-5-((me thylamino)methyl)pyridin-2(1*H*)-one**

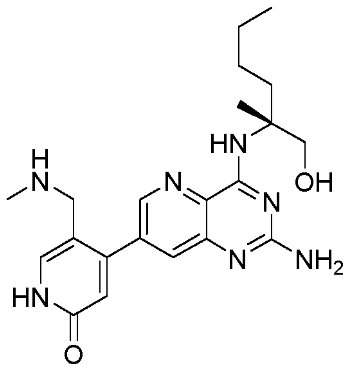

2-Fluoro-4-iodo-5-methylpyridine and $CH_3NHBoc$ were used as raw materials to obtain the compound of 4-iodo-5-((methylamino)methyl)pyridin-2(1*H*)-one according to the synthesis method of steps 1-3 of example 56, then 4-iodo-5-((methylamino)methyl)pyridin-2(1*H*)-one and (R)-(2-((2,4-dimethoxybenzyl)amino)-4-((1-hydroxy-2-methylhexan-2-yl)amino)pyrido[3,2-d]pyri midin-7-yl)boronic acid were used as raw materials, according to the synthesis method of step 5 of example 56, and the title compound can be obtained as a yellow solid with a total yield of 16%.

MS (ESI, pos.ion) m/z: 412.2 $[M+H]^+$;
$^1H$ NMR (400 MHz, $CD_3OD$): δ (ppm) 8.64 (d, *J* = 1.4 Hz, 1H), 7.94 (d, *J* = 1.4 Hz, 1H), 7.88 (s, 1H), 6.60 (s, 1H), 4.09 (s, 2H), 4.01 (d, *J* = 11.1 Hz, 1H), 3.75 (d, *J* = 11.2 Hz, 1H), 2.59 (s, 3H), 2.19 - 2.08 (m, 1H), 2.06 - 1.93 (m, 1H), 1.59 (s, 3H), 1.38 (s, 4H), 0.95 (t, *J*= 6.5 Hz, 3H). **Example** 50:

**(*R*)-4-(2-Amino-4-((1-hydroxy-2-methylhexan-2-yl)amino)pyrido[3,2-*d*]pyrimidin-7-yl)-5-((di methylamino) methyl)pyridin-2(1*H*)-one**

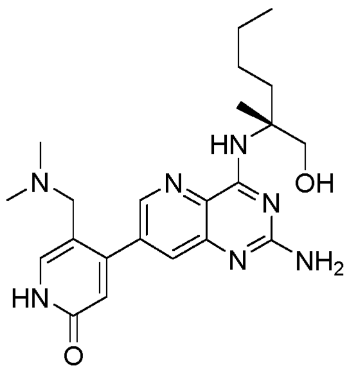

2-Fluoro-4-iodo-5-methylpyridine and dimethylamine were used as raw materials to obtain the compound of 5-((dimethylamino)methyl)-4-iodopyridine-2(1*H*)-one according to the synthesis method of steps 1-3 of example 56, then 5-((dimethylamino)methyl)-4-iodopyridine-2(1*H*)-one and (R)-(2-((2,4-dimethoxybenzyl)amino)-4-((1-hydroxy-2-methylhexan-2-yl)amino)pyrido[3,2-*d*]pyri midin-7-yl)boronic acid were used as raw materials, according to the synthesis method of step 5 of example 56, and the title compound can be obtained as a yellow solid with a total yield of 33%.

MS (ESI, pos.ion) m/z: 426.3 $[M+H]^+$;
$^1H$ NMR (400 MHz, $CD_3OD$): δ (ppm) 8.64 (d, *J*= 1.6 Hz, 1H), 7.99 - 7.92 (m, 2H), 6.61 (s, 1H), 4.23 (s, 2H), 4.00 (d, *J* = 11.2 Hz, 1H), 3.75 (d, *J*= 11.2 Hz, 1H), 2.68 (s, 6H), 2.18 - 2.08 (m, 1H), 2.03 - 1.96 (m, 1H), 1.59 (s, 3H), 1.44 - 1.35 (m, 4H), 0.95 (t, *J*= 6.7 Hz, 3H).

**Example 51: (*R*)-4-(2-Amino-4-((1-hydroxy-2-methylhexan-2-yl)amino)pyrido[3,2-*d*]pyrimidin-7-yl)-5-(met hoxymethyl)pyridin-2(1*H*)-one**

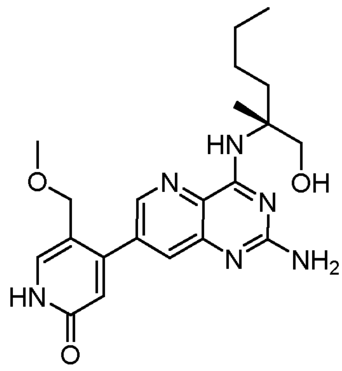

Step 1: 4-Iodo-2-methoxy-5-(methoxymethyl)pyridine

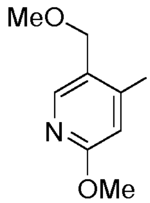

5-(Bromomethyl)-2-fluoro-4-iodopyridine (1.10 g, 3.48 mmol) was dissolved in a solution of sodium methoxide in methanol (6.57 mL, 34.8 mmol). The mixture was stirred at room temperature for about 3 hours, then concentrated *in vacuo* to remove the solvent, the residue was diluted with water (20 mL) and ethyl acetate (20 mL), the resulted mixture was stirred and the resulted phases were separated, the aqueous phase was extracted with ethyl acetate (20 mL × 2), the organic phases were combined, washed with saturated brine (20 mL), dried over anhydrous sodium sulfate, filtered, and the solvent was concentrated to obtain the title compound as a yellow solid (0.88 g, yield 90.30%).

Step 2: 4-Iodo-5-(methoxymethyl)pyridin-2(1H)-one

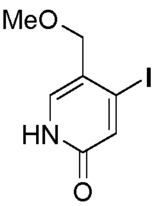

Sodium iodide (2.35 g, 15.70 mmol) was suspended in ACN (20 mL), and trimethylsilyl chloride (1.71 g, 15.70 mmol) was added at 0 °C. After the addition was complete, the mixture was moved to room temperature and stirred for 15 min. 4-iodo-2-methoxy-5-(methoxymethyl)pyridine (0.877 g, 3.14 mmol) was added to the reaction system. After the addition was complete, the reaction mixture was stirred for about 17 hours, then concentrated to remove the solvent, the residue was diluted with water (10 mL) and dichloromethane (10 mL), the resulted mixture was stirred and the phases were separated, the aqueous phase was extracted with dichloromethane (10 mL × 3), the organic phases were combined, washed with saturated brine (10 mL × 3), dried over anhydrous sodium sulfate, and the solvent was concentrated. The residue was purified by silica gel column chromatography (DCM/MeOH (V/V) = 15/1) to give the title compound as a light yellow solid (0.38 g, yield 45.78%).

Step 3: (*R*)-4-(2-Amino-4-((1-hydroxy-2-methylhexan-2-yl)amino)pyrido[3,2-*d*]pyrimidin-7-yl)-5-(methox ymethyl)pyridin-2(1*H*)-one

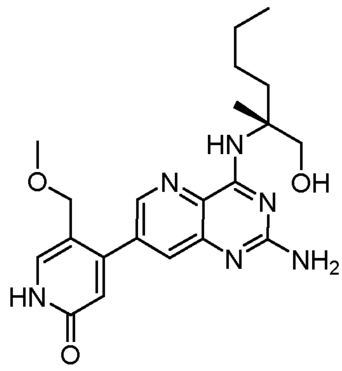

4-Iodo-5-(methoxymethyl)pyridin-2(1*H*)-one and (*R*)-(2-((2,4-dimethoxybenzyl)amino)-4-((1-hydroxy-2-methylhexan-2-yl)amino)pyrido[3,2-d]pyri midin-7-yl)boronic acid were used as raw materials, according to the synthesis method of steps 10-11 of example 1, and the title compound can be obtained as a white solid with a total yield of 46%.

MS (ESI, *pos.ion*) *m*/*z:* 413.2 $[M+H]^+$,

$^1H$ NMR (400 MHz, $CD_3OD$): δ (ppm) 8.67 (s, 1H), 7.95 (s, 1H), 7.64 (s, 1H), 6.57 (s, 1H), 4.12 (s, 2H), 4.00 (d, *J* = 11.2 Hz, 1H), 3.74 (d, *J* = 11.2 Hz, 1H), 3.25 (s, 3H), 2.19 - 2.07 (m, 1H), 2.02 - 1.88 (m, 1H), 1.57 (s, 3H), 1.37 (s, 4H), 0.93 (t, *J* = 6.3 Hz, 3H).

**Example 52: (*R*)-4-(2-Amino-4-((1-hydroxy-2-methylhexan-2-yl)amino)pyrido[3,2-*d*]pyrimidin-7-yl)-5-((me thylthio)methyl)pyridin-2(1*H*)-one**

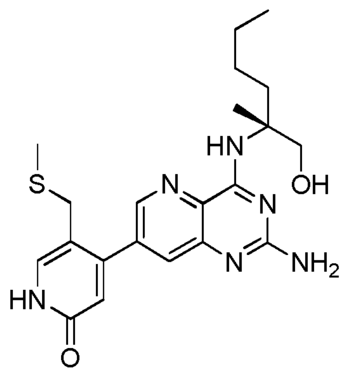

Step 1: 4-Iodo-2-methoxy-5-methylpyridine

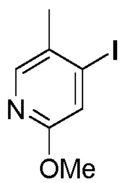

2-Fluoro-4-iodo-5-methylpyridine (4.0 g, 16.88 mmol) was dissolved in a solution of sodium methoxide in methanol (9.54 mL, 50.47 mmol) and stirred at room temperature for about 22 hours. The reaction was stopped, the solvent was concentrated, water (20 mL) and ethyl acetate (20 mL) were added, the mixture was stirred and the resulted phases were separated, the aqueous phase was extracted with ethyl acetate (20 mL × 2), the organic phases were combined, washed with saturated brine (20 mL), dried over anhydrous sodium sulfate, filtered, and the solvent was concentrated to obtain the title compound as a yellow solid (3.22 g, yield 76.55%).

MS *(ESI,pos.ion) m/z*: 250.0 $[M+H]^+$.

Step 2: 5-(Bromomethyl)-4-iodo-2-methoxypyridine

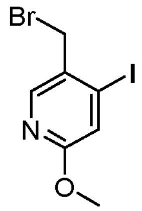

4-Iodo-2-methoxy-5-methylpyridine (3.20 g, 12.85 mmol), NBS (2.29 g, 12.85 mmol) and AIBN (2.11 g, 12.85 mmol) were dissolved in carbon tetrachloride (30 mL). The reaction mixture was degassed and refilled with $N_2$ three times, and stirred at 70 °C for about 4 hours. The stirring was stopped, the heating was turned off, the mixture was cooled to room temperature, filtered, a saturated sodium thiosulfate solution (20 mL) was added to the filtrate, the aqueous phase was extracted with dichloromethane (20 mL × 3), the organic layers were combined and washed with saturated brine (20 mL), the organic layers were combined and dried over anhydrous sodium sulfate, filtered and concentrated under reduced pressure, and the residue was purified by silica gel column chromatography (PE/EA (V/V) = 20/1) to give the title compound as a white solid (3.59 g, yield 85.19%).

Step 3: 4-Iodo-2-methoxy-5-((methylthio)methyl)pyridine

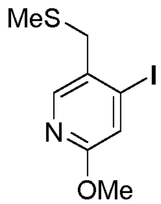

5-(Bromomethyl)-4-iodo-2-methoxypyridine (3.20 g, 9.76 mmol) and sodium thiomethoxide (0.75 g, 10.74 mmol) were added to DMF (16 L) and stirred at room temperature for about 3 hours. The reaction was stopped, ethyl acetate (20 mL) and water (20 mL) were added, the mixture was stirred and the resulted phases were separated, the aqueous phase was extracted with ethyl acetate (20 mL × 2), the organic phases were combined, washed with saturated brine (20 mL×3), dried over anhydrous sodium sulfate, filtered, and the solvent was concentrated. The residue was purified by silica gel column chromatography (PE/EA (V/V) = 10/1) to obtain the title compound as a white solid (0.54 g, yield 18.75%).
MS (ESI, *pos. ion*) *m/z*: 296.00 $[M+H]^+$.

Step 4: Synthesis of 4-iodo-5-((methylthio)methyl)pyridin-2(1*H*)-one

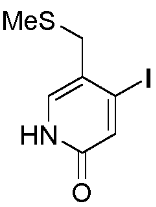

Sodium iodide (1.37 g, 9.15 mmol) was suspended in ACN (10 mL), and trimethylsilyl chloride (0.99 g, 9.15 mmol) was added at 0 °C. After the addition was complete, the mixture was moved to room temperature and stirred for 15 min. 4-Iodo-2-methoxy-5-((methylthio)methyl)pyridine (0.54 g, 1.83 mmol) was added to the reaction system. After the addition was complete, the reaction was stirred for about 23 hours. The reaction was stopped, the solvent was concentrated, water

(10 mL) and dichloromethane (10 mL) were added, the mixture was stirred and the resulted phases were separated, the aqueous phase was extracted with dichloromethane (10 mL × 3), the organic phases were combined, washed with saturated brine (10 mL × 3), dried over anhydrous sodium sulfate, filtered, and the solvent was concentrated. The residue was purified by silica gel column chromatography (DCM/MeOH (V/V) = 10/1) to give the title compound as a light yellow solid (0.11 g, yield 22.16%).
MS (ESI, *pos.ion*) *m*/*z:* 282.1 $[M+H]^+$.

Step 5: (*R*)-4-(2-Amino-4-((1-hydroxy-2-methylhexan-2-yl)amino)pyrido[3,2-*6*]pyrimidin-7-yl)-5-((methyl thio)methyl) pyridin-2(1*H*)-one

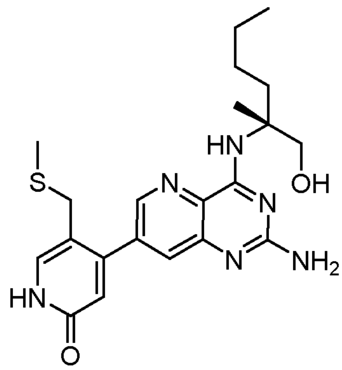

4-Iodo-5-((methylthio)methyl)pyridin-2(1*H*)-one and (*R*)-(2-((2,4-dimethoxybenzyl)amino)-4-((1-hydroxy-2-methylhexan-2-yl)amino)pyrido[3,2-d]pyri midin-7-yl)boronic acid were used as raw materials, according to the synthesis method of steps 10-11 of example 1, and the title compound can be obtained as a white solid with a total yield of 13%.

MS (ESI, *pos.ion) m*/z: 429.4 $[M+H]^+$,
$^1$H NMR (400 MHz, $CD_3OD$) δ (ppm) 8.62 (d, *J* = 1.4 Hz, 1H), 7.89 (d, *J* = 1.3 Hz, 1H), 7.50 (s, 1H), 6.50 (s, 1H), 3.96 (d, *J* = 11.3 Hz, 1H), 3.71 (d, *J* = 11.3 Hz, 1H), 3.44 (s, 2H), 3.27 (d, *J* = 1.4 Hz, 2H), 2.09 (d, *J* = 14.4 Hz, 1H), 1.90 (s, 3H), 1.53 (s, 3H), 1.33 (s, 4H), 0.90 (t, *J* = 6.5 Hz, 3H).

**Example 53: (*R*)-4-(2-Amino-4-((1-methoxy-2-methylhexan-2-yl)amino)pyrido[3,2-*d*]pyrimidin-7-yl)-5-((bu tyl(methyl)amino)methyl)pyridin-2(1*H*)-one**

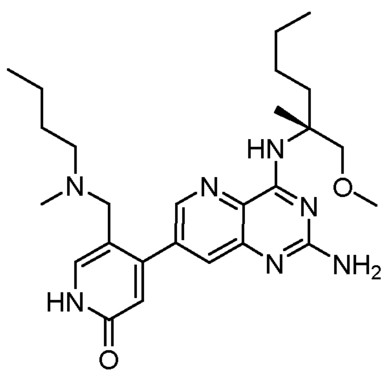

Step 1: Tert-butyl (*R*)-(1-methoxy-2-methylhexan-2-yl)carbamate

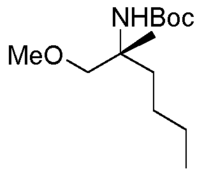

Sodium hydride (0.10 g, 4.30 mmol) was dissolved in DMF (5 mL) and stirred at 0 °C under $N_2$. Tert-butyl (R)-(1-hydroxy-2-methylhexane-2-yl)carbamate (0.50 g, 2.14 mmol) dissolved in DMF (5 mL) was slowly added dropwise. Stirring was continued for about 30 minutes under this condition. Methyl iodide (0.36 g, 2.57 mmol) was added and the mixture was stirred at room temperature for about 1 hour. The reaction was stopped, ethyl acetate (20 mL) and water (20 mL) were added, the mixture was stirred and the resulted phases were separated, the aqueous phase was extracted with ethyl acetate (20 mL × 2), the organic phases were combined, washed with saturated brine (20 mL×3), dried over anhydrous sodium sulfate, filtered, and concentrated to give a yellow oil (0.46 g, 88.18%).
MS (ESI, *pos. ion*) *m*/*z*: 146.3 [M+H-Boc]$^+$.

Step 2: (*R*)-4-(2-Amino-4-((1-methoxy-2-methylhexan-2-yl)amino)pyrido[3,2-*d*]pyrimidin-7-yl)-5-((butyl( methyl)amino) methyl)pyridin-2(1*H*)-one

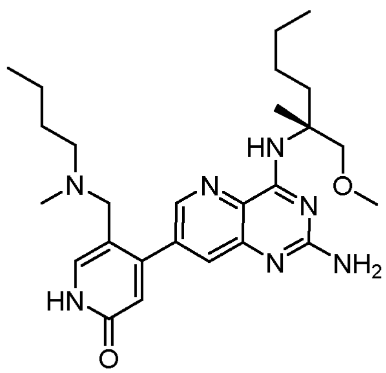

7-Bromo-2,4-dichloropyrido[3,2-*d*]pyrimidine and tert-butyl (*R*)-(1-methoxy-2-methylhexan-2-yl)carbamate were used as raw materials to obtain the compound of (*R*)-(2-((2,4-dimethoxybenzyl)amino)-4-((1-methoxy-2-methylhexan-2-yl)amino)pyrido[3,2-d]pyri midin-7-yl)boronic acid according to the synthesis method of steps 2-5 of example 1, then (*R*)-(2-((2,4-dimethoxybenzyl)amino)-4-((1-methoxy-2-methylhexan-2-yl)amino)pyrido[3,2-d]pyri midin-7-yl)boronic acid and 5-((butyl(methyl)amino)methyl)-4-iodopyridin-2(1*H*)-one were used as raw materials, according to the synthesis method of steps 10-11 of example 1, and the title compound can be obtained as a white solid with a total yield of 10.3%.

MS (ESI, *pos.ion*) *m*/*z*: 482.5 [M+H]$^+$,
$^1$H NMR (400 MHz, $CD_3OD$) δ (ppm) 8.64 (d, *J* = 1.5 Hz, 1H), 7.95 (s, 2H), 6.61 (s, 1H), 3.82 (d, *J* = 9.3 Hz, 1H), 3.58 (d, *J* = 9.4 Hz, 1H), 3.43 (s, 3H), 3.32 (s, 2H), 3.31 (s, 2H), 2.62 (s, 3H), 2.08 (t, *J* = 7.4 Hz, 2H), 1.60 (s, 3H), 1.48 - 1.32 (m, 6H), 1.28 - 1.18 (m, 2H), 0.93 (t, *J* = 6.6 Hz, 3H), 0.86 (t, *J* = 7.3 Hz, 3H).

**Example 54: (R)-4-(2-Amino-4-((1-hydroxy-2-methylhexan-2-yl)amino)quinazolin-7-yl)-5-(((2-methoxyethy l)(methyl)amino)methyl)pyridin-2(1*H*)-one**

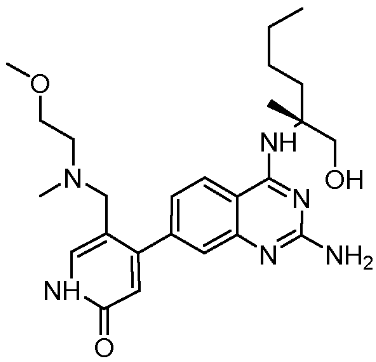

Step 1: 5-(Bromomethyl)-2-fluoro-4-iodopyridine

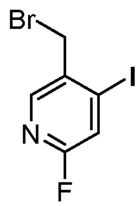

2-Fluoro-4-iodo-5-methylpyridine (20.00 g, 84.38 mmol), N-bromosuccinimide (16.52 g, 92.82 mmol) and 2,2'-azobis(2-methylpropionitrile) (1.39 g, 8.44 mmol) were weighed and mixed in carbon tetrachloride (200 mL). Under $N_2$, the resulted mixture was refluxed in an oil bath at 80 °C for about 14 hours. The stirring was stopped and saturated sodium thiosulfate solution (30 mL) was added to the reaction system, the mixture was extracted with dichloromethane (100 mL × 3). The organic phases were combined, washed with saturated brine (50 mL), dried over anhydrous sodium sulfate, and concentrated under reduced pressure. The residue was purified by silica gel column chromatography (PE/EA (V/V) = 20/1) to obtain the title compound as a white solid (19.56 g, yield 73.38%).
$^1$HNMR(400MHz, $CDCl_3$) δ (ppm) 8.25 (s, 1H), 7.51 (d, *J*= 3.1Hz, 1H), 4.58 (s, 2H).

Step 2: *N*-((6-Fluoro-4-iodopyridin-3-yl)methyl)-2-methoxy-*N*-methylethan-1-amine

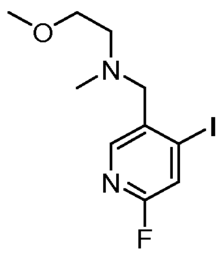

5-(Bromomethyl)-2-fluoro-4-iodopyridine (1.00 g, 3.17 mmol), *N*-(2-methoxyethyl)methylamine (0.93 g, 10.46 mmol) and DIPEA (4.10 g, 31.69 mmol) were weighed separately and mixed in dichloromethane (50 mL). The mixture was stirred at room temperature for about 2 hours, then concentrated under reduced pressure, and the residue was purified by silica gel column chromatography (PE/EA (V/V) = 3/1) to obtain the title compound as a colorless oily liquid (0.76 g, yield 73.97%).
MS (ESI, *pos.ion*) *m*/z: 325.2 [M+H]$^+$.

Step 3: 4-Iodo-5-(((2-methoxyethyl)(methyl)amino)methyl)pyridin-2(1*H*)-one

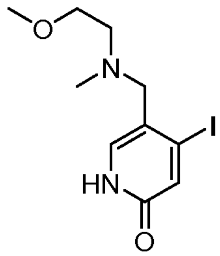

*N*-((6-Fluoro-4-iodopyridin-3-yl)methyl)-2-methoxy-*N*-methylethan-1-amine (0.65 g, 2.01 mmol) and potassium tert-butoxide (0.90 g, 8.02 mmol) were weighed and mixed in tert-butanol (30 mL),.The mixture was heated in an oil bath for about 18 hours at 90 °C, then the pH of the mixture was adjusted to 2-3 with 4 M hydrochloric acid in 1,4-dioxane , and stirred for about 15 minutes; then saturated sodium bicarbonate solution was added to the system to adjust the pH to 7-8, and the mixture was extracted with dichloromethane (30 mL × 3), the organic phases were combined, washed with saturated brine (30 mL), dried over anhydrous sodium sulfate, and concentrated under reduced pressure to obtain the title compound as a light yellow solid (0.63 g, yield 97.30%).
MS (ESI, *pos.ion*) *m*/*z*: 323.0 [M+H]+.

Step 4: (*R*)-4-(2-Amino-4-((1-hydroxy-2-methylhexan-2-yl)amino)quinazolin-7-yl)-5-(((2-methoxyethyl)( methyl)amino) methyl)pyridin-2(1*H*)-one

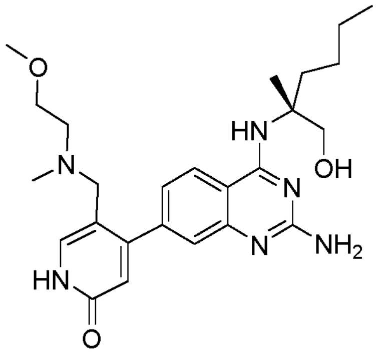

4-Iodo-5-(((2-methoxyethyl)(methyl)amino)methyl)pyridin-2(1*H*)-one and (*R*)-(2-((2,4-dimethoxybenzyl)amino)-4-((1-hydroxy-2-methylhexan-2-yl)amino)quinazolin-7-yl)bo ronic acid were used as raw materials, according to the synthesis method of steps 10-11 of example 1, and the title compound can be obtained as a white solid with a total yield of 39%.

MS (ESI, *pos. ion) m*lz: 469.3 [M+H]+,

$^1$H NMR (400 MHz, $CD_3OD$): $\delta$ (ppm) 8.41 (d, *J* = 8.4 Hz, 1H), 7.92 (s, 1H), 7.50 (s, 1H), 7.44 (d, *J* = 8.4 Hz, 1H), 6.55 (s, 1H), 4.30 (s, 1H), 4.20 (d, *J* = 11.2 Hz, 1H), 3.73 (d, *J* = 11.2 Hz, 1H), 3.55 (t, *J*= 4.9 Hz, 2H), 3.39 - 3.31 (m, 3H), 3.26 (s, 3H), 3.17 (s, 2H), 2.63 (s, 3H), 2.34 - 2.23 (m, 1H), 1.91 - 1.78 (m, 1H), 1.57 (s, 3H), 1.44 - 1.27 (m, 4H), 0.93 (t, *J*= 6.9 Hz, 3H).

**Example 55: (*R*)-4-(2-Amino-4-((1-hydroxy-2-methylhexan-2-yl)amino)quinazolin-7-yl)-5-(((2-methoxyethy l)(methyl)amino)methyl)-1-methylpyridin-2(1*H*)-one**

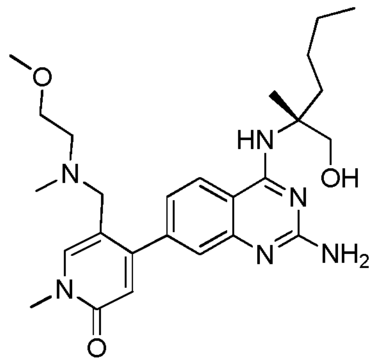

4,6-Dichloronicotinaldehyde and 2-methoxyethane-1-amine were used as raw materials to obtain the compound of 4-iodo-5-(((2-methoxyethyl)(methyl)amino)methyl)-1-methylpyridin-2(1*H*)-one according to the synthesis method of steps 6-9 of example 1, then, 4-iodo-5-(((2-methoxyethyl)(methyl)amino)methyl)-1-methylpyridin-2(1*H*)-one and (*R*)-(2-((2,4-dimethoxybenzyl)amino)-4-((1-hydroxy-2-methylhexan-2-yl)amino)quinazolin-7-yl)bo ronic acid were used as raw materials, according to the synthesis method of steps 10-11 of example 1, and the title compound can be obtained as a white solid with a total yield of 24%.

MS (ESI, *pos.ion*) *m*/*z*: 483.3 [M+H]$^+$,
$^1$H NMR (400 MHz, $CD_3OD$): δ(ppm) 8.41 (d, *J* = 8.4 Hz, 1H), 8.12 (s, 1H), 7.50 (s, 1H), 7.43 (d, *J* = 8.4 Hz, 1H), 6.57 (s, 1H), 4.30 (s, 1H), 4.20 (d, *J* = 11.3 Hz, 1H), 3.73 (d, *J* = 11.2 Hz, 1H), 3.69 (s, 3H), 3.55 (t, *J* = 4.9 Hz, 2H), 3.33 (s, 2H), 3.26 (s, 3H), 3.18 (s, 2H), 2.64 (s, 3H), 2.35 - 2.20 (m, 1H), 1.91 - 1.79 (m, 1H), 1.56 (s, 3H), 1.44 - 1.25 (m, 4H), 0.93 (t, *J* = 6.8 Hz, 3H).

**Example 56: (R)-4-(2-Amino-4-((1-hydroxy-2-methylhexan-2-yl)amino)quinazolin-7-yl)-5-((butyl(methyl)a mino) methyl)-1-methylpyridin-2(1*H*)-one**

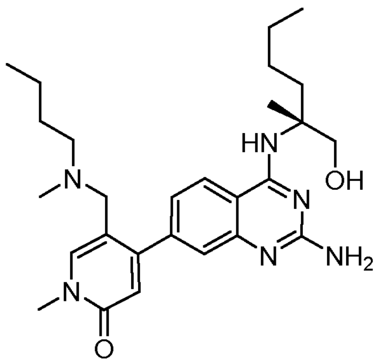

Step 1: 5-(Bromomethyl)-2-fluoro-4-iodopyridine

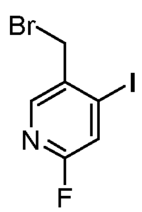

2-Fluoro-4-iodo-5-methylpyridine (20.00 g, 84.38 mmol), N-bromosuccinimide (16.52 g, 92.82 mmol) and 2,2'-azobis(2-methylpropionitrile) (1.39 g, 8.44 mmol) were weighed and mixed in carbon tetrachloride (200 mL). Under $N_2$, the mixture was refluxed in an oil bath at 80 °C for about 14 hours, then the temperature of the mixture was cooled to room

temperature. Saturated sodium thiosulfate solution (30 mL) was added to the reaction system, the mixture was extracted with dichloromethane (100 mL × 3). The organic phases were combined, washed with saturated brine (50 mL), dried over anhydrous sodium sulfate, and concentrated under reduced pressure. The residue was purified by silica gel column chromatography (PE/EA (V/V) = 20/1) to obtain the title compound as a white solid (19.56 g, yield 73.38%).
$^1$HNMR (400MHz, $CDCl_3$): $\delta$ (ppm) 8.25 (s, 1H), 7.51 (d, *J*=3.1Hz, 1H), 4.58 (s, 2H).

Step 2: *N*-((6-Fluoro-4-iodopyridin-3-yl)methyl)-*N*-methylbutan-1-amine

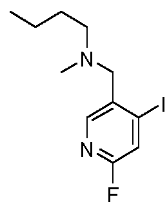

5-(Bromomethyl)-2-fluoro-4-iodopyridine (6.90 g, 21.84 mmol), *N*-methyl-1-butylamine (5.71 g, 65.52 mmol) and DIPEA (14.11 g, 109.18 mmol) were weighed separately and mixed in dichloromethane (100 mL). The mixture was stirred at room temperature for about 30 min, then concentrated under reduced pressure, and the residue was purified by silica gel column chromatography (PE/EA (V/V) = 20/1) to obtain the title compound as a light yellow oily liquid (6.52 g, yield 92.67%).
MS (ESI, *pos.ion*) *m*/*z:* 323.1 [M+H]$^+$.

Step 3: 5-((Butyl(methyl)amino)methyl)-4-iodopyridin-2(1*H*)-one

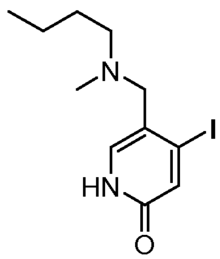

*N*-((6-Fluoro-4-iodopyridin-3-yl)methyl)-*N*-methylbutan-1-amine (6.50 g, 20.18 mmol) was weighed and dissolved in tert-butanol (50 mL), then potassium tert-butoxide (9.05 g, 80.69 mmol) was added and stirred at room temperature for about 47 hours. After the reaction was stopped, the pH of the reaction mixture was adjusted to 1-3 with 6 M hydrochloric acid in 1,4-dioxane, and the resulted mixture was stirred at room temperature for about 30 minutes; then saturated sodium bicarbonate solution was added to adjust the pH to 7-9, the mixture was extracted with ethyl acetate (30 mL × 3), the organic phases were combined, washed with saturated brine (35 mL), dried over anhydrous sodium sulfate, and concentrated under reduced pressure to obtain the title compound as an off-white solid (5.14 g, yield 79.55%).
MS (ESI, *pos.ion*) *m*/*z:* 321.1 [M+H]$^+$.

Step 4: 5-((Butyl(methyl)amino)methyl)-4-iodo-1-methylpyridin-2(1*H*)-one

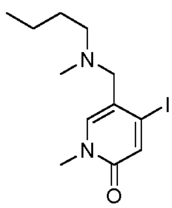

5-((Butyl(methyl)amino)methyl)-4-iodopyridin-2(1*H*)-one (2.46 g, 10.42 mmol), cesium carbonate (0.60 g, 1.83 mmol) and iodomethane (0.21 g, 1.46 mmol) were weighed separately and mixed in DMF (30 mL). The mixture was stirred at room temperature for about 4 hours. Water (30 mL) was added for dilution, and then the mixture was extracted with ethyl acetate (30 mL × 3). The organic phases were combined, washed with saturated brine (30 mL × 3), dried over anhydrous sodium sulfate, and concentrated under reduced pressure. The residue was purified by silica gel column chromatography (PE/EA(V/V) = 10/1) to obtain the title compound as a light yellow oily liquid (0.23 g, 56.41%).
MS *(ESI, pos.ion) m*/*z:* 335.1 [M+H]$^+$.

Step 5: (*R*)-4-(2-Amino-4-((1-hydroxy-2-methylhexan-2-yl)amino)quinazolin-7-yl)-5-((butyl(methyl)amin o)methyl)-1-methylpyridin-2(1*H*)-one

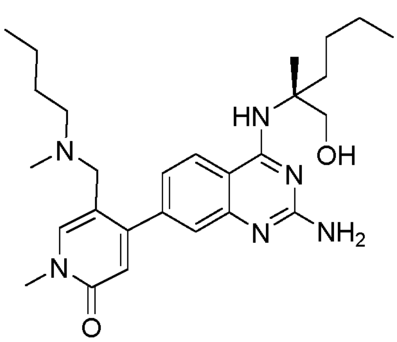

5-((Butyl(methyl)amino)methyl)-4-iodo-1-methylpyridin-2(1*H*)-one and (*R*)-(2-((2,4-dimethoxybenzyl)amino)-4-((1-hydroxy-2-methylhexan-2-yl)amino)quinazolin-7-yl)bo ronic acid were used as raw materials, according to the synthesis method of steps 10-11 of example 1, and the title compound can be obtained as a white solid with a total yield of 16%.

MS (ESI, *pos.ion) m*/z: 481.4 [M+H]$^+$,
$^1$H NMR (400 MHz, $CD_3OD$): δ (ppm) 8.42 (d, *J* = 8.4 Hz, 1H), 8.15 (s, 1H), 7.51 (d, *J*= 0.9 Hz, 1H), 7.45 (d, *J* = 8.4 Hz, 1H), 6.57 (s, 1H), 4.36 (s, 1H), 4.19 (d, *J* = 11.2 Hz, 1H), 3.72 (d, *J* = 11.3 Hz, 1H), 3. 68 (s, 3H), 3.33 (t, *J* = 1.6 Hz, 2H), 2.99 - 2.78 (m, 2H), 2.64 (s, 3H), 2.34 - 2.23 (m, 1H), 1.91 - 1.80 (m, 1H), 1.57 (s, 3H), 1.45 - 1.18 (m, 8H), 0.93 (t, *J*= 6.9 Hz, 3H), 0.84 (t, *J*= 7.3 Hz, 3H).

Example 57: (*R*)-4-(2-Amino-4-((1-hydroxy-2-methylhexan-2-yl)amino)quinazolin-7-yl)-5-(((2-methoxyethyl)a mino) methyl)pyridin-2(1*H*)-one

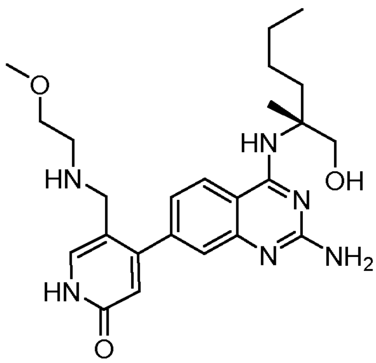

5-(Bromomethyl)-2-fluoro-4-iodopyridine and 2-methoxyethane-1-amine were used as raw materials to obtain the compound of 4-iodo-5-(((2-methoxyethyl)methyl)amino)pyridin-2(1*H*)-one according to the synthesis method of steps 2-3 of example 56, then, 4-iodo-5-(((2-methoxyethyl)methyl)amino)pyridin-2(1*H*)-one and (*R*)-(2-((2,4-dimethoxybenzyl) amino)-4-((1-hydroxy-2-methylhexan-2-yl)amino)quinazolin-7-yl)bo ronic acid were used as raw materials, according to the synthesis method of step 5 of example 56, and the title compound can be obtained as a yellow solid with a total yield of

6.4%.

MS *(ESI, pos. ion) m/z*: 455.6 [M+H]+,
$^1$H NMR (400 MHz, $CD_3OD$): δ (ppm) 8.39 (d, *J* = 8.4 Hz, 1H), 7.84 (s, 1H), 7.49 - 7.39 (m, 2H), 6.53 (s, 1H), 4.19 (d, *J* = 11.2 Hz, 1H), 4.08 (s, 2H), 3.71 (d, *J* = 11.2 Hz, 1H), 3. 49 (t, *J* = 5.0 Hz, 2H), 3.24 (s, 3H), 3.06 (t, *J* = 4.8 Hz, 2H), 2.26 (t, *J* = 10.5 Hz, 1H), 1.87 - 1.77 (m, 1H), 1.55 (s, 3H), 1.40 - 1.29 (m, 4H), 0.92 (t, *J*= 6.9 Hz, 3H).

**Example** 58: **(R)-4-(2-Amino-4-((1-hydroxy-2-methylhexan-2-yl)amino)quinazolin-7-yl)-5-((butylamino)met hyl) pyridin-2(1*H*)-one**

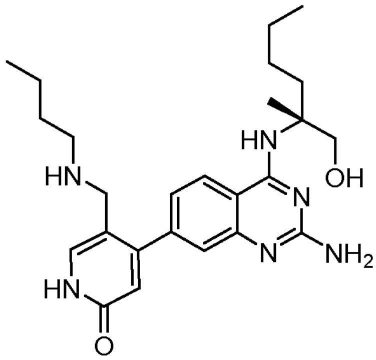

5-(Bromomethyl)-2-fluoro-4-iodopyridine and n-butylamine were used as raw materials to obtain the compound of 5-((butylamino)methyl)-4-iodopyridine-2(1*H*)-one according to the synthesis method of steps 2-3 of example 56, then 5-((butylamino)methyl)-4-iodopyridine-2(1*H*)-one and (R)-(2-((2,4-dimethoxybenzyl)amino)-4-((1-hydroxy-2-methylhexan-2-yl)amino)quinazolin-7-yl)bo ronic acid were used as raw materials, according to the synthesis method of step 5 of example 56, and the title compound can be obtained as a yellow solid with a total yield of 3.8%.

MS (ESI, *pos.ion*) *m/z*: 453.5 [M+H]+,
$^1$H NMR (400 MHz, $CD_3OD$): δ (ppm) 8.38 (d, *J* = 8.4 Hz, 1H), 7.85 (s, 1H), 7.52 - 7.37 (m, 2H), 6.53 (s, 1H), 4.18 (d, *J* = 11.2 Hz, 1H), 4.05 (s, 2H), 3.71 (d, *J* = 11.3 Hz, 1H), 2.82 (t, *J* = 8.0 Hz, 2H), 2.32 - 2.20 (m, 1H), 1.89 - 1.76 (m, 1H), 1.55 (s, 3H), 1.50 - 1.42 (m, 2H), 1.41 - 1.20 (m, 8H), 0.92 (t, *J* = 6.8 Hz, 3H), 0.86 (t, *J* = 7.3 Hz, 3H).

**Example** 59: **(*R*)-4-(2-Amino-4-((1-hydroxy-2-methylhexan-2-yl)amino)quinazolin-7-yl)-5-((methylamino)m ethyl)pyridin-2(1*H*)-one**

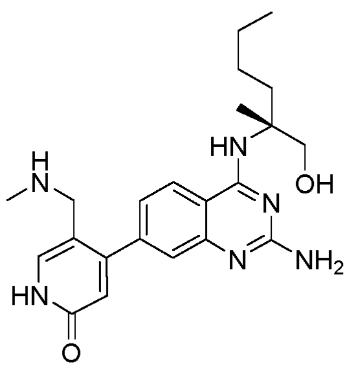

5-(Bromomethyl)-2-fluoro-4-iodopyridine and Boc methylamine were used as raw materials to obtain the compound of tert-butyl ((4-iodo-6-oxo-1,6-dihydropyridin-3-yl)methyl)(methyl)carbamate according to the synthesis method of steps 2-3 of example 56, then, tert-butyl ((4-iodo-6-oxo-1,6-dihydropyridin-3-yl)methyl)(methyl)carbamate and (*R*)-(2-((2,4-dimethoxybenzyl)amino)-4-((1-hydroxy-2-methylhexan-2-yl)amino)quinazolin-7-yl)bo ronic acid were used as raw materials, according to the synthesis method of step 5 of example 56, and the title compound can be obtained

as a yellow solid with a total yield of 77%.

MS (ESI, *pos.ion) m/z*: 411.5 $[M+H]^+$,
$^1H$ NMR (400 MHz, $CD_3OD$): δ (ppm) 8.40 (d, *J* = 8.4 Hz, 1H), 7.85 (s, 1H), 7.47 (s, 1H), 7.43 (d, *J* = 8.3 Hz, 1H), 6.54 (s, 1H), 4.20 (d, *J* = 11.3 Hz, 1H), 4.07 (s, 2H), 3.73 (d, *J* = 11.2 Hz, 1H), 2.56 (s, 3H), 2.30 (d, *J* = 12.8 Hz, 1H), 1.84 (t, *J* = 10.7 Hz, 1H), 1.57 (s, 3H), 1.44 - 1.32 (m, 4H), 0.94 (t, *J* = 6.8 Hz, 3H).

**Example 60: (*R*)-4-(2-Amino-4-((1-hydroxy-2-methylhexan-2-yl)amino)quinazolin-7-yl)-5-(((2-hydroxyethyl ) (methyl)amino)methyl)pyridin-2(1*H*)-one**

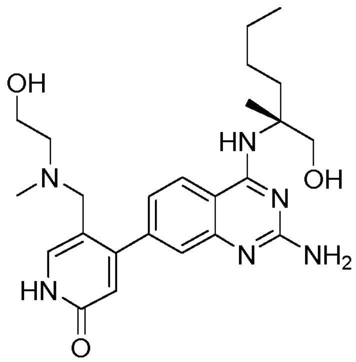

5-(Bromomethyl)-2-fluoro-4-iodopyridine and 2-(methylamino)ethane-1-ol were used as raw materials to obtain the compound of 5-(((2-hydroxyethyl)(methyl)amino)methyl)-4-iodopyridin-2(1*H*)-one according to the synthesis method of steps 2-3 of example, then, 5-(((2-hydroxyethyl)(methyl)amino)methyl)-4-iodopyridin-2(1*H*)-one and (*R*)-(2-((2,4-dimethoxybenzyl)amino)-4-((1-hydroxy-2-methylhexan-2-yl)amino)quinazolin-7-yl)bo ronic acid were used as raw materials, according to the synthesis method of step 5 of example 56, and the title compound can be obtained as a white solid with a total yield of 19%.

MS (ESI, *pos. ion) m/z*: 455.3 $[M+H]^+$,
$^1H$ NMR (400 MHz, $CD_3OD$): δ (ppm) 8.40 (d, *J* = 8.4 Hz, 1H), 7.95 (s, 1H), 7.51 (s, 1H), 7.43 (d, *J*= 8.4 Hz, 1H), 6.55 (s, 1H), 4.32 (s, 2H), 4.19 (d, *J* = 11.3 Hz, 1H), 3.67 - 3.79 (m, 3H), 3.08 (s, 2H), 2.66 (s, 3H), 2.34 - 2.22 (m, 1H), 1.91 - 1.78 (m, 1H), 1.57 (s, 3H), 1.43 - 1.27 (m, 4H), 0.94 (t, J = 6.8 Hz, 3H).

**Example 61: (*R*)-4-(2-Amino-4-((1-hydroxy-2-methylhexan-2-yl)amino)quinazolin-7-yl)-5-(((2-methoxyethy l) amino)methyl)-1-methylpyridin-2(1*H*)-one**

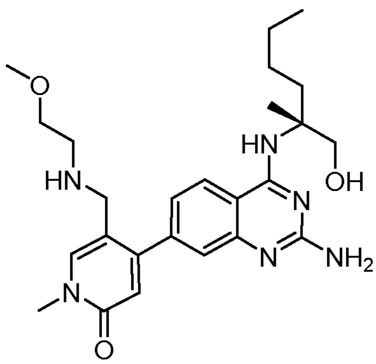

5-(Bromomethyl)-2-fluoro-4-iodopyridine, 2-methoxyethane-1-amine and (*R*)-(2-((2,4-dimethoxybenzyl)amino)-4-((1-hydroxy-2-methylhexan-2-yl)amino)quinazolin-7-yl)bo ronic acid were used as raw materials, according to the synthesis method of steps 2-5 of example 56, and the title compound can be obtained as a white solid with a total yield of 24%.

MS (ESI, *pos.ion) m*/z: 469.5 [M+H]$^+$,
$^1$H NMR (400 MHz, $CD_3OD$): δ (ppm) 8.38 (d, *J* = 8.4 Hz, 1H), 8.05 (s, 1H), 7.49 (s, 1H), 7.41 (d, *J* = 8.4 Hz, 1H), 6.53 (s, 1H), 4.18 (d, *J* = 11.3 Hz, 1H), 4.09 (s, 2H), 3.71 (d, *J* = 11.3 Hz, 1H), 3.65 (s, 3H), 3.49 (t, *J* = 5.0 Hz,2H), 3.24 (s, 3H), 3.06 (t, *J* = 11.3 Hz,2H), 2.32 - 2.20 (m, 1H), 1.87 - 1.76 (m, 1H), 1.55 (s, 3H), 1.42 - 1.23 (m, 4H), 0.91 (t, *J*= 6.9 Hz, 3H).

**Example** 62: **(R)-4-(2-Amino-4-((1-hydroxy-2-methylhexan-2-yl)amino)quinazolin-7-yl)-5-((butylamino)met hyl)-1-methylpyridin-2(1*H*)-one**

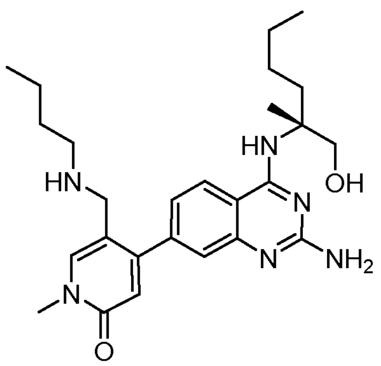

5-(Bromomethyl)-2-fluoro-4-iodopyridine, n-butylamine and (*R*)-(2-((2,4-dimethoxybenzyl)amino)-4-((1-hydroxy-2-methylhexan-2-yl)amino)quinazolin-7-yl)bo ronic acid were used as raw materials, according to the synthesis method of steps 2-5 of example 56, and the title compound can be obtained as a white solid with a total yield of 11%.

MS (ESI, *pos.ion*) *m*/*z*: 467.5 [M+H]$^+$,
$^1$H NMR (400 MHz, $CD_3OD$): δ (ppm) 8.38 (d, *J* = 8.4 Hz, 1H), 8.06 (s, 1H), 7.49 (s, 1H), 7.41 (dd, *J* = 8.4, 1.2 Hz, 1H), 6.53 (s, 1H), 4.18 (d, *J* = 11.3 Hz, 1H), 4.06 (s, 2H), 3.71 (d, *J* = 11.3 Hz, 1H), 3.64 (s, 3H), 3.35 (s, 2H), 2.87 - 2.78 (m, 2H), 2.31 - 2.19 (m, 1H), 1.90 - 1.76 (m, 1H), 1.53 (s, 3H), 1.50 - 1.42 (m, 2H), 1.42 - 1.21 (m, 7H), 0.91 (t, *J* = 6.8 Hz, 3H), 0.85 (t, *J* = 7.3 Hz, 3H).

**Example 63: (*R*)-4-(2-Amino-4-((1-hydroxy-2-methylhexan-2-yl)amino)quinazolin-7-yl)-1-methyl-5-((methy lamino)methyl)pyridin-2(1*H*)-one**

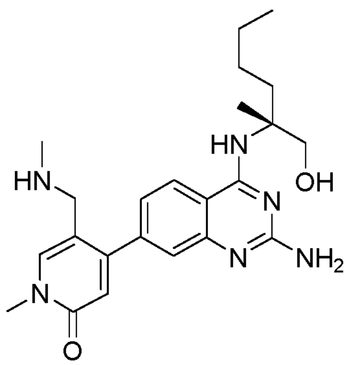

5-(Bromomethyl)-2-fluoro-4-iodopyridine and Boc methylamine were used as raw materials to obtain the compound of tert-butyl ((4-iodo-6-oxo-1,6-dihydropyridin-3-yl)methyl)(methyl)carbamate according to the synthesis method of steps 2-3 of example 56, then, tert-butyl ((4-iodo-6-oxo-1,6-dihydropyridin-3-yl)methyl)(methyl)carbamate and (*R*)-(2-((2,4-dimethoxybenzyl)amino)-4-((1-hydroxy-2-methylhexan-2-yl)amino)quinazolin-7-yl)bo ronic acid were used as raw materials, according to the synthesis method of steps 4-5 of example 56, and the title compound can be obtained as a white solid with a total yield of 57%.
MS (ESI, *pos. ion*) *m*/*z*: 425.5 [M+H]$^+$.

**Example** 64: **(*R*)-4-(2-Amino-4-((1-hydroxy-2-methylhexan-2-yl)amino)quinazolin-7-yl)-5-(((2-hydroxyethyl ) (methyl)amino)methyl)-1-methylpyridin-2(1*H*)-one**

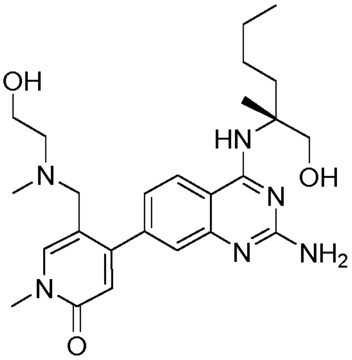

5-(Bromomethyl)-2-fluoro-4-iodopyridine, 2-(methylamino)ethane-1-ol and (*R*)-(2-((2,4-dimethoxybenzyl)amino)-4-((1-hydroxy-2-methylhexan-2-yl)amino)quinazolin-7-yl)bo ronic acid were used as raw materials, according to the synthesis method of steps 2-5 of example 56, and the title compound can be obtained as a white solid with a total yield of 16%.

MS (ESI, *pos.ion) m/z*: 469.3 $[M+H]^+$,
$^1H$ NMR (400 MHz, $CD_3OD$): δ (ppm) 8.40 (d, *J* = 8.4 Hz, 1H), 8.14 (s, 1H), 7.50 (s, 1H), 7.43 (d, *J* = 8.4 Hz, 1H), 6.57 (s, 1H), 4.31 (s, 2H), 4.19 (d, *J* = 11.3 Hz, 1H), 3.78 - 3.70 (m, 3H), 3.69 (s, 3H), 3.09 (s, 2H), 2.66 (s, 3H), 2.34 - 2.21 (m, 1H), 1.91 - 1.79 (m, 1H), 1.57 (s, 3H), 1.44 - 1.25 (m, 4H), 0.94 (t, *J* = 6.8 Hz, 3H).

**Example** 65: **(R)-4-(2-Amino-4-((1-hydroxy-2-methylhexan-2-yl)amino)quinazolin-7-yl)-5-(((2-methoxyethy l) (methyl)amino)methyl)pyridine-2(1*H*)-thione**

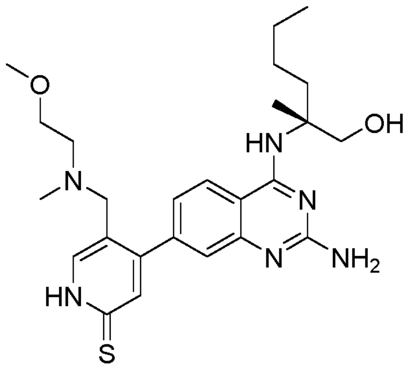

Step 1: *N*-((6-Fluoro-4-iodopyridin-3-yl)methyl)-2-methoxy-*N*-methylethan-1-amine

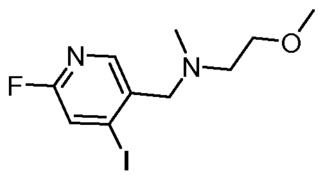

5-(Bromomethyl)-2-fluoro-4-iodopyridine and 2-methoxyethane-1-amine were used as raw materials, according to the synthesis method of step 2 of example 56, and the title compound can be obtained as a light yellow oily liquid with a

total yield of 90%.
MS (ESI, *pos.ion*) *m*/*z*: 325.1 $[M+H]^+$.

Step 2: *N*-((4-Iodo-6-((4-methoxybenzyl)thio)pyridin-3-yl)methyl)-2-methoxy-*N*-methylethan-1-amine

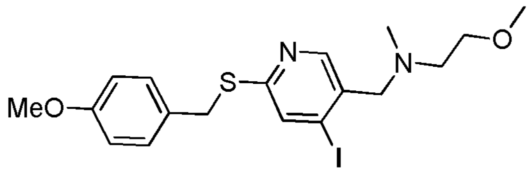

*N*-((6-Fluoro-4-iodopyridin-3-yl)methyl)-2-methoxy-*N*-methylethan-1-amine (1.00 g, 3.09 mmol) was weighed and dissolved in tetrahydrofuran (20 mL). Sodium hydride (0.15 g, 3.60 mmol, 60%) was slowly added at 0 °C and stirred for about 10 minutes. Then 4-methoxybenzylamine (0.48 g, 3.09 mmol) was added and the reaction was continued to stir at 0 °C. The temperature was naturally raised to room temperature and the reaction was continued for about 2 hours. Water (20 mL) was added to quench the reaction, and then the mixture was extracted with EA (15 mL × 3). The organic phases were combined, washed with saturated brine (30 mL), dried over anhydrous sodium sulfate, and concentrated under reduced pressure. The residue was purified by silica gel column chromatography (PE/EA (V/V) = 5/1) to obtain the title compound as a colorless oily liquid (0.76 g, yield 53.66%)
MS (ESI,*pos.ion*) *m*/*z*: 459.1 $[M+H]^+$.

Step 3: 2-((2-((2,4-Dimethoxybenzyl)amino)-7-(2-((4-methoxybenzyl)thio)-5-(((2-methoxyethyl)(methyl)a mino)methyl) pyridin-4-yl)quinazolin-4-yl)amino)-2-methylhexan-1-ol

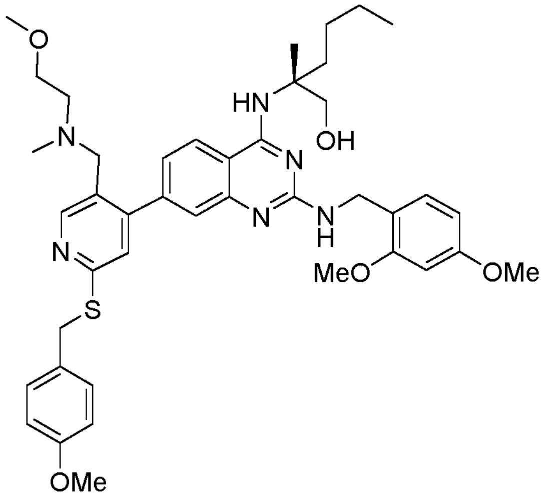

(*R*)-(2-((2,4-Dimethoxybenzyl)amino)-4-((1-hydroxy-2-methylhexan-2-yl)amino) quinazolin-7-yl)boronic acid (0.69 g, 0.72 mmol), *N*-((4-iodo-6-((4-methoxybenzyl)thio)pyridin-3-yl)methyl)-2-methoxy-*N*-methylethan-1-amine (0.36 g, 0.79 mmol), potassium carbonate (0.30 g, 2.16 mmol) and $PdCl_2$(dppf) (0.053 g, 0.072 mmol) were weighed separately and mixed in 1,4-dioxane/water (V/V) = 5/1 (30 mL), under $N_2$, the reaction mixture was heated in an oil bath at 80 °C for about 29 hours. The mixture was cooled to room temperature and concentrated under reduced pressure. The residue was purified by silica gel column chromatography (DCM/MeOH (V/V) = 15/1) to give the title compound as a brown-black solid (0.54 g, yield 99.34%).
MS (ESI,*pos.ion*) *m*/*z*: 755.4 $[M+H]^+$.

Step 4: (*R*)-4-(2-Amino-4-((1-hydroxy-2-methylhexan-2-yl)amino)quinazolin-7-yl)-5-(((2-methoxyethyl)( methyl)amino) methyl)pyridin-2(1*H*)-thione

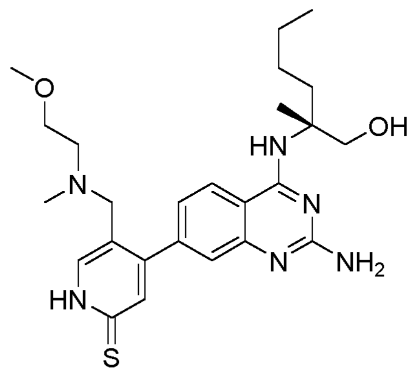

2-((2-((2,4-Dimethoxybenzyl)amino)-7-(2-((4-methoxybenzyl)thio)-5-(((2-methox yethyl)(methyl)amino) methyl)pyridin-4-yl)quinazolin-4-yl)amino)-2-methylhexan-1-ol was weighed and added into trifluoroacetic acid (3 mL), and the reaction mixture was stirred at 60 °C for about 18 hours. The stirring was stopped, the temperature was lowered to room temperature, and the mixture was concentrated under reduced pressure. Potassium carbonate (0.44 g, 3.18 mmol) and methanol (5 mL) were added in sequence. The resulted mixture was stirred at room temperature for about 6 hours, filtered, and concentrated under reduced pressure. Acetonitrile (2 mL), water (2 mL) and trifluoroacetic acid (0.5 mL) were added to the residue for dilution, and then filtered. The filtrate was purified by preparative chromatography (eluted with 15%-50% acetonitrile/water for 30 minutes, containing one thousandth of trifluoroacetic acid), concentrated under reduced pressure to remove acetonitrile, and then freeze-dried on a freeze dryer to obtain the title compound as a yellow solid (0.13 g, yield 33.95%).

MS (ESI, *pos. ion) m*/*z*: 485.3 $[M+H]^+$,
$^1H$ NMR (400 MHz, $CD_3OD$) δ (ppm) 8.42 (d, *J* = 8.4 Hz, 1H), 8.04 (s, 1H), 7.51 (s, 1H), 7.46 - 7.40 (m, 2H), 4.34 (s, 2H), 4.20 (d, *J* = 11.3 Hz, 1H), 3.72 (d, *J* = 11.2 Hz, 1H), 3.56 (t, *J* = 4.9 Hz, 2H), 3.27 (s, 3H), 3.24 - 3.14 (m, 2H), 2.64 (s, 3H), 2.32 - 2.21 (m, 1H), 1.92 - 1.77 (m, 1H), 1.56 (s, 3H), 1.42 - 1.30 (m, 4H), 0.93 (t, *J* = 6.8 Hz, 3H).

**Example 66: (*R*)-4-(2-Amino-4-((1-hydroxy-2-methylhexan-2-yl)amino)pyrido[3,2-*d*]pyrimidin-7-yl)-5-(((2-methoxyethyl)(methyl)amino)methyl)pyridine-2(1*H*)-thione**

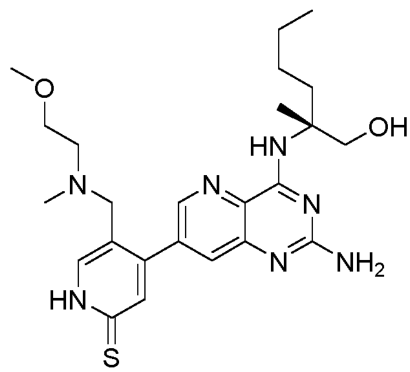

*N*-((4-Iodo-6-((4-methoxybenzyl)thio)pyridin-3-yl)methyl)-2-methoxy-*N*-methyle than-1-amine and (*R*)-(2-((2,4-dimethoxybenzyl)amino)-4-((1-hydroxy-2-methylhexan-2-yl)amino)pyrido[3,2-d*]*pyri midin-7-yl)boronic acid were used as raw materials, according to the synthesis method of steps 3-4 of example 65, and the title compound can be obtained as a white solid with a total yield of 8%.

MS (ESI, *pos.ion*) *m*/z: 486.5 $[M+H]^+$,

$^1H$ NMR (400 MHz, $CD_3OD$): δ (ppm) 8.65 (d, *J* = 1.6 Hz, 1H), 8.40 (s, 1H), 8.06 (s, 1H), 7.99 (d, *J* = 1.7 Hz, 1H), 7.46 (s, 1H), 4.33 (s, 2H), 4.00 (d, *J* = 11.2 Hz, 1H), 3.74 (d, *J* = 11.2 Hz, 1H), 3.63 - 3.53 (m, 2H), 3.38 - 3.31 (m, 3H), 3.28 (s, 3H), 3.21 (s, 2H), 2.68 (s, 3H), 2.13 (d, *J* = 14.5 Hz, 1H), 2.00 (dd, *J* = 15.2, 9.5 Hz, 1H), 1.58 (s, 3H), 1.35 (d, *J* = 25.3 Hz, 5H), 0.95 (t, *J* = 6.7 Hz, 3H).

**Example** 67: **(*R*)-4-(2-Amino-4-((1-hydroxy-2-methylhexan-2-yl)amino)quinazolin-7-yl)-5-((dimethylamino) methyl)pyridin-2(1*H*)-one**

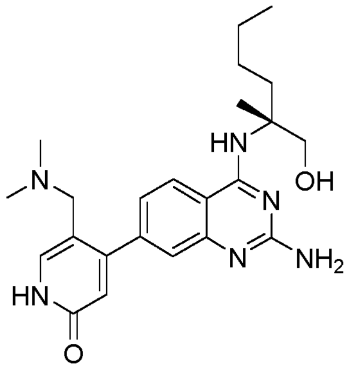

5-((Dimethylamino)methyl)-4-iodopyridin-2(1*H*)-one and (R)-(2-((2,4-dimethoxybenzyl)amino)-4-((1-hydroxy-2-methylhexan-2-yl)amino)quinazolin-7-yl)bo ronic acid were used as raw materials, according to the synthesis method of step 5 of example 56, and the title compound can be obtained as a light yellow solid with a total yield of 54.27%.

MS (ESI, *pos.ion*) *m*/*z*: 425.5 [M+H]+,

$^1H$ NMR (400 MHz, $CD_3OD$) δ (ppm) 7.99 (d, *J* = 8.4 Hz, 1H), 7.50 (s, 1H), 7.28 (d, *J* = 1.4 Hz, 1H), 7.14 (dd, *J* = 8.4, 1.7 Hz, 1H), 6.48 (s, 1H), 3.94 (d, *J* = 11.4 Hz, 1H), 3.75 (d, *J* = 11.4 Hz, 1H), 3.23 (s, 2H), 2.24 - 2.13 (m, 1H), 2.13 (s, 6H), 1.92 - 1.78 (m, 1H), 1.45 (s, 3H), 1.40 - 1.26 (m, 4H), 0.92 (t, *J* = 7.0 Hz, 3H).

**Example 68: (*R*)-4-(2-Amino-4-((*1*-hydroxy-2-methylhexan-2-yl)amino)quinazolin-7-yl)-5-(methoxymethyl) pyridin-2(1*H*)-one**

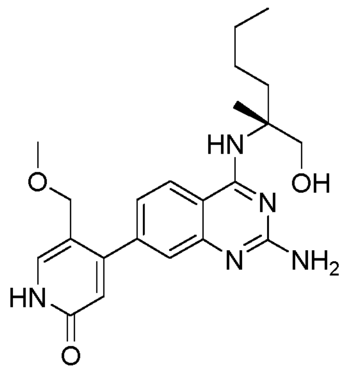

4-Iodo-5-(methoxymethyl)pyridin-2(1*H*)-one and (*R*)-(2-((2,4-dimethoxybenzyl)amino)-4-((1-hydroxy-2-methylhexan-2-yl)amino)quinazolin-7-yl)bo ronic acid were used as raw materials, according to the synthesis method of step 5 of example 56, and the title compound can be obtained as a light yellow solid with a total yield of 83.88%.

MS (ESI, *pos.ion) m*/*z*: 412.5 [M+H]+,

$^1H$ NMR (400 MHz, $CD_3OD$) δ (ppm) 8.21 (d, *J* = 8.4 Hz, 1H), 7.60 (s, 1H), 7.41 (s, 1H), 7.37 (d, *J*= 8.4 Hz, 1H), 6.51 (s, 1H), 4.14 (s, 2H), 4.11 (d, *J* = 7.9 Hz, 1H), 3.75 (d, *J* = 11.3 Hz, 1H), 3.26 (s, 3H), 1.53 (s, 3H), 2.32 - 2.21 (m, 1H), 1.87 - 1.76 (m, 1H), 1.45 - 1.31 (m, 4H), 0.93 (t, *J*= 6.9 Hz, 3H).

**Example 69: 4-(2-Amino-4-((1-hydroxy-2-methylhexan-2-yl)amino)pyrido[3,2-*d*]pyrimidin-7-yl)-5-((butyl( methyl)amino)methyl)pyridin-2(1*H*)-one**

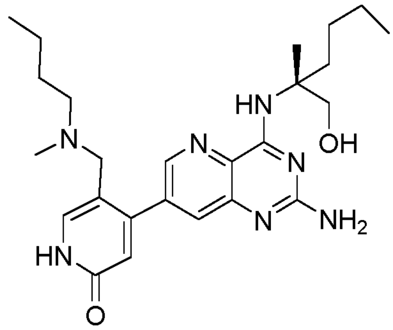

5-((Butyl(methyl)amino)methyl)-4-iodopyridin-2(1*H*)-one and (*R*)-(2-((2,4-dimethoxybenzyl)amino)-4-((1-hydroxy-2-methylhexan-2-yl)amino)pyrido[3,2-d]pyri midin-7-yl)boronic acid were used as raw materials, according to the synthesis method of step 5 of example 56, and the title compound was obtained as a yellow solid with a total yield of 59.03%.

MS (ESI, *pos. ion) m*/*z*: 468.50 $[M+H]^+$,
$^1$H NMR (400 MHz, $CD_3OD$) δ (ppm) 8.38 (d, *J* = 1.6 Hz, 1H), 7.63 (d, *J* = 1.6 Hz, 1H), 7.49 (s, 1H), 6.52 (s, 1H), 3.96 (d, *J* = 11.2 Hz, 1H), 3.77 (d, *J* = 11.3 Hz, 1H), 3.25 (s, 2H), 2.17 (t, *J* = 7.1 Hz, 2H), 2.14 -2.06 (m, 1H), 2.03 (s, 3H), 1.97 - 1.82 (m, 1H), 1.50 (s, 3H), 1.42 - 1.33 (m, 4H), 1.19 - 1.09 (m, 2H), 1.08 - 0.98 (m, 2H), 0.93 (t, *J*= 6.9 Hz, 3H), 0.73 (t, *J*= 7.2 Hz, 3H).

**Example 70: (*R*)-4-(2-Amino-4-((1-hydroxy-2-methylhexan-2-yl)amino)pyrido[3,2-d]pyrimidin-7-yl)-1-met hyl-5-(piperidin-1-ylmethyl)pyridin-2(1*H*)-one**

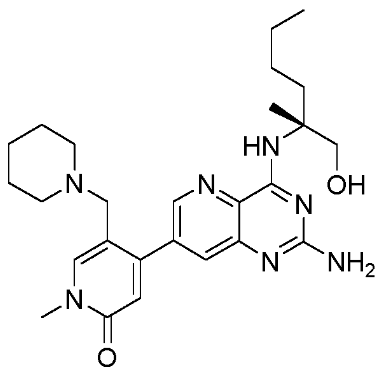

Step 1: 4-Iodo-6-methoxynicotinonitrile

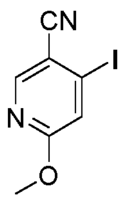

6-Methoxynicotinonitrile (1.0 g, 7.45 mmol) was dissolved in THF (30 mL). Under $N_2$, a solution of lithium

diisopropylamide in tetrahydrofuran (4.86 mL, 9.71 mmol) was slowly added dropwise at -78 °C. After the addition was completed, the mixture was stirred and reacted at -78 °C for 1 h. Under the same condition, a solution of iodine (3.03 g, 11.92 mmol) in THF (5 mL) was added. After the addition was completed, the reaction was continued for 1 h. Saturated ammonium chloride solution (20 mL) and ethyl acetate (30 mL) were added, the aqueous phase was extracted with ethyl acetate (20 mL × 2), the organic phases were combined, dried over anhydrous sodium sulfate, filtered, and concentrated under reduced pressure. The residue was purified by silica gel column chromatography (PE/EA (V/V) = 5/1) to obtain 0.919 g of a white solid compound with a yield of 47.44%.

MS (ESI, *pos. ion*) *m*/*z*: 260.9 $[M+H]^+$,
$^1H$ NMR (400 MHz, $CDCl_3$) δ (ppm) 8.34 (s, 1H), 7.34 (s, 1H), 3.97 (s, 3H).

Step 2: 4-Bromo-6-oxo-1,6-dihydropyridine-3-carbonitrile

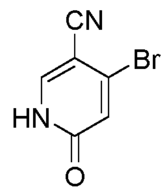

4-Iodo-6-methoxynicotinonitrile (7.0 g, 26.92 mmol) was dissolved in DMF (105 mL), pyridine hydrobromide (86.15 g, 538.40 mmol) was added, and the mixture was heated to 100 °C and stirred for 23 h. The mixture was cooled to room temperature and concentrated to remove the solvent to obtain a yellow solid. The solid was diluted with Water (50 mL) and the reaction solution was stirred in an ice bath. The product precipitated and was filtered off with suction. The filter cake was washed with water (50 mL) and dried to obtain 3.15 g of a white solid compound with a yield of 58.80%.
MS (ESI, *pos. ion) m*/*z*: 198.9$[M+H]^+$.

Step 3: 4-Bromo-6-oxo-1,6-dihydropyridine-3-carbaldehyde

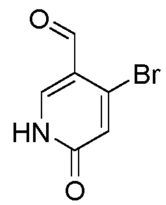

4-Bromo-6-oxo-1,6-dihydropyridine-3-carbonitrile (0.400 g, 2.01 mmol) was dissolved in formic acid (6 mL), and Raney nickel (0.35 g, 6.03 mmol) was added and heated under reflux for 3 h. The mixture was filtered through celite, and the filter cake was washed with dichloromethane (20 mL). The solvent was concentrated to obtain a green solid. The solid was triturated with Ethanol (10 mL) to obtain 0.270 g of a white solid compound with a yield of 66.50%.
MS (ESI, *pos. ion*) *mlz:* 202.0 $[M+H]^+$.

Step 4: 4-Bromo-5-(piperidin-1-ylmethyl)pyridin-2(1*H*)-one

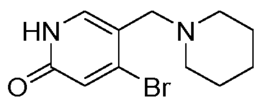

4-Bromo-6-oxo-1,6-dihydropyridine-3-carbaldehyde (0.248 g, 1.23 mmol) was dissolved in DCE (10 mL), piperidine (0.19 mL, 1.88 mmol) and triethylamine (0.34 mL, 2.47 mmol) were added. The reaction mixture was stirred at room temperature for 1 h, and then sodium triacetoxyborohydride (1.04 g, 4.91 mmol) was added under ice bath. After

the addition was complete, the mixture was stirred at room temperature for 12 h. After the reaction was completed, saturated sodium bicarbonate (20 mL) and dichloromethane (10 mL) were added, the resulted phases were were separated, the aqueous phase was extracted with dichloromethane (20 mL × 2), the organic phases were combined, dried over anhydrous sodium sulfate, concentrated, and the residue was purified by silica gel column chromatography (DCM/MeOH (V/V) = 10/1) to obtain 0.250 g of a yellow solid compound with a yield of 74.96%.
MS (ESI, *pos. ion) m*/*z*: 271.0 $[M+H]^+$.

Step 5: 4-Bromo-1-methyl-5-(piperidin-1-ylmethyl)pyridin-2(1*H*)-one

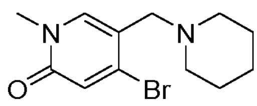

4-Bromo-5-(piperidin-1-ylmethyl)pyridin-2(1*H*)-one and iodomethane were used as raw materials, according to the synthesis method of step 9 of example 1, and the title compound can be obtained 0.18 g of a white solid with a total yield of 78.53%.
MS (ESI, *pos.ion*) *m*/*z*: 285.1 $[M+H]^+$.

Step 6: (*R*)-4-(2-Amino-4-((1-hydroxy-2-methylhexan-2-yl)amino)pyrido[3,2-*d*]pyrimidin-7-yl)-1-methyl-5-(piperidin-1-ylmethyl)pyridin-2(1*H*)-one

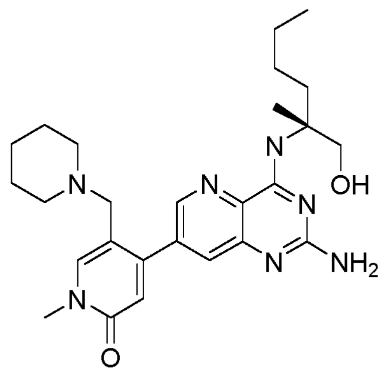

4-Bromo-1-methyl-5-(piperidin-1-ylmethyl)pyridin-2(1*H*)-one and (*R*)-(2-((2,4-dimethoxybenzyl)amino)-4-((1-hydroxy-2-methylhexan-2-yl)amino)pyrido[3,2-*d*]pyri midin-7-yl)boronic acid were used as raw materials, according to the synthesis method of steps 10-11 of example 1, and the title compound can be obtained as a off-yellow solid with a total yield of 40.75%.

MS (ESI, *pos.ion*) *m*/*z*: 480.3 $[M+H]^+$,
$^1H$ NMR (400 MHz, $CD_3OD$) δ (ppm) 8.40 (d, *J* = 1.7 Hz, 1H), 7.70 (s, 1H), 7.64 (d, *J* = 1.7 Hz, 1H), 6.52 (s, 1H), 3.98 (d, *J* = 11.2 Hz, 1H), 3.78 (d, *J* = 11.3 Hz, 1H), 3.65 (s, 3H), 3.19 (s, 2H), 2.28 - 2.11 (m, 5H), 1.91 - 1.80 (m, 1H),1.51 (s, 3H), 1.43 - 1.32 (m, 10H), 0.93 (t, *J* = 6.8 Hz, 3H).

**Biological test**

**Test 1: Test of the agonistic activity of human TLR7 and TLR8**

Purpose: HEK-Blue™ hTLR7 and HEK-Blue™ hTLR8 cells were used to detect the agonist and cytotoxic activities of compounds against hTLR7 and hTLR8.
The experimental steps were as shown below:

1) Preparation of compound: The compound was prepared into a 20 mM stock solution with DMSO, and then the stock solution was diluted 3-fold in a gradient and added to a 96-well plate, for a total of 10 concentrations, with duplicate

wells for each concentration. For negative control wells, 0.5 μL DMSO was added to each well. The final DMSO concentration was 0.5%.

2) HEK-Blue™ hTLR7 or HEK-Blue™ hTLR8 cells were suspended in culture medium and then seeded into 96-well plates containing compounds at a cell count of 50,000 per well. The compounds and cells were incubated for 24 h at 37 °C, 5% $CO_2$.

3) QUANTI-Blue was used for the detection solution: to a mixture of 1 mL of QB reagent and 1 mL of QB buffer was added 98 mL of sterile water, the mixture was mixed and dissolved, and then placed at room temperature for 10 min.

4) Activity test of the compound: 20 μL of the culture supernatant in step 2) was added to each well of a 96-well plate containing 180 μL of QUANTI-Blue detection solution. After incubation at 37 °C for 1 h, the absorbance at 650 nm ($OD_{650}$) was detected using a multifunctional microplate reader Flextation III.

5) Detection of cell activity: The chemiluminescent signal (RLU) was detected using the multifunctional microplate reader Flextation III according to the instructions of Celltiter-Glo.

6) Data analysis

Activity of compounds: $OD_{650}$ values were analyzed using GraphPad Prism software, and the compound dose-effect curves were fitted to calculate the $EC_{50}$ values of the compounds.

Detection of cell activity: The calculation formula for cell activity % is as follows. The cell activity % values were analyzed using GraphPad Prism software, and the compound dose-effect curves were fitted to calculate the $CC_{50}$ values of the compounds.

$$\text{Cell activity \%} = \text{RLU compound / RLU DMSO Control} * 100\%$$

The compounds of the present invention have good agonist activity and selective activation effect on hTLR8. The test results of the agonist activity of the compounds of the present invention on human TLR7 and TLR8 are shown in Table A. The compounds of the present invention have low toxicity to cells. The toxicity results of the compounds of the present invention on cells are shown in Table A-1.

Table A: Agonistic activity of the compounds of the present invention on human TLR7 and TLR8

| Example No. | hTLR 8 $EC_{50}$(μM) | hTLR 7 $EC_{50}$ ($_{[}$tM) |
|---|---|---|
| 1 | 1.819 | 72.96 |
| 2 | 0.1 | >100 |
| 3 | 1.455 | >100 |
| 4 | 1.068 | >100 |
| 5 | 2.722 | >100 |
| 6 | 3.896 | >100 |
| 7 | 0.315 | >100 |
| 8 | 0.042 | >100 |
| 9 | 0.020 | 31.78 |
| 11 | 1.651 | N/A |
| 12 | 0.019 | 50.85 |
| 13 | 1.734 | 81.48 |
| 14 | 0.288 | >100 |
| 16 | 0.104 | 65.85 |
| 17 | 0.22 | >100 |
| 18 | 0.132 | >100 |
| 19 | 0.255 | >100 |
| 20 | 2.849 | >100 |
| 21 | 0.034 | 90.33 |

(continued)

| Example No. | hTLR 8 $EC_{50}$($\mu$M) | hTLR 7 $EC_{50}$ ($_[$tM) |
|---|---|---|
| 22 | 0.98 | >100 |
| 23 | 0.291 | >100 |
| 24 | 0.239 | >100 |
| 25 | 0.13 | >100 |
| 26 | 0.845 | 54.6 |
| 27 | 4.254 | >100 |
| 28 | 0.063 | 29.45 |
| 29 | 0.267 | 23.22 |
| 31 | 0.209 | 56.74 |
| 32 | 0.399 | 47.15 |
| 35 | 0.079 | 68.62 |
| 36 | 0.066 | >100 |
| 37 | 0.397 | >100 |
| 38 | 2.987 | >100 |
| 40 | 1.524 | >100 |
| 41 | 0.354 | 29.9 |
| 42 | 0.595 | >100 |
| 44 | 0.166 | >100 |
| 45 | 0.097 | >100 |
| 46 | 0.569 | >100 |
| 50 | 0.016 | >100 |
| 51 | 0.012 | >100 |
| 52 | 0.030 | 18.36 |
| 53 | 0.68 | N/A |
| 54 | 0.106 | >100 |
| 55 | 0.039 | >100 |
| 56 | 0.025 | 11.11 |
| 57 | 1.856 | >100 |
| 58 | 0.918 | >100 |
| 60 | 0.292 | >100 |
| 61 | 0.418 | >100 |
| 62 | 0.198 | >100 |
| 64 | 0.077 | >100 |
| 65 | 0.212 | >100 |
| 66 | 0.183 | >100 |
| 67 | 0.056 | >100 |
| 68 | 0.042 | 14.23 |
| 69 | 0.023 | 12.02 |
| Note: N/A means not tested | | |

Conclusion: The experimental data show that most of the compounds of the present invention have good agonistic activity and selective activation effect on hTLR8.

Table A-1: Toxicity of the compounds of the present invention to cells

| Example No. | hTLR 8 $CC_{50}$ ($\mu$M) |
|---|---|
| 5 | >100 |
| 7 | >100 |
| 8 | 98.29 |
| 9 | >100 |
| 13 | 97.27 |
| 14 | >100 |
| 15 | >100 |
| 17 | >100 |
| 18 | >100 |
| 19 | >100 |
| 20 | >100 |
| 21 | >100 |
| 22 | >100 |
| 23 | >100 |
| 24 | >100 |
| 25 | >100 |
| 27 | >100 |
| 28 | >100 |
| 35 | >100 |
| 36 | >100 |
| 38 | >100 |
| 40 | >100 |
| 41 | >100 |
| 42 | >100 |
| 43 | >100 |
| 44 | >100 |
| 45 | >100 |
| 46 | >100 |
| 50 | >100 |
| 51 | >100 |
| 52 | >100 |
| 54 | >100 |
| 55 | >100 |
| 57 | >100 |
| 58 | >100 |
| 60 | >100 |
| 61 | >100 |

(continued)

| Example No. | hTLR 8 $CC_{50}$ (μM) |
|---|---|
| 62 | >100 |
| 64 | >100 |
| 65 | >100 |
| 66 | >100 |
| 67 | >100 |
| 68 | >100 |

Conclusion: The experimental data show that the compounds of the present invention have low toxicity to cells.

**Test 2: Detection of the effect of compounds on hERG channel currents stably expressed in HEK293 cells using automated patch clamp**

Experimental purpose: The concentration-effect relationship of the blocking effect of the compounds on hERG channels was determined using the patch clamp technique and HEK-293 cells stably expressing hERG channels to evaluate their risk of inhibitory effect on cardiac hERG potassium channels.

Experimental method: hERG cells were clamped using the patch clamp technique to form a high-resistance seal and then the membrane was broken. The whole-cell recording mode was used to record the hERG current. Each drug concentration was set to be administered twice for at least 5 minutes. The test concentrations were 0.3 μM, 1 μM, 3 μM, 10 μM and 30 μM, and the changes of the test compounds on hERG current were observed. The test results are shown in Table B.

Table B: hERG IC50 of compounds of the present invention

| Tested compounds | hERG $IC_{50}$(μM) |
|---|---|
| Comparative compounds | 3.3 |
| Example 8 | >30 |
| Example 9 | 24.68 |
| Example 21 | >30 |
| Example 28 | >30 |
| Note: The comparative compound is | |

(*R*)-2-((2-amino-7-fluoropyrido[3,2-*d*]pyrimidin-4-yl)amino)-2-methylhexan-1-ol

Conclusion: The hERG test experimental data show that the compounds of the present invention have low toxicity to the heart.

**Test 3: Inhibitory effect of the compounds of the present invention on human liver microsomal CYP enzymes**

Experimental purpose: The inhibitory effects of the test compounds on the major metabolizing enzymes CYP1A2, CYP2C19, CYP2D6 and CYP3A4 in microsomes were evaluated using the human liver microsomal system.

Experimental method: The test substance was incubated with a suspension of human liver microsomes containing cytochrome P450 enzymes CYP1A2, CYP2C19, CYP2D6 and CYP3A4 at a concentration of 10 μM in the final incubation system. The known substrates were metabolized by the corresponding single enzymes to generate specific metabolites, and the metabolites were determined by UPLC-MS/MS method. The relative inhibition rate (%) was calculated by the percentage reduction of metabolite production by the test compound and solvent DMSO in the same time period.

$$\text{Relative inhibition rate }\%=(1-(N_{+inh}/N_{veh}))*100$$

N is the concentration value of the probe substrate metabolite and it is assumed that the blood product with or without inhibitor at 0 min is 0. $N_{+inh}$ is the metabolite concentration in the group with inhibitor, and $N_{veh}$ is the metabolite concentration in the group without inhibitor. The results of the inhibition test of the compounds of the present invention on human liver microsomal CYP enzymes are shown in Table C.

Table C: Experimental data of the inhibition test of the compounds of the present invention on human liver microsomal CYP enzymes

| Tested compounds | Relative inhibition rate (%) | | | | |
|---|---|---|---|---|---|
| | CYP1 A2 | CYP2C 19 | CYP2 D6 | CYP3A4 (Midazola m) | CYP3A4 (Testostero ne) |
| Example 8 | -10.5 | 9.3 | 18.1 | 9.6 | 6.3 |
| Example 21 | -4.1 | 10.5 | 9.5 | 15.3 | 18.0 |
| Example 28 | 5.1 | 30.5 | 12.9 | 31.1 | 17.9 |
| Example 35 | 2.7 | 9.7 | 31.4 | 23.9 | 11.5 |
| Example 36 | 9.0 | 7.0 | 20.1 | 25.9 | 7.5 |
| Example 50 | 10.7 | 11.4 | 9.7 | 13.7 | 11.6 |
| Example 51 | 11.1 | 22.1 | 9.6 | 7.4 | 34.1 |
| Example 54 | 7.9 | 0.8 | 12.5 | 22.5 | 8.9 |
| Example 55 | 9.1 | 17.0 | 19.1 | 38.6 | 17.1 |
| Example 56 | 10.7 | 10.4 | 31.7 | 33.6 | 15.6 |
| Example 64 | 8.9 | 2.4 | 13.8 | 17.6 | 8.8 |

Conclusion: The experimental data show that the compounds of the present invention have basically no inhibitory effect on the main human liver microsomal CYP enzymes.

**Test 4: Induction of the compounds of the present invention on human liver microsomal CYP enzymes**

Experimental purpose: Using frozen human hepatocytes as the test system, the induction effects of the test substances on CYP1A2, CYP2B6 and CYP3A4 were evaluated from two aspects of enzyme activity and mRNA level.

Experimental method: 24 hours after cell plating, induction was started by adding medium containing the test compound (test concentrations were 10, 1, and 0.1 μM), and fresh drug-containing medium was replaced every 24 hours. After 72 hours of induction, the medium was replaced with medium containing CYP1A2, CYP2B6, and CYP3A4 specific substrates for 30 minutes of incubation. 100 μL was taken for treatment, and the labeled metabolites of the substrates were detected to evaluate the enzyme activity level. Finally, the cells were lysed and reverse transcribed, and the expression levels of CYP1A2, CYP2B6 and CYP3A4 genes in the cells were evaluated by real-time quantitative PCR.

The induction fold of the test compound compared with the blank control and the induction percentage of the test compound compared with the positive control were used as the basis for evaluating the induction potential of the test compound. Among them, the positive control for CYP1A2 is Omeprazole; the positive control for CYP2B6 is Phenobarbital; the positive control for CYP3A4 is Rifampicin. The results of the induction test on human liver microsomal CYP enzymes are shown in Table D.

Table D: Experimental data of the induction test of the compounds of the present invention on human liver microsomal CYP enzymes

| Tested compounds | CYP1A2 | | CYP2B6 | | CYP3A4 | |
|---|---|---|---|---|---|---|
| | Induction percentage relative to positive control (%) | Induction fold relative to blank control | Induction percentage relative to positive control (%) | Induction fold relative to blank control | Induction percentage relative to positive control (%) | Induction fold relative to blank control |
| Comparative compounds | 10.5 | 2.6 | 24.5 | 3.6 | 15.8 | 9.5 |

(continued)

| Tested compounds | CYP1A2 | | CYP2B6 | | CYP3A4 | |
|---|---|---|---|---|---|---|
| | Induction percentage relative to positive control (%) | Induction fold relative to blank control | Induction percentage relative to positive control (%) | Induction fold relative to blank control | Induction percentage relative to positive control (%) | Induction fold relative to blank control |
| Example 8 | -10.5 | 0.7 | -4.1 | 0.6 | -1.5 | 0.6 |
| Example 9 | -0.1 | 1.0 | 4.2 | 1.3 | 2.0 | 4.2 |
| Example 21 | -15.4 | 0.6 | -4.8 | 0.5 | 1.5 | 1.4 |
| Example 28 | -4.3 | 0.8 | -0.7 | 0.9 | 2.0 | 2.0 |
| Example 35 | -22.2 | 0.4 | -5.6 | 0.4 | 0.9 | 1.3 |
| Note: The comparative compound is (*R*)-2-((2-amino-7-fluoropyrido[3,2-d]pyrimidin-4-yl)amino)-2-methylhexan-1-ol | | | | | | |

Conclusion: The experimental data show that the compounds of the present invention have basically no induction effect on the hepatic enzymes CYP1A2, CYP2B6 and CYP3A4.

**Test 5: Pharmacokinetic study of the compounds of the present invention in beagle dogs, mice, rats or cynomolgus monkeys**

**(1) PK test experiment in beagle dogs**

PK determination experimental method of the compound of the present invention in beagle dogs (purchased from Hunan Slake Jingda Experimental Animal Co., Ltd., body weight 10-12 kg, male, age 10-12 months, oral administration 3 per group, intravenous injection 3 per group)

Beagle dogs were given the test compound of 2.5 mg/kg or 5 mg/kg by oral gavage or 0.5 mg/kg, 1 mg/kg or 2 mg/kg by intravenous injection.

Venous blood was collected at different time points (0.083, 0.25, 0.5, 1, 2, 4, 6, 8 and 24 hours) after administration and collected in anticoagulant tubes containing EDTA-$K_2$. After liquid-liquid extraction, plasma samples were quantitatively analyzed on a triple quadrupole tandem mass spectrometer using multiple reaction ion monitoring (MRM) mode. Pharmacokinetic parameters were calculated using a noncompartmental method by WinNonLin 6.3 software.

Conclusion: The pharmacokinetic experimental data show that the compounds of the present invention have good pharmacokinetic properties in beagle dogs and has a good application prospect in anti-HBV

**(2) PK test experiment in mice:**

PK determination experimental method of the compound of the present invention in ICR mice (purchased from Hunan Slake Jingda Experimental Animal Co., Ltd., body weight 20-25 g, male, age 45-60 days, oral administration 3 per group, intravenous injection 3 per group)

ICR mice were given the test compound of 10 mg/kg by oral gavage or 2 mg/kg or 10 mg/kg by tail vein injection.

Blood was collected from the orbital vein at different time points (0.083, 0.25, 0.5, 1, 2, 4, 6, 8 and 24 hours) after administration and placed in anticoagulant tubes containing EDTA-$K_2$. After liquid-liquid extraction, plasma samples were quantitatively analyzed on a triple quadrupole tandem mass spectrometer using multiple reaction ion monitoring (MRM) mode. Pharmacokinetic parameters were calculated using a noncompartmental method by WinNonLin 6.3 software.

Conclusion: The pharmacokinetic experimental data show that the compounds of the present invention have good pharmacokinetic properties in mice and has a good application prospect in anti-HBV.

**(3) PK test experiment in SD rats:**

PK determination experimental method of the compound of the present invention in SD rats (purchased from Hunan Slake Jingda Experimental Animal Co., Ltd., body weight 200-250 g, male, age 2-3 months, oral administration 3 per group, intravenous injection 3 per group)

Rats were given the test compound of 2.5 mg/kg or 5 mg/kg by oral gavage or 1 mg/kg by intravenous injection.

Venous blood was collected at different time points (0.083, 0.25, 0.5, 1, 2, 5, 7, 8 and 24 hours) after administration and collected in anticoagulant tubes containing EDTA-$K_2$. After liquid-liquid extraction, plasma samples were quantitatively analyzed on a triple quadrupole tandem mass spectrometer using multiple reaction ion monitoring (MRM) mode. Pharmacokinetic parameters were calculated using a noncompartmental method by WinNonLin 6.3 software.

Conclusion: The pharmacokinetic experimental data show that the compounds of the present invention have a large exposure in SD rats, indicating that the compounds of the present invention are well absorbed in SD rats and has good bioavailability, and has a good application prospect in anti-HBV

**(4) PK test experiment in cynomolgus monkeys:**

PK determination experimental method of the compound of the present invention in cynomolgus monkeys (ppurchased from Guangdong Chunsheng Biotechnology Development Co., Ltd., body weight 3-6 kg, male, age 4-6 years old, oral administration 3 per group, intravenous injection 3 per group)

Cynomolgus monkeys were given the test compound of 2.5 mg/kg or 5 mg/kg by oral gavage or 0.5 mg/kg, 1 mg/kg by intravenous injection.

Venous blood was collected at different time points (0.083, 0.25, 0.5, 1, 2, 4, 6, 8 and 24 hours) after administration and collected in anticoagulant tubes containing EDTA-$K_2$. After liquid-liquid extraction, plasma samples were quantitatively analyzed on a triple quadrupole tandem mass spectrometer using multiple reaction ion monitoring (MRM) mode. Pharmacokinetic parameters were calculated using a noncompartmental method by WinNonLin 6.3 software.

Conclusion: The pharmacokinetic experimental data show that the compounds of the present invention have good pharmacokinetic properties in cynomolgus monkeys and has a good application prospect in anti-HBV

Although the present invention has been described in detail above by means of general description, specific implementation methods and experiments, it is obvious to those skilled in the art that some modifications or improvements may be made based on the present invention. Therefore, all modifications or improvements made without departing from the spirit of the present invention belong to the scope of protection required by the present invention.

**Claims**

**1.** A compound having Formula (I) or a stereoisomer, a tautomer, an N-oxide, a solvate, a metabolite, a pharmaceutically acceptable salt or a prodrug thereof,

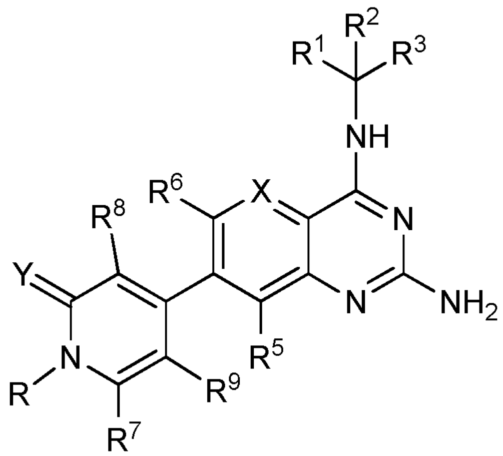

(I)

wherein, X is N or $CR^4$;

each $R^1$, $R^2$, $R^4$, $R^5$, $R^6$, $R^7$ and $R^8$ is independently H, D, F, Cl, Br, I, -OH, -CN, -$NH_2$, $C_{1-4}$ alkylamino, $C_{1-6}$ alkoxy or $C_{1-6}$ alkyl, wherein each $C_{1-4}$ alkylamino, $C_{1-6}$ alkoxy and $C_{1-6}$ alkyl is independently unsubstituted or substituted with 1, 2, 3 or 4 groups independently selected from F, Cl, Br, I, -OH, -CN, -$NH_2$ and $C_{1-4}$ alkyl;

Yis O or S;

$R^3$ is -$C_{1-6}$ alkylene-$R^{10}$, wherein the -$C_{1-6}$ alkylene of -$C_{1-6}$ alkylene-$R^{10}$ is unsubstituted or substituted with 1, 2, 3, 4 or 5 $R^{w1}$;

each $R^{10}$ is independently $C_{1-6}$ alkoxy, -OH, $-NH_2$, -O-C(=O)-$R^{10a}$ or -$NR^c$-C(=O)-$R^{10b}$, wherein the $C_{1-6}$ alkoxy is unsubstituted or substituted with 1, 2, 3 or 4 groups independently selected from F, Cl, Br, I, -OH, -CN, $-NH_2$ and $C_{1-4}$ alkyl;

each $R^c$ is independently H, D or $C_{1-4}$ alkyl, wherein the $C_{1-4}$ alkyl is unsubstituted or substituted with 1, 2, 3 or 4 groups independently selected from F, Cl, Br, I, -OH, -CN, $-NH_2$ and $C_{1-4}$ alkyl;

each $R^{10a}$ and $R^{10b}$ is independently $C_{1-6}$ alkyl, $C_{3-7}$ cycloalkyl, heteroaryl consisting of 5-12 ring atoms or heterocyclyl consisting of 3-12 ring atoms, wherein each $C_{1-6}$ alkyl, $C_{3-7}$ cycloalkyl, heteroaryl consisting of 5-12 ring atoms and heterocyclyl consisting of 3-12 ring atoms is independently unsubstituted or substituted with 1, 2, 3, 4 or 5 $R^{W2}$;

R is H, D, $C_{1-6}$ alkyl or heterocyclyl consisting of 3-6 ring atoms, wherein each $C_{1-6}$ alkyl and heterocyclyl consisting of 3-6 ring atoms is independently unsubstituted or substituted with 1, 2, 3, 4 or 5 $R^{w3}$;

$R^9$ is H, D, -L-$R^{11}$, F, Cl, Br, I, -OH, -CN, $-NH_2$, $C_{1-4}$ alkylamino, $C_{1-6}$ alkoxy or $C_{1-6}$ alkyl, wherein each $C_{1-4}$ alkylamino, $C_{1-6}$ alkoxy and $C_{1-6}$ alkyl is independently unsubstituted or substituted with 1, 2, 3 or 4 groups independently selected from F, Cl, Br, I, -OH, -CN, $-NH_2$ and $C_{1-4}$ alkyl;

L is -$C_{1-6}$ alkylene or -C(=O)-, wherein the -$C_{1-6}$ alkylene is independently unsubstituted or substituted with 1, 2, 3 or 4 groups independently selected from F, Cl, Br, I, -OH, -CN, $-NH_2$, methyl, ethyl, n-propyl or isopropyl;

$R^{11}$ is -$NR^aR^b$, $C_{1-12}$ alkyl, -C(=O)$R^{9a}$, -$OR^{9b}$, -$S(=O)_tR^{9c}$, $C_{3-7}$ cycloalkyl, heteroaryl consisting of 5-12 ring atoms or heterocyclyl consisting of 3-12 ring atoms, wherein each $C_{1-12}$ alkyl, $C_{3-7}$ cycloalkyl, heteroaryl consisting of 5-12 ring atoms and heterocyclyl consisting of 3-12 ring atoms is independently unsubstituted or substituted with 1, 2, 3, 4 or 5 $R^{w4}$;

each $R^a$ and $R^b$ is independently H, D, -C(=O)$R^{9d}$, $C_{1-12}$ alkyl, $C_{2-12}$ alkenyl, $C_{2-12}$ alkynyl or -$C_{1-6}$ alkylene-$R^{d1}$, wherein each $C_{1-12}$ alkyl, $C_{2-12}$ alkenyl, $C_{2-12}$ alkynyl and -$C_{1-6}$ alkylene of -$C_{1-6}$ alkylene-$R^{d1}$ is independently unsubstituted or substituted with 1, 2, 3, 4 or 5 $R^{w5}$;

or, $R^a$, $R^b$ and together with the N atom to which they are attached, form heterocyclyl consisting of 3-8 ring atoms, wherein the heterocyclyl consisting of 3-8 ring atoms is unsubstituted or substituted with 1, 2, 3 or 4 $R^{w4}$;

each $R^{9a}$, $R^{9b}$, $R^{9c}$ and $R^{9d}$ is independently $C_{1-12}$ alkyl, $C_{2-12}$ alkenyl, $C_{2-12}$ alkynyl or -$C_{1-6}$ alkylene-$R^{d2}$, wherein each $C_{1-12}$ alkyl, $C_{2-12}$ alkenyl, $C_{2-12}$ alkynyl and -$C_{1-6}$ alkylene of -$C_{1-6}$ alkylene-$R^{d2}$ is independently unsubstituted or substituted with 1, 2, 3, 4 or 5 $R^{w6}$;

each $R^{d1}$ and $R^{d2}$ is independently -OH, $C_{1-6}$ alkoxy, $C_{6-10}$ aryl, heteroaryl consisting of 5-12 ring atoms, $C_{3-6}$ cycloalkyl or $C_{1-6}$ alkylamino, wherein each $C_{1-6}$ alkoxy, $C_{6-10}$ aryl, heteroaryl consisting of 5-12 ring atoms, $C_{3-6}$ cycloalkyl and $C_{1-6}$ alkylamino is independently unsubstituted or substituted with 1, 2, 3, 4 or 5 $R^{w7}$;

each $R^{w1}$, $R^{w2}$, $R^{w3}$, $R^{w4}$ and $R^{w7}$ is independently D, F, Cl, Br, I, =O, -OH, -CN, $-NH_2$, $C_{1-6}$ alkylamino, $C_{1-6}$ alkoxy, $C_{1-6}$ alkyl, $C_{1-6}$ haloalkyl, -C(=O)-$C_{1-4}$ alkyl, $C_{3-6}$ cycloalkyl, heterocyclyl consisting of 3-6 ring atoms or $C_{6-10}$ aryl, wherein each $C_{1-6}$ alkylamino, $C_{1-6}$ alkoxy, $C_{1-6}$ alkyl, $C_{1-6}$ haloalkyl, -C(=O)-$C_{1-4}$ alkyl, $C_{3-6}$ cycloalkyl, heterocyclyl consisting of 3-6 ring atoms and $C_{6-10}$ aryl is independently unsubstituted or substituted with 1, 2, 3 or 4 groups independently selected from F, Cl, Br, I, -OH, -CN, $-NH_2$, $C_{1-4}$ alkyl and $C_{1-4}$ alkylamino;

each $R^{w5}$ and $R^{w6}$ is independently D, F, Cl, Br, I, =O, -OH, -CN, $-NH_2$, $C_{1-6}$ alkylamino, $C_{1-6}$ alkoxy, $C_{1-6}$ alkyl, $C_{1-6}$ haloalkyl, -C(=O)-$C_{1-4}$ alkyl, $C_{2-12}$ alkenyl, $C_{2-12}$ alkynyl, $C_{3-6}$ cycloalkyl, heterocyclyl consisting of 3-6 ring atoms, heteroaryl consisting of 5-6 ring atoms or $C_{6-10}$ aryl, wherein each $-NH_2$, $C_{1-6}$ alkylamino, $C_{1-6}$ alkoxy, $C_{1-6}$ alkyl, $C_{1-6}$ haloalkyl, -C(=O)-$C_{1-4}$ alkyl, $C_{2-12}$ alkenyl, $C_{2-12}$ alkynyl, $C_{3-6}$ cycloalkyl, heterocyclyl consisting of 3-6 ring atoms, heteroaryl consisting of 5-6 ring atoms and $C_{6-10}$ aryl is independently unsubstituted or substituted with 1, 2, 3 or 4 groups independently selected from F, Cl, Br, I, -OH, -CN, $-NH_2$, $C_{1-4}$ alkyl and $C_{1-4}$ alkylamino;

t is 0, 1 or 2.

**2.** The compound of claim 1, wherein, each $R^1$, $R^2$, $R^4$, $R^5$, $R^6$, $R^7$and $R^8$ is independently H, D, F, Cl, Br, I, -OH, -CN, $-NH_2$, *N*-methylamino, *N*-ethylamino, *N*,*N*-dimethylamino, *N*,*N*-diethylamino, methoxy, ethoxy, 1-propoxy, 2-propoxy, 1-butoxy, 2-methyl-1-propoxy, 2-butoxy, methyl, ethyl, *n*-propyl, isopropyl, *n*-butyl, *i*-butyl, *sec*-butyl, *tert*-butyl, *n*-pentyl or *n*-hexyl, wherein each *N*-methylamino, *N*-ethylamino, *N*,*N*-dimethylamino, *N*,*N*-diethylamino, methoxy, ethoxy, 1-propoxy, 2-propoxy, 1-butoxy, 2-methyl-1-propoxy, 2-butoxy, methyl, ethyl, *n*-propyl, isopropyl, *n*-butyl, *i*-butyl, *sec*-butyl, *tert*-butyl, *n*-pentyl and *n*-hexyl is independently unsubstituted or substituted with 1, 2, 3 or 4 groups independently selected from F, Cl, Br, I, -OH, -CN, $-NH_2$, methyl, ethyl, *n*-propyl, isopropyl, *n*-butyl, *i*-butyl and *sec*-butyl;

R is H, D, methyl, ethyl, *n*-propyl, isopropyl, *n*-butyl, *i*-butyl, *sec*-butyl, *tert*-butyl, *n*-pentyl, *n*-hexyl, aziridinyl, azetidinyl, oxetanyl, thietanyl, pyrrolidyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl or piperazinyl, wherein each methyl, ethyl, *n*-propyl, isopropyl, *n*-butyl, *i*-butyl, *sec*-butyl, *tert*-butyl, *n*-pentyl, *n*-hexyl, aziridinyl, azetidinyl, oxetanyl, thietanyl, pyrrolidyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl and piperazinyl is independently unsubstituted or substituted with 1,

2, 3, 4 or 5 $R^{w3}$.

**3.** The compound of claim 1 or 2, wherein, $R^3$ is $-CH_2-R^{10}$, $-(CH_2)_2-R^{10}$, $-(CH_2)_3-R^{10}$, $-CH(CH_3)CH_2-R^{10}$, $-CH_2CH(CH_3)-R^{10}$ or $-(CH_2)_4-R^{10}$, wherein each the $-CH_2-$ of $CH_2-R^{10}$, $-(CH_2)_2$-of $-(CH_2)_2-R^{10}$, $-(CH_2)_3-$ of $-(CH_2)_3-R^{10}$, $-CH(CH_3)CH_2-$ of $-CH(CH_3)CH_2-R^{10}$, $-CH_2CH(CH_3)$ - of $-CH_2CH(CH_3)-R^{10}$ and $-(CH_2)_4-$ of $-(CH_2)_4-R^{10}$ is independently unsubstituted or substituted with 1, 2, 3, 4 or 5 $R^{w1}$;

each $R^{10}$ is independently methoxy, ethoxy, 1-propoxy, 2-propoxy, 1-butoxy, 2-methyl-1-propoxy, 2-butoxy, -OH, $-NH_2$, $-O-C(=O)-R^{10a}$ or $-NR^c-C(=O)-R^{10b}$, wherein each methoxy, ethoxy, 1-propoxy, 2-propoxy, 1-butoxy, 2-methyl-1-propoxy and 2-butoxy is independently unsubstituted or substituted with 1, 2, 3 or 4 groups independently selected from F, Cl, Br, I, -OH, -CN, $-NH_2$, methyl, ethyl, *n*-propyl, isopropyl, *n*-butyl, *i*-butyl and *sec*-butyl;
each $R^{10a}$ and $R^{10b}$ is independently $C_{1-4}$ alkyl, $C_{3-6}$ cycloalkyl, heteroaryl consisting of 5-6 ring atoms or heterocyclyl consisting of 3-6 ring atoms, wherein each $C_{1-4}$ alkyl, $C_{3-6}$ cycloalkyl, heteroaryl consisting of 5-6 ring atoms and heterocyclyl consisting of 3-6 ring atoms is independently unsubstituted or substituted with 1, 2, 3, 4 or 5 $R^{w2}$;
$R^c$ is H, D, methyl, ethyl, n-propyl or isopropyl, wherein each methyl, ethyl, n-propyl and isopropyl is independently unsubstituted or substituted with 1, 2, 3 or 4 groups independently selected from F, Cl, Br, I, -OH, -CN, $-NH_2$, methyl, ethyl, *n*-propyl and isopropyl.

**4.** The compound of any one of claims 1 to 3, wherein, each $R^{10a}$ and $R^{10b}$ is independently methyl, ethyl, *n*-propyl, isopropyl, *n*-butyl, *i*-butyl, *sec*-butyl, *tert*-butyl, cyclopropyl, cyclobutyl, cyclopentyl, cyclohexyl, furyl, pyrrolyl, pyridinyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidyl, aziridinyl, azetidinyl, oxetanyl, thietanyl, pyrrolidyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl or piperazinyl, wherein each methyl, ethyl, *n*-propyl, isopropyl, *n*-butyl, *i*-butyl, *sec*-butyl, *tert-butyl,* cyclopropyl, cyclobutyl, cyclopentyl, cyclohexyl, furyl, pyrrolyl, pyridinyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidyl, aziridinyl, azetidinyl, oxetanyl, thietanyl, pyrrolidyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl and piperazinyl is independently unsubstituted or substituted with 1, 2, 3, 4 or 5 $R^{w2}$.

**5.** The compound of any one of claims 1 to 4, wherein, $R^9$ is H, D, $-L-R^{11}$, F, Cl, Br, I, -OH, -CN, $-NH_2$, $C_{1-4}$ alkylamino, $C_{1-4}$ alkoxy or $C_{1-4}$ alkyl, wherein each $C_{1-4}$ alkylamino, $C_{1-4}$ alkoxy and $C_{1-4}$ alkyl isindependently unsubstituted or substituted with 1, 2, 3 or 4 groups independently selected from F, Cl, Br, I, -OH, -CN, $-NH_2$ and $C_{1-4}$ alkyl;

L is $-CH_2-$, $-(CH_2)_2-$, $-(CH_2)_3-$, $-CH_2CH(CH_3)-$, $-CH(CH_3)CH_2-$, $-(CH_2)_4-$ or $-C(=O)-$, wherein each $-CH_2-$, $-(CH_2)_2-$, $-(CH_2)_3-$, $-CH_2CH(CH_3)-$, $-CH(CH_3)CH_2-$ and $-(CH_2)_4-$is independently unsubstituted or substituted with 1, 2, 3 or 4 groups independently selected from F, Cl, Br, I, -OH, -CN, $-NH_2$, methyl, ethyl, n-propyl or isopropyl;
$R^{11}$ is $-NR^aR^b$, $C_{1-10}$ alkyl, $-C(=O)R^{9a}$, $-OR^{9b}$, $-S(=O)_tR^{9c}$, $C_{3-6}$ cycloalkyl, heteroaryl consisting of 5-6 ring atoms or heterocyclyl consisting of 3-6 ring atoms, wherein each $C_{1-10}$ alkyl, $C_{3-6}$ cycloalkyl, heteroaryl consisting of 5-6 ring atoms and heterocyclyl consisting of 3-6 ring atoms is independently unsubstituted or substituted with 1, 2, 3, 4 or 5 $R^{w4}$.

**6.** The compound of any one of claims 1 to 5, wherein, $R^9$ is independently H, D, $-L-R^{11}$, F, Cl, Br, I, -OH, -CN, $-NH_2$, *N*-methylamino, *N*-ethylamino, *N*,*N*-dimethylamino, *N*,*N*-diethylamino, methoxy, ethoxy, 1-propoxy, 2-propoxy, 1-butoxy, 2-methyl-1-propoxy, 2-butoxy, methyl, ethyl, *n*-propyl, isopropyl, *n*-butyl, *i*-butyl, *sec*-butyl, *tert*-butyl, *n*-pentyl or *n*-hexyl, wherein each *N*-methylamino, *N*-ethylamino, *N*,*N*-dimethylamino, *N*,*N*-diethylamino, methoxy, ethoxy, 1-propoxy, 2-propoxy, 1-butoxy, 2-methyl-1-propoxy, 2-butoxy, methyl, ethyl, *n*-propyl, isopropyl, *n*-butyl, *i*-butyl, sec-butyl, *tert*-butyl, *n*-pentyl and n-hexyl is independently unsubstituted or substituted with 1, 2, 3 or 4 groups independently selected from F, Cl, Br, I, -OH, -CN, $-NH_2$, methyl, ethyl, *n*-propyl, isopropyl, *n*-butyl, i-butyl and *sec*-butyl;
$R^{11}$ is $-NR^aR^b$, methyl, ethyl, n-propyl, isopropyl, *n*-butyl, *i*-butyl, *sec*-butyl, *tert*-butyl, *n*-pentyl, *n*-hexyl, $-C(=O)R^{9a}$, $-OR^{9b}$, $-S(=O)_tR^{9c}$, cyclopropyl, cyclobutyl, cyclopentyl, cyclohexyl, furyl, pyrrolyl, pyridinyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidyl, aziridinyl, azetidinyl, oxetanyl, thietanyl, pyrrolidyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl or piperazinyl, wherein each methyl, ethyl, *n*-propyl, isopropyl, *n*-butyl, *i*-butyl, *sec*-butyl, *tert*-butyl, *n*-pentyl, *n*-hexyl, cyclopropyl, cyclobutyl, cyclopentyl, cyclohexyl, furyl, pyrrolyl, pyridinyl, pyrazolyl, imidazolyl, triazolyl, tetrazolyl, oxazolyl, isoxazolyl,

oxadiazolyl, 1,3,5-triazinyl, thiazolyl, thienyl, pyrazinyl, pyridazinyl, pyrimidyl, aziridinyl, azetidinyl, oxetanyl, thietanyl, pyrrolidyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl and piperazinyl is independently unsubstituted or substituted with 1, 2, 3, 4 or 5 $R^{w4}$.

**7.** The compound of any one of claims 1 to 6, wherein, each $R^a$ and $R^b$ is independently H, D, -C(=O)$R^{9d}$, $C_{1-6}$ alkyl, $C_{2-6}$ alkenyl, $C_{2-6}$ alkynyl or -$C_{1-4}$ alkylene-$R^{d1}$, wherein each $C_{1-6}$ alkyl, $C_{2-6}$ alkenyl, $C_{2-6}$ alkynyl and -$C_{1-4}$ alkylene of -$C_{1-4}$ alkylene-$R^{d1}$ is independently unsubstituted or substituted with 1, 2, 3, 4 or 5 $R^{w5}$;
or, $R^a$, $R^b$ and together with the N atom to which they are attached, form heterocyclyl consisting of 3-6 ring atoms, wherein the heterocyclyl consisting of 3-6 ring atoms is unsubstituted or substituted with 1, 2, 3 or 4 $R^{w4}$.

**8.** The compound of any one of claims 1 to 7, wherein, each $R^a$ and $R^b$ is independently H, D, -C(=O)$R^{9d}$, methyl, ethyl, n-propyl, isopropyl, *n*-butyl, *i*-butyl, *sec*-butyl, *tert*-butyl, *n*-pentyl, n-hexyl, $-CH=CH_2$, $-CH=CHCH_3$, $-CH_2CH=CH_2$, $-CH_2CH_2CH=CH_2$, -C=CH, $-CH_2C=CH$, $-C\equiv C-CH_3$, $-CH_2CH_2C\equiv CH$, $-CH_2C\equiv CCH_3$, $-C\equiv CCH_2CH_3$, $-CH_2-R^{d1}$, $-(CH_2)_2-R^{d1}$, $-(CH_2)_3-R^{d1}$, $-CH_2CH(CH_3)-R^{d1}$, $-CH(CH_3)CH_2-R^{d1}$ or $-(CH_2)_4-R^{d1}$, wherein, each methyl, ethyl, *n*-propyl, isopropyl, *n*-butyl, *i*-butyl, *sec*-butyl, *tert*-butyl, *n*-pentyl, *n*-hexyl, $-CH=CH_2$, $-CH=CHCH_3$, $-CH_2CH=CH_2$, $-CH_2CH_2CH=CH_2$, -C=CH, $-CH_2C=CH$, $-C\equiv C-CH_3$, $-CH_2CH_2C\equiv CH$, $-CH_2C\equiv CCH_3$, $-C\equiv CCH_2CH_3$, $-CH_2-$ of $-CH_2-R^{d1}$, $-(CH_2)_2-$ of $-(CH_2)_2-R^{d1}$, $-(CH_2)_3-$ of $-(CH_2)_3-R^{d1}$, $-CH_2CH(CH_3)-$ of $-CH_2CH(CH_3)-R^{d1}$, $-CH(CH_3)CH_2-$ of $-CH(CH_3)CH_2-R^{d1}$ and $-(CH_2)_4-$ of $-(CH_2)_4-R^{d1}$ is independently unsubstituted or substituted with 1, 2, 3, 4 or 5 $R^{w5}$;
or, $R^a$, $R^b$ and together with the N atom to which they are attached, form aziridinyl, azetidinyl, pyrrolidyl, pyrazolidinyl, imidazolidinyl, piperidinyl, morpholinyl, thiomorpholinyl or piperazinyl, wherein each aziridinyl, azetidinyl, pyrrolidyl, pyrazolidinyl, imidazolidinyl, piperidinyl, morpholinyl, thiomorpholinyl and piperazinyl is independently unsubstituted or substituted with 1, 2, 3 or 4 $R^{w4}$.

**9.** The compound of any one of claims 1 to 8, wherein, each $R^{9a}$, $R^{9b}$, $R^{9c}$ and $R^{9d}$ is independently $C_{1-6}$ alkyl, $C_{2-6}$ alkenyl, $C_{2-6}$ alkynyl or -$C_{1-4}$ alkylene-$R^{d2}$, wherein each $C_{1-6}$ alkyl, $C_{2-6}$ alkenyl, $C_{2-6}$ alkynyl and -$C_{1-4}$ alkylene of -$C_{1-4}$ alkylene-$R^{d2}$ is independently unsubstituted or substituted with 1, 2, 3, 4 or 5 $R^{w6}$;
each $R^{d1}$ and $R^{d2}$ is independently -OH, $C_{1-4}$ alkoxy, phenyl, heteroaryl consisting of 5-6 ring atoms, cyclopropyl, cyclobutyl, cyclopentyl, cyclohexyl or $C_{1-4}$ alkylamino, wherein each $C_{1-4}$ alkoxy, phenyl, heteroaryl consisting of 5-6 ring atoms, cyclopropyl, cyclobutyl, cyclopentyl, cyclohexyl and $C_{1-4}$ alkylamino is independently unsubstituted or substituted with 1, 2, 3, 4 or 5 $R^{w7}$.

**10.** The compound of any one of claims 1 to 9, wherein, each $R^{9a}$, $R^{9b}$, $R^{9c}$ and $R^{9d}$ is independently methyl, ethyl, *n*-propyl, isopropyl, *n*-butyl, *i*-butyl, *sec*-butyl, *tert*-butyl, *n*-pentyl, *n*-hexyl, $-CH=CH_2$, $-CH=CHCH_3$, $-CH_2CH=CH_2$, $-CH_2CH_2CH=CH_2$, -C=CH, $-CH_2C=CH$, $-C\equiv C-CH_3$, $-CH_2CH_2C\equiv CH$, $-CH_2C\equiv CCH_3$, $-C\equiv CCH_2CH_3$, $-CH_2-R^{d2}$, $-(CH_2)_2-R^{d2}$, $-(CH_2)_3-R^{d2}$, $-CH_2CH(CH_3)-R^{d2}$, $-CH(CH_3)CH_2-R^{d2}$ or $-(CH_2)_4-R^{d2}$, wherein, each methyl, ethyl, *n*-propyl, isopropyl, *n*-butyl, *i*-butyl, *sec*-butyl, *tert*-butyl, *n*-pentyl, *n*-hexyl, $-CH=CH_2$, $-CH=CHCH_3$, $-CH_2CH=CH_2$, $-CH_2CH_2CH=CH_2$, -C=CH, $-CH_2C=CH$, $-C\equiv C-CH_3$, $-CH_2CH_2C\equiv CH$, $-CH_2C\equiv CCH_3$, $-C\equiv CCH_2CH_3$, $-CH_2-$ of $-CH_2-R^{d2}$, $-(CH_2)_2-$ of $-(CH_2)_2-R^{d2}$, $-(CH_2)_3-$ of $-(CH_2)_3-R^{d2}$, $-CH_2CH(CH_3)-$ of $-CH_2CH(CH_3)-R^{d2}$, $-CH(CH_3)CH_2-$ of $-CH(CH_3)CH_2-R^{d2}$ and $-(CH_2)_4-$ of $-(CH_2)_4-R^{d2}$ is independently unsubstituted or substituted with 1, 2, 3, 4 or 5 $R^{w6}$;
each $R^{d1}$ and $R^{d2}$ is independently -OH, methoxy, ethoxy, 1-propoxy, 2-propoxy, 1-butoxy, 2-methyl-1-propoxy, 2-butoxy, phenyl, furyl, pyrrolyl, pyridinyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidyl, cyclopropyl, cyclobutyl, cyclopentyl, cyclohexyl, *N*-methylamino, *N*-ethylamino, *N*,*N*-dimethylamino or *N*,*N*-diethylamino, wherein each methoxy, ethoxy, 1-propoxy, 2-propoxy, 1-butoxy, 2-methyl-1-propoxy, 2-butoxy, phenyl, furyl, pyrrolyl, pyridinyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidyl, cyclopropyl, cyclobutyl, cyclopentyl, cyclohexyl, *N*-methylamino, *N*-ethylamino, *N*,*N*-dimethylamino and *N*,*N*-diethylamino is independently unsubstituted or substituted with 1, 2, 3, 4 or 5 $R^{w7}$.

**11.** The compound of any one of claims 1 to 10, wherein, each $R^{w1}$, $R^{w2}$, $R^{w3}$, $R^{w4}$ and $R^{w7}$ is independently D, F, Cl, Br, I, =O, -OH, -CN, $-NH_2$, *N*-methylamino, *N*-ethylamino, *N*,*N*-dimethylamino, *N*,*N*-diethylamino, methoxy, ethoxy, 1-propoxy, 2-propoxy, 1-butoxy, 2-methyl-1-propoxy, 2-butoxy, methyl, ethyl, n-propyl, isopropyl, n-butyl, i-butyl, sec-butyl, tert-butyl, $-CH_2F$, $-CH_2Cl$, $-CF_3$, $-CHF_2$, $-CHCl_2$, $-CH_2CH_2F$, $-CH_2CH_2Cl$, $-CH_2CHF_2$, $-CH_2CHCl_2$, $-CHFCH_2F$, $-CHClCH_2Cl$, $-CH_2CF_3$, $-CH(CF_3)_2$, $-CF_2CH_2CH_3$, $-CH_2CH_2CH_2F$, $-CH_2CH_2CHF_2$, $-CH_2CH_2CF_3$, $-C(=O)-CH_3$, $-C(=O)-CH_2CH_3$, cyclopropyl, cyclobutyl, cyclopentyl, cyclohexyl, aziridinyl, azetidinyl, oxetanyl, thietanyl, pyrrolidyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl, piperazinyl or phenyl, wherein each *N*-methylamino, *N*-ethylamino, *N*,*N*-dimethylamino, *N*,*N*-diethylamino, methoxy, ethoxy, 1-propoxy, 2-propoxy, 1-butoxy, 2-methyl-1-propoxy, 2-butoxy,

methyl, ethyl, n-propyl, isopropyl, n-butyl, i-butyl, sec-butyl, tert-butyl, $-CH_2F$, $-CH_2Cl$, $-CHF_2$, $-CHCl_2$, $-CH_2CH_2F$, $-CH_2CH_2Cl$, $-CH_2CHF_2$, $-CH_2CHCl_2$, $-CHFCH_2F$, $-CHClCH_2Cl$, $-CH_2CF_3$, $-CH(CF_3)_2$, $-CF_2CH_2CH_3$, $-CH_2CH_2CH_2F$, $-CH_2CH_2CHF_2$, $-CH_2CH_2CF_3$, $-C(=O)-CH_3$, $-C(=O)-CH_2CH_3$, cyclopropyl, cyclobutyl, cyclopentyl, cyclohexyl, aziridinyl, azetidinyl, oxetanyl, thietanyl, pyrrolidyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl, piperazinyl and phenyl is independently unsubstituted or substituted with 1, 2, 3 or 4 groups independently selected from F, Cl, Br, I, -OH, -CN, $-NH_2$, methyl, ethyl, n-propyl, isopropyl, n-butyl, i-butyl, $-N(CH_3)_2$, $-NHCH_3$, $-N(CH_2CH_3)_2$, $-N(CH_3)CH_2CH_3$ and $-NHCH_2CH_3$;

each $R^{w5}$ and $R^{w6}$ is independently D, F, Cl, Br, I, =O, -OH, -CN, $-NH_2$, $C_{1-4}$ alkylamino, $C_{1-4}$ alkoxy, $C_{1-4}$ alkyl, $C_{1-4}$ haloalkyl, $-C(=O)-C_{1-4}$ alkyl, $C_{2-6}$ alkenyl, $C_{2-6}$ alkynyl, cyclopropyl, cyclobutyl, cyclopentyl, cyclohexyl, heterocyclyl consisting of 3-6 ring atoms, heteroaryl consisting of 5-6 ring atoms or phenyl, wherein each $-NH_2$, $C_{1-4}$ alkylamino, $C_{1-4}$ alkoxy, $C_{1-4}$ alkyl, $C_{1-4}$ haloalkyl, $-C(=O)-C_{1-4}$ alkyl, $C_{2-6}$ alkenyl, $C_{2-6}$ alkynyl, cyclopropyl, cyclobutyl, cyclopentyl, cyclohexyl, heterocyclyl consisting of 3-6 ring atoms, heteroaryl consisting of 5-6 ring atoms and phenyl is independently unsubstituted or substituted with 1, 2, 3 or 4 groups independently selected from F, Cl, Br, I, -OH, -CN, $-NH_2$, $C_{1-4}$ alkyl and $C_{1-4}$ alkylamino.

**12.** The compound of any one of claims 1 to 11, wherein, each $R^{w5}$ and $R^{w6}$ is independently D, F, Cl, Br, I, =O, -OH, -CN, $-NH_2$, *N*-methylamino, *N*-ethylamino, *N*,*N*-dimethylamino, *N*,*N*-diethylamino, methoxy, ethoxy, 1-propoxy, 2-propoxy, 1-butoxy, 2-methyl-1-propoxy, 2-butoxy, methyl, ethyl, n-propyl, isopropyl, n-butyl, i-butyl, sec-butyl, tert-butyl, $-CH_2F$, $-CH_2Cl$, $-CF_3$, $-CHF_2$, $-CHCl_2$, $-CH_2CH_2F$, $-CH_2CH_2Cl$, $-CH_2CHF_2$, $-CH_2CHCl_2$, $-CHFCH_2F$, $-CHClCH_2Cl$, $-CH_2CF_3$, $-CH(CF_3)_2$, $-CF_2CH_2CH_3$, $-CH_2CH_2CH_2F$, $-CH_2CH_2CHF_2$, $-CH_2CH_2CF_3$, $-C(=O)-CH_3$, $-C(=O)-CH_2CH_3$, $-C(=O)-CH_2CH_2CH_3$, $-CH=CH_2$, $-CH=CHCH_3$, $-CH_2CH=CH_2$, $-CH_2CH_2CH=CH_2$, $-C\equiv CH$, $-CH_2C\equiv CH$, $-C\equiv C-CH_3$, $-CH_2CH_2C\equiv CH$, $-CH_2C\equiv CCH_3$, $-C\equiv CCH_2CH_3$, cyclopropyl, cyclobutyl, cyclopentyl, cyclohexyl, aziridinyl, azetidinyl, oxetanyl, thietanyl, pyrrolidyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl, piperazinyl, furyl, pyrrolyl, pyridinyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidyl or phenyl, wherein each $-NH_2$, *N*-methylamino, *N*-ethylamino, *N*,*N*-dimethylamino, *N*,*N*-diethylamino, methoxy, ethoxy, 1-propoxy, 2-propoxy, 1-butoxy, 2-methyl-1-propoxy, 2-butoxy, methyl, ethyl, n-propyl, isopropyl, n-butyl, i-butyl, sec-butyl, tert-butyl, $-CH_2F$, $-CH_2Cl$, $-CHF_2$, $-CHCl_2$, $-CH_2CH_2F$, $-CH_2CH_2Cl$, $-CH_2CHF_2$, $-CH_2CHCl_2$, $-CHFCH_2F$, $-CHClCH_2Cl$, $-CH_2CF_3$, $-CH(CF_3)_2$, $-CF_2CH_2CH_3$, $-CH_2CH_2CH_2F$, $-CH_2CH_2CHF_2$, $-CH_2CH_2CF_3$, $-C(=O)-CH_3$, $-C(=O)-CH_2CH_3$, $-C(=O)-CH_2CH_2CH_3$, $-CH=CH_2$, $-CH=CHCH_3$, $-CH_2CH=CH_2$, $-CH_2CH_2CH=CH_2$, $-C\equiv CH$, $-CH_2C\equiv CH$, $-C\equiv C-CH_3$, $-CH_2CH_2C\equiv CH$, $-CH_2C\equiv CCH_3$, $-C\equiv CCH_2CH_3$, cyclopropyl, cyclobutyl, cyclopentyl, cyclohexyl, aziridinyl, azetidinyl, oxetanyl, thietanyl, pyrrolidyl, pyrazolidinyl, imidazolidinyl, tetrahydrofuranyl, tetrahydrothiophenyl, tetrahydropyranyl, tetrahydrothiopyranyl, piperidinyl, morpholinyl, thiomorpholinyl, piperazinyl, furyl, pyrrolyl, pyridinyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidyl and phenyl is independently unsubstituted or substituted with 1, 2, 3 or 4 groups independently selected from F, Cl, Br, I, -OH, -CN, $-NH_2$, methyl, ethyl, n-propyl, isopropyl, n-butyl, i-butyl, $-N(CH_3)_2$, $-NHCH_3$, $-N(CH_2CH_3)_2$, $-N(CH_3)CH_2CH_3$ and $-NHCH_2CH_3$.

**13.** The compound of any one of claims 1 to 12 comprising one of the following structures:

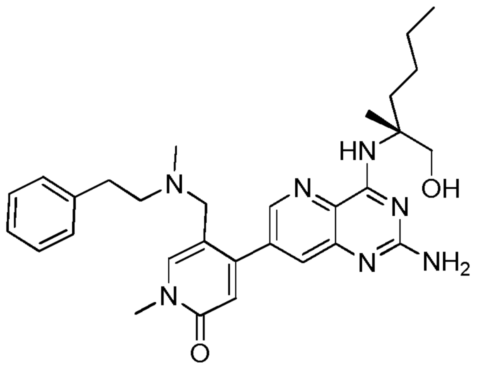
(1),

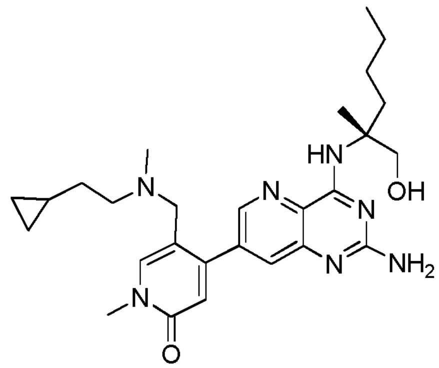
(2),

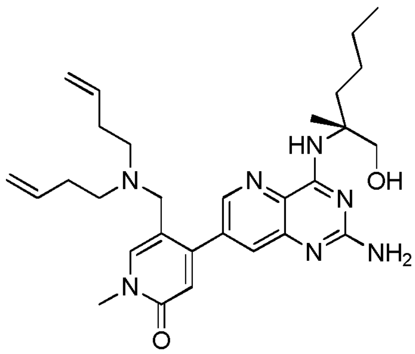

(3),

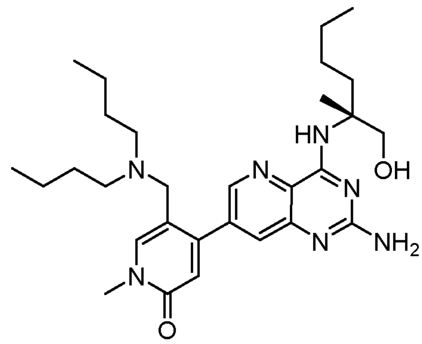

(4),

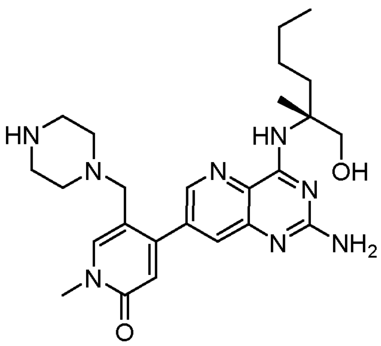

(5),

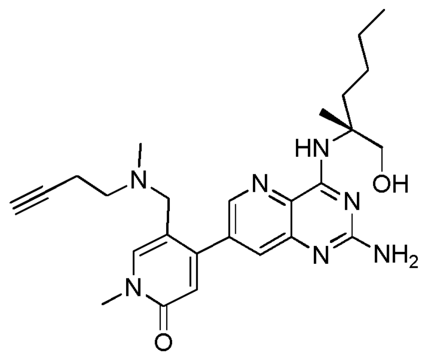

(6),

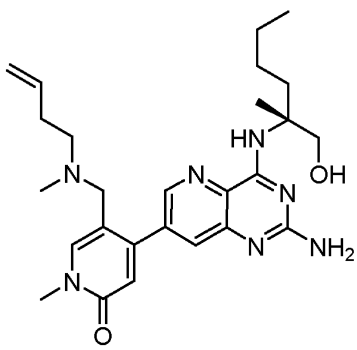

(7),

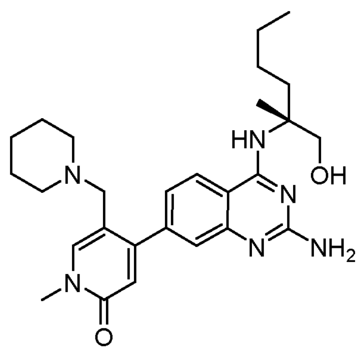

(8),

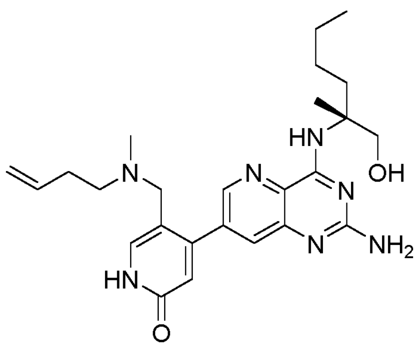

(9),

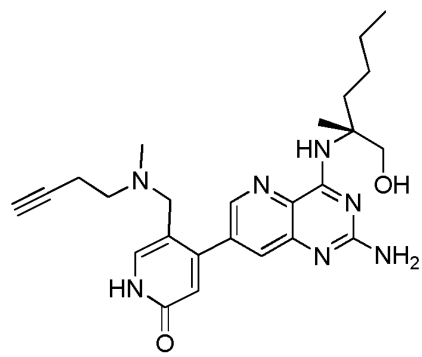

(10),

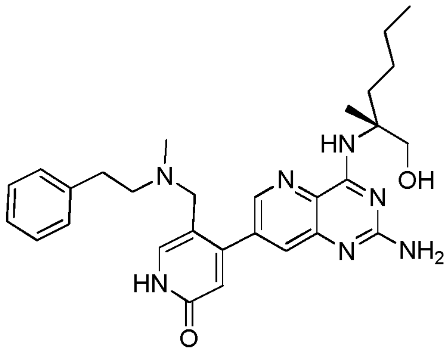
(11),

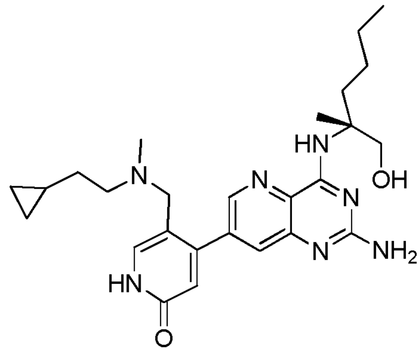
(12),

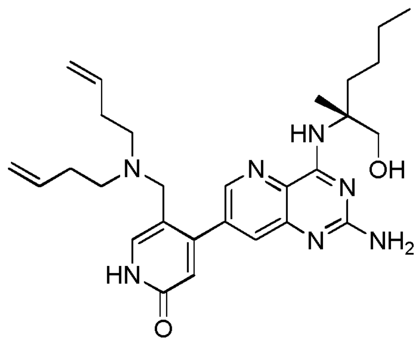
(13),

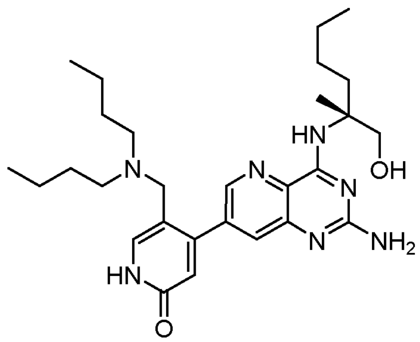
(14),

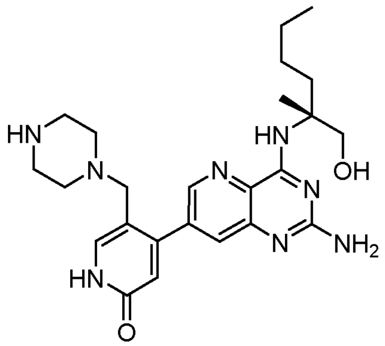
(15),

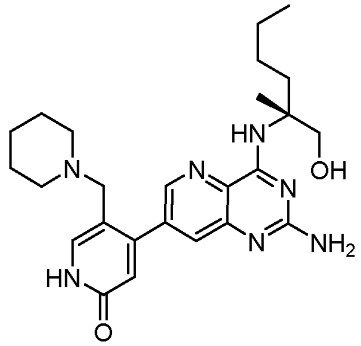
(16),

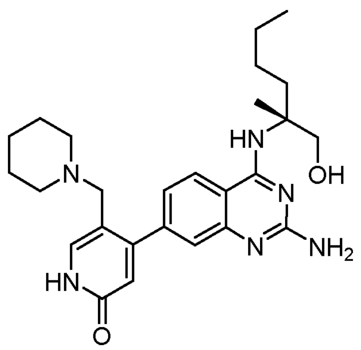
(17),

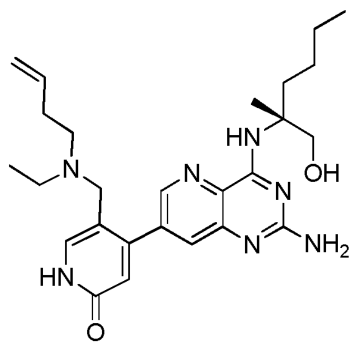
(18),

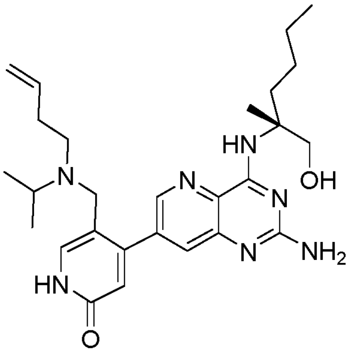

(19),

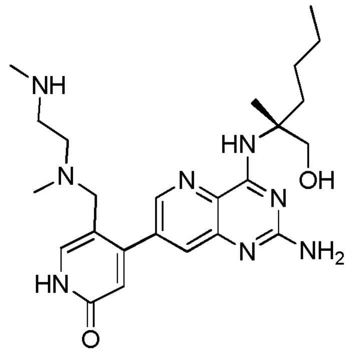

(20),

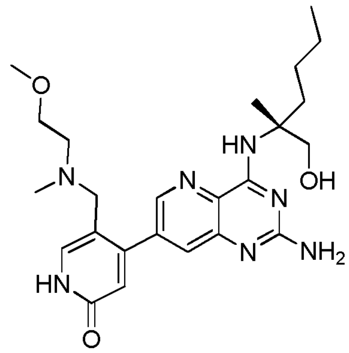

(21),

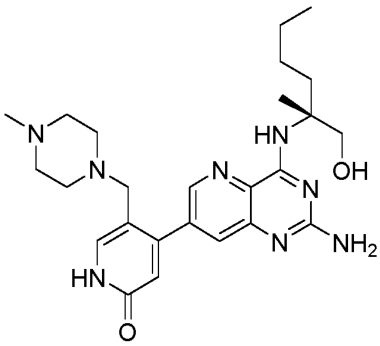

(22),

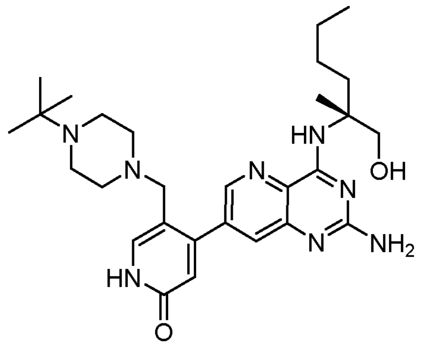

(23),

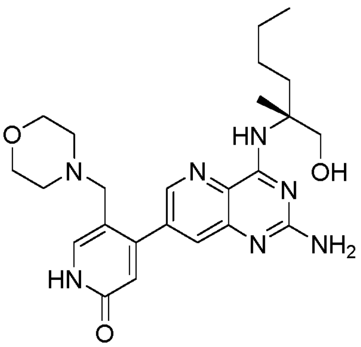

(24),

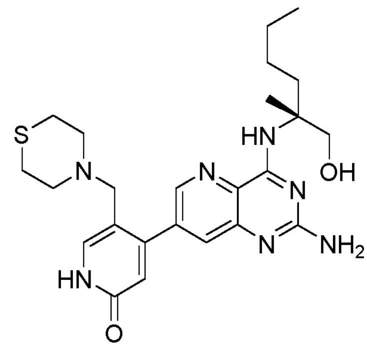

(25),

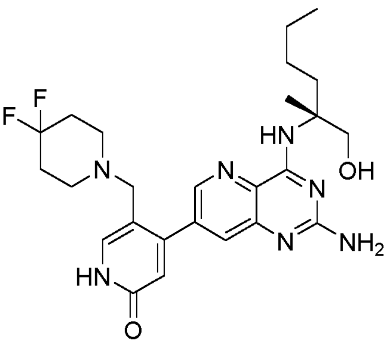

(26),

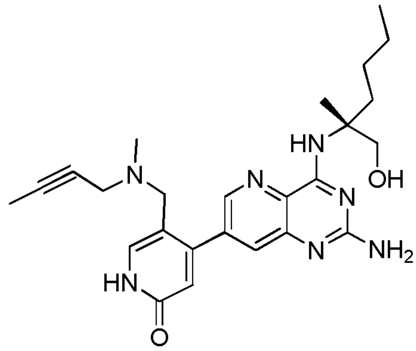

(27),

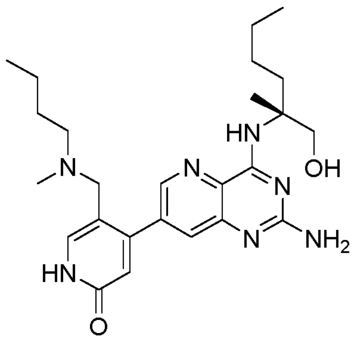
(28),

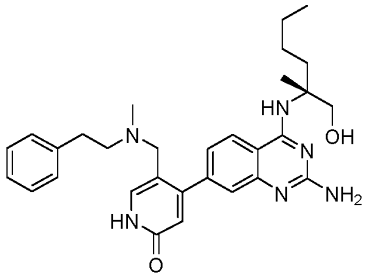
(29),

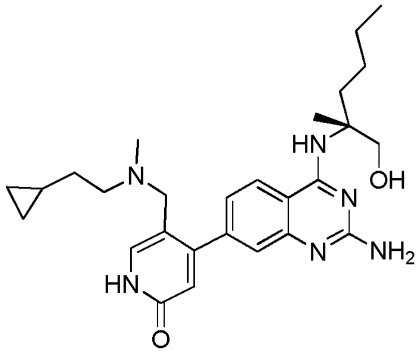
(30),

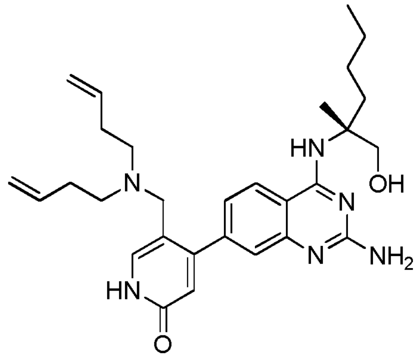
(31),

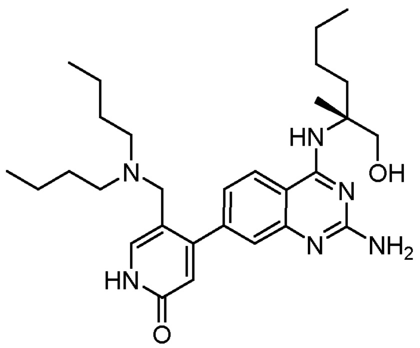
(32),

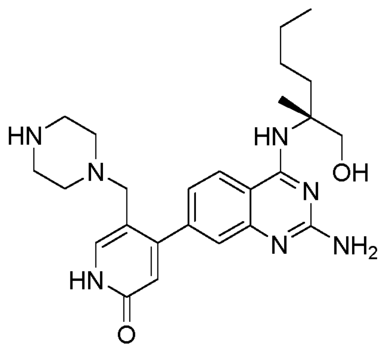
(33),

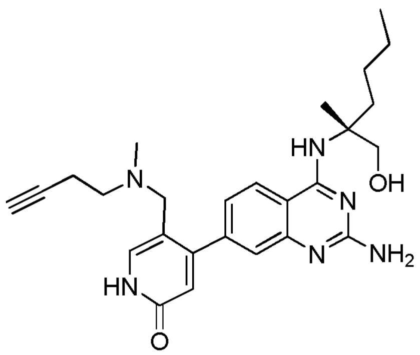
(34),

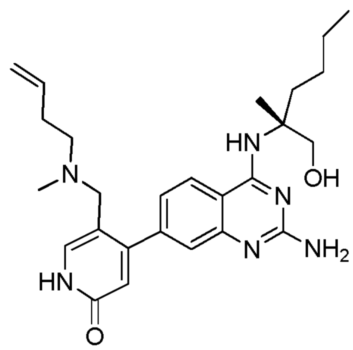
(35),

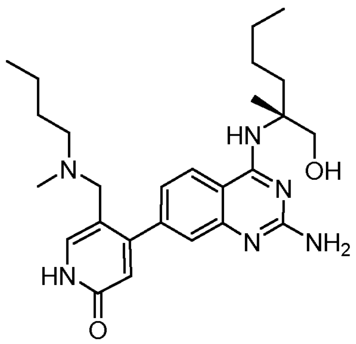 (36),

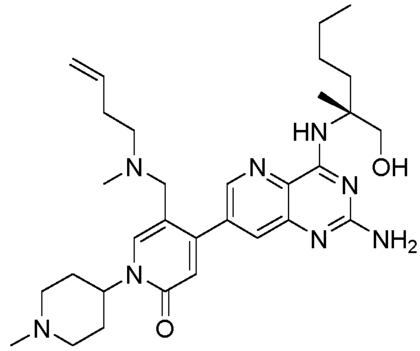 (37),

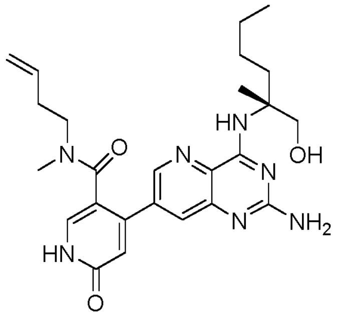 (38),

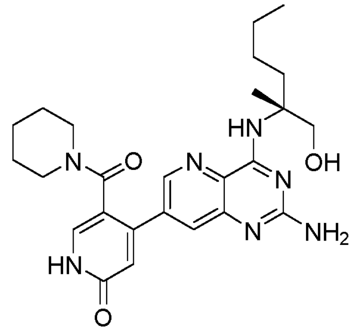 (39),

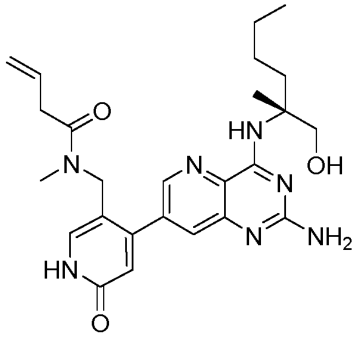 (40),

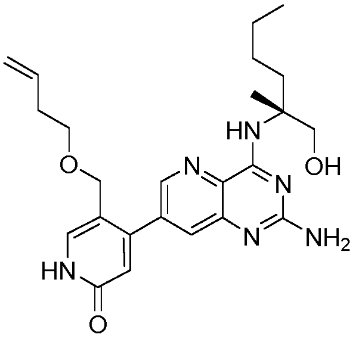 (41),

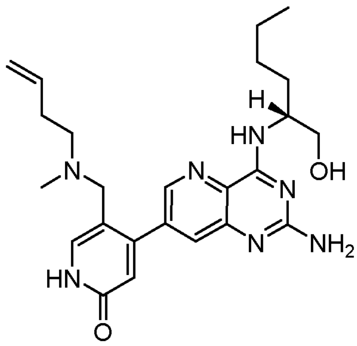 (42),

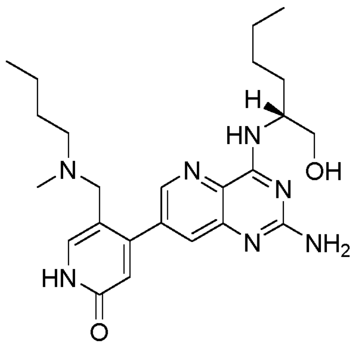 (43),

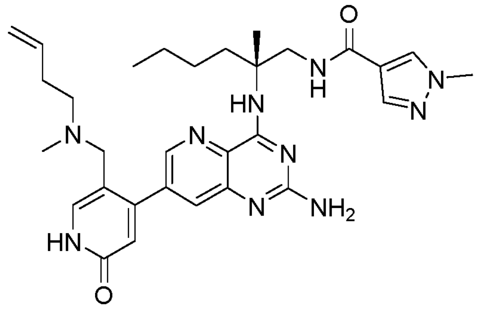
(44),

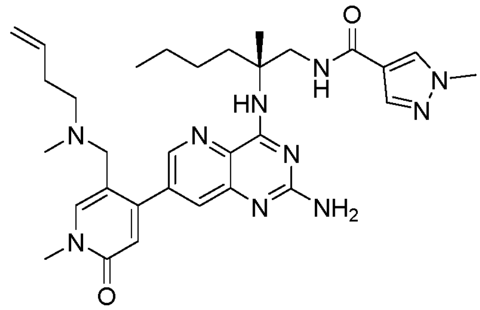
(45),

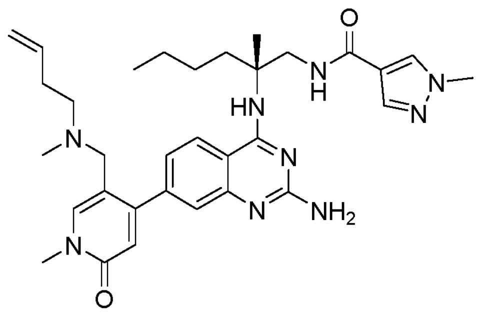
(46),

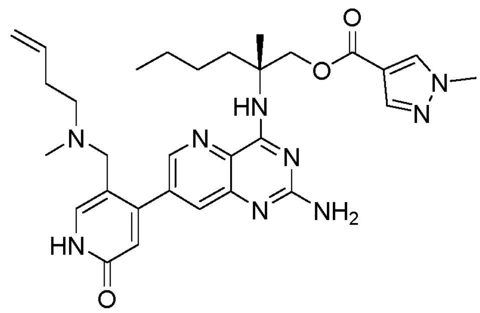
(47),

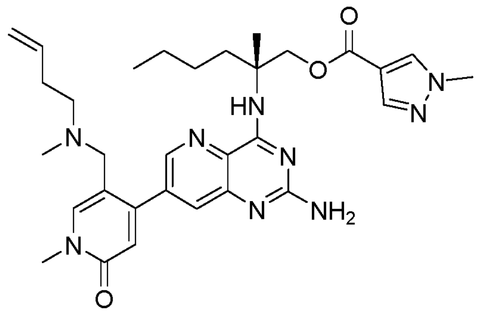
(48),

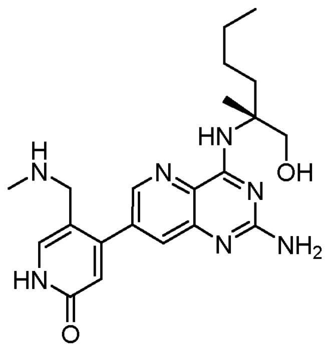
(49),

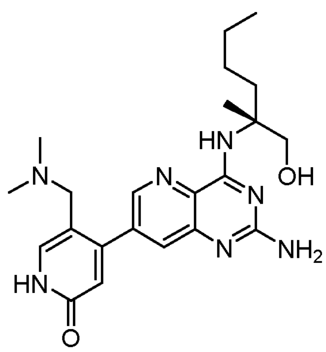
(50),

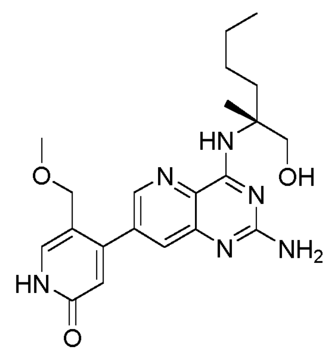
(51),

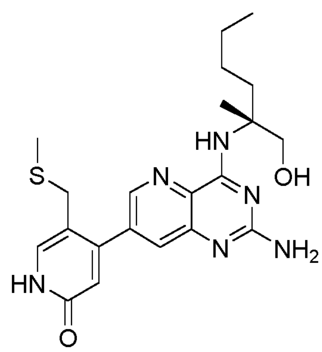
(52),

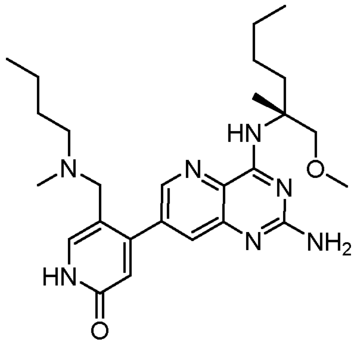

(53),

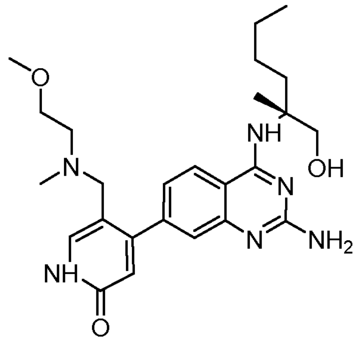

(54),

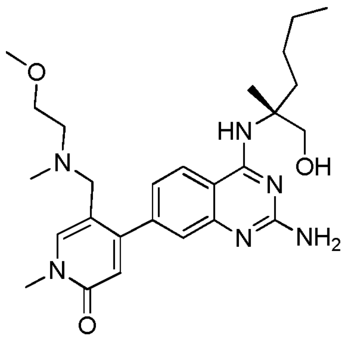

(55),

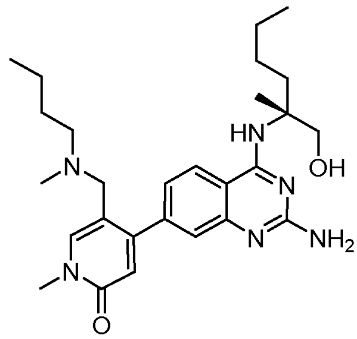

(56),

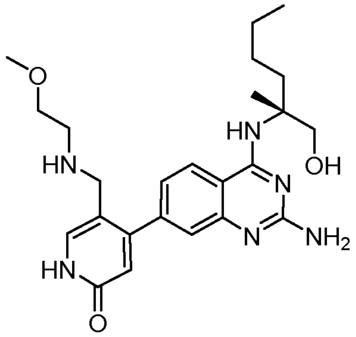

(57),

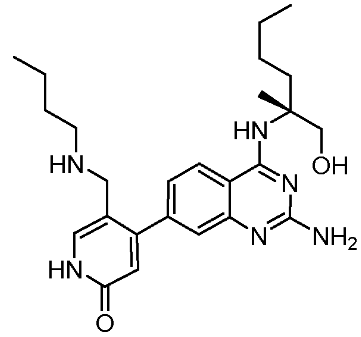

(58),

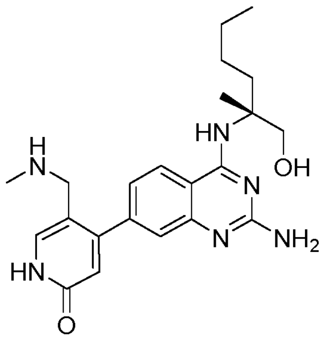

(59),

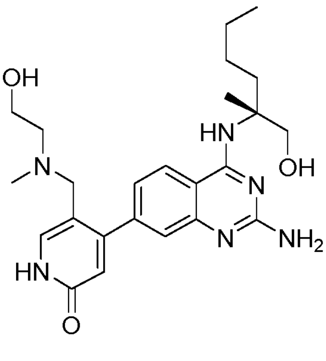

(60),

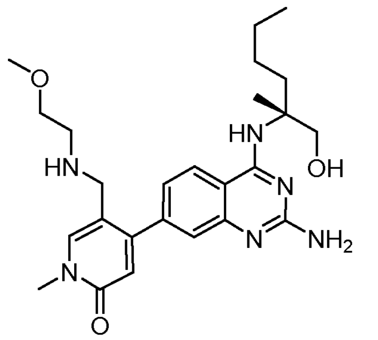

(61),

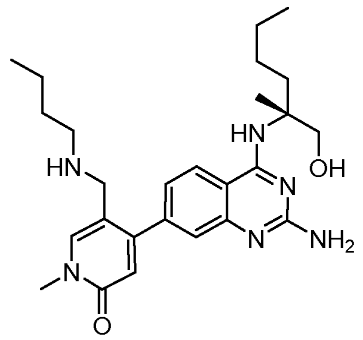

(62),

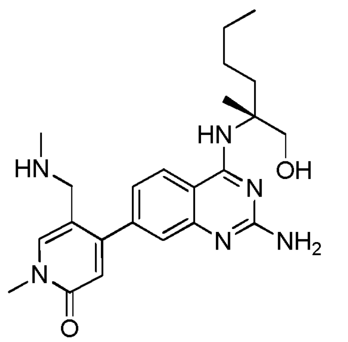

(63),

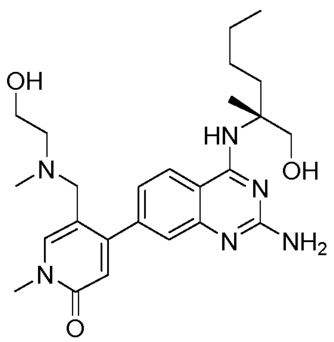

(64),

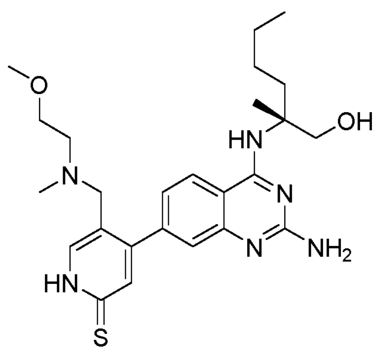

(65),

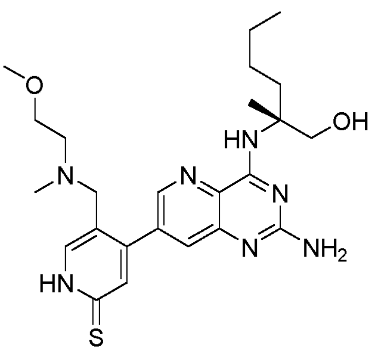

(66),

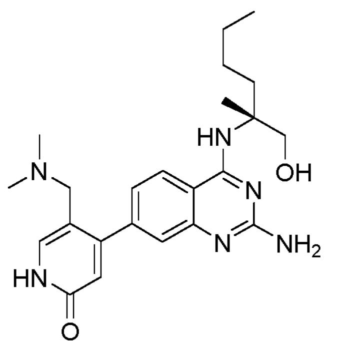

(67),

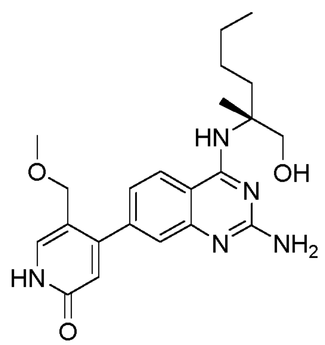

(68),

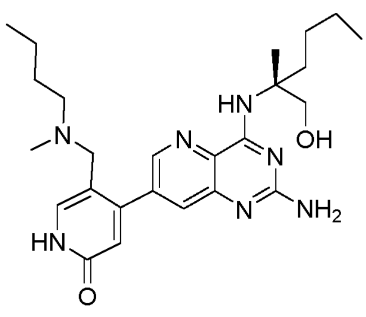

(69),

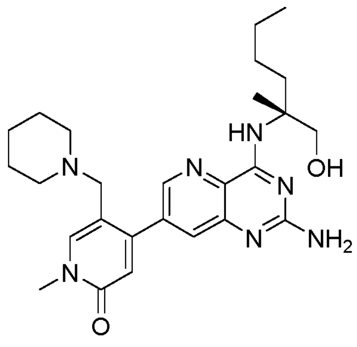

(70),

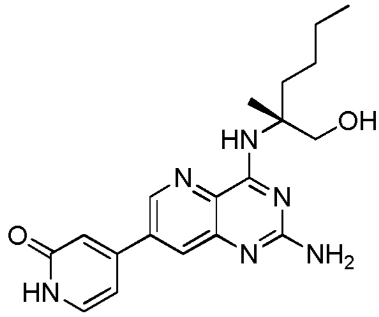

(71)

or a stereoisomer, tautomer, nitrogen oxide, solvate, metabolite, pharmaceutically acceptable salt or prodrug thereof.

**14.** A pharmaceutical composition comprising the compound of any one of claims 1 to 13 and pharmaceutically acceptable adjuvant.

**15.** The pharmaceutical composition of claim 14, the pharmaceutical composition further comprises one or more other therapeutic agent, wherein the therapeutic agent is HBV DNA polymerase inhibitor, Toll Like Receptor 7 conditioning agent, Toll Like Receptor 8 conditioning agent, Toll Like Receptor 7 and 8 conditioning agent, Toll Like Receptor 3 conditioning agent, interferon $\alpha$ ligand, HBsAg inhibitor, compound of HbcAg-targeting, cyclophilin inhibitor, HBV therapeutic vaccine, HBV prophylactic vaccine, HBV virus entry inhibitor, NTCP inhibitor, antisense oligonucleotides targeting viral mRNA, short interfering RNA, hepatitis Be antigen inhibitor, HBx inhibitor, cccDNA inhibitor, HBV antibody, thymosin agonist, cytokine, nuclear protein inhibitor, stimulator of retinoic acid-inducible gene 1, NOD2 stimulator, recombinant thymosin $\alpha$-1, hepatitis Be antigen replication inhibitor, hepatitis B surface antigen secretion or assembly inhibitor, IDO inhibitor or combination thereof.

**16.** The pharmaceutical composition of claim 14, wherein the therapeutic agent is Lamivudine, Sebivo, Tenofovir, Entecavir, Adefovir Dipivoxil, Tenofovir Alafenamide, Tenofovir disoproxil, Tenofovir alafenamide fumarate, Tenofovir alafenamide hemifumarate, Alfaferone, Alloferon, Celmoleukin, Clevudine, Emtricitabine, Famciclovir, interferon, hepatect CP, Intefen, Interleukin-2, Mivotilate, Nitazoxanide, Ribavirin, Roferon-A, Schizophyllan, Euforavac, Ampligen, Phosphazid, Heplisav, Recombinant Human Interleukin-2 Injection, levamisole or Proxigermanium.

**17.** Use of the compound of any one of claims 1 to 13 or the pharmaceutical composition of any one of claims 14 to 16 in the manufacture a medicament for preventing, managing, treating or lessening diseases mediated by TLR8 in patients.

**18.** The use of claim 17, wherein the diseases mediated by TLR8 are hepatitis B virus infection, hepatitis C virus infection, influenza virus infection, herpes virus infection, HIV infection, allergic diseases, rheumatoid arthritis, allergic asthma, chronic fatigue, type II diabetes mellitus, hay fever, lupus erythrosus, multiple sclerosis, melanoma, lung cancer, liver cancer, basal cell carcinoma, kidney cancer, myeloma, biliary tract cancer, brain cancer, breast cancer, cervical cancer, chorionic cancer, colon cancer, rectal cancer, head and neck cancer, Peritoneal tumors, fallopian tube cancer, endometrial cancer, esophageal cancer, gastric cancer, leukemia, lymphoma, sarcoma, neuroblastoma, oral cancer, ovarian cancer, pancreatic cancer, prostate cancer, testicular cancer, skin cancer or thyroid cancer.

**19.** The compound of any one of claims 1 to 13 or the pharmaceutical composition of any one of claims 14 to 16 for use in preventing, managing, treating or lessening diseases mediated by TLR8 in patients.

**20.** The compound or the pharmaceutical composition of claim 19, wherein, the diseases mediated by TLR8 are hepatitis B virus infection, hepatitis C virus infection, influenza virus infection, herpes virus infection, HIV infection, allergic diseases, rheumatoid arthritis, allergic asthma, chronic fatigue, type II diabetes mellitus, hay fever, lupus erythrosus, multiple sclerosis, melanoma, lung cancer, liver cancer, basal cell carcinoma, kidney cancer, myeloma, biliary tract cancer, brain cancer, breast cancer, cervical cancer, chorionic cancer, colon cancer, rectal cancer, head and neck cancer, Peritoneal tumors, fallopian tube cancer, endometrial cancer, esophageal cancer, gastric cancer, leukemia, lymphoma, sarcoma, neuroblastoma, oral cancer, ovarian cancer, pancreatic cancer, prostate cancer, testicular cancer, skin cancer or thyroid cancer.

**21.** A method for preventing, managing, treating or lessening diseases mediated by TLR8 in patients, comprising administering to the patient an effective therapeutic amount of the compound of any one of claims 1 to 13 or the pharmaceutical composition of any one of claims 14 to 16.

**22.** The method of claim 21, wherein, the diseases mediated by TLR8 are hepatitis B virus infection, hepatitis C virus infection, influenza virus infection, herpes virus infection, HIV infection, allergic diseases, rheumatoid arthritis, allergic asthma, chronic fatigue, type II diabetes mellitus, hay fever, lupus erythrosus, multiple sclerosis, melanoma, lung cancer, liver cancer, basal cell carcinoma, kidney cancer, myeloma, biliary tract cancer, brain cancer, breast cancer, cervical cancer, chorionic cancer, colon cancer, rectal cancer, head and neck cancer, Peritoneal tumors, fallopian tube cancer, endometrial cancer, esophageal cancer, gastric cancer, leukemia, lymphoma, sarcoma, neuroblastoma, oral cancer, ovarian cancer, pancreatic cancer, prostate cancer, testicular cancer, skin cancer or thyroid cancer.

| INTERNATIONAL SEARCH REPORT | International application No. PCT/CN2023/077368 |
|---|---|

**A. CLASSIFICATION OF SUBJECT MATTER**

C07D471/04(2006.01)i;A61P35/00(2006.01)i;A61P35/02(2006.01)i;A61P31/14(2006.01)i;A61P31/20(2006.01)i;A61K31/519(2006.01)i

According to International Patent Classification (IPC) or to both national classification and IPC

**B. FIELDS SEARCHED**

Minimum documentation searched (classification system followed by classification symbols)

C07D A61P A61K

Documentation searched other than minimum documentation to the extent that such documents are included in the fields searched

Electronic data base consulted during the international search (name of data base and, where practicable, search terms used)

CNKI; CNABS; CNTXT; WOTXT; EPTXT; USTXT; JPTXT; VEN; WPABS; ISI-WEB OF SCIENCE; STN; 读秀, DUXIU: 广东东阳光药业, 任青云, 刘为顺, 张英俊, 颜光华, 嘧啶, 吡啶, 氨基, 酮, TLR8, 癌, 肿瘤, +pyrimid+, +pyridin+, +amino+, +one, cancer, tumor, tumour, 结构式(I), structural formula (I)

**C. DOCUMENTS CONSIDERED TO BE RELEVANT**

| Category* | Citation of document, with indication, where appropriate, of the relevant passages | Relevant to claim No. |
|---|---|---|
| A | WO 2020007275 A1 (JIANGSU HENGRUI MEDICINE CO., LTD. et al.) 09 January 2020 (2020-01-09)<br>claims 1-21 | 1-22 |
| A | CN 101010299 A (BRISTOL MYERS SQUIBB CO.) 01 August 2007 (2007-08-01)<br>entire document | 1-22 |
| A | CN 112225738 A (BEIJING XINKAIYUAN PHARMACEUTICALS CO., LTD.) 15 January 2021 (2021-01-15)<br>entire document | 1-22 |
| A | CN 112390795 A (SHANGHAI ZHIMENG PHARMACEUTICAL TECHNOLOGY CO., LTD.) 23 February 2021 (2021-02-23)<br>claims 1-11 | 1-22 |
| A | CN 108069963 A (TSINGHUA UNIVERSITY) 25 May 2018 (2018-05-25)<br>claims 1-20 | 1-22 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **19 April 2023** | **30 April 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/077368**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **19, 21**
because they relate to subject matter not required to be searched by this Authority, namely:

Claim 19 relates to the use of the compound of any one of claims 1-13 or the pharmaceutical composition of any one of claims 14-16 for preventing, disposing, treating or relieving TLR8-mediated diseases in a patient, and claim 21 relates to a method for preventing, disposing, treating or relieving TLR8-mediated diseases in a patient, which are both therapeutic methods practised on the living human or animal body for the direct purpose of disease treatment, and falls within the cases set out in PCT Rule 39.1(iv) for which an international search is not required.

The search for claims 19 and 21 is performed on the basis of the following reasonably expected amendment: the subject matter of claim 19 is amended to "the use of the compound of any one of claims 1-13 or the pharmaceutical composition of any one of claims 14-16 for preparing a drug for preventing, disposing, treating or alleviating TLR8-mediated diseases in a patient", and the subject matter of claim 21 is amended to "a method for preparing a drug for preventing, disposing, treating or alleviating TLR8-mediated diseases in a patient".

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2023/077368**

| Patent document cited in search report | Publication date (day/month/year) | Patent family member(s) | Publication date (day/month/year) |
|---|---|---|---|
| WO 2020007275 A1 | 09 January 2020 | CA 3105286 A1 | 09 January 2020 |
| | | CN 111094289 A | 01 May 2020 |
| | | TW 202019925 A | 01 June 2020 |
| | | AU 2019299609 A1 | 07 January 2021 |
| | | IN 202127001409 A | 26 February 2021 |
| | | KR 20210028208 A | 11 March 2021 |
| | | BR 112020026890 A2 | 30 March 2021 |
| | | VN 77421 A | 26 April 2021 |
| | | EP 3822271 A1 | 19 May 2021 |
| | | US 2021269439 A1 | 02 September 2021 |
| | | JP 2021529190 A | 28 October 2021 |
| | | EP 3822271 A4 | 13 April 2022 |
| | | RU 2021100972 A | 03 August 2022 |
| | | RU 2778524 C2 | 22 August 2022 |
| | | CN 111094289 B | 22 November 2022 |
| | | CN 115710266 A | 24 February 2023 |
| CN 101010299 A | 01 August 2007 | WO 2005123680 A1 | 29 December 2005 |
| | | US 2006009455 A1 | 12 January 2006 |
| | | TW 200612952 A | 01 May 2006 |
| | | AU 2005255029 A1 | 11 January 2007 |
| | | SG 128025 A1 | 30 January 2007 |
| | | KR 20070022867 A | 27 February 2007 |
| | | MX 2006014799 A1 | 01 March 2007 |
| | | NO 20070201 A | 12 March 2007 |
| | | EP 1773775 A1 | 18 April 2007 |
| | | IN 200607496 P1 | 17 August 2007 |
| | | JP 2008502692 A | 31 January 2008 |
| | | US 7429604 B2 | 30 September 2008 |
| | | CN 101010299 B | 20 April 2011 |
| | | EP 1773775 B1 | 17 August 2011 |
| | | ES 2370053 T3 | 12 December 2011 |
| | | JP 4834663 B2 | 14 December 2011 |
| | | IN 289259 B | 10 November 2017 |
| CN 112225738 A | 15 January 2021 | CN 112225738 B | 13 April 2021 |
| CN 112390795 A | 23 February 2021 | CA 3147836 A1 | 25 February 2021 |
| | | WO 2021031960 A1 | 25 February 2021 |
| | | SG 11202201524 A | 30 March 2022 |
| | | AU 2020333708 A1 | 31 March 2022 |
| | | KR 20220047607 A | 18 April 2022 |
| | | BR 112022003090 A2 | 17 May 2022 |
| | | HK 40062138 A0 | 10 June 2022 |
| | | EP 4019517 A1 | 29 June 2022 |
| | | IN 202217014685 A | 01 July 2022 |
| | | US 2022340563 A1 | 27 October 2022 |
| | | JP 2022545872 A | 01 November 2022 |
| | | EP 4019517 A4 | 18 January 2023 |
| | | CN 114222742 A | 22 March 2022 |
| CN 108069963 A | 25 May 2018 | WO 2019095455 A1 | 23 May 2019 |
| | | CN 108069963 B | 14 January 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Handbook of Chemical Agents. 1994
- **THOMAS SORRELL**. Organic Chemistry. University Science Books, 1999
- **MICHAEL B. SMITH** ; **JERRY MARCH**. March's Advanced Organic Chemistry. John Wiley&Sons, 2007
- Pro-drugs as Novel Delivery Systems. **T. HIGUCHI** ; **V. STELLA**. Bioreversible Carriers in Drug Design. American Pharmaceutical Association and Pergamon Press, 1987, vol. 14
- **J. RAUTIO et al.** Prodrugs: Design and Clinical Applications. *Nature Review Drug Discovery*, 2008, vol. 7, 255-270
- **S. J. HECKER et al.** Prodrugs of Phosphates and Phosphonates. *Journal of Medicinal Chemistry*, 2008, vol. 51, 2328-2345
- McGraw-Hill Dictionary of Chemical Terms. McGraw-Hill Book Company, 1984
- **ELIEL et al.** Stereochemistry of Organic Compounds. John Wiley & Sons, Inc., 1994
- **BERGE et al.** *J. Pharmacol Sci*, 1977, vol. 66, 1-19
- **T. W. GREENE**. Protective Groups in Organic Synthesis. John Wiley & Sons, 1991
- **P. J. KOCIENSKI**. Protecting Groups. Thieme, 2005
- **TROY et al.** Remington: The Science and Practice of Pharmacy. Lippincott Williams & Wilkins, 2005
- **SWARBRICK et al.** Encyclopedia of Pharmaceutical Technology. Marcel Dekker, 1988